# EUROPEAN PATENT APPLICATION

(11) **EP 4 838 190 A2**
(43) Date of publication of application: **11.11.2026**
(21) Application number: 26193334.5
(22) Date of filing: 15.11.2022
(51) Int. Cl.: G01K 13/024

(54) **MOISTURE DETECTION AND MANAGEMENT IN A GASES SUPPLY SYSTEM**

(30) Priority: 15.11.2021 US 202163264088 P
(62) Document number(s) of the earlier application(s) in accordance with Art. 76 EPC: 22892263.9 / 4 433 125
(71) Applicant: Fisher & Paykel Healthcare Limited, 2013 Auckland (NZ)
(72) Inventor: SEEKUP, Peter Alan, 2013 Auckland (NZ); STOKS, Elmo Benson, 2013 Auckland (NZ); AU, Eugena Ming-Yee, 2013 Auckland (NZ); DEVERICK, Eric, 2013 Auckland (NZ); LIU, Po-Yen, 2013 Auckland (NZ); TENG, Ivan Chih-Fan, 2013 Auckland (NZ); BUDHIRAJA, Nimansha, 2013 Auckland (NZ); RAZALI, Naeem Bin, 2013 Auckland (NZ); GEMMELL, Luke Morgan, 2013 Auckland (NZ); SUROWIEZ, Ree Isaac, 2013 Auckland (NZ); EDWARDS, Thomas James, 2013 Auckland (NZ)
(74) Representative: Forresters IP LLP

(57) **Abstract**

Moisture conditions in a conduit, particularly a gases supply system conduit for supplying respiratory or surgical gases, can be detected using an electrical property of the conduit. Moisture conditions can also be detected in another component of the gases supply system. The detected moisture conditions can be managed to reduce and/or remove excess moisture in the system.

## Description

### FIELD OF THE DISCLOSURE

The present disclosure relates to detecting moisture in a conduit. More particularly, the present disclosure describes a respiratory or surgical gases conveying system which is capable of detecting the presence, volume and/or location of one or more of condensation, humidity and/or bodily fluids.

### BACKGROUND

Respiratory assistance apparatuses and surgical insufflators provide a flow of gases or a flow of pressurized gases through a conduit system to a patient. For a range of applications using these and similar devices, it is beneficial to humidify the supplied gases. These applications include where the gases are inspired and/or where the gas is being supplied during surgery to a surgery site of a patient. A downside to providing humidified gases through a conduit is the potential for condensation to form within the conduit. In addition to condensation, other types of moisture may also be introduced into a conduit from the patient, an optional heat and moisture exchanger (HME), an optional nebulizer, and/or the environment (such as via a room-entraining ventilator, or through a liquid- or vapor-permeable conduit wall, for example). Moisture may include bodily fluids such as saliva, blood mucus, or the like. This application may refer to water as an example, but it will be appreciated that water may be replaced with moisture, more generally, or any other liquid as well.

### SUMMARY OF THE DISCLOSURE

Humidified gases can cool as they pass through a conduit system between a gases source and the gases delivery destination. This can result in moisture (or liquid) such as condensate forming inside the conduit as the gases cool. In addition to condensate, moisture can refer, water, bodily fluids such as saliva, blood or mucus, or the presence of any liquid in the conduit. The formation of moisture is typically undesirable in respiratory assistance apparatuses and surgical insufflators. For example, condensation in a conduit can lead to a condition referred to as "rain out". Rain out occurs when moisture forms and potentially runs down the walls of the conduit system. The moisture can pool in a low part of the conduit system or it can run out of the conduit system into the patient's respiratory system, body, back into the gases source or into a ventilator return or other device connected to a conduit of a respiratory assistance apparatus or surgical insufflator. All of these rain out effects are undesirable and can cause numerous complications. Similarly, fluids from other sources (including the patient, other equipment and/or the environment) may be undesirable, and/or should be monitored for other reasons.

As used herein, the phrase "conduit system" encompasses any conduits (also referred to herein as tubes), connectors or patient interfaces that convey gases between a gases source and a patient and/or convey expired gases from the patient to another component of the respiratory assistance apparatus or surgical insufflator. For example, as discussed in further detail below, the conduit system can include one or more of inspiratory tube(s), dry lines, gases supply tubes, expiratory tube(s), insufflation tube(s), connector(s), Y-piece(s), patient tube(s), and/or patient interface(s) (including masks, nasal cannulas, nasal pillows, endotracheal tubes, tracheostomy tubes, surgical cannulas, etc.). Further, moisture, condensate, condensation and liquid are generally used synonymously in the present disclosure as would be understood by a person of skill in the art from the contextual usage of those terms herein. It should be appreciated by one of skill in the art that the term dryline (or dry line) may not always convey dry gases (for instance, when an air-entraining ventilator or air-entraining blow system is used, or in the case of reverse flow through the system, or the like).

Respiratory assistance apparatuses and surgical insufflators (collectively referred to herein as gases supply systems) can employ one or more heating wires within the conduit system, such as in the walls of any or all components of the conduit system to provide a heat source. The one or more heating wires allow the conduit to control the temperature and/or relative humidity of the gases to a desired value or range as the gases pass through the conduit system, reducing the potential for condensation. One or more sensor wires can also be included within the lumen or in the walls of the conduit system as well. The one or more sensor wires are typically used to convey temperature measurement information of the humidified gases flowing through a conduit and/or patient interface from one or more temperature sensors back to a controller of the gases supply system. The gases supply system can use the temperature measurement information in a feedback control system to adjust the amount of heat provided by the one or more heating wires or other components of the gases supply system.

Even with heating wires, condensation and rain out can still occur, and fluids from other sources may still be introduced into the circuit system. The present disclosure provides for detecting moisture in the circuit system. Moisture can be detected by measuring or inferring capacitance, reactance and/or impedance or a change in capacitance, reactance and/or impedance of two or more spaced electrical conductors within the conduit system or embedded in the conduit system walls. These electrical conductors can be, for example, one or more conductive wires, such as the one or more heating wires and/or the one or more sensor wires. Alternatively, or additionally, dedicated conductors may be provided within, or embedded in, the conduit system including the lumen of the conduit or the walls thereof. The capacitance can be an intrinsic/parasitic capacitance. The measure can use a time response and/or a frequency response of the wire(s). Moisture can also be detected by measuring a change in resistance as disclosed herein and/or a change to a wireless signal such as an RF signal.

The present disclosure provides a humidifier system useable in a gases supply system, the humidifier system comprising a humidifier; a conduit comprising a first electrically conductive element and a second electrically conductive element; and a controller configured to monitor a signal using one or more of the first electrically conductive element and the second electrically conductive element to determine a value indicative of moisture in the conduit based at least in part on the signal.

In some configurations, the signal can be indicative of a capacitance between the first electrically conductive element and the second electrically conductive element.

In some configurations, the signal can be indicative of a change in capacitance between the first electrically conductive element and the second electrically conductive element.

In some configurations, the controller can comprise a signal generator.

In some configurations, the controller can comprise one or more hardware and/or software processors.

In some configurations, the first electrically conductive element and the second electrically conductive element can be separated by a distance configured to allow for a capacitive charge to be sensed between the first electrically conductive element and the second electrically conductive element.

In some configurations, the humidifier system can also comprise a dielectric material located between the first electrically conductive element and the second electrically conductive element.

In some configurations, the dielectric material can be vapor or liquid permeable.

In some configurations, the vapor permeable dielectric material can allow evaporation of water to ambient air while inhibiting passage of liquid water and breathing gases to ambient air.

In some configurations, the controller can be configured to determine the value indicative of moisture on a comparison of a measurement of the first electrically conductive element and/or the second electrically conductive element.

In some configurations, the value indicative of moisture can comprise a time constant of a circuit comprising the first electrically conductive element or the second electrically conductive element in series with the reference resistor.

In some configurations, the signal can be indicative of a time constant or a resonant frequency of a circuit comprising the first electrically conductive element and/or the second electrically conductive element.

In some configurations, the signal can be indicative of a change in a time constant or a change in a resonant frequency of a circuit comprising the first electrically conductive element and/or the second electrically conductive element.

In some configurations, the value inductive of moisture in the conduit can correspond to an inductance of the conduit.

In some configurations, the value inductive of moisture in the conduit can correspond to a change in inductance of the conduit.

In some configurations, the humidifier system can also comprise a resonant circuit wherein an inductor is placed in parallel with a capacitor.

In some configurations, the resonant circuit can be electrically connected in parallel with the first electrically conductive element, the second electrically conductive element, or both the first electrically conductive element and the second electrically conductive element.

In some configurations, the resonant circuit can be tuned to exhibit resonant behavior when sufficiently excited by the signal.

In some configurations, the resonant circuit can be tuned to exhibit resonant behavior when excited by the signal, wherein the signal has been selected to excite the resonant circuit.

In some configurations, the controller can be configured to apply additional power to the first electrically conductive element in conjunction with a normal control power.

In some configurations, the humidifier system can also comprise an AC power supply.

In some configurations, the humidifier system can also comprise a DC power supply.

In some configurations, the signal can be indicative of a temperature of the first electrically conductive element or the second electrically conductive element.

In some configurations, the signal can be indicative of a change in temperature of the first electrically conductive element or the second electrically conductive element.

In some configurations, the signal can be indicative of a thermal conductivity of a medium between the first electrically conductive element and the second electrically conductive element, or the signal can be indicative of a thermal conductivity of a medium proximal to the first electrically conductive element or the second electrically conductive element.

In some configurations, the signal can be indicative of a change in thermal conductivity of a medium between the first electrically conductive element and the second electrically conductive element, or the signal can be indicative of a change in thermal conductivity of a medium proximal to the first electrically conductive element or the second electrically conductive element.

In some configurations, a change in temperature of the first electrically conductive element or the second electrically conductive element can be substantially linear.

In some configurations, the signal can be indicative of a temperature difference between the first electrically conductive element and the second electrically conductive element.

In some configurations, the second electrically conductive element can measure the signal.

In some configurations, the signal can correspond to a resistance of the second electrically conductive element, the resistance of the second electrically conductive element varying with the temperature of the second electrically conductive element.

In some configurations, the first electrically conductive element or the second electrically conductive element can further comprise a thermistor.

In some configurations, the first electrically conductive element or the second electrically conductive element can further comprise a diode.

In some configurations, the diode can be electrically connected in parallel with the thermistor.

In some configurations, the diode can be electrically connected in parallel, and positioned substantially adjacent, with the thermistor.

In some configurations, the first electrically conductive element and the second electrically conductive element can be adjacent to each other.

In some configurations, the first electrically conductive element and the second electrically conductive element can be not adjacent to each other.

In some configurations, the first electrically conductive element and the second electrically conductive element can be within a bead of the conduit.

In some configurations, the first electrically conductive element can measure the signal.

In some configurations, the signal can be indicative of a resistance of the first electrically conductive element or the second electrically conductive element.

In some configurations, the signal can be indicative of a resistance of a medium between the first electrically conductive element and the second electrically conductive element.

In some configurations, the first electrically conductive element or the second electrically conductive element can comprise at least two portions that are electrically disconnected from one another.

In some configurations, the first electrically conductive element and the second electrically conductive element can be electrically insulated from other electrically conductive elements for a portion of a length of the first electrically conductive element and for a portion of a length of the second electrically conductive element.

In some configurations, the at least two portions can protrude into a lumen of the conduit. The at least two portions can be flush with an inner wall of the conduit.

In some configurations, the at least two portions can be arranged within the tube wall and are pneumatically coupled with the lumen of the conduit.

In some configurations, the at least two portions that can be electrically disconnected from one another can be in series with one another.

In some configurations, the at least two portions that can be electrically disconnected from one another can be in parallel with one another.

In some configurations, the controller can determine a value indicative of moisture in the conduit based at least in part on a magnitude and/or phase of the signal.

In some configurations, the humidifier system can also comprise a signal generator.

In some configurations, the signal can have a frequency between 30 Hz and 300 GHz.

In some configurations, the signal can have a frequency between 1 MHz and 100 MHz.

In some configurations, the signal can have a frequency of about 10 MHz.

In some configurations, the first electrically conductive element and/or the second electrically conductive element can be a quarter of the wavelength of the signal.

In some configurations, the wavelength of the signal can be four times larger than the length of the first electrically conductive element and/or the second electrically conductive element.

In some configurations, the signal generator can inject the signal into the first electrically conductive element.

In some configurations, the first electrically conductive element can be configured to be a transmitter.

In some configurations, the second electrically conductive element can be configured to be a receiver to receive the signal transmitted by the first electrically conductive element.

In some configurations, the magnitude and/or phase of the signal can be measured by a radio-frequency transducer.

In some configurations, the radio-frequency transducer can be an AM receiver, RF sampling ADC, or RF rectifier.

In some configurations, the humidifier system can also comprise a filter to filter the signal.

In some configurations, the filter can comprise a high pass or bandpass filter.

In some configurations, the filter can be configured to filter out the mains frequency.

In some configurations, the filter can be configured to filter out frequencies between 50 - 60 Hz.

In some configurations, the transmitter can comprise a loop antenna.

In some configurations, the receiver can comprise a loop antenna.

In some configurations, the transmitter can comprise a monopole antenna.

In some configurations, the transmitter can comprise a monopole antenna.

In some configurations, the first electrically conductive element can be electrically coupled to a first switch.

In some configurations, the second electrically conductive element can be electrically coupled to a second switch.

In some configurations, the first switch can be configured to electrically disconnect one end of the first electrically conductive element.

In some configurations, the second switch can be configured to electrically disconnect one end of the second electrically conductive element.

In some configurations, the first switch and/or the second switch can be located in any one of the following: a heater base, a sensor cartridge, the conduit, an external component, or an intermediate connector.

In some configurations, the controller can be configured to output an alarm if the value indicative of moisture falls below a first threshold value.

In some configurations, the controller can be configured to output an alarm if the value indicative of condensation exceeds a second threshold value.

In some configurations, the alarm indicates an unacceptable level of moisture.

In some configurations, the controller can be configured to automatically reduce humidification of breathing or insufflation gases in response to the value indicative of moisture and/or humidity in the conduit.

In some configurations, the reduction of humidity delivered to the patient can be achieved by a reduction in heater plate power.

In some configurations, the conduit can be a composite conduit.

In some configurations, the conduit can comprise a vapor and/or liquid permeable bead.

In some configurations, the permeable bead can allow evaporation of water to ambient air while inhibiting passage of liquid water and breathing gases to ambient air.

In some configurations, the permeable bead can be one or more of an activated perfluorinated polymer material having extreme hydrophilic properties, hydrophilic thermoplastic, breathable thermoplastic copolyester, woven treated fabric exhibiting breathable characteristics, or a hydrophilic polyester block copolymer.

In some configurations, the first conductive element and the second electrically conductive element can be spirally wound about at least a length of the conduit.

In some configurations, the first conductive element and the second electrically conductive element can be spirally wound within, through or around the conduit.

In some configurations, the first conductive element and the second electrically conductive element can form part of the conduit walls.

In some configurations, the first electrically conductive element can be a sensing wire.

In some configurations, the first electrically conductive element can be a heater wire.

In some configurations, the second electrically conductive element can be a sensing wire.

In some configurations, the second electrically conductive element can be a heater wire.

The present disclosure provides a method of detecting an indication of moisture in a conduit of a gases supply system used to transport respiratory or surgical gases, the method comprising determining a presence and/or level of moisture based at least in part on property of the conduit.

In some configurations, the determination of the presence or level of moisture can be inferred from a dielectric property of the conduit.

In some configurations, the determination of the property can comprise applying a signal to a first electrically conductive element in the conduit.

In some configurations, the determination of the property can comprise measuring a capacitance between the first electrically conductive element and a second electrically conductive element.

In some configurations, the determination of the property can comprise measuring a capacitance between the first electrically conductive element and a second electrically conductive element based on the applied signal.

In some configurations, the determination of the property comprises measuring an indication of a time constant or a resonant frequency of a circuit comprising the first electrically conductive element.

In some configurations, the determination of the property can comprise processing a value indicative of an inductance.

In some configurations, the determination of the property can further comprise measuring an indication of a resistance of the first electrically conductive element.

In some configurations, the determination of the property can further comprise measuring an indication of a temperature.

In some configurations, the determination of the property can further comprise measuring an indication of a thermal conductivity.

In some configurations, the determination of the property can further comprise measuring a magnitude and/or phase of a signal.

In some configurations, the conduit can be the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing two electrically conductive elements separated by a dielectric and located within, around or on the conduit and measuring a capacitance or change in capacitance to indicate a measure of moisture or condensate within the conduit.

The present disclosure provides a method of detecting moisture in a conduit utilized to transport humidified gases, the method comprising providing two electrically conductive elements located within, around or on the conduit and measuring a resistance or a change in resistance to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing two electrically conductive elements located within, around or on the conduit and measuring a time constant, a resonant frequency, a change in a time constant, or a change in a resonant frequency to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing two electrically conductive elements located within, around or on the conduit and measuring a resistance or a change in resistance to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing two electrically conductive elements located within, around or on the conduit and measuring a temperature or a change in temperature to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing two electrically conductive elements and located within, around or on the conduit and measuring a thermal conductivity or a change in thermal conductivity to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing two electrically conductive elements and located within, around or on the conduit and measuring a magnitude and/or phase of a signal or a change in a magnitude and/or phase of a signal to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides external accessories. An example of an external accessory is a cartridge for use with a humidifier in a respiratory or surgical humidification system.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least a first electrical terminal or pad and a second electrical terminal or pad configured to make an electrical coupling with a first electrically conductive element and a second electrically conductive element extending along at least a portion of a length of the inspiratory conduit; and a controller communicatively coupled with the one or more sensors and the first electrically conductive element and the second electrical connectors.

In some configurations, the cartridge can be removably attachable to the humidifier and the controller can be configured to, in use, measure a signal indicative of a capacitance between the first electrically conductive element and the second electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least a first electrical terminal or pad and a second electrical terminal or pad configured to make an electrical coupling with a first electrically conductive element and a second electrically conductive element extending along at least a portion of a length of the inspiratory conduit; and a controller communicatively coupled with the one or more sensors and the first electrically conductive element and the second electrical connectors.

In some configurations, the cartridge can be removably attachable to the humidifier and the controller can be configured to, in use, measure a signal indicative of a time constant or a resonant frequency of a circuit comprising the first electrically conductive element and the second electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least a first electrical terminal or pad and a second electrical terminal or pad configured to make an electrical coupling with a first electrically conductive element and a second electrically conductive element extending along at least a portion of a length of the inspiratory conduit; and a controller communicatively coupled with the one or more sensors and the first electrically conductive element and the second electrical connectors.

In some configurations, the cartridge can be removably attachable to the humidifier and the controller can be configured to, in use, measure a signal indicative of a resistance of the first electrically conductive element or the second electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least a first electrical terminal or pad and a second electrical terminal or pad configured to make an electrical coupling with a first electrically conductive element and a second electrically conductive element extending along at least a portion of a length of the inspiratory conduit; and a controller communicatively coupled with the one or more sensors and the first electrically conductive element and the second electrical connectors.

In some configurations, the cartridge can be removably attachable to the humidifier and the controller can be configured to, in use, measure a signal indicative of a temperature of the first electrically conductive element or the second electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least a first electrical terminal or pad and a second electrical terminal or pad configured to make an electrical coupling with a first electrically conductive element and a second electrically conductive element extending along at least a portion of a length of the inspiratory conduit; and a controller communicatively coupled with the one or more sensors and the first electrically conductive element and the second electrical connectors.

In some configurations, the cartridge can be removably attachable to the humidifier and the controller can be configured to, in use, measure a signal indicative of a thermal conductivity of a medium between the first electrically conductive element and the second electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least a first electrical terminal or pad and a second electrical terminal or pad configured to make an electrical coupling with a first electrically conductive element and a second electrically conductive element extending along at least a portion of a length of the inspiratory conduit; and a controller communicatively coupled with the one or more sensors and the first electrically conductive element and the second electrical connectors.

In some configurations, the cartridge can be removably attachable to the humidifier and the controller can be configured to, in use, measure magnitude and/or phase of a signal or a change in a magnitude and/or phase of a signal between the first electrically conductive element and the second electrically conductive element of the removable inspiratory conduit.

The present disclosure provides a humidifier useable in a gases supply system, the humidifier comprising a humidification chamber configured to humidify a gases supply; an inspiratory conduit connector configured to connection with an inspiratory conduit including a first electrically conductive element and a second electrically conductive element; a controller configured to monitor a signal using one or more of the first electrically conductive element and the second electrically conductive element to determine a value indicative of moisture in the conduit based at least in part on the signal.

In some configurations, the controller can be further configured to monitor the signal.

In some configurations, the signal can be indicative of a capacitance between the first electrically conductive element and the second electrically conductive element.

In some configurations, the signal can be indicative of a change in capacitance between the first electrically conductive element and the second electrically conductive element.

In some configurations, the signal can be indicative of a time constant or a resonant frequency of the first electrically conductive element or the second electrically conductive element.

In some configurations, the signal can be indicative of a temperature of the first electrically conductive element or the second electrically conductive element.

In some configurations, the signal can be indicative of a change in temperature of the first electrically conductive element or the second electrically conductive element.

In some configurations, the signal can be indicative of a thermal conductivity of a medium between the first electrically conductive element or the second electrically conductive element, or the signal or the signal is indicative of a thermal conductivity of a medium proximal to the first electrically conductive element or the second electrically conductive element.

In some configurations, the signal can be indicative of a change in thermal conductivity of a medium between the first electrically conductive element or the second electrically conductive element, or the signal or the signal is indicative of a change in thermal conductivity of a medium proximal to the first electrically conductive element or the second electrically conductive element.

In some configurations, the signal can be indicative of a temperature difference between the first electrically conductive element and the second electrically conductive element.

In some configurations, the value indicative of moisture can be a magnitude and/or phase of a signal or a change in a magnitude and/or phase of a signal.

In some configurations, the controller can comprise a signal generator.

In some configurations, the controller can comprise one or more hardware and/or software processors.

In some configurations, the humidifier can further comprise the conduit of any of conduit implementations disclosed herein.

The present disclosure provides a conduit used with a respiratory or surgical gases supply system, the conduit comprising a first electrically conductive element; a second electrically conductive element spaced apart from the first electrically conductive element at a distance configured to allow a capacitive effect to exist between the first electrically conductive element and the second electrically conductive element such that the capacitive effect changes in the presence of moisture; and a material separating the first conductive element from the second electrically conductive element.

In some configurations, at least one of the electrically conductive elements can be one or more of a heater wire or sensor wire.

In some configurations, the conduit can further comprise a controller configured to determine a presence and/or indication of moisture within the conduit by determining a capacitance or change in capacitance between the first electrically conductive element and the second electrically conductive element.

In some configurations, the controller can be one or more microprocessors.

In some configurations, the controller can use the first electrically conductive element and second electrically conductive element to determine the presence or the indication of moisture within the conduit by measuring a capacitive reactance and/or inductance existing between the first electrically conductive element and the second electrically conductive element.

In some configurations, the first electrically conductive element and second electrically conductive element can be placed close enough to allow for a measurable capacitance, but far enough apart to allow for a measurable change in capacitance due to a presence of moisture.

The present disclosure provides a conduit used with a respiratory or surgical gases supply system, the conduit comprising a first electrically conductive element; a second electrically conductive element, wherein one or more of the first electrically conductive element and the second electrically conductive element can be configured to provide a measurement of a time constant or a resonant frequency indicative of a presence or amount of moisture in a conduit.

In some configurations, the conduit can further comprise a resonant circuit wherein an inductive element is electrically connected in parallel with a capacitive element.

In some configurations, one or more of the first electrically conductive element and the second electrically conductive element can be configured to be electrically connected in parallel with a resonant circuit wherein an inductive element is electrically connected in parallel with a capacitive element.

In some configurations, the resonant circuit can be external to the conduit.

In some configurations, the resonant circuit can be tuned to exhibit resonant behavior when excited by a signal.

In some configurations, one or more of the first electrically conductive element and the second electrically conductive element can be configured to be electrically connected in parallel with a signal generator.

In some configurations, the conduit can comprise a controller configured to determine a presence and/or indication of moisture within the conduit by determining a time constant, a resonant frequency, a change in time constant, or a change in resonant frequency.

In some configurations, one or more of the first electrically conductive element and the second electrically conductive element can be configured to be electrically connected in parallel with the controller.

In some configurations, the controller can comprise a signal generator.

In some configurations, the controller can be one or more microprocessors.

The present disclosure provides a conduit used with a respiratory or surgical gases supply system, the conduit comprising a first electrically conductive element; a second electrically conductive element, wherein one or more of the first electrically conductive element and the second electrically conductive element are configured to provide a measurement of a resistive property indicative of a presence or amount of moisture in a conduit.

In some configurations, the first electrically conductive element or the second electrically conductive element can comprise at least two portions that are electrically disconnected from one another.

In some configurations, the at least two portions can protrude into a lumen of the conduit.

In some configurations, the at least two portions can be flush with an inner wall of the conduit.

In some configurations, the at least two portions can be arranged within the tube wall and can be pneumatically coupled with the lumen of the conduit.

In some configurations, the at least two portions that can be electrically disconnected from one another can be in series with one another.

In some configurations, the at least two portions that can be electrically disconnected from one another can be in parallel with one another.

In some configurations, the conduit can further comprise a controller configured to determine a presence and/or indication of moisture within the conduit by determining a resistance or change in resistance.

In some configurations, the controller can be one or more microprocessors.

The present disclosure provides a conduit used with a respiratory or surgical gases supply system, the conduit comprising a first electrically conductive element; a second electrically conductive element, wherein one or more of the first electrically conductive element and the second electrically conductive element are configured to provide a measurement of a temperature or thermal conductivity property indicative of a presence or amount of moisture in a conduit.

In some configurations, the first electrically conductive element or the second electrically conductive element can further comprise a thermistor.

In some configurations, the first electrically conductive element or the second electrically conductive element can further comprise a diode.

In some configurations, the diode can be electrically connected in parallel with the thermistor.

In some configurations, the diode can be electrically connected in parallel, and positioned substantially adjacent, with the thermistor.

In some configurations, the first electrically conductive element and the second electrically conductive element can be adjacent to each other.

In some configurations, the first electrically conductive element and the second electrically conductive element can be not adjacent to each other.

In some configurations, the first electrically conductive element and the second electrically conductive element can be within a bead of the conduit.

In some configurations, the conduit can further comprise a controller configured to determine a presence and/or indication of moisture within the conduit by determining a temperature, a thermal conductivity, a change in temperature, or a change in thermal conductivity of the first electrically conductive element or the second electrically conductive element.

In some configurations, the controller can be configured to apply additional power to the first electrically conductive element in conjunction with a normal control power.

In some configurations, the controller can be one or more microprocessors.

The present disclosure provides a conduit used with a respiratory or surgical gases supply system, the conduit comprising a first electrically conductive element; a second electrically conductive element, wherein one or more of the first electrically conductive element and the second electrically conductive element are configured to measure a magnitude and/or phase of a signal or a change in a magnitude and/or phase of a signal indicative of a presence or amount of moisture in a conduit.

In some configurations, the first electrically conductive element can be configured to be a transmitter.

In some configurations, the second electrically conductive element can be configured to be a receiver to receive a signal transmitted by the first electrically conductive element.

In some configurations, the transmitter can comprise a loop antenna.

In some configurations, the receiver can comprise a loop antenna.

In some configurations, the transmitter can comprise a monopole antenna.

In some configurations, the receiver can comprise a monopole antenna.

In some configurations, the first electrically conductive element can be electrically coupled to a first switch.

In some configurations, the second electrically conductive element can be electrically coupled to a second switch.

In some configurations, the first switch can be configured to electrically disconnect one end of the first electrically conductive element and the second switch can be configured to electrically disconnect one end of the second electrically conductive element.

In some configurations, the conduit can further comprise a controller configured to determine a presence and/or indication of moisture within the conduit by determining a magnitude and/or phase of a signal or a change in a magnitude and/or phase of a signal in the first electrically conductive element or the second electrically conductive element.

In some configurations, the controller can be one or more microprocessors.

In some configurations, the material can be a fluid permeable material.

Throughout the present disclosure, the "liquid" and "fluid" are used interchangeably.

In some configurations, the first electrically conductive element and second electrically conductive element can be elongate filaments.

In some configurations, the elongate filament can be surrounded by an electrically insulating jacket.

In some configurations, the first electrically conductive element and second electrically conductive element can be spirally wound about at least a portion of a length of the conduit.

In some configurations, the first electrically conductive element and second electrically conductive element extend from one end of the conduit to the other end of the conduit.

In some configurations, the first electrically conductive element and second electrically conductive element extend only a portion of a length from one end of the conduit to the other end of the conduit.

In some configurations, the conduit can include and/or communicate with a controller configured to determine a presence and/or indication of moisture within the conduit by determining a capacitance or change in capacitance between the first electrically conductive element and the second electrically conductive element.

In some configurations, the conduit can be a composite conduit.

In some configurations, the first electrically conductive element and second electrically conductive element form part of a wall of the conduit.

In some configurations, the first electrically conductive element and second electrically conductive element can form part of a bead disposed in a composite conduit.

In some configurations, alternatively, the first conductive element and second electrically conductive element can be disposed in the conduit such that the first conductive element and second electrically conductive element can freely move within the conduit.

In some configurations, the material can be a vapor and/or liquid permeable material.

In some configurations, the material can allow evaporation of water to ambient air while inhibiting passage of liquid water and breathing gases to ambient air.

In some configurations, the material can be a one or more of an activated perfluorinated polymer material having extreme hydrophilic properties, hydrophilic thermoplastic, breathable thermoplastic copolyester, woven treated fabric exhibiting breathable characteristics, or a hydrophilic polyester block copolymer.

In some configurations, the conduit can further comprise microstructures configured to use capillary action to move moisture.

In some configurations, the vapor and/or liquid permeable material can be a dielectric material.

In some configurations, the conduit further can comprise microstructures configured to wick moisture across a portion of the first electrically conductive element and/or the second electrically conductive element.

In some configurations, the conduit further can comprise openings configured to convey moisture by capillary action between the first electrically conductive element and the second electrically conductive element.

In some configurations, the conduit further can comprise a wicking material configured to convey moisture between the first electrically conductive element and the second electrically conductive element.

In some configurations, the first electrically conductive element and the second electrically conductive element can be ribbon wires.

In some configurations, the first electrically conductive element and the second electrically conductive element can be comprised within a permeable, non-permeable or partially permeable and non-permeable bead.

In some configurations, the first electrically conductive element and the second electrically conductive element and bead can be coextruded.

In some configurations, the conduit can further comprise an electrically conductive mesh.

In some configurations, a spacing between the first electrically conductive element and the second electrically conductive element can be variable depending a presence and/or amount of moisture present within the conduit.

In some configurations, the material can cause the first electrically conductive element and the second electrically conductive element to touch or separate based on a presence of moisture.

In some configurations, the material can comprise an opening, keyhole, dip, channel and/or void configured to allow moisture between the first electrically conductive element and the second electrically conductive element and affect a capacitive effect between the first electrically conductive element and the second electrically conductive element.

In some configurations, the first electrically conductive element and the second electrically conductive element can be sensitive to touching of the conduit.

In some configurations, the material can comprise an accordion shape that expands or contracts in the presence of moisture, thereby moving the first electrically conductive element and the second electrically conductive element further apart or closer together.

In some configurations, the material can cause the first electrically conductive element and the second electrically conductive element to touch or separate based on a presence of moisture.

The present disclosure provides a humidifier system useable in a gases supply system, the humidifier system comprising: a humidifier; a conduit comprising a conductive element; and a controller configured to monitor a signal using the electrically conductive element to determine a value indicative of moisture in the conduit based at least in part on the signal.

In some configurations, the signal can be indicative of a time constant or a resonant frequency of the electrically conductive element.

In some configurations, the signal can be indicative of a change in a time constant or a change in a resonant frequency of the electrically conductive element.

In some configurations, the value can be indicative of moisture in the conduit corresponds to an inductance of the conduit.

In some configurations, the value indicative of moisture in the conduit can correspond to a change in inductance of the conduit.

In some configurations, the humidifier system can further comprise a resonant circuit wherein an inductive element is electrically connected in parallel with a capacitive element.

In some configurations, the resonant circuit can be electrically connected in parallel with the electrically conductive element.

In some configurations, the humidifier system can further comprise a signal generator.

In some configurations, the controller can comprise a signal generator.

In some configurations, the controller can comprise one or more hardware and/or software processors.

In some configurations, the resonant circuit can be tuned to exhibit resonant behavior when sufficiently excited by the signal.

In some configurations, the resonant circuit can be tuned to exhibit resonant behavior when excited by the signal, wherein the signal has been selected to excite the resonant circuit.

In some configurations, the controller can be configured to apply additional power to the electrically conductive element in conjunction with a normal control power.

In some configurations, the humidifier system can further comprise an AC power supply.

In some configurations, the humidifier system can further comprise a DC power supply.

In some configurations, the signal can be indicative of a temperature of the electrically conductive element.

In some configurations, the signal can be indicative of a change temperature of the electrically conductive element.

In some configurations, the signal can be indicative of a thermal conductivity of a medium proximal to the electrically conductive element.

In some configurations, the signal can be indicative of a change in thermal conductivity of a medium proximal to the electrically conductive element.

In some configurations, a change in temperature of the electrically conductive element can be substantially linear.

In some configurations, the electrically conductive element can further comprise a thermistor.

In some configurations, the electrically conductive element can further comprise a diode.

In some configurations, the diode can be electrically connected in parallel with the thermistor.

In some configurations, the diode can be electrically connected in parallel, and positioned substantially adjacent, with the thermistor.

In some configurations, the electrically conductive element can be within a bead of the conduit.

In some configurations, the electrically conductive element can measure the signal.

In some configurations, the controller can be configured to output an alarm if the value indicative of moisture falls below a first threshold value.

In some configurations, the controller can be configured to output an alarm if the value indicative of moisture exceeds a second threshold value.

In some configurations, the alarm indicates an unacceptable level of moisture.

In some configurations, the alarm indicates an unacceptable level of moisture.

In some configurations, the controller can be configured to automatically reduce humidification of breathing or insufflation gases in response to the value indicative of moisture and/or humidity in the conduit.

In some configurations, the reduction of humidity delivered to the conduit can be achieved by a reduction in heater plate power.

In some configurations, the conduit can be a composite conduit.

In some configurations, the conduit can comprise a vapor and/or liquid permeable bead.

In some configurations, the permeable bead can allow evaporation of water to ambient air while inhibiting passage of liquid water and breathing gases to ambient air.

In some configurations, the permeable bead can be one or more of an activated perfluorinated polymer material having extreme hydrophilic properties, hydrophilic thermoplastic, breathable thermoplastic copolyester, woven treated fabric exhibiting breathable characteristics, or a hydrophilic polyester block copolymer.

In some configurations, the electrically conductive element can be spirally wound about at least a length of the conduit.

In some configurations, the electrically conductive element can be spirally wound within, through or around the conduit.

In some configurations, the electrically conductive element can form part of the conduit walls.

In some configurations, the electrically conductive element can be a sensing wire. The electrically conductive element can be a heater wire.

The present disclosure provides a method of detecting moisture in a conduit utilized to transport humidified gases, the method comprising providing an electrically conductive elements located within, around or on the conduit and measuring a time constant, a resonant frequency, a change in a time constant, or a change in a resonant frequency to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing an electrically conductive elements located within, around or on the conduit and measuring a temperature or a change in temperature to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a method of detecting moisture in a conduit utilized to transport humidified gases, the method comprising providing an electrically conductive elements and located within, around or on the conduit and measuring a thermal conductivity or a change in thermal conductivity to indicate a measure of moisture or condensate within the conduit.

In some configurations, the method uses the conduit of any of conduit implementations disclosed herein.

In some configurations, the method uses the humidifier system of any humidifier system disclosed herein.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least one electrical terminal or pad configured to make an electrical coupling with an electrically conductive element extending along at least a portion of a length of the inspiratory conduit; and a controller communicatively coupled with the one or more sensors and the first and the second electrical connectors.

In some configurations, the cartridge can be removably attachable to the humidifier, and the controller can be configured to, in use, measure a signal indicative of a time constant or a resonant frequency of a circuit comprising the electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least one electrical terminal or pad configured to make an electrical coupling with an electrically conductive element extending along at least a portion of a length of the inspiratory conduit; and a controller communicatively coupled with the one or more sensors and the first and the second electrical connectors.

In some configurations, the cartridge can be removably attachable to the humidifier, and the controller can be configured to, in use, measure a signal indicative of a temperature of the electrically conductive element of the removable inspiratory conduit.

The present disclosure provides a cartridge for use with a humidifier in a respiratory or surgical humidification system, the cartridge comprising one or more sensors for sensing a property of a gases flow in a removable humidification chamber of the humidifier; a first electrical connector configured to make an electrical connection with a corresponding electrical connector of the humidifier; a second electrical connector configured to make an electrical connection with a corresponding electrical connector of an inspiratory conduit removably engageable with the cartridge, wherein the second electrical connector can comprise at least one electrical terminal or pad configured to make an electrical coupling with an electrically conductive element extending along at least a portion of a length of the inspiratory conduit; and a controller communicatively coupled with the one or more sensors and the first and the second electrical connectors.

In some configurations, the cartridge can be removably attachable to the humidifier, and the controller can be configured to, in use, measure a signal indicative of a thermal conductivity of a medium proximal to the electrically conductive element of the removable inspiratory conduit.

The present disclosure provides a humidifier useable in a gases supply system, the humidifier comprising a humidification chamber configured to humidify a gases supply; an inspiratory conduit connector configured to connection with an inspiratory conduit including an electrically conductive element; a controller configured to monitor a signal using the electrically conductive element to determine a value indicative of moisture in the conduit based at least in part on the signal.

In some configurations, the signal can be indicative of a time constant or a resonant frequency of the electrically conductive element.

In some configurations, the signal can be indicative of a temperature of the electrically conductive element.

In some configurations, the signal can be indicative thermal conductivity of a medium proximal to the electrically conductive element.

In some configurations, the controller can comprise a signal generator.

In some configurations, the controller can comprise one or more hardware and/or software processors.

In some configurations, the humidifier can further comprise the conduit of any of conduit implementations disclosed herein.

The present disclosure provides a conduit used with a respiratory or surgical gases supply system, the conduit comprising an electrically conductive element, wherein the electrically conductive element is configured to provide a measurement of a time constant or a resonant frequency indicative of a presence or amount of moisture in a conduit.

In some configurations, the conduit can further comprise a resonant circuit wherein an inductive element is electrically connected in parallel with a capacitive element.

In some configurations, the resonant circuit can be external to the conduit.

In some configurations, the resonant circuit can be tuned to exhibit resonant behavior when sufficiently excited by the signal.

In some configurations, the resonant circuit can be tuned to exhibit resonant behavior when excited by the signal, wherein the signal has been selected to excite the resonant circuit.

In some configurations, the electrically conductive element can be configured to be electrically connected in parallel with a signal generator.

In some configurations, the conduit can further comprise a controller configured to determine a presence and/or indication of moisture within the conduit by determining a time constant, a resonant frequency, a change in time constant, or a change in resonant frequency.

In some configurations, the electrically conductive can be configured to be electrically connected in parallel with the controller.

In some configurations, the controller can comprise a signal generator.

In some configurations, the controller can comprise one or more microprocessors.

The present disclosure provides a conduit used with a respiratory or surgical gases supply system, the conduit comprising an electrically conductive element, wherein the electrically conductive element is configured to provide a measurement of a temperature or thermal conductivity property indicative of a presence or amount of moisture in a conduit.

In some configurations, the electrically conductive element can further comprise a thermistor.

In some configurations, the electrically conductive element can further comprise a diode.

In some configurations, the diode can be electrically connected in parallel with the thermistor.

In some configurations, the diode can be electrically connected in parallel, and positioned substantially adjacent, with the thermistor.

In some configurations, the electrically conductive element can be within a bead of the conduit.

In some configurations, the conduit can further comprise a controller configured to determine a presence and/or indication of moisture within the conduit by determining a temperature and/or a change in temperature of the electrically conductive element, and/or by determining a thermal conductivity of a medium proximal to the electrically conductive element and/or a change in thermal conductivity of a medium proximal to the electrically conductive element.

In some configurations, the controller can be configured to apply additional power to the electrically conductive element in conjunction with a normal control power.

In some configurations, the controller can be one or more microprocessors

The present disclosure can be applied to any known conduit with two electrically conductive elements.

In some configurations, the material can be a fluid permeable material.

In some configurations, the electrically conductive element can be elongate filaments.

In some configurations, the elongate filament can be surrounded by an electrically insulating jacket.

In some configurations, the electrically conductive element can be spirally wound about at least a portion of a length of the conduit.

In some configurations, the electrically conductive element may extend from one end of the conduit to the other end of the conduit.

In some configurations, the electrically conductive element may extend only a portion of a length from one end of the conduit to the other end of the conduit.

In some configurations, the conduit can be a composite conduit.

In some configurations, the electrically conductive element can form part of a wall of the conduit.

In some configurations, the electrically conductive element can form part of a bead disposed in a composite conduit. Alternatively, the first conductive element and second electrically conductive element can be disposed in the conduit such that the first conductive element and second electrically conductive element can freely move within the conduit.

In some configurations, the material can be a vapor and/or liquid permeable material.

In some configurations, the material can allow evaporation of water to ambient air while inhibiting passage of liquid water and breathing gases to ambient air.

In some configurations, the material can be a one or more of an activated perfluorinated polymer material having extreme hydrophilic properties, hydrophilic thermoplastic, breathable thermoplastic copolyester, woven treated fabric exhibiting breathable characteristics, or a hydrophilic polyester block copolymer.

In some configurations, the conduit can further comprise microstructures configured to use capillary action to move moisture.

In some configurations, the vapor and/or liquid permeable material can be a dielectric material.

In some configurations, the conduit further can comprise microstructures configured to wick moisture across a portion of the electrically conductive element.

In some configurations, the conduit further can comprise openings configured to convey moisture by capillary action. The conduit further can comprise a wicking material configured to convey moisture.

In some configurations, the electrically conductive element can be ribbon wires.

In some configurations, the electrically conductive element can be comprised within a permeable, non-permeable or partially permeable and non-permeable bead.

In some configurations, the electrically conductive element and bead can be coextruded.

In some configurations, the conduit can further comprise an electrically conductive mesh.

In some configurations, the electrically conductive element can be sensitive to touching of the conduit.

A humidifier system according to the present disclosure may deliver a flow of gases to a user. The humidifier may provide a humidifier that may heat and humidify the gases, a conduit to transport the gases from the humidifier to the user, at least one sensor that may output at least one sensor signal based on an amount of moisture present in a component of the system, and a controller to control operation of the humidifier. The controller may determine at least one value indicative of the amount of moisture present in the component based at least in part on the at least one sensor signal and execute one or more moisture management responses based on the at least one value indicative of the amount of moisture present in the component.

In some configurations, the one or more moisture management may include one or more of the following: generating at least one notification and/or alarm, changing power supplied to a humidifier heater, changing power suppled to at least one conduit heater, changing a mode of the humidifier, and/or changing a parameter of a ventilator which may be part of the humidification system.

In some configurations, the component may include the conduit, a portion of the conduit, a connector, a portion of a chamber that may be exposed to the flow of gases, and/or a patient interface.

In some configurations, the moisture present in the component of the system may include condensate, and/or water in a vapor permeable and/or liquid permeable material of the component of the system.

In some configurations, the vapor permeable material may be a breathable material.

In some configurations, the at least one value indicative of the amount of moisture present in the component may include at least one of a value indicative of the amount of condensate in the component of the system, and/or a value indicative of the amount of water in the vapor permeable and/or liquid permeable material of the component of the system. The value indicative of the amount of moisture present in the component may be a sum of the value indicative of the amount of condensate in the component of the system and the value indicative of the amount of water in the vapor permeable and/or liquid permeable material of the component of the system.

In some configurations, the controller may determine at least one value indicative of the amount of moisture present in the component based on a humidity output of the humidifier.

In some configurations, the controller may determine that a predetermined level of moisture may be present in the component based on the at least one value indicative of the amount of moisture present in the component.

In some configurations, the controller may determine that the predetermined level of moisture may be present in the component based on comparing the at least one value indicative of the amount of moisture present in the component and at least one threshold.

In some configurations, the predetermined level of moisture may be determined to be present in response to the at least one the at least one value being above the at least one threshold.

In some configurations, the predetermined level of moisture may be determined to be present in response to the at least one value being below the at least one threshold.

In some configurations, the at least one threshold mat include an absolute value threshold, a value change threshold, a percentage threshold of a maximum sensor output, a percentage change threshold, a percentage change over time threshold, a gradient threshold, and/or a crossing threshold.

In some configurations, the maximum sensor output may provide an output indicating that the vapor and/or liquid material of the conduit may be saturated with moisture.

In some configurations, the maximum sensor output may provide an output indicating that the predetermined or acceptable amount of moisture may be present.

In some configurations, the percentage threshold may be 80%.

In some configurations, the percentage threshold may be 90%

In some configurations, the percentage threshold may be 100%.

In some configurations, the crossing threshold may occur when the output crosses a particular threshold a number of times. This may optionally be over a predetermined time period.

In some configurations, the system may generate at least one notification and/or alarm in response to the predetermined level of moisture determined to be present in the component.

In some configurations, the controller may determine the presence of condensate in the component based on a comparison between the at least one value indicative amount of moisture present in the component and at least one condensate threshold.

In some configurations, the controller may determine the presence of a predetermined amount of water in a vapor permeable and/or liquid permeable material of the component of the system based on a comparison between the at least one value indicative of the amount of moisture present in the component and at least one water amount threshold. The predetermined amount of water may optionally be indicative of saturation of the vapor and/or liquid permeable material.

In some configurations, the controller may control the heater of the humidifier and/or the heater of the conduit to achieve a humidity target.

In some configurations, the humidity target may include a dew point target, an absolute target, and/or a relative humidity target.

In some configurations, the controller may decrease humidity target, and optionally the absolute humidity and/or relative humidity target, of the flow of gases in response to a predetermined amount of moisture being determined to be present in the component.

In some configurations, the humidifier may include a heater configured to heat the humidification liquid.

In some configurations, the heater may include a heater plate.

In some configurations, the conduit may include at least one heater wire to heat the flow of gases in the conduit.

In some configurations, the controller may decrease the absolute humidity of the flow of gases by changing at least one operating parameter of the heater of the humidifier.

In some configurations, the controller may decrease the relative humidity of the flow of gases by changing the at least one operating parameter of the heater of the humidifier.

In some configurations, the changing includes decreasing or limiting or disabling the relative humidity of the flow of gases.

In some configurations, the at least one operating parameter may include a heater temperature set point and/or a power to a heater.

In some configurations, the controller may decrease the relative humidity target of the flow of gases by changing at least one operating parameter of the heater of the conduit. In some configurations, the changing may include increasing the operating parameter.

In some configurations, the operating parameter of the at least one heater of the conduit may be a patient end temperature set point and/or a power to the at least one heater of the conduit.

In some configurations, the operating parameter of the heater of the humidifier and/or the heater of the conduit may be changed based on the at least one value indicative of the amount of moisture present in the component.

In some configurations, the controller may further decrease an absolute humidity targe, and/or a relative humidity target in response to determining that the predetermined level of moisture may be still present in the component a predetermined time period after initially determining that the predetermined level of moisture may be present.

In some configurations, the controller may return to a normal operation, and/or revert to an original absolute humidity and/or relative humidity target of the flow of gases, in response to determining that the predetermined level of moisture may be no longer present in the component after initially determining that the predetermined level of moisture may be present.

In some configurations, the controller may return to normal operation, and/or revert to an original absolute humidity and/or relative humidity target of the flow of gases, in response to determining that the predetermined level of moisture may be no longer present in the component after initially determining that the predetermined level of moisture may be present.

In some configurations, the controller may determine that the predetermined level of moisture may be no longer present in the component in response to detecting an absence of moisture or a small amount of moisture present in the component.

In some configurations, the controller may determine that the predetermined level of moisture may be no longer present in the component in response to detecting an absence of condensate or a small amount of condensate present in the component.

In some configurations, at least a portion of the conduit or another component of the system may include a vapor permeable and/or liquid permeable material.

In some configurations, the conduit may include at least a first electrically conductive element and a second electrically conductive element, the at least one sensor signal comprising a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element are spirally wound within, through, or around the conduit.

In some configurations, the first electrically conductive element and the second electrically conductive element for part of conduit walls.

In some configurations, the first electrically conductive element may be a sensing wire

In some configurations, the first electrically conductive element may be at least one heater wire.

In some configurations, the second electrically conductive element may be at least one heater wire.

In some configurations, the vapor permeable and/or liquid permeable material may be a dielectric material.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the other component may include a patient interface.

In some configurations, the other component may include connectors and/or adaptors.

In some configurations, the conduit may include a composite conduit.

In some configurations, the conduit may include a vapor and/or liquid permeable bead.

In some configurations, the vapor permeable bead allows evaporation of water to ambient air while inhibiting passage of liquid water and breathing gases to ambient air.

In some configurations, the vapor permeable bead may be an activated perfluorinated polymer material having extreme hydrophilic properties, hydrophilic thermoplastic, breathable thermoplastic copolyester, woven treated fabric exhibiting breathable characteristics, and/or a hydrophilic polyester block copolymer.

In some configurations, the signal may be indicative of a capacitance between the first electrically conductive element and the second electrically conductive element.

In some configurations, the signal may be indicative of a change in capacitance between the first electrically conductive element and the second electrically conductive element.

In some configurations, the first electrically conductive element and the second electrically conductive element are separated by a distance configured to allow for a capacitive charge to be sensed between the first electrically conductive element and the second electrically conductive element.

In some configurations, the humidifier system may further include a dielectric material located between the first electrically conductive element and the second electrically conductive element.

In some configurations, the controller may determine the at least one value indicative of moisture based on a comparison of a measurement of the first electrically conductive element and/or the second electrically conductive element.

In some configurations, the signal may be indicative of a temperature of the first electrically conductive element or the second electrically conductive element.

In some configurations, the signal may be indicative of a change in temperature of the first electrically conductive element or the second electrically conductive element.

In some configurations, the signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the signal may be indicative of a resistance of the first electrically conductive element or the second electrically conductive element.

In some configurations, the first electrically conductive element or the second electrically conductive element comprise at least two portions that are electrically disconnected from one another.

In some configurations, the two portions may be in series with one another.

In some configurations, the humidifier system may further include a signal generator wherein the controller may determine that at least one value indicative of the amount of moisture in the conduit based at least in part on a magnitude and/or phase of the at least one signal.

In some configurations, the at least one value indicative of the amount of moisture in the conduit may correspond to an inductance of the conduit.

In some configurations, the at least one value indicative of the amount of moisture in the conduit corresponds to a change in inductance of the conduit.

In some configurations, the predetermined level of moisture present in the component may be indicative of a predetermined amount of water in a vapor permeable and/or liquid permeable material of the component of the system.

In some configurations, the controller may initially change an operating parameter of the at least one heater wire of the conduit in response to the predetermined level of moisture being determined to be present in the component. In some configurations, the changing may include decreasing, limiting, or disabling the operating parameter. In some configurations, the decreasing or limiting includes decreasing or limiting power to the at least one heater wire to a range of 3.0W/m to 7.0W/m.

In some configurations, the controller may maintain the initially changed operating parameter of the heater wire of the conduit.

In some configurations, maintaining the initially changed operating parameter of the at least one heater wire of the conduit may increase a moisture saturation of the vapor permeable and/or liquid permeable material. This change may optionally be an increase to a predetermined moisture saturation.

In some configurations, the controller may further change the operating parameter of the at least one heater wire of the conduit.

In some configurations, the controller may further change the operating parameter of the heater wire of the conduit a predetermined time after having maintained the initially changed operating parameter.

In some configurations, the further change may be an increase of the operating parameter.

In some configurations, the controller may change an operating parameter of the heater of the conduit in response to the predetermined level of moisture being determined to be present in the component.

In some configurations, the predetermined level may be a percentage threshold of a maximum sensor output. In some configurations, the percentage threshold may be 80%. In some configurations, the percentage threshold may be 90%. In some configurations, the percentage threshold may be 100%. A fully saturated permeable material may be at 100%.

In some configurations, the maximum sensor output may be an output indicating that a bead of the conduit may be saturated with moisture.

In some configurations, the maximum sensor output may be an output indicating that the predetermined or acceptable amount of moisture may be present.

In some configurations, the controller may maintain the changed operating parameter of the at least one heater wire of the conduit to allow for drying of the vapor permeable and/or liquid permeable material via evaporation to the atmosphere.

In some configurations, the humidifier system may maintain the changed operating parameter increase a rate at which the moisture may be transferred across the vapor permeable material to the atmosphere.

In some configurations, the changed operating parameter may include an increased power to between 10W/m and 14W/m to the at least one heater wire.

In some configurations, the controller may return to a normal or original operating parameter of the at least one heater of the conduit a predetermined time after having maintained the changed operating parameter.

In some configurations, the controller may repeat increasing and maintain the operating parameter and then decreasing and maintaining the operating parameter a number of times before returning to the normal or original operating parameter.

In some configurations, the controller may return to a normal or original operating parameter of the at least one heater of the conduit in response to the predetermined level of moisture in the component being determined to fall below a threshold. In some configurations, the threshold may be a percentage threshold. In some configurations, the percentage threshold may be 20%. In some configurations, the percentage threshold may be 15%. In some configurations, the percentage threshold may be 0%.

In some configurations, the controller may return to the normal or original operating parameter of the at least one heater wire of the conduit in response to the predetermined quantity of water molecules in the vapor permeable and/or liquid permeable material being determined to fall below a water molecule threshold.

In some configurations, the controller may return to the normal or original operating parameter of the at least one heater wire of the conduit in response to the predetermined level of condensate in the component being determined to fall below a condensate threshold.

In some configurations, the operating parameter of the at least one heater wire of the conduit may include a patient end temperature set point, and/or a power to the at least one heater wire.

In some configurations, the controller may increase a temperature of the vapor permeable material via a heater of the conduit to increases a rate at which water passes from one side of the material to an opposite side of the material.

In some configurations, the controller may increase a temperature of the liquid permeable material via a heater of the conduit to increases an evaporation rate of liquid water in the liquid permeable material to atmosphere.

In some configurations, the controller may control a relative humidity gradient between the vapor permeable and/or liquid permeable material and the atmosphere to be greater than a relative humidity gradient between the vapor permeable and/or liquid permeable material and a lumen of the conduit.

In some configurations, the humidifier system may include a moisture draining assembly configured to collect condensate and/or transport the condensate back towards the humidification chamber.

In some configurations, the moisture draining assembly may include a water trap configured to collect the condensate.

In some configurations, the moisture draining assembly comprises at least one valve electronically coupled to the controller. The controller may actuate the at least one valve based on the at least one sensor signal.

In some configurations, the controller may actuate the at least one valve into an open position in response to a predetermined level of moisture being determined to be present in the component.

In some configurations, the controller may actuate the at least one valve into an open position in response to a predetermined level of condensate being determined to be present in the component.

In some configurations, the moisture draining assembly may include a moisture conveying assembly configured to convey moisture collected in the water trap or the conduit back to the humidification chamber.

In some configurations, the moisture draining assembly may further include a conveying conduit, and/or a pump. In some configurations, the pump may be a peristaltic pump.

In some configurations, the controller may determine a chamber overflow event based on the at least one value indicative of the amount of moisture present in the component.

In some configurations, the component may be located near an outlet of a humidification chamber of the humidifier.

In some configurations, the sensor may include a temperature sensor located near a distal end of the conduit.

In some configurations, the controller may determine a dew point temperature or a value indicative thereof of the flow of gases based on a temperature of the flow of gases sensed by the temperature sensor in response to determining the presence of a predetermined amount of moisture.

In some configurations, the controller may determine the dew point temperature or a value indicative thereof based on the temperature of the flow of gases sensed by the temperature sensor in response to determining the presence of a predetermined amount of condensate.

In some configurations, the dew point temperature or a value indicative thereof may be the temperature of the flow of gases sensed by the temperature sensor.

In some configurations, the controller may control a heater of the humidifier or a heater of the conduit based on the determined dew point temperature or a value indicative thereof.

In some configurations, the controller may decrease an operating parameter of the heater of the conduit to increase a relative humidity in the conduit and induce formation of condensate.

In some configurations, the controller may control the gases exiting the humidifier to reach a dew point temperature set point.

In some configurations, the controller may determine that a source of the gases for the humidifier may be the ambient air or from a room-entraining ventilator in response to determining that a patient end temperature may be greater than the dew point temperature set point and that a predetermined level of moisture may be present.

In some configurations, when the controller determines that a source of the gases for the humidifier is the ambient air or from a room-entraining ventilator, the controller may control a temperature set point of the humidifier to a constant value.

In some configurations, the temperature set point can be a chamber outlet set point.

In some configurations, the chamber outlet set point may correspond to the dew point of the gases.

In some configurations, the controller may determine the presence of a predetermined level of moisture in the conduit based on the at least one value indicative of the amount of moisture in the conduit in response to the conduit being connected to a humidifier and/or prior to operation of the humidifier.

In some configurations, the controller may determine the presence of the predetermined level of moisture in the conduit based on a comparison between a threshold and the at least one value indicative of the amount of moisture in the conduit.

In some configurations, the controller may determine the presence of the predetermined level of moisture in the conduit when the conduit may be first connected to the humidifier.

In some configurations, the predetermined level of moisture present in the conduit may be a level for which a new unused conduit may be needed or the conduit needs to be checked for faults or dried further.

In some configurations, the controller may generate an alarm in response to determining the presence of the predetermined level of moisture in the conduit.

In some configurations, the alarm can include a visual and/or audio indication.

In some configurations, the controller may prevent use of the conduit with the humidifier in response to determining the presence of the predetermined level of moisture in the conduit by preventing powering of the heater of the conduit and/or preventing use of the humidifier while connected to the conduit.

In some configurations, the conduit may not include a patient end sensor.

In some configurations, the at least one sensor signal based on the amount of moisture present in the component may further include a signal relating to a humidity of the gases in the component.

In some configurations, the signal relating to the humidifier of the gases in the component may be filtered from the at least one signal or ignored by the controller.

In some configurations, the controller may determine at least one value indicative of the amount of moisture present in the component based on the at least one sensor signal and control a function of the gases source to reduce moisture in response to a predetermined amount of moisture determined to be present in the component based on the value.

In some configurations, the function may include reducing a tidal volume, increasing an inspiratory rise time, reducing inspiratory to expiratory ratio, increasing an inlet gases temperature, and/or reducing an amount of entrained air and/or switching to a wall or bottle gases source.

In some configurations, the gases source may include a ventilator.

In some configurations, the gases source may include a glow generator.

In some configurations, the controller may determine at least one value indicative of the amount of moisture present in the component based on the at least one sensor signal, and determine a water-out condition of the humidification chamber based on the at least one value indicative of the amount of moisture present in the component.

In some configurations, no water-out condition may be determined in response to detecting an increase in the amount of moisture and/or humidity in the component.

In some configurations, no water-out condition may be determined in response to the increase in the amount of moisture and/or humidity in the component being above a threshold.

In some configurations, a water-out condition may be determined in response to detecting no increase in the amount of moisture and/or humidity in the component.

In some configurations, a water-out condition may be determined in response to the increase in the amount of moisture and/or humidity in the component being below a threshold.

In some configurations, the water-out condition comprises no water present or a predetermined level of water present.

In some configurations, the controller may increase power to a heater plate of the humidifier and/or reduce power to at least one heater wire of a conduit configured to provide the gases from the humidifier to the user so as to increase humidity and/or increase moisture.

In some configurations, the increased moisture may be condensate or water in vapor permeable and/or liquid permeable material.

In some configurations, the controller may determine the water-out condition subsequent to increasing the power to the heater plate and/or reducing the power to the at least one heater wire in response to the predetermined amount of moisture and/or humidity being determined to be below a threshold.

A humidifier system may deliver a flow of gases to a user. The humidifier system may include a humidifier to heat and humidify the gases, at least one sensor to output at least one sensor signal based on an amount of moisture and/or humidity present in a component of the system, and a controller to controller operation of the humidifier, wherein the controller may determine at least one value indicative of the amount of moisture and/or humidity present in the component based on the at least one sensor signal, and determine a humidity of the flow of gases based on the at least one value indicative of the amount of moisture and/or humidity present in the component.

In some configurations, the controller may assume 100% relative humidity upon detection of presence of moisture based on the value.

In some configurations, the temperature sensor located near a distal end of a conduit may transport the gases from the humidifier to the user. The controller may determine the presence of a predetermined level of moisture based on the at least one value indicative of the amount of moisture and/or humidity present in the component. The controller may determine a dew point of the flow of gases, as the humidity of the flow of gases.

In some configurations, the humidity may be a dew point temperature, a relative humidity value, or an absolute humidity value.

In some configurations, the controller may control a heater of the humidifier and/or conduit based on the humidity to control a target humidity.

In some configurations, the controller may determine the presence of a predetermined level of moisture based on the at least one value indicative of the amount of moisture and/or humidity present in the component.

In some configurations, the controller may control at least one operating parameter of the humidifier system based on the determined dew point temperature or a value indicative thereof.

In some configurations, the controller may control a heater of the humidifier and a heater of the conduit based on the determined dew point temperature or a value indicative thereof.

In some configurations, the operating parameter of the heater of the humidifier may include at least one of a heater plate temperature set point, and a power to a heater plate.

In some configurations, the operating parameter of the heater of the conduit may include at least one of a patient end temperature set point, and a power to at least one heater wire.

In some configurations, the determined dew point temperature or a value indicative thereof may be the temperature of the flow of gases sensed by the temperature sensor.

In some configurations, the controller may decrease an operating parameter of a heater of the conduit to increase a relative humidity in the conduit and induce the formation of condensate.

In some configurations, the controller may control the heater of the conduit to reach a patient end temperature set point.

In some configurations, the target patient end temperature may be greater than the determined dew point temperature or a value indicative thereof.

In some configurations, the determined humidity of the flow of gases may be a relative humidity or an absolute humidity.

In some configurations, an output of the at least one sensor may be filtered to obtain a portion of the at least one signal that may be related to the humidity in the component.

In some configurations, the humidifier may not include a patient end sensor.

In some configurations, the humidifier system may include a flow generator.

In some configurations, the flow generator and the humidifier may be in a single housing.

In some configurations, the conduit may include at least a first electrically conductive element and a second electrically conductive element. The at least one sensor signal may include a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element may be spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be at least one heater wire.

In some configurations, the second electrically conductive element may be a sensing wire.

In some configurations, the second electrically conductive element may be at least one heater wire.

In some configurations, the at least one signal may be indicative of a capacitance between the first electrically conductive element and the second electrically conductive element.

In some configurations, the at least one signal may be indicative of a change in capacitance between the first electrically conductive element and the second electrically conductive element.

In some configurations, the first electrically conductive element and the second electrically conductive element may be separated by a distance that may allow for a capacitive charge to be sensed between the first electrically conductive element and the second electrically conductive element.

In some configurations, the humidifier system may include a dielectric material located between the first electrically conductive element and the second electrically conductive element.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the controller may determine the at least one value indicative of moisture based on a comparison of a measurement of the first electrically conductive element and/or the second electrically conductive element.

In some configurations, the at least one signal may be indicative of a temperature of the first electrically conductive element or the second electrically conductive element.

In some configurations, the at least one signal may be indicative of a change in temperature of the first electrically conductive element or the second electrically conductive element.

In some configurations, the at least one signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the at least one signal may be indicative of a resistance of the first electrically conductive element or the second electrically conductive element.

In some configurations, the first electrically conductive element or the second electrically conductive element may include at least two portions that are electrically disconnected from one another.

In some configurations, the at least two portions may be in series with one another.

In some configurations, the at least two portions may be in parallel with one another.

In some configurations, the humidifier system may further include a signal generator. The controller may determine the at least one value indicative of the amount of moisture in the conduit based at least in part on a magnitude and/or phase of the at least one signal.

In some configurations, the at least one value indicative of the amount of moisture in the conduit corresponds to an inductance of the conduit.

In some configurations, the at least one value indicative of the amount of moisture in the conduit may correspond to a change in inductance of the conduit.

A humidifier system may deliver flow of gases to a user. The humidifier system may include a humidifier to heat and humidify the gases, a first sensor associated with a first location, a second sensor associated with a second location, and a controller to control operation of the humidifier. The first sensor may output at least one first sensor signal based on an amount of moisture present at the first location. The second sensor may output at least one second sensor signal based on an amount of moisture present at the second location. The controller may determine a first value indicative of an amount of humidity and/or moisture present at the first location based on a first sensor signal, and a second value indicative of the amount of humidity and/or moisture present at the second location based on a second sensor.

In some configurations, the first location may be associated with a first component of the system.

In some configurations, the second location may be associated with a second component of the system.

In some configurations, the first and/or second component may include a conduit, a portion of the conduit, a connector, a portion of a chamber that may be exposed to the flow of gases, and/or a patient interface.

In some configurations, the connector may include one or more adapters.

In some configurations, the first location may be upstream of the second location during normal operation of the system.

In some configurations, the controller may determine that no flow and/or a flow rate below a threshold may be occurring in the system by detecting a first predetermined amount of moisture at the first location, and a predetermined time period after detecting the predetermined amount of moisture at the first location, determining that a second predetermined amount of moisture may be not detected at a second location.

In some configurations, the controller may determine that a flow rate of the gases may be in a range based on readings from a first temperature sensor at the first location and a second temperature sensor at the second location in response to detecting the first predetermined amount of moisture at the first location and not detecting the second predetermined amount of moisture at the second location a predetermined time period after detecting the predetermined amount of moisture at the first location.

In some configurations, the first temperature sensor may be located at a chamber outlet of the humidifier.

In some configurations, the second temperatures sensor may be located at or near a patient end of an inspiratory conduit or a distal end of a patient supply conduit configured to transport the gases from the humidifier to the user.

In some configurations, the controller may determine that the flow direction may be correct in response to the moisture at an upstream location being greater than the moisture at a downstream location.

In some configurations, an inspiratory conduit may include the upstream location.

In some configurations, the conduit positioned between the gases source and the humidifier may include the downstream location.

In some configurations, the humidifier system may include a third sensor associated with a third location of the system. The third sensor may output at least one third sensor signal based on an amount of moisture present at the third location.

In some configurations, the third location may be upstream of the first location and the second location.

In some configurations, the first location may be at an inspiratory conduit.

In some configurations, the third location may be at an expiratory conduit.

In some configurations, the second location may be at a chamber inlet of a humidification chamber.

In some configurations, the controller may be configured to determine a flow direction based on a direction of the moisture detected from the humidifier. The controller may be configured to detect a reverse flow condition in response to moisture being detected in a component upstream of the humidifier when correctly connected.

A humidifier system according to the present disclosure may deliver a flow of gases to a user. The humidifier may provide a humidifier that may heat and humidify the gases, a conduit to transport the gases from the humidifier to the user, at least one sensor that may output at least one sensor signal based on an amount of moisture present in a component of the system, and a controller to control operation of the humidifier. The controller may determine a value indicative of the amount of moisture present in the component based on the at least one sensor signal, and control a function of the gases source to reduce moisture in response to a predetermined amount of moisture determined to be present in the component based on the value.

In some configurations, the function may include reducing a tidal volume, increasing an inspiratory rise time, reducing inspiratory to expiratory ratio, increasing an inlet gases temperature, and/or reducing an amount of entrained air and/or switching to a wall or bottle gases source.

In some configurations, the gases source may include a ventilator.

In some configurations, the gases source may include a glow generator.

In some configurations, the conduit may include at least a first electrically conductive element and a second electrically conductive element. The at least one sensor signal may include a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element may be spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be at least one heater wire.

In some configurations, the second electrically conductive element may be a sensing wire.

In some configurations, the second electrically conductive element may be at least one heater wire.

In some configurations, the at least one signal may be indicative of a capacitance between the first electrically conductive element and the second electrically conductive element.

In some configurations, the at least one signal may be indicative of a change in capacitance between the first electrically conductive element and the second electrically conductive element.

In some configurations, the first electrically conductive element and the second electrically conductive element may be separated by a distance that may allow for a capacitive charge to be sensed between the first electrically conductive element and the second electrically conductive element.

In some configurations, the humidifier system may include a dielectric material located between the first electrically conductive element and the second electrically conductive element.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the controller may determine the at least one value indicative of moisture based on a comparison of a measurement of the first electrically conductive element and/or the second electrically conductive element.

In some configurations, the at least one signal may be indicative of a temperature of the first electrically conductive element or the second electrically conductive element.

In some configurations, the at least one signal may be indicative of a change in temperature of the first electrically conductive element or the second electrically conductive element.

In some configurations, the at least one signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the at least one signal may be indicative of a resistance of the first electrically conductive element or the second electrically conductive element.

In some configurations, the first electrically conductive element or the second electrically conductive element may include at least two portions that are electrically disconnected from one another.

In some configurations, the at least two portions may be in series with one another.

In some configurations, the at least two portions may be in parallel with one another.

In some configurations, the humidifier system may further include a signal generator. The controller may determine the at least one value indicative of the amount of moisture in the conduit based at least in part on a magnitude and/or phase of the at least one signal.

In some configurations, the at least one value indicative of the amount of moisture in the conduit corresponds to an inductance of the conduit.

In some configurations, the at least one value indicative of the amount of moisture in the conduit may correspond to a change in inductance of the conduit.

A humidifier system according to the present disclosure may deliver a flow of gases to a user. The humidifier may provide a humidifier that may heat and humidify the gases, a conduit to transport the gases from the humidifier to the user, at least one sensor that may output at least one sensor signal based on an amount of moisture present in a component of the system, and a controller to control operation of the humidifier. The controller may determine at least one value indicative of the amount of moisture present in the component based on the at least one sensor signal, and determine a water-out condition of the humidification chamber based on the at least one value indicative of the amount of moisture present in the component.

In some configurations, no water-out condition may be determined in response to detecting an increase in the amount of moisture and/or humidity in the component.

In some configurations, no water-out condition may be determined in response to the increase in the amount of moisture and/or humidity in the component being above a threshold.

In some configurations, a water-out condition may be determined in response to detecting no increase in the amount of moisture and/or humidity in the component.

In some configurations, a water-out condition may be determined in response to the increase in the amount of moisture and/or humidity in the component being below a threshold.

In some configurations, the water-out condition comprises no water present or a predetermined level of water present.

In some configurations, the controller may increase power to a heater plate of the humidifier and/or reduce power to at least one heater wire of a conduit configured to provide the gases from the humidifier to the user so as to increase humidity and/or increase moisture.

In some configurations, the increased moisture may be condensate or water in vapor permeable and/or liquid permeable material.

In some configurations, the controller may determine the water-out condition subsequent to increasing the power to the heater plate and/or reducing the power to the at least one heater wire in response to the predetermined amount of moisture and/or humidity being determined to be below a threshold.

In some configurations, the conduit may include at least a first electrically conductive element and a second electrically conductive element. The at least one sensor signal may include a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element may be spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be at least one heater wire.

In some configurations, the second electrically conductive element may be a sensing wire.

In some configurations, the second electrically conductive element may be at least one heater wire.

In some configurations, the at least one signal may be indicative of a capacitance between the first electrically conductive element and the second electrically conductive element.

In some configurations, the at least one signal may be indicative of a change in capacitance between the first electrically conductive element and the second electrically conductive element.

In some configurations, the first electrically conductive element and the second electrically conductive element may be separated by a distance that may allow for a capacitive charge to be sensed between the first electrically conductive element and the second electrically conductive element.

In some configurations, the humidifier system may include a dielectric material located between the first electrically conductive element and the second electrically conductive element.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the controller may determine the at least one value indicative of moisture based on a comparison of a measurement of the first electrically conductive element and/or the second electrically conductive element.

In some configurations, the at least one signal may be indicative of a temperature of the first electrically conductive element or the second electrically conductive element.

In some configurations, the at least one signal may be indicative of a change in temperature of the first electrically conductive element or the second electrically conductive element.

In some configurations, the at least one signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the at least one signal may be indicative of a resistance of the first electrically conductive element or the second electrically conductive element.

In some configurations, the first electrically conductive element or the second electrically conductive element may include at least two portions that are electrically disconnected from one another.

In some configurations, the at least two portions may be in series with one another.

In some configurations, the at least two portions may be in parallel with one another.

In some configurations, the humidifier system may further include a signal generator. The controller may determine the at least one value indicative of the amount of moisture in the conduit based at least in part on a magnitude and/or phase of the at least one signal.

In some configurations, the at least one value indicative of the amount of moisture in the conduit corresponds to an inductance of the conduit.

In some configurations, the at least one value indicative of the amount of moisture in the conduit may correspond to a change in inductance of the conduit.

A humidifier system may be used in a gases supply system. The humidifier system may include a humidifier to heat and humidify the gases, a conduit to transport the gases from the humidifier to the user. The conduit may include a first electrically conductive element, a second electrically conductive element, and a controller configured to monitor capacitance and/or capacitance change of a capacitor formed between the first electrically conductive element and the second electrically conductive element to detect presence of skin in close vicinity or contact with the conduit.

In some configurations, the capacitance and/or capacitance change includes measuring a time constant, a resonant frequency, a change in a time constant, or a change in a resonant frequency of the capacitor formed between the first electrically conductive element and the second electrically conductive element.

In some configurations, the controller may detect a change in permittivity of the capacitor based on the monitored capacitance and/or capacitance change.

In some configurations, the controller may compare the detected change in permittivity with one or more permittivity thresholds.

In some configurations, the controller may detect the presence of skin in close vicinity or contact with the conduit in response to the detected change in permittivity exceeding the one or more permittivity thresholds.

In some configurations, the controller may output an alarm in response to detecting the presence of skin in close vicinity or contact with the conduit.

In some configurations, the controller may output the alarm in response to detecting the presence of skin in close vicinity or contact with the conduit continuously or intermittently for a predefined duration.

In some configurations, the predefined duration may be variable based on an expected conduit surface temperature.

In some configurations, the conduit may include a dielectric material between the first electrically conductive element and the second electrically conductive element.

In some configurations, the first electrically conductive element and the second electrically conductive element are spirally wound about at least a length of the conduit.

In some configurations, the first electrically conductive element and the second electrically conductive element are spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be a heater wire.

In some configurations, the second electrically conductive element may be a sensing wire.

In some configurations, the second electrically conductive element may be a heater wire.

In some configurations, the first electrically conductive element and the second electrically conductive element may be separated by a distance to allow for a capacitive charge to be sensed between the first electrically conductive element and the second electrically conductive element.

In some configurations, the first electrically conductive element or the second electrically conductive element may include at least two portions that are electrically disconnected from one another.

In some configurations, the at least two portions may be series with one another.

In some configurations, the at least two portions may be in parallel with one another.

A method of delivering a flow of gases to a user according to the present disclosure may use a controller of a humidifier that may be part of a humidifier system. The humidifier may provide a humidifier that may heat and humidify the gases, a conduit to transport the gases from the humidifier to the user, at least one sensor that may output at least one sensor signal based on an amount of moisture present in a component of the system, and a controller to control operation of the humidifier. The method include determining at least one value indicative of the amount of moisture present in the component based at least in part on the at least one sensor signal and executing one or more moisture management responses based on the at least one value indicative of the amount of moisture present in the component.

In some configurations, the one or more moisture management may include one or more of the following: generating at least one notification and/or alarm, changing power supplied to a humidifier heater, changing power suppled to at least one conduit heater, changing a mode of the humidifier, and/or changing a parameter of a ventilator which may be part of the humidification system.

In some configurations, the component may include the conduit, a portion of the conduit, a connector, a portion of a chamber that may be exposed to the flow of gases, and/or a patient interface.

In some configurations, the moisture present in the component of the system may include condensate, and/or water in a vapor permeable and/or liquid permeable material of the component of the system.

In some configurations, the vapor permeable material may be a breathable material.

In some configurations, the at least one value indicative of the amount of moisture present in the component may include at least one of a value indicative of the amount of condensate in the component of the system, and/or a value indicative of the amount of water in the vapor permeable and/or liquid permeable material of the component of the system. The value indicative of the amount of moisture present in the component may be a sum of the value indicative of the amount of condensate in the component of the system and the value indicative of the amount of water in the vapor permeable and/or liquid permeable material of the component of the system.

In some configurations, the method may further include determining at least one value indicative of the amount of moisture present in the component based on a humidity output of the humidifier.

In some configurations, the method may further include determining that a predetermined level of moisture may be present in the component based on the at least one value indicative of the amount of moisture present in the component.

In some configurations, the method may further include determining that the predetermined level of moisture may be present in the component based on comparing the at least one value indicative of the amount of moisture present in the component and at least one threshold.

In some configurations, the predetermined level of moisture may be determined to be present in response to the at least one the at least one value being above the at least one threshold.

In some configurations, the predetermined level of moisture may be determined to be present in response to the at least one value being below the at least one threshold.

In some configurations, the at least one threshold mat include an absolute value threshold, a value change threshold, a percentage threshold of a maximum sensor output, a percentage change threshold, a percentage change over time threshold, a gradient threshold, and/or a crossing threshold.

In some configurations, the maximum sensor output may provide an output indicating that the vapor and/or liquid material of the conduit may be saturated with moisture.

In some configurations, the maximum sensor output may provide an output indicating that the predetermined or acceptable amount of moisture may be present.

In some configurations, the percentage threshold may be 80%.

In some configurations, the percentage threshold may be 90%

In some configurations, the percentage threshold may be 100%.

In some configurations, the crossing threshold may occur when the output crosses a particular threshold a number of times. This may optionally be over a predetermined time period.

In some configurations, the method may further include generating at least one notification and/or alarm in response to the predetermined level of moisture determined to be present in the component.

In some configurations, the method may further include determining the presence of condensate in the component based on a comparison between the at least one value indicative amount of moisture present in the component and at least one condensate threshold.

In some configurations, the method may further include determining the presence of a predetermined amount of water in a vapor permeable and/or liquid permeable material of the component of the system based on a comparison between the at least one value indicative of the amount of moisture present in the component and at least one water amount threshold. The predetermined amount of water may optionally be indicative of saturation of the vapor and/or liquid permeable material.

In some configurations, the method may further include controlling the heater of the humidifier and/or the heater of the conduit to achieve a humidity target.

In some configurations, the humidity target may include a dew point target, an absolute target, and/or a relative humidity target.

In some configurations, the method may further include decreasing humidity target, and optionally the absolute humidity and/or relative humidity target, of the flow of gases in response to a predetermined amount of moisture being determined to be present in the component.

In some configurations, the humidifier may include a heater configured to heat the humidification liquid.

In some configurations, the heater may include a heater plate.

In some configurations, the conduit may include at least one heater wire to heat the flow of gases in the conduit.

In some configurations, the method may further include decreasing the absolute humidity of the flow of gases by changing at least one operating parameter of the heater of the humidifier.

In some configurations, the method may further include decreasing the relative humidity of the flow of gases by changing the at least one operating parameter of the heater of the humidifier.

In some configurations, the changing includes decreasing or limiting or disabling the relative humidity of the flow of gases.

In some configurations, the at least one operating parameter may include a heater temperature set point and/or a power to a heater.

In some configurations, the method may further include decreasing the relative humidity target of the flow of gases by changing at least one operating parameter of the heater of the conduit. In some configurations, the changing may include increasing the operating parameter.

In some configurations, the operating parameter of the at least one heater of the conduit may be a patient end temperature set point and/or a power to the at least one heater of the conduit.

In some configurations, the operating parameter of the heater of the humidifier and/or the heater of the conduit may be changed based on the at least one value indicative of the amount of moisture present in the component.

In some configurations, the method may further include decreasing an absolute humidity target, and/or a relative humidity target in response to determining that the predetermined level of moisture may be still present in the component a predetermined time period after initially determining that the predetermined level of moisture may be present.

In some configurations, the method may further include returning to a normal operation, and/or revert to an original absolute humidity and/or relative humidity target of the flow of gases, in response to determining that the predetermined level of moisture may be no longer present in the component after initially determining that the predetermined level of moisture may be present.

In some configurations, the method may further include returning to normal operation, and/or revert to an original absolute humidity and/or relative humidity target of the flow of gases, in response to determining that the predetermined level of moisture may be no longer present in the component after initially determining that the predetermined level of moisture may be present.

In some configurations, the method may further include determining that the predetermined level of moisture may be no longer present in the component in response to detecting an absence of moisture or a small amount of moisture present in the component.

In some configurations, the method may further include determining that the predetermined level of moisture may be no longer present in the component in response to detecting an absence of condensate or a small amount of condensate present in the component.

In some configurations, at least a portion of the conduit or another component of the system may include a vapor permeable and/or liquid permeable material.

In some configurations, the conduit may include at least a first electrically conductive element and a second electrically conductive element, the at least one sensor signal comprising a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element are spirally wound within, through, or around the conduit.

In some configurations, the first electrically conductive element and the second electrically conductive element for part of conduit walls.

In some configurations, the first electrically conductive element may be a sensing wire

In some configurations, the first electrically conductive element may be at least one heater wire.

In some configurations, the second electrically conductive element may be at least one heater wire.

In some configurations, the vapor permeable and/or liquid permeable material may be a dielectric material.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the other component may include a patient interface.

In some configurations, the other component may include connectors and/or adaptors.

In some configurations, the conduit may include a composite conduit.

In some configurations, the conduit may include a vapor and/or liquid permeable bead.

In some configurations, the vapor permeable bead allows evaporation of water to ambient air while inhibiting passage of liquid water and breathing gases to ambient air.

In some configurations, the vapor permeable bead may be an activated perfluorinated polymer material having extreme hydrophilic properties, hydrophilic thermoplastic, breathable thermoplastic copolyester, woven treated fabric exhibiting breathable characteristics, and/or a hydrophilic polyester block copolymer.

In some configurations, the signal may be indicative of a capacitance between the first electrically conductive element and the second electrically conductive element.

In some configurations, the signal may be indicative of a change in capacitance between the first electrically conductive element and the second electrically conductive element.

In some configurations, the first electrically conductive element and the second electrically conductive element are separated by a distance configured to allow for a capacitive charge to be sensed between the first electrically conductive element and the second electrically conductive element.

In some configurations, the humidifier system may further include a dielectric material located between the first electrically conductive element and the second electrically conductive element.

In some configurations, the method may further include determining the at least one value indicative of moisture based on a comparison of a measurement of the first electrically conductive element and/or the second electrically conductive element.

In some configurations, the signal may be indicative of a temperature of the first electrically conductive element or the second electrically conductive element.

In some configurations, the signal may be indicative of a change in temperature of the first electrically conductive element or the second electrically conductive element.

In some configurations, the signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the signal may be indicative of a resistance of the first electrically conductive element or the second electrically conductive element.

In some configurations, the first electrically conductive element or the second electrically conductive element comprise at least two portions that are electrically disconnected from one another.

In some configurations, the two portions may be in series with one another.

In some configurations, the humidifier system may further include a signal generator wherein the controller may determine that at least one value indicative of the amount of moisture in the conduit based at least in part on a magnitude and/or phase of the at least one signal.

In some configurations, the at least one value indicative of the amount of moisture in the conduit may correspond to an inductance of the conduit.

In some configurations, the at least one value indicative of the amount of moisture in the conduit corresponds to a change in inductance of the conduit.

In some configurations, the predetermined level of moisture present in the component may be indicative of a predetermined amount of water in a vapor permeable and/or liquid permeable material of the component of the system.

In some configurations, the method may further include initially changing an operating parameter of the at least one heater wire of the conduit in response to the predetermined level of moisture being determined to be present in the component. In some configurations, the changing may include decreasing, limiting, or disabling the operating parameter. In some configurations, the decreasing or limiting includes decreasing or limiting power to the at least one heater wire to a range of 3.0W/m to 7.0W/m.

In some configurations, the method may further include maintaining the initially changed operating parameter of the heater wire of the conduit.

In some configurations, maintaining the initially changed operating parameter of the at least one heater wire of the conduit may increase a moisture saturation of the vapor permeable and/or liquid permeable material. This change may optionally be an increase to a predetermined moisture saturation.

In some configurations, the method may further include changing the operating parameter of the at least one heater wire of the conduit.

In some configurations, the method may further include changing the operating parameter of the heater wire of the conduit a predetermined time after having maintained the initially changed operating parameter.

In some configurations, the further change may be an increase of the operating parameter.

In some configurations, the method may further include changing an operating parameter of the heater of the conduit in response to the predetermined level of moisture being determined to be present in the component.

In some configurations, the predetermined level may be a percentage threshold of a maximum sensor output. In some configurations, the percentage threshold may be 80%. In some configurations, the percentage threshold may be 90%. In some configurations, the percentage threshold may be 100%. A fully saturated permeable material may be at 100%.

In some configurations, the maximum sensor output may be an output indicating that a bead of the conduit may be saturated with moisture.

In some configurations, the maximum sensor output may be an output indicating that the predetermined or acceptable amount of moisture may be present.

In some configurations, the method may further include maintaining the changed operating parameter of the at least one heater wire of the conduit to allow for drying of the vapor permeable and/or liquid permeable material via evaporation to the atmosphere.

In some configurations, the method may further include maintaining the changed operating parameter increase a rate at which the moisture may be transferred across the vapor permeable material to the atmosphere.

In some configurations, the changed operating parameter may include an increased power to between 10W/m and 14W/m to the at least one heater wire.

In some configurations, the method may further include returning to a normal or original operating parameter of the at least one heater of the conduit a predetermined time after having maintained the changed operating parameter.

In some configurations, the method may further include repeating increasing and maintain the operating parameter and then decreasing and maintaining the operating parameter a number of times before returning to the normal or original operating parameter.

In some configurations, the method may further include returning to a normal or original operating parameter of the at least one heater of the conduit in response to the predetermined level of moisture in the component being determined to fall below a threshold. In some configurations, the threshold may be a percentage threshold. In some configurations, the percentage threshold may be 20%. In some configurations, the percentage threshold may be 15%. In some configurations, the percentage threshold may be 0%.

In some configurations, the method may further include returning to the normal or original operating parameter of the at least one heater wire of the conduit in response to the predetermined quantity of water molecules in the vapor permeable and/or liquid permeable material being determined to fall below a water molecule threshold.

In some configurations, the method may further include returning to the normal or original operating parameter of the at least one heater wire of the conduit in response to the predetermined level of condensate in the component being determined to fall below a condensate threshold.

In some configurations, the operating parameter of the at least one heater wire of the conduit may include a patient end temperature set point, and/or a power to the at least one heater wire.

In some configurations, the method may further include increasing a temperature of the vapor permeable material via a heater of the conduit to increases a rate at which water passes from one side of the material to an opposite side of the material.

In some configurations, the method may further include increasing a temperature of the liquid permeable material via a heater of the conduit to increases an evaporation rate of liquid water in the liquid permeable material to atmosphere.

In some configurations, the method may further include controlling a relative humidity gradient between the vapor permeable and/or liquid permeable material and the atmosphere to be greater than a relative humidity gradient between the vapor permeable and/or liquid permeable material and a lumen of the conduit.

In some configurations, the humidifier system may include a moisture draining assembly configured to collect condensate and/or transport the condensate back towards the humidification chamber.

In some configurations, the moisture draining assembly may include a water trap configured to collect the condensate.

In some configurations, the moisture draining assembly may include at least one valve electronically coupled to the controller. The method may further include actuating the at least one valve based on the at least one sensor signal.

In some configurations, the method may further include actuating the at least one valve into an open position in response to a predetermined level of moisture being determined to be present in the component.

In some configurations the method may further include actuating the at least one valve into an open position in response to a predetermined level of condensate being determined to be present in the component.

In some configurations, the moisture draining assembly may include a moisture conveying assembly configured to convey moisture collected in the water trap or the conduit back to the humidification chamber.

In some configurations, the moisture draining assembly may further include a conveying conduit, and/or a pump. In some configurations, the pump may be a peristaltic pump.

In some configurations, the method may further include determining a chamber overflow event based on the at least one value indicative of the amount of moisture present in the component.

In some configurations, the component may be located near an outlet of a humidification chamber of the humidifier.

In some configurations, the sensor may include a temperature sensor located near a distal end of the conduit.

In some configurations, the method may further include determining a dew point temperature or a value indicative thereof of the flow of gases based on a temperature of the flow of gases sensed by the temperature sensor in response to determining the presence of a predetermined amount of moisture.

In some configurations, the method may further include determining the dew point temperature or a value indicative thereof based on the temperature of the flow of gases sensed by the temperature sensor in response to determining the presence of a predetermined amount of condensate.

In some configurations, the dew point temperature or a value indicative thereof may be the temperature of the flow of gases sensed by the temperature sensor.

In some configurations, the method may further include controlling a heater of the humidifier or a heater of the conduit based on the determined dew point temperature or a value indicative thereof.

In some configurations, the method may further include decreasing an operating parameter of the heater of the conduit to increase a relative humidity in the conduit and induce formation of condensate.

In some configurations, the method may further include controlling the gases exiting the humidifier to reach a dew point temperature set point.

In some configurations, the method may further include determining that a source of the gases for the humidifier may be the ambient air or from a room-entraining ventilator in response to determining that a patient end temperature may be greater than the dew point temperature set point and that a predetermined level of moisture may be present.

In some configurations, the method may further include controlling a temperature set point of the humidifier to a constant value after determining that a source of the gases for the humidifier is the ambient air or from a room-entraining ventilator.

In some configurations, the temperature set point can be a chamber outlet set point.

In some configurations, the chamber outlet set point may correspond to the dew point of the gases.

In some configurations, the method may further include determining the presence of a predetermined level of moisture in the conduit based on the at least one value indicative of the amount of moisture in the conduit in response to the conduit being connected to a humidifier and/or prior to operation of the humidifier.

In some configurations, the method may further include determining the presence of the predetermined level of moisture in the conduit based on a comparison between a threshold and the at least one value indicative of the amount of moisture in the conduit.

In some configurations, the method may further include determining the presence of the predetermined level of moisture in the conduit when the conduit may be first connected to the humidifier.

In some configurations, the predetermined level of moisture present in the conduit may be a level for which a new unused conduit may be needed or the conduit needs to be checked for faults or dried further.

In some configurations, the method may further include generating an alarm in response to determining the presence of the predetermined level of moisture in the conduit.

In some configurations, the alarm can include a visual and/or audio indication.

In some configurations, the method may further include preventing use of the conduit with the humidifier in response to determining the presence of the predetermined level of moisture in the conduit by preventing powering of the heater of the conduit and/or preventing use of the humidifier while connected to the conduit.

In some configurations, the conduit may not include a patient end sensor.

In some configurations, the at least one sensor signal based on the amount of moisture present in the component may further include a signal relating to a humidity of the gases in the component.

In some configurations, the signal relating to the humidifier of the gases in the component may be filtered from the at least one signal or ignored by the controller.

In some configurations, the method may further include determining at least one value indicative of the amount of moisture present in the component based on the at least one sensor signal and control a function of the gases source to reduce moisture in response to a predetermined amount of moisture determined to be present in the component based on the value.

In some configurations, the function may include reducing a tidal volume, increasing an inspiratory rise time, reducing inspiratory to expiratory ratio, increasing an inlet gases temperature, and/or reducing an amount of entrained air and/or switching to a wall or bottle gases source.

In some configurations, the gases source may include a ventilator.

In some configurations, the gases source may include a glow generator.

In some configurations, the method may further include determining at least one value indicative of the amount of moisture present in the component based on the at least one sensor signal, and determine a water-out condition of the humidification chamber based on the at least one value indicative of the amount of moisture present in the component.

In some configurations, the water-out condition may be determined in response to detecting an increased amount of moisture and/or humidity in the component.

In some configurations, the water-out condition may be determined in response to the increase in the amount of moisture and/or humidity in the component being above a threshold.

In some configurations, the water-out condition may include no water present or a predetermined level of water present.

In some configurations, the method may further include increasing power to a heater plate of the humidifier and/or reduce power to at least one heater wire of a conduit configured to provide the gases from the humidifier to the user so as to increase humidity and/or increase moisture.

In some configurations, the increased moisture may be condensate or water in vapor permeable and/or liquid permeable material.

In some configurations, the method may further include determining the water-out condition subsequent to increasing the power to the heater plate and/or reducing the power to the at least one heater wire in response to the predetermined amount of moisture and/or humidity being determined to be below a threshold.

A method of delivering a flow of gases to a user according to the present disclosure may use a controller of a humidifier that may be part of a humidifier system. The humidifier system may include the humidifier to heat and humidify the gases, at least one sensor to output at least one sensor signal based on an amount of moisture and/or humidity present in a component of the system, and a controller to controller operation of the humidifier. The method may include determining at least one value indicative of the amount of moisture and/or humidity present in the component based on the at least one sensor signal, and determining a humidity of the flow of gases based on the at least one value indicative of the amount of moisture and/or humidity present in the component.

In some configurations, the method may further include assuming 100% relative humidity upon detection of presence of moisture based on the value.

In some configurations, the temperature sensor located near a distal end of a conduit may transport the gases from the humidifier to the user. The method may further include determining the presence of a predetermined level of moisture based on the at least one value indicative of the amount of moisture and/or humidity present in the component. The method may further include determining a dew point of the flow of gases, as the humidity of the flow of gases.

In some configurations, the humidity may be a dew point temperature, a relative humidity value, or an absolute humidity value.

In some configurations, the controller may control a heater of the humidifier and/or conduit based on the humidity to control a target humidity.

In some configurations, the method may further include determining the presence of a predetermined level of moisture based on the at least one value indicative of the amount of moisture and/or humidity present in the component.

In some configurations, the method may further include controlling at least one operating parameter of the humidifier system based on the determined dew point temperature or a value indicative thereof.

In some configurations, the method may further include controlling a heater of the humidifier and a heater of the conduit based on the determined dew point temperature or a value indicative thereof.

In some configurations, the operating parameter of the heater of the humidifier may include at least one of a heater plate temperature set point, and a power to a heater plate.

In some configurations, the operating parameter of the heater of the conduit may include at least one of a patient end temperature set point, and a power to at least one heater wire.

In some configurations, the determined dew point temperature or a value indicative thereof may be the temperature of the flow of gases sensed by the temperature sensor.

In some configurations, the method may further include decreasing an operating parameter of a heater of the conduit to increase a relative humidity in the conduit and induce the formation of condensate.

In some configurations, the method may further include controlling the heater of the conduit to reach a patient end temperature set point.

In some configurations, the target patient end temperature may be greater than the determined dew point temperature or a value indicative thereof.

In some configurations, the determined humidity of the flow of gases may be a relative humidity or an absolute humidity.

In some configurations, an output of the at least one sensor may be filtered to obtain a portion of the at least one signal that may be related to the humidity in the component.

In some configurations, the humidifier system may include a flow generator.

In some configurations, the flow generator and the humidifier may be in a single housing.

In some configurations, the conduit may include at least a first electrically conductive element and a second electrically conductive element. The at least one sensor signal may include a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element may be spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be at least one heater wire.

In some configurations, the second electrically conductive element may be a sensing wire.

In some configurations, the second electrically conductive element may be at least one heater wire.

In some configurations, the at least one signal may be indicative of a capacitance between the first electrically conductive element and the second electrically conductive element.

In some configurations, the at least one signal may be indicative of a change in capacitance between the first electrically conductive element and the second electrically conductive element.

In some configurations, the first electrically conductive element and the second electrically conductive element may be separated by a distance that may allow for a capacitive charge to be sensed between the first electrically conductive element and the second electrically conductive element.

In some configurations, the humidifier system may include a dielectric material located between the first electrically conductive element and the second electrically conductive element.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the method may further include determining the at least one value indicative of moisture based on a comparison of a measurement of the first electrically conductive element and/or the second electrically conductive element.

In some configurations, the at least one signal may be indicative of a temperature of the first electrically conductive element or the second electrically conductive element.

In some configurations, the at least one signal may be indicative of a change in temperature of the first electrically conductive element or the second electrically conductive element.

In some configurations, the at least one signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the at least one signal may be indicative of a resistance of the first electrically conductive element or the second electrically conductive element.

In some configurations, the first electrically conductive element or the second electrically conductive element may include at least two portions that are electrically disconnected from one another.

In some configurations, the at least two portions may be in series with one another.

In some configurations, the at least two portions may be in parallel with one another.

In some configurations, the humidifier system may further include a signal generator. The method may further include determining the at least one value indicative of the amount of moisture in the conduit based at least in part on a magnitude and/or phase of the at least one signal.

In some configurations, the at least one value indicative of the amount of moisture in the conduit corresponds to an inductance of the conduit.

In some configurations, the at least one value indicative of the amount of moisture in the conduit may correspond to a change in inductance of the conduit.

A method of delivering a flow of gases to a user according to the present disclosure may use a controller of a humidifier that may be part of a humidifier system. The humidifier system may include the humidifier to heat and humidify the gases, a first sensor associated with a first location, a second sensor associated with a second location, and a controller to control operation of the humidifier. The first sensor may output at least one first sensor signal based on an amount of moisture present at the first location. The second sensor may output at least one second sensor signal based on an amount of moisture present at the second location. The method may include determining a first value indicative of an amount of humidity and/or moisture present at the first location based on a first sensor signal, and a second value indicative of the amount of humidity and/or moisture present at the second location based on a second sensor.

In some configurations, the first location may be associated with a first component of the system.

In some configurations, the second location may be associated with a second component of the system.

In some configurations, the first and/or second component may include a conduit, a portion of the conduit, a connector, a portion of a chamber that may be exposed to the flow of gases, and/or a patient interface.

In some configurations, the connector may include one or more adapters.

In some configurations, the first location may be upstream of the second location during normal operation of the system.

In some configurations, the method may further include determining that no flow and/or a flow rate below a threshold may be occurring in the system by detecting a first predetermined amount of moisture at the first location, and a predetermined time period after detecting the predetermined amount of moisture at the first location, determining that a second predetermined amount of moisture may be not detected at a second location.

In some configurations, the method may further include determining that a flow rate of the gases may be in a range based on readings from a first temperature sensor at the first location and a second temperature sensor at the second location in response to detecting the first predetermined amount of moisture at the first location and not detecting the second predetermined amount of moisture at the second location a predetermined time period after detecting the predetermined amount of moisture at the first location.

In some configurations, the first temperature sensor may be located at a chamber outlet of the humidifier.

In some configurations, the second temperatures sensor may be located at or near a patient end of an inspiratory conduit or a distal end of a patient supply conduit configured to transport the gases from the humidifier to the user.

In some configurations, the method may further include determining that the flow direction may be correct in response to the moisture at an upstream location being greater than the moisture at a downstream location.

In some configurations, an inspiratory conduit may include the upstream location.

In some configurations, the conduit positioned between the gases source and the humidifier may include the downstream location.

In some configurations, the humidifier system may include a third sensor associated with a third location of the system. The third sensor may output at least one third sensor signal based on an amount of moisture present at the third location.

In some configurations, the third location may be upstream of the first location and the second location.

In some configurations, the first location may be at an inspiratory conduit.

In some configurations, the third location may be at an expiratory conduit.

In some configurations, the second location may be at a chamber inlet of a humidification chamber.

In some configurations, the method may further include determining a flow direction based on a direction of the moisture detected from the humidifier. The method may further include detecting a reverse flow condition in response to moisture being detected in a component upstream of the humidifier when correctly connected.

A method of delivering a flow of gases to a user according to the present disclosure may use a controller of a humidifier that may be part of a humidifier system. The humidifier may provide a humidifier that may heat and humidify the gases, a conduit to transport the gases from the humidifier to the user, at least one sensor that may output at least one sensor signal based on an amount of moisture present in a component of the system, and a controller to control operation of the humidifier. The method may include determining a value indicative of the amount of moisture present in the component based on the at least one sensor signal, and controlling a function of the gases source to reduce moisture in response to a predetermined amount of moisture determined to be present in the component based on the value.

In some configurations, the function may include reducing a tidal volume, increasing an inspiratory rise time, reducing inspiratory to expiratory ratio, increasing an inlet gases temperature, and/or reducing an amount of entrained air and/or switching to a wall or bottle gases source.

In some configurations, the gases source may include a ventilator.

In some configurations, the gases source may include a glow generator.

In some configurations, the conduit may include at least a first electrically conductive element and a second electrically conductive element. The at least one sensor signal may include a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element may be spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be at least one heater wire.

In some configurations, the second electrically conductive element may be a sensing wire.

In some configurations, the second electrically conductive element may be at least one heater wire.

In some configurations, the at least one signal may be indicative of a capacitance between the first electrically conductive element and the second electrically conductive element.

In some configurations, the at least one signal may be indicative of a change in capacitance between the first electrically conductive element and the second electrically conductive element.

In some configurations, the first electrically conductive element and the second electrically conductive element may be separated by a distance that may allow for a capacitive charge to be sensed between the first electrically conductive element and the second electrically conductive element.

In some configurations, the humidifier system may include a dielectric material located between the first electrically conductive element and the second electrically conductive element.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the method may further include determining the at least one value indicative of moisture based on a comparison of a measurement of the first electrically conductive element and/or the second electrically conductive element.

In some configurations, the at least one signal may be indicative of a temperature of the first electrically conductive element or the second electrically conductive element.

In some configurations, the at least one signal may be indicative of a change in temperature of the first electrically conductive element or the second electrically conductive element.

In some configurations, the at least one signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the at least one signal may be indicative of a resistance of the first electrically conductive element or the second electrically conductive element.

In some configurations, the first electrically conductive element or the second electrically conductive element may include at least two portions that are electrically disconnected from one another.

In some configurations, the at least two portions may be in series with one another.

In some configurations, the at least two portions may be in parallel with one another.

In some configurations, the humidifier system may further include a signal generator. The method may further include determining the at least one value indicative of the amount of moisture in the conduit based at least in part on a magnitude and/or phase of the at least one signal.

In some configurations, the at least one value indicative of the amount of moisture in the conduit corresponds to an inductance of the conduit.

In some configurations, the at least one value indicative of the amount of moisture in the conduit may correspond to a change in inductance of the conduit.

A method of delivering a flow of gases to a user according to the present disclosure may use a controller of a humidifier that may be part of a humidifier system. The humidifier may provide a humidifier that may heat and humidify the gases, a conduit to transport the gases from the humidifier to the user, at least one sensor that may output at least one sensor signal based on an amount of moisture present in a component of the system, and a controller to control operation of the humidifier. The method may include determining at least one value indicative of the amount of moisture present in the component based on the at least one sensor signal, and determining a water-out condition of the humidification chamber based on the at least one value indicative of the amount of moisture present in the component.

In some configurations, the water-out condition may be determined in response to detecting an increased amount of moisture and/or humidity in the component.

In some configurations, the water-out condition may be determined in response to the increase in the amount of moisture and/or humidity in the component being above a threshold.

In some configurations, the water-out condition comprises no water present or a predetermined level of water present.

In some configurations, the method may further include increasing power to a heater plate of the humidifier and/or reduce power to at least one heater wire of a conduit configured to provide the gases from the humidifier to the user so as to increase humidity and/or increase moisture.

In some configurations, the increased moisture may be condensate or water in vapor permeable and/or liquid permeable material.

In some configurations, the method may further include determining the water-out condition subsequent to increasing the power to the heater plate and/or reducing the power to the at least one heater wire in response to the predetermined amount of moisture and/or humidity being determined to be below a threshold.

In some configurations, the conduit may include at least a first electrically conductive element and a second electrically conductive element. The at least one sensor signal may include a signal generated using one or more of the at least first and second electrically conductive elements.

In some configurations, the first electrically conductive element and the second electrically conductive element may be spirally wound about at least a length of the conduit.

In some configurations, the first electrically conductive element and the second electrically conductive element may be spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be at least one heater wire.

In some configurations, the second electrically conductive element may be a sensing wire.

In some configurations, the second electrically conductive element may be at least one heater wire.

In some configurations, the at least one signal may be indicative of a capacitance between the first electrically conductive element and the second electrically conductive element.

In some configurations, the at least one signal may be indicative of a change in capacitance between the first electrically conductive element and the second electrically conductive element.

In some configurations, the first electrically conductive element and the second electrically conductive element may be separated by a distance that may allow for a capacitive charge to be sensed between the first electrically conductive element and the second electrically conductive element.

In some configurations, the humidifier system may include a dielectric material located between the first electrically conductive element and the second electrically conductive element.

In some configurations, the dielectric material may be vapor or liquid permeable.

In some configurations, the vapor permeable dielectric material may allow for evaporation of water to ambient air while inhibiting passage of liquid water and breathing gases to ambient air.

In some configurations, the method may further include determining the at least one value indicative of moisture based on a comparison of a measurement of the first electrically conductive element and/or the second electrically conductive element.

In some configurations, the at least one signal may be indicative of a temperature of the first electrically conductive element or the second electrically conductive element.

In some configurations, the at least one signal may be indicative of a change in temperature of the first electrically conductive element or the second electrically conductive element.

In some configurations, the at least one signal may be indicative of a thermal conductivity of a medium between the first electrically conductive element and the second electrically conductive element, or the at least one signal may be indicative of a thermal conductivity of a medium proximal to the first electrically conductive element or the second electrically conductive element.

In some configurations, the at least one signal may be indicative of a resistance of the first electrically conductive element or the second electrically conductive element.

In some configurations, the first electrically conductive element or the second electrically conductive element may include at least two portions that are electrically disconnected from one another.

In some configurations, the at least two portions may be in series with one another.

In some configurations, the at least two portions may be in parallel with one another.

In some configurations, the humidifier system may further include a signal generator. The method may further include determining the at least one value indicative of the amount of moisture in the conduit based at least in part on a magnitude and/or phase of the at least one signal.

In some configurations, the at least one value indicative of the amount of moisture in the conduit corresponds to an inductance of the conduit.

In some configurations, the at least one value indicative of the amount of moisture in the conduit may correspond to a change in inductance of the conduit.

A method of delivering a flow of gases to a user according to the present disclosure may use a controller of a humidifier that may be part of a humidifier system. The humidifier system may include a humidifier to heat and humidify the gases, a conduit to transport the gases from the humidifier to the user. The conduit may include a first electrically conductive element, a second electrically conductive element. The method may include monitoring capacitance and/or capacitance change of a capacitor formed between the first electrically conductive element and the second electrically conductive element to detect presence of skin in close vicinity or contact with the conduit.

In some configurations, the capacitance and/or capacitance change includes measuring a time constant, a resonant frequency, a change in a time constant, or a change in a resonant frequency of the capacitor formed between the first electrically conductive element and the second electrically conductive element.

In some configurations, the method may further include detecting a change in permittivity of the capacitor based on the monitored capacitance and/or capacitance change.

In some configurations, the method may further include comparing the detected change in permittivity with one or more permittivity thresholds.

In some configurations, the method may further include detecting the presence of skin in close vicinity or contact with the conduit in response to the detected change in permittivity exceeding the one or more permittivity thresholds.

In some configurations, the method may further include outputting an alarm in response to detecting the presence of skin in close vicinity or contact with the conduit.

In some configurations, the method may further include outputting the alarm in response to detecting the presence of skin in close vicinity or contact with the conduit continuously or intermittently for a predefined duration.

In some configurations, the predefined duration may be variable based on an expected conduit surface temperature.

In some configurations, the conduit may include a dielectric material between the first electrically conductive element and the second electrically conductive element.

In some configurations, the first electrically conductive element and the second electrically conductive element are spirally wound about at least a length of the conduit.

In some configurations, the first electrically conductive element and the second electrically conductive element are spirally wound within, through, or around the conduit.

In some configurations, the first electrically conductive element and the second electrically conductive element form part of conduit walls.

In some configurations, the first electrically conductive element may be a sensing wire.

In some configurations, the first electrically conductive element may be a heater wire.

In some configurations, the second electrically conductive element may be a sensing wire.

In some configurations, the second electrically conductive element may be a heater wire.

In some configurations, the first electrically conductive element and the second electrically conductive element may be separated by a distance to allow for a capacitive charge to be sensed between the first electrically conductive element and the second electrically conductive element.

In some configurations, the first electrically conductive element or the second electrically conductive element may include at least two portions that are electrically disconnected from one another.

In some configurations, the at least two portions may be series with one another.

In some configurations, the at least two portions may be in parallel with one another.

The present disclosure includes a method of determining a condition of a medical humidifier configured to provide gases to a patient. The method can comprise: reducing power to a heater wire of a breathing tube coupled to the medical humidifier, wherein the medical humidifier can comprise a heater plate and a temperature based flow sensor; measuring a condensate metric; determining whether the measured condensate metric satisfies an expected condensate metric; and outputting the condition of the medical humidifier to a controller of the medical humidifier in response to the measured condensate metric not satisfying the expected condensate metric, wherein outputting the condition of the medical humidifier can comprise disregarding reading from the temperature based flow sensor for control of the medical humidifier.

In some configurations, the method can further include determining whether the measured condensate metric satisfies the expected condensate metric comprises comparing a condensate level in the breathing tube to a predetermined threshold.

In some configurations, the measured condensate metric not satisfying the expected condensate metric can include the condensate level in the breathing tube being greater than the predetermined threshold.

In some configurations, wherein outputting the condition of the medical humidifier can include disabling the temperature based flow sensor.

In some configurations, wherein reducing power to the heater wire can include disabling power to the heater wire.

In some configurations, wherein outputting the condition of the medical humidifier can include implementing an alternative method to determine a value indicative of a flow rate of the gases.

In some configurations, wherein the alternative method can include an algorithmic method that does not require further input from sensors.

In some configurations, wherein outputting the condition of the medical humidifier can include adjusting a setting of the medical humidifier to be displayed on a user interface of the medical humidifier.

In some configurations, wherein adjusting the setting of the medical humidifier can include at least one of: adjusting PID coefficients; increasing a control loop timing; and/or applying a maximum increase in a setpoint of the heater plate.

In some configurations, wherein the condition can be a Heliox mode.

In some configurations, wherein outputting the condition of the medical humidifier can include determining a gases source type.

In some configurations, wherein the power to the heater wire of the breathing tube can be reduced or disabled for a predetermined period of time.

A method of determining a condition of a medical humidifier configured to provide gases to a patient, the method comprising: reducing power to a heater wire of a breathing tube coupled to the medical humidifier, wherein the medical humidifier comprises a heater plate; measuring a condensate metric; determining whether the measured condensate metric satisfies an expected condensate metric; and outputting the condition of the medical humidifier to a controller of the medical humidifier in response to the measured condensate metric not satisfying the expected condensate metric.

In some configurations, wherein reducing the power to the heater wire of the breathing tube can include disabling the power to the heater wire.

In some configurations, wherein the power to the heater wire of the breathing tube can be reduced for a predetermined period of time.

In some configurations, wherein determining whether the measured condensate metric satisfies the expected condensate metric can include comparing a condensate level in the breathing tube to a predetermined threshold.

In some configurations, wherein the measured condensate metric not satisfying the expected condensate metric can include the condensate level in the breathing tube being greater than the predetermined threshold.

In some configurations, wherein the measured condensate metric not satisfying the expected condensate metric can include the condensate level in the breathing tube being less than the predetermined threshold.

In some configurations, wherein the condition can include a reverse flow condition.

In some configurations, wherein the measured condensate metric not satisfying the expected condensate metric can include a condensate level of a dryline connected to the medical humidifier and a gases source is greater than a condensate level of the breathing tube.

In some configurations, wherein outputting the condition can include outputting a reverse flow alarm.

In some configurations, wherein the condition can include the medical humidifier having just started up or is malfunctioning.

In some configurations, wherein the condition of the medical humidifier can include no presence of flow or water out.

In some configurations, the method can include confirming the determination by performing a flow test or a water out test.

In some configurations, the method can include indicating the condition to the patient in an audio or visual message.

In some configurations, wherein the measured condensate metric not satisfying the expected condensate metric can include a duration of time to dry condensation in the breathing tube being greater than a predetermined threshold duration of time.

In some configurations, wherein in response to the duration of time to dry condensation in the breathing tube being greater than a predetermined threshold duration of time, outputting the condition comprising the breathing tube being covered.

In some configurations, wherein measuring a condensate metric can include measuring a first condensate metric in a first section of an inspiratory limb and a second condensate metric in a second section of the inspiratory limb; wherein determining whether the measured condensate metric satisfies an expected condensate metric comprises comparing the first condensate metric of the first section of the inspiratory limb and the second condensate metric of the second section of the inspiratory limb, wherein the condition comprises an incubator being used with the medical humidifier.

In some configurations, wherein the incubator can be determined to be used in response to the first condensate metric of the first section being greater than the second condensate metric of the second section.

In some configurations, wherein the first condensate metric and the second condensate metric can each correspond to a presence of condensation.

In some configurations, wherein the presence of condensation in the first section of the inspiratory limb and no presence of condensation in the second section of the inspiratory limb can be indicative of the incubator being in use.

In some configurations, the method can include decreasing power delivered to the heater wire at the second section of the inspiratory limb when the incubator is used.

In some configurations, the method can include increasing power delivered to the heater wire at the first section of the inspiratory limb when the incubator is used.

In some configurations, wherein the condition can be an orientation of the breathing tube.

In some configurations, wherein the measured condensate metric not satisfying the expected condensate metric can include the measured condensate metric at a patient end of the breathing tube being greater than the measured condensate metric at an outlet of a humidification chamber of the medical humidifier.

In some configurations, wherein the measured condensate metric not satisfying the expected condensate metric can include the measured condensate metric at the patient end being greater than the measured condensate metric at a region between the patient end and the outlet.

A method to determine a condition of a humidifier configured to provide gases to a patient, the method comprising: determining a startup condition; reducing power to a heater plate of a medical humidifier coupled to a breathing tube and comprising a heater plate; determining whether a measured condensate metric satisfies an expected condensate metric; and outputting the condition of the humidifier to a controller of the humidifier based one whether the measured condensate metric not satisfying the expected condensate metric.

In some configurations, wherein the startup condition can include determining a duration of time for which the humidifier has been powered on and determining the duration of time indicating the humidifier has just turned on.

In some configurations, the method can include resuming a state or normal control in response to the measured condensate metric satisfies the expected condensate metric.

In some configurations, wherein the condition can include an alarm that the breathing tube is contaminated.

In some configurations, wherein the humidifier may have been just powered on.

In some configurations, the method can include powering off the heater plate and ensuring the heater plate is at a substantially cool temperature.

In some configurations, the method can include raising a circuit moisture alarm when condensate is detected.

A method of controlling power to a heating element of an inspiratory tube coupled to a humidifier of a humidifier system, the method comprising: measuring a condensate metric of the humidifier system; determining whether the measured condensate metric satisfies an expected condensate metric; and controlling power to the heating element based on at least whether the measured condensate metric satisfies the expected condensate metric.

In some configurations, wherein determining whether the measured condensate metric satisfies the expected condensate metric can include detecting whether the measured condensate metric is greater than a predetermined threshold.

In some configurations, the method further can include increasing power to the heating element in response to the measured condensate metric being greater than the predetermined threshold.

In some configurations, the method can include decreasing power to the heating element in response to the measured condensate metric being less than the predetermined threshold.

In some configurations, the method can include determining whether a power to the heating element is at a maximum.

In some configurations, the method can include reducing a setpoint of a heater plate of the humidifier of the humidifier system when the power to the heating element is at the maximum.

In some configurations, wherein reducing a setpoint of the heater plate can be based on determining that the measured condensate satisfies the expected condensate metric.

In some configurations, wherein the inspiratory tube can be sensorless at a patient end.

In some configurations, wherein whether the measured condensate metric satisfies the expected condensate metric can be determined without an input from the sensor located at the patient end.

In some configurations, wherein the humidifier system can be sensorless at an outlet of a humidification chamber of the system.

A method of identifying a tube type of a humidifier system can include determining a capacitance value of a tube and mapping the capacitance value to the tube type.

In some configurations, the method can be performed at start-up of the humidifier system.

Although discussed mainly with respect to respiratory assistance apparatuses and surgical insufflators, it is to be understood that the moisture detection disclosure provided by the present application can also apply to other medical or non-medical uses of a conduit or humidified gases transport system where it is desirable to detect a presence or extent of moisture.

### BRIEF DESCRIPTION OF THE DRAWINGS

These and other features, aspects, and advantages of the present disclosure are described with reference to the drawings of certain implementations, which are intended to schematically illustrate certain implementations and not to limit the disclosure.
Fig. 1A illustrates schematically an example respiratory humidifier system.
Fig. 1B illustrates an example humidifier.
Fig. 1C illustrates an example heater base and cartridge.
Fig. 1D illustrates an example humidifier with the electropneumatic connector disconnected from the humidifier of FIG. 1B.
Fig, 1E illustrates an example heater base and humidification chamber.
Fig. 1F illustrates an example cartridge.
Fig. 1G illustrates the electropneumatic connector of the humidifier of Fig. 1B.
Fig. 2 illustrates schematically an example surgical humidifier system.
Fig. 3A shows a side-plan view of a section of an example composite conduit.
Fig. 3B shows a longitudinal cross-section of a top portion of a tube similar to the example composite conduit of Fig. 3A.
Fig. 3C shows another longitudinal cross-section illustrating a first elongate member in the composite conduit.
Fig. 4A illustrates condensation interaction with a non-permeable bead of a composite conduit.
Fig. 4B illustrates condensation interaction with a permeable bead of a composite conduit.
Fig. 5A illustrates an example modeled circuit system of a condensation detection system using capacitance to detect condensation.
Fig. 5B illustrates an example modeled circuit system of a condensation detection system using capacitance to detect condensation.
Fig. 6 illustrates an example modeled circuit system of a condensation detection system using a time constant or resonance frequency derived from inductance to detect condensation.
Fig. 7A illustrates an example modeled circuit system of a condensation detection system using resistance to detect condensation.
Fig. 7B illustrates an example modeled circuit system of a condensation detection system using resistance to detect condensation.
Fig. 8A illustrates an example modeled circuit system of a condensation detection system using resistance and short-circuiting to detect condensation.
Fig. 8B illustrates an example modeled circuit system of a condensation detection system using resistance and short-circuiting to detect condensation.
Fig. 9A illustrates schematically an example condensation detection system using signal attenuation to detect condensation.
Fig. 9B illustrates a conduit wall structures configured to detect moisture using signal attenuation.
Fig. 10A illustrates an example modeled circuit system of a condensation detection system using signal attenuation to detect condensation.
Fig. 10B illustrates an example modeled circuit system of a condensation detection system using signal attenuation to detect condensation with monopoles.
Fig. 11A illustrates heat radiating from wires in a bead.
Fig. 11B illustrates an example modeled circuit system of a condensation detection system using temperature or thermal conductivity to detect condensation.
Fig. 12 illustrates a table of resistor voltage vs. time constant in a condensation detection system.
Fig. 13A illustrates a flow chart of a condensation detection mode.
Fig. 13B illustrates a flow chart of a condensation measurement mode.
Fig. 13C illustrates a flow chart of a condensation measurement mode using resonant frequency.
Fig. 13D illustrates a flow chart of a condensation measurement mode using signal attenuation.
Fig. 13E illustrates a flow chart of a condensation measurement mode using thermal conductivity.
Fig. 14 illustrates an example bead with various conduit wall structures configured to detect moisture.
Fig. 15 illustrates an example configuration of a bead with an opening.
Fig. 16 illustrates a second example configuration of a bead with an opening.
Fig. 17A illustrates an example configuration cross section of a part of the tube wall.
Fig. 17B illustrates a second example configuration cross section of a part of the tube wall.
Fig. 18 illustrates a portion of the tube wall with parallel elements.
Fig. 19 illustrates a portion of the tube wall where the elements can pivot.
Fig. 20 illustrates an example of a permeable wall portion of a conduit wall.
Fig. 21 illustrates a second example of a permeable wall portion of a conduit wall.
Fig. 22 illustrates an example conduit configuration where elements are in the same plane, parallel to the surface of the exterior conduit wall.
Fig. 23 illustrates a cross section of an example conduit wherein the elements are provided longitudinally, parallel to the lumen, and equidistantly spaced about the circumference of the tube
Fig. 24 illustrates a cross section of an example conduit wherein an additional conductive element wound about the outside of the conduit wall.
Fig. 25 illustrates a cross section of an example conduit wherein individual strands of two meshes can be insulated and multiplexed.
Fig. 26 illustrates an example method of a humidifier controller enacting various moisture management measures.
Fig. 27 illustrates an example method of absolute humidity reduction for moisture management.
Fig. 28 illustrates an example method of relative humidity reduction for moisture management.
Fig. 29 illustrates an example method of moisture management using drying strategies.
Fig. 30 illustrates an example method of automatic condensate draining for moisture management.
Fig. 31A illustrates an example passive method of humidity sensing.
Fig. 31B illustrates an example active method of humidity sensing.
Fig. 32A illustrates an example method of flow-related sensing using moisture detection disclosed herein.
Fig. 32B illustrates an example method of flow-related sensing using moisture detection disclosed herein.
Fig. 32C illustrates an example method of flow-related sensing using moisture detection disclosed herein.
Fig. 32D illustrates an example method of flow-related sensing using moisture detection disclosed herein.
Fig. 33 illustrates an example water-out detection method.
Fig. 34A illustrates an example humidifier system with correct connections.
Fig. 34B illustrates an example humidifier system with correct connections.
Fig. 35A illustrates an example humidifier system with incorrect connections.
Fig. 35B illustrates an example humidifier system with incorrect connections.
Fig. 35C illustrates an example humidifier system with incorrect connections.
Fig. 35D illustrates an example humidifier system with incorrect connections.
Fig. 36 illustrates an example humidifier system that can provide high flow respiratory support.
Fig. 37 illustrates a flowchart of an example process for gas type and room entraining vent detection based on the condensate detection/measurement methods disclosed herein.
Fig. 38A illustrates a flowchart of an example process for no flow/water-out detection based on the condensate detection/measurement methods disclosed herein.
Fig. 38B illustrates a flowchart of an example process for no flow/water out detection based on the condensate detection/measurement methods disclosed herein.
Fig. 38C illustrates a flowchart of an example process of using detection of no humidity for fault detection.
Fig. 39 illustrates a flowchart of an example process for reverse flow detection based on capacitance measurements disclosed herein.
Fig. 40 illustrates a flowchart of an example process for covered tube detection based on the condensate detection/measurement methods disclosed herein.
Fig. 41 illustrates a flowchart of an example process for contaminated tube detection based on the condensate detection/measurement methods disclosed herein.
Fig. 42 illustrates a flowchart of an example process for incubator use detection based on the condensate detection/measurement methods disclosed herein.
Fig. 43 illustrates a flowchart of an example process for tube type detection based on the condensate detection/measurement methods disclosed herein.
Fig. 44 illustrates a flowchart of an example process for tube orientation detection based on the condensate detection/measurement methods disclosed herein.
Fig. 45 illustrates a flowchart of an example process for calculating power to an inspiratory tube heating element power based on the condensate detection/measurement methods disclosed herein.
Fig. 46 illustrates a flowchart of an example process for calculating power to multiple zones of an inspiratory tube heating element based on the condensate detection/measurement methods disclosed herein.
Fig. 47 illustrates an example longitudinal cross section of a bubble tube 4701 in a quadruple helix arrangement.

### DETAILED DESCRIPTION OF THE DISCLOSURE

Although certain implementations and examples are described below, those of skill in the art will appreciate that the disclosure extends beyond the specifically disclosed implementations and/or uses and obvious modifications and equivalents thereof. Thus, it is intended that the scope of the disclosure herein disclosed should not be limited by any particular implementations described below. For example, the dimensions provided in the present disclosure are examples and not limiting. Similarly, although described mainly with respect to respiratory or surgical humidification systems, the present disclosure is applicable to any tubing arrangement where it is desirable to measure moisture. The following examples describe detection of a presence and, optionally, volume and/or location of moisture for example, condensate, water, bodily fluids such as saliva, blood or mucus, or any liquid in the conduit, but the disclosed methods and apparatuses may alternatively or additionally be applied to detect humidity of the gases and/or the presence of other moisture or fluids within the conduit system.

### Example Gases Supply Systems

Fig, 1 schematically illustrates an example respiratory assistance apparatus including a conduit system comprising one or more conduits 103, 117, a patient interface 115 and a Y-piece 113. The respiratory assistance apparatus may be a ventilator, a continuous, variable, or bi-level positive airway pressure (PAP) system or provide another form of respiratory therapy, such as, for example, high flow therapy.

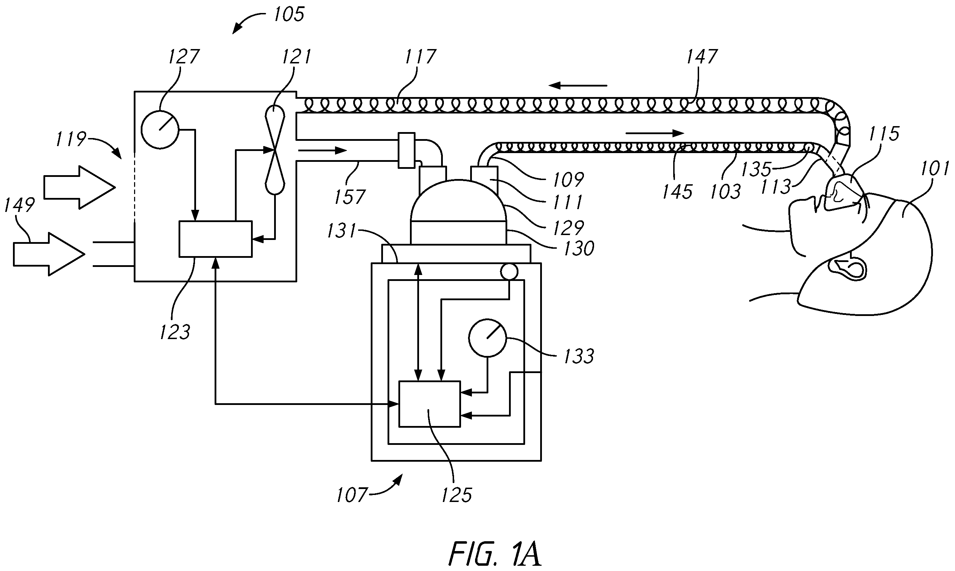

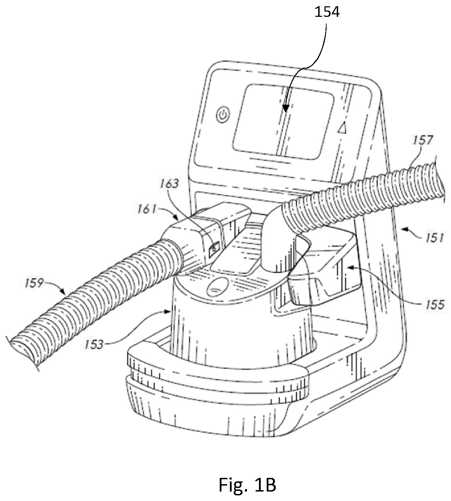

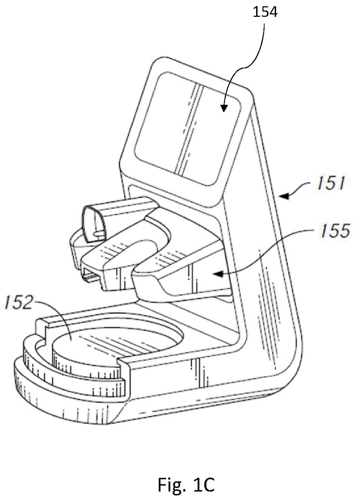

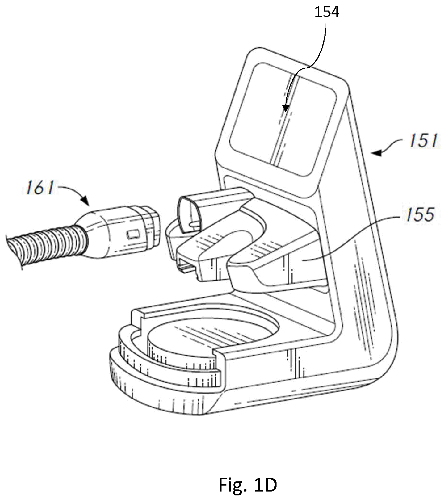

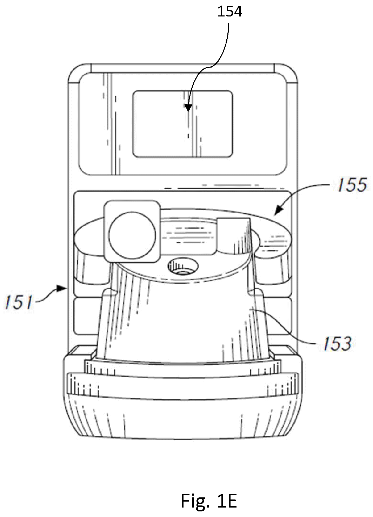

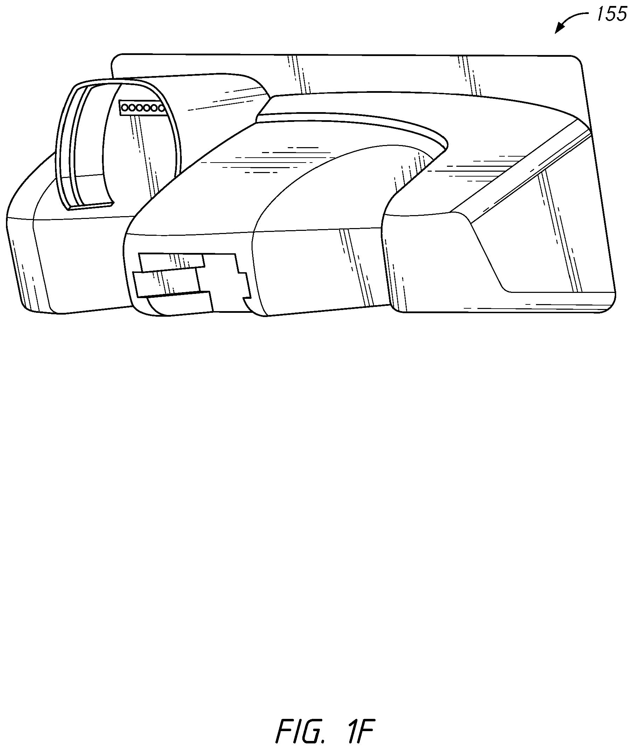

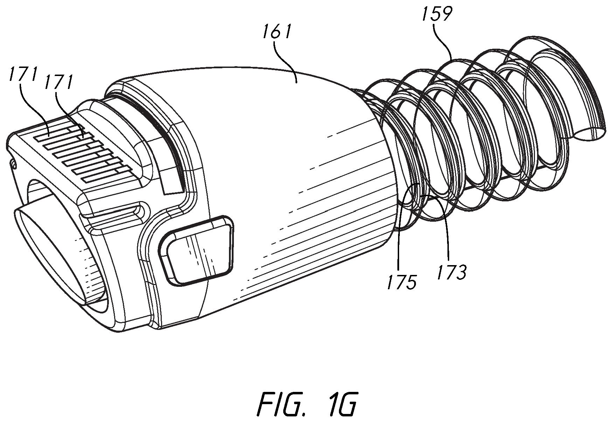

Gases may be transported in the breathing circuit of Fig. 1 as follows: dry or relatively dry gases pass from a gases source 105 through a dry line tube or supply tube 157 to a humidifier 107, which humidifies the dry gases. The gases source 105 may be, for example, a ventilator or a blower. The gases source 105 may be separated from the humidifier 107 or integrated with the humidifier 107 in a single housing.

The humidifier 107 connects to an end 109 of a conduit, such as inspiratory tube 103, via a port 111. The inspiratory tube 103 is connected to a patient 101 through a patient interface 115, optionally using a Y-piece 113. An optional expiratory conduit, such as expiratory tube 117, also connects to the patient interface 115 through the Y-piece 113. The expiratory tube 117 may be configured to move exhaled gases away from the patient 101. As illustrated in Fig. 1, expiratory tube 117 returns exhaled gases from the patient 101 to the gases source 105. Alternatively, the inspiratory tube 103 connects directly to the patient interface 115 without a Y-piece 113. In such an implementation, expired gases are allowed to flow directly to the ambient environment, without requiring an expiratory tube.

Inspiratory tube 103 can include electrically conductive elements such as heater, sensor and/or moisture detection elements 145. Similarly, expiratory tube 117 can include heater, sensor and/or moisture detection elements 147. Further, the Y-piece 113 and patient interface 115 can also include heater, sensor and/or moisture detection elements. As will be explained in further detail below, the heater, sensor and/or moisture detection elements 145, 147 can be wires or filaments.

As shown in the example respiratory assistance apparatus of Fig, 1, dry or relatively dry gases enter the gases source 105 through a vent 119. A fan 121 may improve gas flow into the gases source 105 by drawing air or other gases through the vent 119. The fan 121 may be, for instance, a variable speed fan, where an electronic controller 123 controls the fan speed. The electronic controller 123 may also be controlled by a second electronic controller 125, or vice versa, in some implementations.

The humidifier 107 can include a humidification chamber 129 containing a volume of water 130 or other suitable humidifying liquid. The humidification chamber 129 can be removable from the humidifier 107. The humidification chamber 129 may include a highly heat-conductive base (for example, an aluminum base) contacting or associated with a heater plate 131 on the humidifier 107. The examples in the present disclosure describe a heater plate as a heater of the humidifier and a heater wire as a heater of the tube. It will be appreciated that other heaters of the humidifier are possible (e.g. a heater wire, or other type of heating element) and other heaters of the conduit are possible (e.g. another type of heater element).

The humidifier 107 may also include electronic controls. In Fig. 1, for example, the humidifier 107 includes an electronic, analog, or digital controller 125. The controller 125 may be a microprocessor-based controller executing computer software commands stored in associated memory. In response to humidity, temperature or other feedback values provided via a user interface 133 and/or integrated sensors, the controller 125 determines heat, flow, pressure and/or other variables used to provide humidified gases to a patient (also referred to herein as a user). User interface 133 can be one or more hardware buttons and/or a display or touch screen display. The user interface 133 can provide audio and/or visual feedback to the user. When condensation is detected, any number of alarms, alerts, feedback, guidance or instructions can be provided to the user to indicate the presence, extent or remedies for a condensation condition. For example, the user interface 133 can provide an alarm when condensation is detected. The user interface 133 can provide a visual indication of condensation. The user interface 133 can also provide an animation to instruct a user how to properly drain condensation.

Any suitable patient interface may be used. Patient interface is a broad term and is to be given its ordinary and customary meaning to a person of ordinary skill in the art (that is, it is not to be limited to a special or customized meaning) and includes, without limitation, masks (such as tracheal mask, face masks, and nasal masks), endotracheal tubes, tracheostomy tubes, cannulas, and nasal pillows. A temperature probe 135 may be incorporated in or connected to inspiratory tube 103 near the Y-piece 113, or directly to the Y-piece 113 or the patient interface 115. The temperature probe 135 monitors the temperature of the flow of gases near or at the patient interface 115. A heating wire (such as moisture detection element 145) may be used to adjust the temperature of the patient interface 115, the Y-piece 113, and/or the inspiratory tube 103 to maintain the temperature of the flow of gases above the saturation temperature (that is the dew point temperature of the flow of gases), thereby reducing the opportunity for unwanted condensation, and/or to deliver the gases at optimal temperature for patient therapy (for example, 40°C at the patient end of the inspiratory tube and/or 37°C at the patient for non-invasive therapy). As shown in Fig. 1, exhaled gases are optionally returned from the patient interface 115 to the gases source 105 via the expiratory tube 117.

The system of Fig. 1 may be readily adapted for other applications involving the supply of a heated and/or humidified gas flow to a user or patient, including but not limited to laparoscopy, and the like. Such applications may use alternative gases, operating parameters (e.g., flow, pressure, temperature, or humidity) and patient interfaces. Further, although shown with respect to a separate ventilator and humidifier system, it is to be understood that the present disclosure can also be used with an integrated ventilator/blower and humidifier system.

The system of Fig. 1 can also provide oxygen (O₂) or an O₂ fraction to the user through port 149. The system of Fig. 1 can receive O₂ from a remote source and/or by blending atmospheric air with incoming O₂ from the remote source. The blending of atmospheric air and incoming O₂ can occur via a Venturi or a similar inlet located in gases source 105 or humidifier 107.

Fig, 1B illustrates in more detail an example respiratory humidifier. Aside from the differences described below, the humidifier is otherwise similar to the humidifier 107 of the system illustrated in Fig. 1.

The illustrated humidifier comprises a heater base 151 with a heater plate 152, a user interface 154 and a controller 125 (see Fig. 1); a removable and replaceable humidification chamber 153; and a removable and replaceable cartridge 155. Fig. 1C-1F illustrate in more detail the cartridge 155. As shown in Figs. 1B and 1D, the inspiratory tube 159 can connect to the cartridge 155. The humidification chamber 153 is received by the heater base 151, in thermal contact with the heater plate 152. In some embodiments, the cartridge 155 may also be integral with or permanently connected to the heater base, rather than removeable and replaceable.

The cartridge 155 houses electronics and one or more sensors which sense one or more properties of gases flowing through the humidification chamber 153 in use. The sensors may be provided on probes protruding from the cartridge 155 and through an aperture in the inlet or outlet of the humidification chamber 153, in use. The cartridge 155 also comprises an electrical connector 161 which makes an electrical connection with the heater base 151 for communication (for example, serial communication) with the controller. The cartridge 155 may further house, in part or in whole, electronics configured to determine or infer a capacitance or change in capacitance of the inspiratory tube 159, as described in further detail below, and communicate this to the controller via the electrical connector 161. The cartridge 155 therefore preferably comprises a microcontroller communicatively coupled with the sensor(s) and the controller. Alternatively, or additionally, the controller provided within the heater base 151, in part or in whole, may be configured to determine or infer the capacitance from data received from the sensor(s) via the electrical connection.

In use, the outlet of a dry line tube 157 receiving a flow of gases from a gases source is pneumatically coupled with the inlet of the humidification chamber 153, and an inspiratory tube 159 comprising an electropneumatic connector 161 is electrically coupled with the cartridge 155 and pneumatically coupled with the outlet of the humidification chamber 153 to transport the humidified flow of gases towards the patient. The electropneumatic connector 161 makes a releasable and lockable connection with the humidification chamber 153 and/or cartridge 155, and comprises release buttons 163.

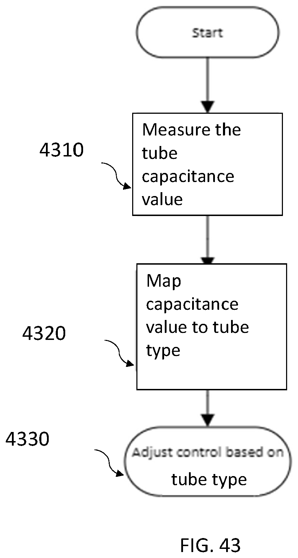

The electropneumatic connector 161, shown in further detail in Fig. 1G comprises electrical terminals or pads 171 respectively coupled with a pair of sensor wires 173 and a pair of heater wires 175 embedded within the inspiratory tube 159, forming respective sensing and heating loops. The electrical terminals or pads 171 can be electrically coupled with an identification resistor or other identification element embedded within the electropneumatic connector 161, which may be used by the humidifier to identify the type of inspiratory tube coupled with the cartridge 155. Alternatively, the type of inspiratory tube coupled with the cartridge 155 may be identified using the method illustrated in Fig. 43 and described further below. As described in further detail, moisture within the inspiratory conduit 159 may be detected from a measure of capacitance between the electrically-isolated heating and sensing loops. The connector 161 may further comprise additional wires or conductors configured to detect moisture within the inspiratory tube 159, alone or in combination with one or more of the sensor or heater wires 173, 175. Corresponding moisture detection terminals or pads 171 can be connected to the additional wires or conductors. . Alternatively, the additional wires or conductors may be electrically coupled with the 'identification' terminals or pads in place of the identification resistor or other identification element, and may optionally have a predetermined resistance (or a resistance within a predetermined range), capacitance, or resonant frequency unique to each tube model. This arrangement provides the dual functionality of identification and moisture detection. For example, a moisture-detection wire having a particular resistance may be used by the humidifier to identify the tube as being configured for capacitive moisture detection, and/or enable calibration of the cartridge and/or heater base for moisture detection with that particular tube model.

The aforementioned electrical terminals or pads 171 of the electropneumatic connector 161 are configured to make an electrical connection with corresponding pads or terminals on the cartridge (155 of Fig. 1B and Fig. 1D). Thus, in an embodiment with a removable and replaceable cartridge 155, existing humidifier bases may be retrofitted with a replacement cartridge comprising any additional electronics and/or electrical pads or terminals required to detect moisture in the inspiratory tube. Similarly, the disclosed humidifier, such as shown in Figs. 1C-1E, may be retrofitted with a replacement cartridge for compatibility with alternative inspiratory conduits, if necessary. Alternatively, the electrical terminals or pads of the electropneumatic connector and cartridges may be arranged so that selected "core" terminals or pads make electrical connections with corresponding terminals or pads of two or more different cartridges, while "optional" terminals or pads make electrical connections only with specific terminals or pads of selected cartridges configured to make use of those connections. A tube may correspond to a particular cartridge based on the terminals or pads.

At a distal (patient) end of the inspiratory conduit 159, there is provided a temperature sensor electrically coupled to the pair of embedded sensing wires 173, forming the sensing loop, and the heating wires 175 are also electrically coupled with each other, forming the heating loop. The additional wire(s) or conductor(s) of the connector 161 described above may similarly be electrically coupled at the distal end of the tube, although this may not be required in at least some implementations.

The cartridge 155 may further comprise a connector and/or cable configured for connection to a corresponding connector of the expiratory conduit (147 of Fig. 1A) to supply power to expiratory heating wire(s). In some implementations, the cartridge 155 and expiratory conduit 147 may alternatively or additionally be configured to detect moisture in the expiratory tube 147, with the connector and/or cable providing the required electrical connections.

Fig, 2 illustrates an example surgical insufflation device that can be used, for example, in a laparoscopy procedure. The laparoscopic cannula 207 can be connected to a gases delivery conduit 206, for example, via a Luer lock connector 4. The cannula 207 can be used to deliver gases into a surgical site, such as within the cavity of the patient 2. The cannula 207 can include one or more passages to introduce gases and/or one or more surgical instruments into the surgical cavity. The surgical instrument can be a scope, electrocautery tool, or any other instrument. The surgical instrument can be coupled to an imaging device, which can have a screen. The imaging device can be part of a surgical stack, which can include a plurality of surgical tools and/or apparatuses.

The humidifier chamber 205 can optionally or preferably be in serial connection to a gases supply 9 via a further conduit 204. The gases supply 9 can provide one or more insufflation gases, such as carbon dioxide, to the humidifier chamber 205. The gases supply can provide a continuous gases flow or an intermittent gases flow. The gases can be humidified as they are passed through the humidifier chamber 205, which can contain a volume of water 220.

A humidifier that incorporates the humidifier chamber 205 can be any suitable type or kind of humidifier. The humidifier chamber 205 can include a plastic formed chamber having a metal or otherwise conductive base sealed thereto. The base can be in contact with the heater plate 212 during use. The volume of water 220 contained in the chamber 205 can be heated by a heater plate 212, which can be under the control of a controller or control means 208 of the humidifier. The volume of water 220 within the chamber 205 can be heated such that it evaporates, mixing water vapor with the gases flowing through the chamber 205 to heat and humidify the gases.

The controller or control means 208 can be housed in a humidifier base unit 221, which can also house the heater plate 212. The heater plate 212 can have an electric heating element therein or in thermal contact therewith. The humidifier base unit 221 and/or the heater plate 212 can be removably engageable with the humidifier chamber 205. The humidifier chamber 205 can also alternatively or additionally include an integral heater.

A temperature sensor can also be located at or near the outlet 209 to monitor a temperature of the humidified gases leaving the humidifier chamber 205 from the outlet 209. Additional sensors can also optionally be incorporated, for example, for sensing characteristics of the gases (such as temperature, humidity, flow, or others) at a patient end and/or anywhere along the gases delivery conduit 206. The temperature sensor can be connected to the controller 208 through a sensor wire within, throughout, or around gases delivery conduit 206

The gases can exit out through the humidifier's outlet 209 and into the gases delivery conduit 206. The gases can move through the gases delivery conduit 206 into the surgical cavity of the patient 2 via the cannula 207, thereby inflating and maintaining the pressure within the cavity. Preferably, the gases leaving the outlet 209 of the humidifier chamber 205 can have a relative humidity of around 100%. The gases travel along the gases delivery conduit 206. As with all of the various example humidifier systems discussed above, "rain out" can occur such that water vapor can condense on a wall of the gases delivery conduit 206. Condensate can have undesirable effects, such as detrimentally reducing the water content of the gases delivered to the patient. In order to reduce and/or minimize the occurrence of condensation within the gases delivery conduit 206, a heater wire 210 can be provided within, throughout, or around the gases delivery conduit 206. The heater wire 210 can be electronically connected to the humidifier base unit 221, for example by an electropneumatic connector of gases delivery conduit 206.

### Composite Tubes

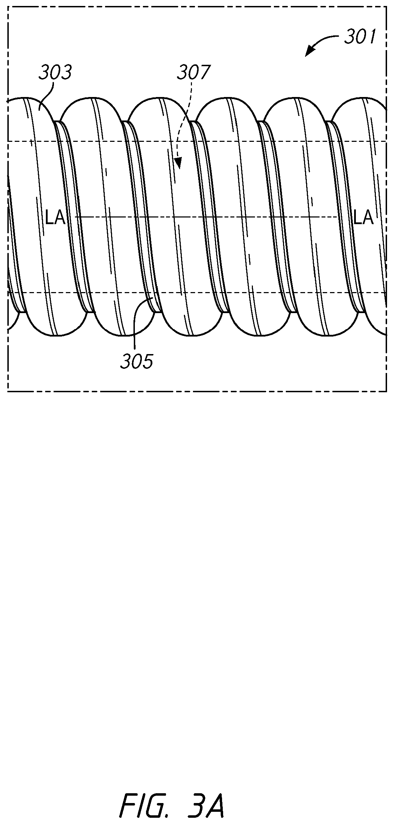

Fig. 3A shows a side-plan view of a section of an example composite conduit or tube 301. In general, the composite tube 301 comprises a first elongate member 303 and a second elongate member 305. Member is a broad term and is to be given its ordinary and customary meaning to a person of ordinary skill in the art, is not to be limited to a special or customized meaning, and includes, without limitation, integral portions, integral components, and distinct components. Thus, although Fig. 3A illustrates an implementation made of two distinct components, it will be appreciated that in other implementations, the first elongate member 303 and second elongate member 305 can also represent regions in a tube formed from a single material. In some configurations, the first elongate member 303 can represent a hollow portion of a tube as described below, while the second elongate member 305 can represent a structural supporting or reinforcement portion of the tube which adds structural support to the hollow portion. The hollow portion and the structural supporting portion can have a spiral configuration, as described herein. The composite tube 301 may be used to form the inspiratory tube and/or the expiratory tube of any type of system as described above, a coaxial tube as described below, or any other tubes as described elsewhere in this disclosure.

In this example, the first elongate member 303 comprises a hollow body spirally wound to form, at least in part, an elongate tube having a longitudinal axis LA-LA and a lumen 307 extending along the longitudinal axis LA-LA. In at least one implementation, the first elongate member 303 is helical tubular member. Preferably, the first elongate member 303 is flexible. Furthermore, the first elongate member 303 is preferably transparent or, at least, semi-transparent or semi-opaque. A degree of optical transparency allows a caregiver or user to inspect the lumen 307 for blockage or contaminants or to confirm the presence of condensate. A variety of plastics, including medical grade plastics, are suitable for the body of the first elongate member 303. Examples of suitable materials include Polyolefin elastomers, Polyether block amides, Thermoplastic co-polyester elastomers, EPDM-Polypropylene mixtures, and Thermoplastic polyurethanes.

The hollow body structure of the first elongate member 303 contributes to the insulating properties of the composite tube 301. An insulating tube 301 is desirable because it reduces heat loss. This can allow the tube 301 to deliver gas from a heater-humidifier to a patient while ameliorating heat loss and condensation formation with less energy consumption than an uninsulated tube (or, a tube lacking a hollow body component for insulation).

The hollow portion of the first elongate member 303 can optionally be filled with a gas. The gas can be air, which is desirable because of its low thermal conductivity (2.62x10⁻² W/mK at 300K) and very low cost. A gas that is more viscous than air may also advantageously be used, as higher viscosity reduces convective heat transfer. Thus, gases such as argon (17.72x10⁻³ W/mK at 300K), krypton (9.43x10⁻³ W/mK at 300K), and xenon (5.65x10⁻³ W/ mK at 300K) can increase insulating performance. Each of these gases is nontoxic, chemically inert, fire-inhibiting, and commercially available. The hollow portion of the first elongated member 303 can be sealed at both ends of the tube, causing the gas within to be substantially stagnant. Alternatively, the hollow portion can be a secondary pneumatic connection, such as a pressure sample line for conveying pressure feedback from the patient-end of the tube to a controller. The first elongate member 303 can be optionally perforated. For instance, the surface of the first elongate member 303 can be perforated on an outward-facing surface, opposite the lumen 307. The hollow portion of the first elongate member 303 can also optionally be filled with a liquid. Examples of liquids can include water or other biocompatible liquids with a high thermal capacity. For instance, nanofluids can be used. An example nanofluid with suitable thermal capacity comprises water and nanoparticles of substances such as aluminum.

The second elongate member 305 is also spirally wound and joined to the first elongate member 303 between adjacent turns of the first elongate member 303. The second elongate member 305 forms at least a portion of the lumen 307 of the elongate tube. The second elongate member 305 acts as structural support for the first elongate member 303.

The second elongate member 305 can optionally be wider at the base (proximal the lumen 307) and narrower at the top. For example, the second elongate member 305 can be generally triangular in shape, generally T-shaped, or generally Y-shaped. However, any shape that meets the contours of the corresponding first elongate member 303 is suitable.

The second elongate member 305 can be flexible, to facilitate bending of the tube. The second elongate member 305 can be less flexible than the first elongate member 303. This improves the ability of the second elongate member 305 to structurally support the first elongate member 303. For example, the modulus of the second elongate member 305 is preferably 30 - 50 MPa (or about 30 - 50 MPa). The modulus of the first elongate member 303 is less than the modulus of the second elongate member 305. The second elongate member 305 can be solid or mostly solid. In addition, the second elongate member 305 can encapsulate or house conductive material, such as heating elements, sensing wires or an antenna. In some embodiments, the second elongate member 305 can be extruded. Heating elements (also referred to herein as heating wires, heating filaments or filaments) can minimize the cold surfaces onto which condensate from moisture-laden air can form. Heating elements can also be used to alter the temperature profile of gases in the lumen 307 of composite tube 301. Sensing wires may be coupled with a sensor, such as a temperature sensor, integrated within or otherwise provided at a distal end of the tube, in use providing measurements which may be used by the gases supply system, such as humidifier 107, in a feedback control system to adjust the amount of heat provided by the one or more heating wires or other components of the gases supply system. A variety of polymers and plastics, including medical grade plastics, are suitable for the body of the second elongate member 305. Examples of suitable materials include Polyolefin elastomers, Polyether block amides, Thermoplastic co-polyester elastomers, EPDM-Polypropylene mixtures, Thermoplastic polyurethanes, Thermoset and Thermochromic materials. In some configurations, the first elongate member 303 and the second elongate member 305 can be made from the same material. The second elongate member 305 can also be made of a different color material from the first elongate member 303, and can be transparent, translucent or opaque. For example, the first elongate member 303 can be made from a clear plastic, and the second elongate member 305 may be made from an opaque blue (or other color) plastic. Further material options are described below, particularly with reference to permeable or breathable materials.

This spirally-wound structure comprising a flexible, hollow body and an integral support can provide crush resistance, while leaving the conduit wall flexible enough to permit short-radius bends without kinking, occluding or collapsing. Preferably, the tube can be bent around a 25 mm diameter metal cylinder without kinking, occluding, or collapsing, as defined in the test for increase in flow resistance with bending according to ISO 5367:2014(E), for example. This structure also can provide a substantially smooth lumen 307 surface (tube bore), which helps keep the tube free from deposits and improves gas flow. The hollow body of the first elongate member 303 has been found to improve the insulating properties of the tube, while allowing the tube to remain light weight. In other implementations, however, a non-smooth lumen surface or tube bore may be preferred, as described below.

The composite tube 301 can be used as an expiratory tube and/or an inspiratory tube in a conduit system, or a portion of a conduit system.

The first elongate members 303 and second elongate member 305 (with encapsulated heating elements and sensing wires) may each be extruded adjacent to each other onto a rotating mandrel in a double-helix arrangement to form a continuous length of tubing. The continuous length of tubing may be cut to any desired lengths appropriate for use in a conduit system (severing the heating elements and sensing wires in the process) and terminated with appropriate connectors at each end. For example, with reference to the system of Fig. 1, an inspiratory conduit may be terminated with a chamber end connector at one end, for pneumatic coupling with a humidification chamber 129 and electrical coupling with a humidifier 107 (or cartridge of a humidifier), and a patient end connector at the other end for pneumatic coupling with a Y-piece 135 or patient interface 115. The patient end connector may comprise an integrated temperature sensor which is electrically coupled with the severed sensing wires to form a sensing circuit. At, or near, the patient end connector, the severed heating elements may also be electrically coupled with each other to form a heating circuit. Alternatively, the heating elements and/or sensing wires may be terminated by respective heating and/or sensing terminals in the patient end connector, for electrical coupling with another component of the conduit system. At the chamber end connector, the severed heating elements and sensing wires may be electrically coupled with respective heating and sensing terminals integrated within the chamber end connector. The chamber end connector may be configured for simultaneous pneumatic coupling with the outlet of the humidification chamber 129 and electrical coupling with the humidifier 107. Alternatively, the chamber end connector may comprise an electrical socket, for example, for independent pneumatic and electrical coupling with the humidification chamber 129 and humidifier 107, respectively.

In some implementations, the tube may be further provided with one or more intermediate connectors, such as a midpoint connector comprising a diode enabling either a first half or section, or an entire length, of the tube to be selectively heated by supplying power to the heater wires in a first or second polarity. For example, the first half or section of the tube may be powered in the first polarity and the entire length of the tube may be powered in the second polarity. The midpoint connector may additionally or alternatively comprise a further sensor, such as a temperature sensor. Alternatively, two or more zones of the tube may be configured to be controlled fully independently of each other, so that any one or more of the zones may be selectively heated and/or sensed.

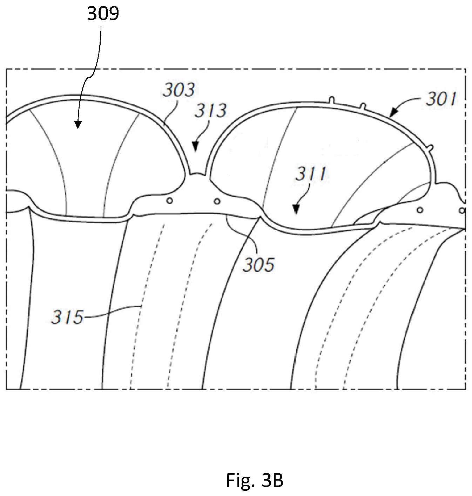

Fig. 3B shows a longitudinal cross-section of a top portion of the example composite tube 301 of Fig. 3A. Fig. 3B has the same orientation as Fig. 3A. This example further illustrates the hollow-body shape of the first elongate member 303. As seen in this example, the first elongate member 303 forms a longitudinal cross-section of enclosed portions 309. This cross-section of the first elongate member 303 appears as a "bubble" in Fig. 3B. Portions 309 of the first elongate member 303 overlap adjacent wraps of the second elongate member 305. A portion 311 of the first elongate member 303 forms the wall of the lumen (tube bore).

A gap 313 between adjacent turns of the first elongate member 303 can improve the overall insulating properties of the composite tube 301. Furthermore, the gap 313 between adjacent bubbles can increase the heat transfer resistivity (the R value) and, accordingly, decreases the heat transfer conductivity of the composite tube 301. This gap configuration also improves the flexibility of the composite tube 301 by permitting shorter-radius bends. A T-shaped second elongate member 305, as shown in Fig. 3B, can help maintain a gap 313 between adjacent bubbles. Nevertheless, adjacent portions of first elongate member 303 can be configured to be touching. For example, adjacent portions can be bonded together or need not be bonded together. In such a bonded configuration or when the adjacent portions are not bonded, an elongate member, such as T-shaped second elongate member 305 or other shaped member, can be included which is water permeable or made of wicking materials in order to improve capacitive measurements in the presence of condensate as described further below.

One or more conductive materials (referred to herein as "elements," "conductive elements" or "filaments") can be disposed in the second elongate member 305 for heating and/or sensing the gases flow. In this example, two elements 315 are encapsulated in the second elongate member 305, one on either side of the vertical portion of the "T." The elements 315 comprise conductive material, such as alloys of Aluminum (Al) and/or Copper (Cu), or conductive polymer. Preferably, the material forming the second elongate member 305 is selected to be non-reactive with the metal in the elements 315 when the elements 315 reach their operating temperature. The elements 315 may be spaced away from lumen 307 so that the elements are not exposed to the lumen 307. At one end of the composite tube, pairs of elements can be formed into a connecting loop.

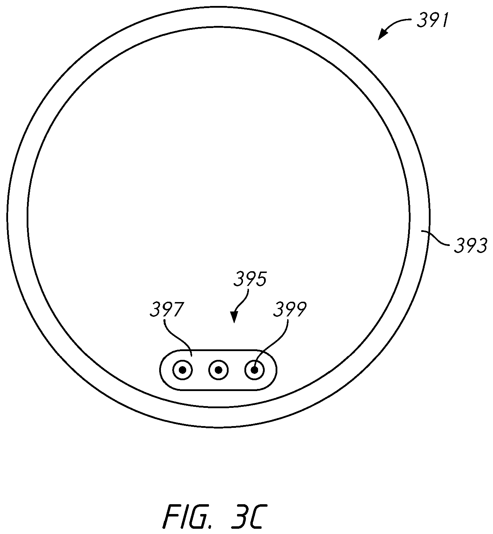

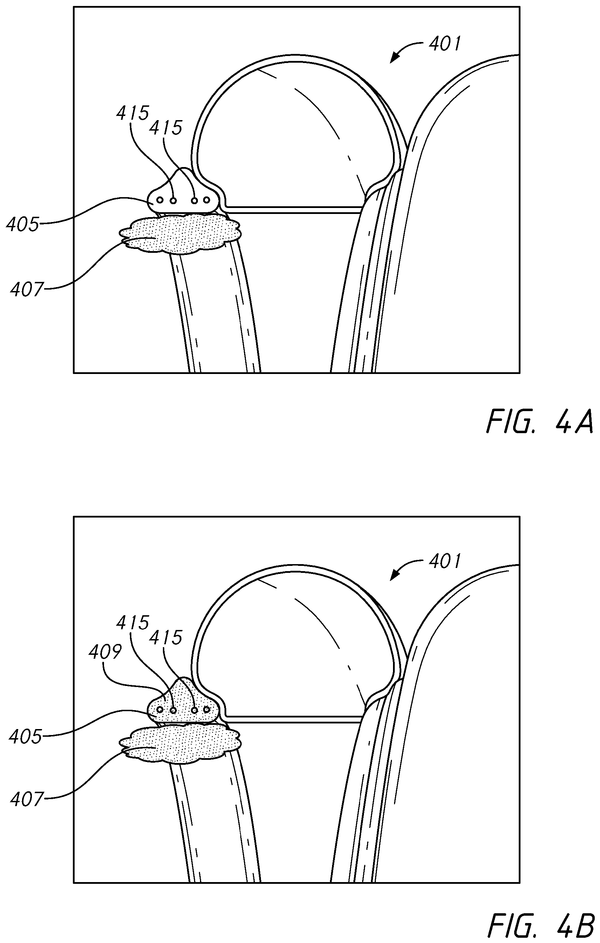

A plurality of elements can be disposed in the second elongate member 305. The elements can be electrically connected together to share a common rail. For example, a first element, such as a heating element, can be disposed on a first side of the second elongate member 305. A second element, such as a sensing element or wire, can be disposed on a second side of the second elongate member 305. An optional third element, such as shown in Fig. 3C as a ground element, can be disposed between the first and second elements. The first, second, and/or third elements can be connected together at one end of the second elongate member 305. The third element may also be configured to dissipate power to heat the tube and/or gases, and the humidifier may comprise a bias generator circuit configured to enable a sensor coupled with the second elongate member 305 to be read regardless of whether or not the heater wire is powered. There can also be four wires, as shown in Figs. 4A-4B, (two heating elements forming a pair and two sensing wires forming a pair) with respective ends of each pair being electrically connected or continuous at a distal end of the tube so that the heating and sensing circuits are independent from each other. Arrangements can include one, two, three, four or more wires. These elements can be connected to the gases supply system using an electrical connector that is either separate from or integrated with a pneumatic connector of the composite tube. A pitch of the helically wound conduit (for example, the pitch of the first and second elongate members) can be varied (for example, decreased) in order to enhance a capacitive measurement in some areas of the conduit, as described in further detail below.

In other implementations, the tube may be provided with one or more additional wires or conductive elements, referred to generally as capacitive wires, provided specifically for the purpose of providing a capacitive coupling with another wire or circuit. The capacitive wire(s) need not necessarily form a closed loop or circuit like the heating and sensing wires generally will. The capacitive wire(s) also need not necessarily extend the full length of the tube. In some implementations, there may be a plurality of capacitive wire(s) of varying length, which may allow a general location of any moisture within the tube to be detected.

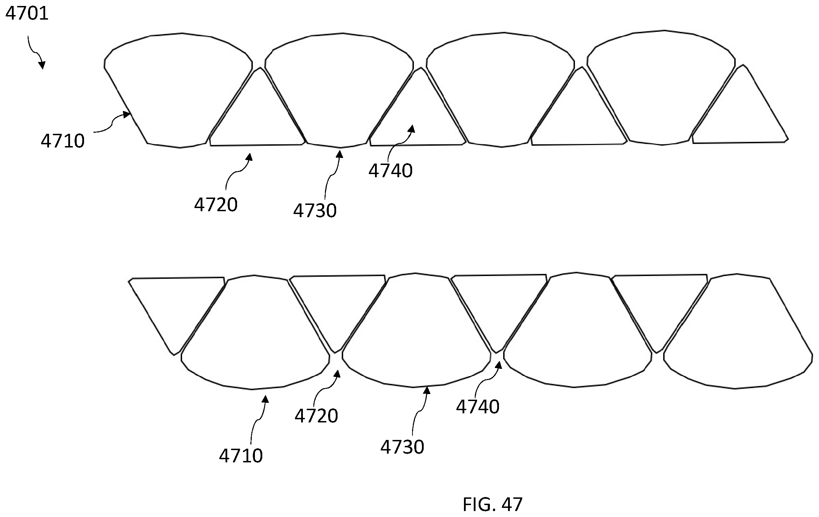

In some implementations, the composite tube may comprise respective pairs of first and second elongate members wound in a quadruple-helix arrangement. Fig. 47 shows a cross section of a bubble tube 4701 in a quadruple helix arrangement. The tube 4701 includes a first pair of first elongate member 4710 and second elongate member 4720, and a second pair of first elongate member 4730 and second elongate member 4740. In particular, sensing wires may be provided in one of the second elongate members (for example, in the second elongate member 4740), with heating wires and at least one capacitive wire provided in the other second elongate member (for example, in the second elongate member 4720), each separated by the pair of first elongate members 4710, 4730 respectively. The pair of first elongate members may be the same as each other or different. Such an arrangement may be preferred to provide a moisture-dependent capacitive coupling between the heating and capacitive wires, while minimizing any capacitive coupling with the sensing wires which may affect sensor readings.

The above description of a composite tube is not meant to be limiting, but is provided as an example only. It is to be understood that any other type of conduit can be used with the condensation detection of the present disclosure. This includes any sized, shaped or constructed tube that incorporates heating, sensor and/or condensate detection elements in the walls of the tube and/or the tube lumen, such as elements which float or dangle freely within the tube or outside of the tube, as shown in Fig. 3C, or are attached to one or more locations within the tube or outside of the tube. As shown in Fig. 3C, tube 391 includes walls 393 and floating or dangling elements 395. It should be appreciated by one of skill in the art that "floating" elements internal to the lumen (not encapsulated in the wall) may be provided as shown, or may be coiled or wound inside the lumen and be held in place in the lumen with a retainer, or could be provided in other configurations as known in the art. As described in further detail below, moisture may be detected from variations in the capacitance between adjacent elements. Different conduit structures have different properties which may be exploited to enhance this detection. For example, helically-wound elements, such as the heating elements and sensing wires described above with respect to Figs. 3A-3B, can have capacitive properties that are relatively high due to the length of the elements and may detect condensate anywhere on the conduit wall. On the other hand, a floating element within the tube may have the advantage that it will tend to settle at the bottom of the conduit where condensation may also accumulate, which may be preferable in some arrangements. A floating element also need not be extruded with the tube, permitting greater flexibility in the design of the floating element (for example, materials, manufacturing methods, and/or variations in dimensions along the length of the floating element). As would be understood by a person of skill in the art, there are many different types of tubes and elements in use and known within the art, any one of which can be used with the present disclosure.

### Condensation Detection

### Capacitance and Inductance Based Condensation Detection

When two or more elements or wires are located within a conduit, a parasitic capacitance (reactance and/or inductance) may exist between them. Although this parasitic capacitance can negatively affect sensor measurements and therefore efforts have been employed to mitigate parasitic capacitance effects (for example, as discussed in WO2018116187A1, incorporated herein in its entirety by reference), it has been discovered that the parasitic capacitance can be dependent on moisture within the conduit, and it can therefore be used as an indication and/or measure of humidity, moisture, fluid and/or condensation (collectively referred to herein as "condensation" for purposes of brevity). Applicants have surprisingly found that in this way, the elements may be treated as the "plates" of a capacitor, and changes to the capacitance due to the presence of moisture may be observed and measured. In some implementations, the measurement of the capacitance does not require any sensor(s) of any type or kind to be exposed to the flow path of the gases in the conduit while in other implementations any known sensor can be used to measure capacitance. By utilizing existing conductive elements within a conduit (for example, existing heating or sensor elements or wires), this discovery provides an inexpensive and accurate solution to detect the presence and/or quantity of condensation within a conduit without substantial change to the conduit or gases supply system. Alternatively, additional conductive elements specifically for this purpose may be included in a tube. This may allow the tube to be designed to minimize negative or undesirable effects from parasitic capacitance with the sensing wires, and/or otherwise enhance the sensitivity to condensate without compromising performance of the heating and/or sensing wires. For example, heating elements and moisture detection elements may be provided in close proximity to each other, with sensing wires spaced apart and/or embedded in a non-permeable material. In another arrangement a tube may comprise sensing wires embedded in the conduit wall, and heating elements and a dedicated condensate detection wire or moisture detection element floating freely within the lumen, as partially shown in Fig. 3C. In another arrangement, as disclosed above, sensing wires may be provided in a separate second elongate member 305 to the heating elements and moisture detection elements or wires in a quadruple helix tube.

The dielectric constant between two electrically isolated conductive elements (for example, for sensing elements, heating elements, or the like), and hence the capacitance, will differ depending on the distance between the elements as well as the existence or amount of condensate on the inner wall surface of the conduit (or, in the case of a vapor permeable wall material, the amount of individual water molecules that move into the wall). Generally, dielectric constant and capacitance are positively related. Moisture near the conductive elements, for example, on the inner wall surface of the conduit, can have a parasitic or peripheral effect on capacitance. In other words, the inherent capacitance of the conduit can change in accordance to the amount and proximity of condensation present in the tube if the elements are at a fixed distance. The distance that the elements are separated requires design considerations in order to properly balance element usage. For example, the elements should be close enough to create a detectable capacitance, yet spaced enough so that there is sufficient moisture change to create the detectable change in capacitance. Accordingly, the distance that the elements are separated may be designed such that the elements are close enough to create a detectable capacitance, yet spaced enough so that there is sufficient moisture change to create the detectable change in capacitance.

Although the conductive elements (referred to herein as "elements" for brevity) are described mainly with respect to wires or filaments, such as heater wires or sensor wires, it is to be understood that the elements can be something other than physical wires. For example, the elements can be conductive plates, polymers, tapes or ribbons, conductive ink, conductive thread or any other conductive materials.

Fig. 4A schematically illustrates a conduit wall 401, this time comprising respective pairs of conductive heating and sensing elements 415, and how condensate 407 which is present on the inner wall of the tube will affect the dielectric between any two wires which are electrically isolated. In some implementations, the elongate member 305 in Figs. 3A-3B is a bead 405. For example in Fig. 4A, bead 405 is not permeable to fluid. Condensate 407 in this arrangement is therefore adjacent to, rather than directly between, the heating and sensing elements 415, but has been found to result in a measurable change in the parasitic capacitance between the conductive elements. Alternatively, or additionally, as described in further detail below, the profile of the bead 405 may be modified to enhance sensitivity to condensate by, for example, providing a channel between adjacent conductive elements which is open to the lumen to receive the condensate 407 between two of the elements 415.

To increase the capacitance between the elements 415, the wires may be provided side-by-side in an alternating or interleaved arrangement, that is in the order heating-sensing-heating-sensing. Other arrangements, such as heating-sensing-sensing-heating or heating-heating-sensing-sensing, may alternatively be used.

Fig. 4B schematically illustrates a second example where the bead 405 is either vapor permeable and/or fluid permeable. It has been found that the vapor permeable material enhances the effect of condensation upon the parasitic capacitive coupling between the wires, so that it can be detected with greater precision. In one example, the material can be vapor permeable such that the material allows evaporation of water molecules to ambient air while effectively blocking passage of liquid water and breathing gases to ambient air. Although using a vapor or fluid permeable material in an inspiratory tube is generally undesirable as it will reduce the humidity of gases delivered to the patient, if it is only used in the bead 405, this drying of the gases will be minimized. Further, this material can be covered on the outside by another material that is not vapor or fluid permeable material to allow penetration of vapor or fluid for measurement purposes without allowing the vapor or fluid to leak to ambient. For example, in Fig. 3B, if the proximate portions of elongate members 303 are joined together, a vapor or liquid permeable elongate member 305 would not leak to ambient.

A bead 405 can be made from, for example, one or more of an activated perfluorinated polymer material having extreme hydrophilic properties (such as NAFION branded products), hydrophilic thermoplastic, woven treated fabric exhibiting breathable characteristics, a hydrophilic polyester block copolymer (such as SYMPATEX branded products), a breathable thermoplastic copolyester (TPC) (more specifically a breathable copolyester with a polyether soft segment) (including materials such as ARNITEL^{®} VT 3108 or those with similar or greater breathable properties), or any other materials which allows evaporation of water vapor to ambient air while inhibiting or blocking passage of liquid water and breathing gases to ambient. Using such materials, individual molecules 409 can pass through the bead by diffusion, directly affecting the dielectric constant between any two electrically isolated wires or elements. The dielectric constant will also be affected due to the presence of condensate on the inner wall of the tube. It should be noted that one of skill in the art would appreciate that the wires can be insulated by a sheath to prevent short-circuiting and/or corrosion.

Hereinafter and throughout the description, a material that allows the passage of water molecules through a monolithic wall of the material via the solution-diffusion mechanism, without allowing the bulk passage of liquid water or bulk flow of respiratory gases all the way through the wall is described as a "breathable" material. It should be appreciated by one of skill in the art that the water molecules in the wall are molecularly dispersed in the media, and are therefore without a state (solid, liquid, or gas), although they are sometimes referred to in the art as vapor (e.g. the rate of transfer is often referred to as a water vapor transmission rate or the like). It should further be appreciated that a monolithic wall does not contain open channels or through holes from one major surface to another, such that viruses could be carried through such channels or holes alongside air or liquid water drops via the pore flow mechanism. It should yet further be appreciated that, like all polymers, some small molecule transport of respiratory gases (such as oxygen, carbon dioxide or nitrogen) may occur in trace or de minimis amounts (i.e. not "bulk" flow), which, for a breathable material as defined herein, would typically be at a rate at least an order of magnitude lower than that for water molecules. Furthermore, of particular relevance for breathing gases being delivered to or from a patient, such small molecule transport of respiratory gases would be of an amount less than that allowed for compliance with the relevant standards, for example, in the leakage test of ISO 2367:2014 at Section 5.4 tested via the method set out in Annex E, which is hereby incorporated by reference in its entirety.

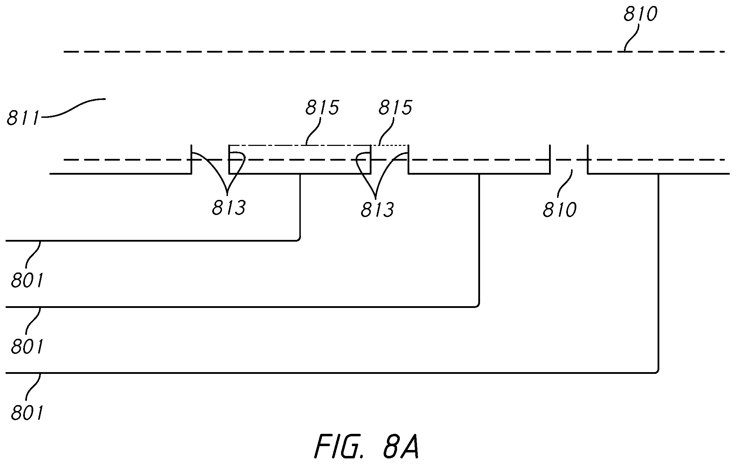

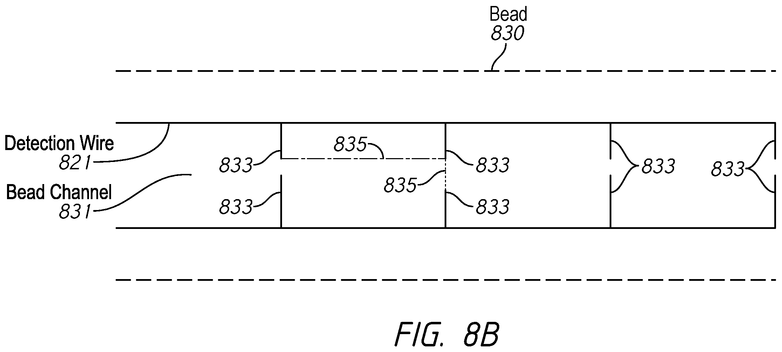

Other element structures within a conduit can also be used. For example, the elements do not need to be comprised within the wall of the conduit, but could be allowed to float within the conduit. In such a configuration, the elements are likely to rest in condensate at the lowest part of the tube which can allow for improved condensate detection. Further, a vapor and/or liquid permeable material could be used to surround and join the elements in order to improve condensate measurements. Alternatively, as shown in Fig. 3C, a liquid water absorptive, wicking, and/or hydrophilic material 397 such as cotton, organic or inorganic fabrics or fibers, or an open-cell foam material could be used to surround and join the elements 399 in order to improve condensate measurements. Such a material also provides the advantage of inhibiting mobility of the condensate towards sensors, the patient or the ventilator or other gases source where condensate is most undesirable. Additionally, or alternatively, the conduit can include microstructures, such as channels, to transport liquid condensate towards and/or along an appropriate measurement element by capillary action. Further examples of these structures are described below with respect to Figs. 8-18.

The inner wall of the tube may also have openings (including, for example, dips, corrugations, valleys, square channels, and/or undulations of varying sizes and shapes) to encourage condensate to accumulate in the openings. For example, this can include parallel or helical corrugations or longitudinal channels with valleys adjacent the relevant elements so that condensate accumulates where it will influence capacitance between the elements. This may provide safety for the patient by reducing the likelihood of condensation flowing into the patient interface, and may also provide a specific measurement site.

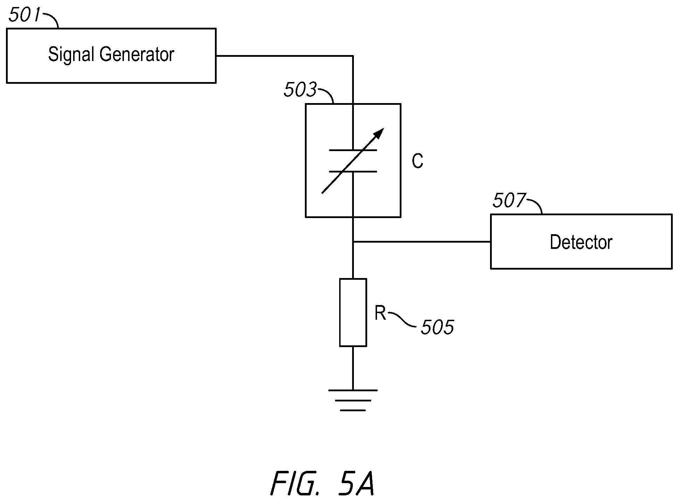

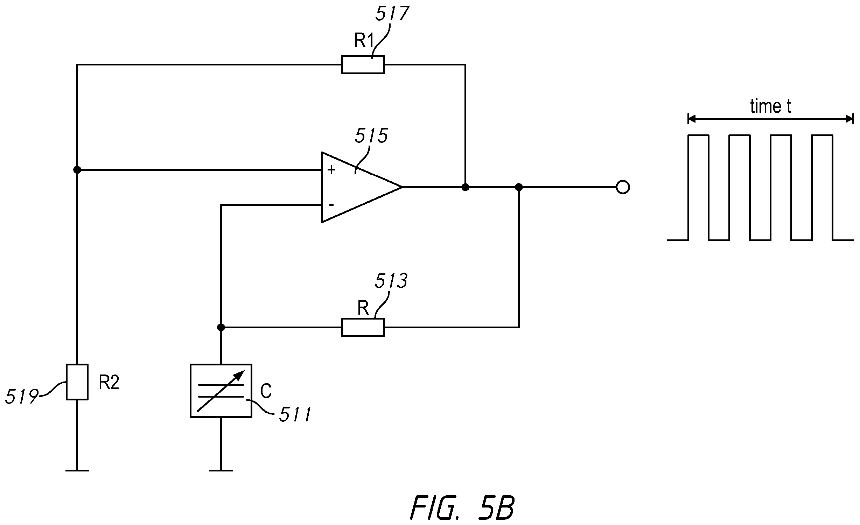

In some implementations, a capacitance measurement can be performed by generating a signal which passes through the tube along one or more elements. This is used to detect and measure, by a detector 507, a time constant dependent on the inherent capacitance "C" between that element and one or more adjacent elements. This process is schematically represented in Fig. 5. A supplied power step change or pulse, or a series of pulses, can be used as the generated signal to measure the change in capacitance. These measurements can either coincide or be interleaved with either or both of temperature sensing measurements or heating wire usage. Capacitance can also be measured by any known method of measuring or determining capacitance.

Fig. 5A schematically illustrates a signal generator 501, a conduit system 503, a resistor R 505 and a detector 507. The signal generator 501, resistor 505 and detector 507 would generally be integrated within a humidifier in electrical communication with the conduit system 503, such as in the cartridge and/or the heater base as described above. The conduit system 503 is represented by the variable capacitor C. The conductive elements of the conduit system will also have their own electrical resistance, for example caused by the heater, sensor and/or capacitance measurement element, but this is omitted for clarity. The series resistance of the element and resistor R 505 in conjunction with the capacitance C of the conduit system 503 form a circuit with a characteristic time constant. The time constant can be mathematically represented as τ = RC, where τ represents the time required to charge the capacitance, through the resistor, from an initial charge voltage of zero to approximately 63.2% (1 - e⁻¹) of the applied voltage, or to discharge the capacitor through the same resistor to approximately 36.8% (e⁻¹) of its initial charge voltage. If the resistance R is made significantly large compared to the element resistance, then the element resistance can be ignored, and an approximate time constant τ will equal the resistance R multiplied by the capacitance C of conduit. If the resistance R is constant, then the time constant is proportional to the capacitance of the conduit.

Capacitance will vary in accordance with the amount of condensate in the conduit. The capacitance and amount of condensate in the conduit can be configured through conduit design to be positively related. Therefore, the presence of condensate in the conduit may be inferred from a comparison of a measured value indicative of the capacitance (such as the time constant τ) with a predetermined threshold. Alternatively, or additionally, an approximate volume of condensate in the conduit can be inferred from an absolute measurement of the measured value indicative of the capacitance. Alternatively, or additionally, whether water is presently condensing or evaporating in the tube may be inferred by comparing two or more measurements of a value indicative of capacitance over time.

The voltage across the resistor R, *V_{R}*, is: ${V}_{R} = {V}_{in} {e}^{- t / RC}$ [1]

Where R is the value of the resistor, C is the capacitance of the tube, and *Vᵢₙ* is the signal generator output.

It can also be seen that: ${V}_{in} = {V}_{C} + {V}_{R}$ [2] ${V}_{R} = {V}_{in} - {V}_{C}$ [3]

The voltage across the resistor R may be measured by any appropriate means, for example by using a detector, whose output may be read by using a general purpose input/output (GPIO) pin of a micro-controller. Additionally or alternatively, frequency domain techniques such as a fast Fourier transform (FFT) can be used to infer capacitance. For example, signal generator 501 could be configured to generate a signal at a certain frequency. At any given frequency the capacitance will exhibit a reactance X_{C} which impedes current flow, where *X_{C}* = 1/(2*πfC*) and *f* is the frequency. Conduit system 503 and resistor R 505 then form a voltage divider whereby detector 507 can be configured to detect a certain voltage level representative of a certain capacitance or condensation level.

Alternatively, the tube capacitance can be included in an RC oscillator circuit to determine the presence and/or amount of condensation. Fig. 5B is an example RC oscillator circuit, although any comparable RC oscillator circuit can be used. The example RC oscillator circuit in Fig. 5B may have a variable tube capacitance *C* 511, an oscillator resistor *R* 513, an operational amplifier 515, a first resistor 517, and a second resistor 519 which are all electrically connected. The oscillator circuit may be electrically connected to a frequency sensor. The frequency sensor can be located in the heater base, an external accessory, the sensor cartridge, an intermediate tube connector, or within the tube itself.

The frequency sensor will determine the frequency of the output of the oscillator circuit. For example, the frequency sensor could measure the frequency of the oscillator output by counting how many pulses occur over a fixed time-window, which in turn can be used to determine the frequency of the oscillator circuit. The frequency of the output of the oscillator circuit can be proportional to changes in the variable tube capacitance - specifically, if capacitance increases, the oscillation frequency will decrease as shown in the below equation, where *k* is a constant that represents a ratio between resistors R1 and R2. $f ≅ k \frac{1}{2 πRC}$ [4]

Tube capacitance will vary in accordance with the amount of condensate in the tube. The presence of condensate in the tube may be inferred from a comparison of a measured value indicative of the capacitance (such as frequency *f*) with a predetermined threshold. Alternatively, or additionally, an approximate volume of condensate in the conduit can be inferred from an absolute measurement of the measured value indicative of the capacitance. Alternatively, or additionally, whether water is presently condensing or evaporating in the tube may be inferred by comparing two or more measurements of a value indicative of capacitance over time.

Alternatively, a fixed inductor may be added to the circuit and the capacitance calculated or inferred from a resonant frequency of the resistor-inductor-capacitor (RLC) or inductor-capacitor (LC) circuit. In an inductance-based detection system, the inductance of a wire or wires in the breathing tube can be measured to detect the presence of condensation. For example, the embedded wires in the bead (for example, the bead 405 of Fig. 4A) of the breathing tube can be configured to function as an inductor. The inductance of the inductor is a function of the permeability of the media present within the core of the inductor. Air and water have different relative permeabilities, thus the inductance of a breathing tube, which can be measured at one end, may vary due to changes in moisture content within the breathing tube.

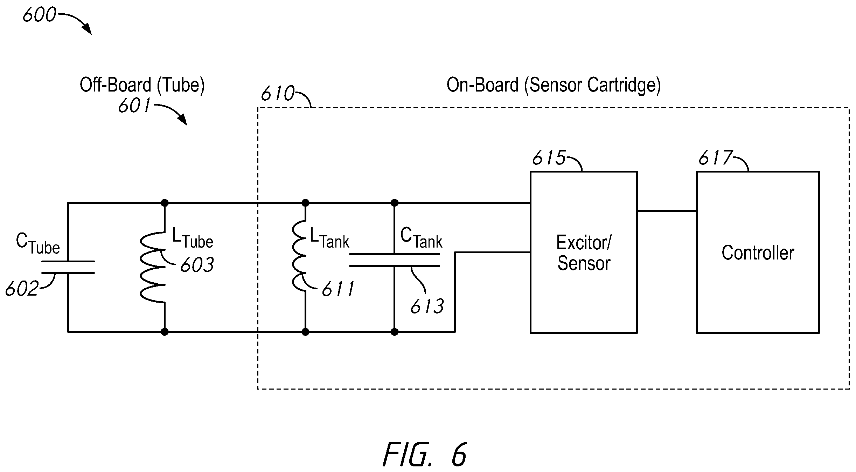

Fig. 6 is an example inductance-based condensation detection system, which schematically illustrates an LC type circuit 600 which can have a first component 601 electrically connected to a second component 610. The first component 601 can be modeled as first inductor *L_{tube}* 603 in parallel with a first capacitor *C_{tube}* 602. The second component can be modeled as a second inductor *Lₜₐₙₖ* 611 in parallel with a second capacitor *Cₜₐₙₖ* 613, an exciter module 615 (for example, a sensor), and a controller 617. In some implementations, the first inductor *L_{tube}* 603, the first capacitor *C_{tube}* 602, the second inductor *Lₜₐₙₖ* 611, the second capacitor *Cₜₐₙₖ* 613, or any combination thereof are actual electrical components such as inductors and capacitors. In some implementations, the first inductor *L_{tube}* 603, the first capacitor *C_{tube}* 602, the second inductor *Lₜₐₙₖ* 611, the second capacitor *Cₜₐₙₖ* 613, or any combination thereof are the result of intrinsic capacitance or inductance. The combination of the second inductor *Lₜₐₙₖ* 611 and the second capacitor *Cₜₐₙₖ* 613 can be referred to as a resonant tank, resonant circuit, tank circuit, tuned circuit, LC network, or LC oscillator, or any other parallel combination of inductor and capacitor which will exhibit resonant behavior when excited. The resonant tank can be electrically connected to the exciter module 615, which is in turn electrically connected to the controller 617.

In some implementations, the second component 610 is on-board and can be located in the humidifier heater base (for example, the heater base 151 of Fig. 1B) while the first component 601 is off-board and may be in a tube (for example, the inspiratory tube 159 of Fig. 1B). As such, the resonant tank can be located in the humidifier heater base and is connected in parallel to one of the embedded wires and an exciter module 615. The resonant tank can be excited by an injection of energy. One example of an injection of energy is a step change in current, which can be injected by the exciter module 615 at the direction of the controller 617. Oscillations in current and voltage occurring at a frequency known as the 'resonant frequency' (*ω*) will thus be observed.

Alternatively, the resonant tank, the exciter module 615, the controller 617, or any combination of the resonant tank, the exciter module 615, and the controller 617 can be located in an external accessory, the sensor cartridge, an intermediate tube connector, or within the tube itself.

In some embodiments, the first component 601 and the second component 610 are not electrically connected. For example, the resonant tank may not be electrically connected to the first component 601 but may include a separate wire wound around the tube. This may create high inductance for the resonant tank. In some embodiments, the separate wire implementation of the resonant tank is within the walls of the tube, but external to the bead (in embodiments where the first component is embedded in the bead 405).

The resonant frequency is dependent on the inductance and capacitance in the circuit. *Lₜₐₙₖ* and *Cₜₐₙₖ* are known and fixed by design; therefore, changes in resonant frequency can be inferred from changes in the first inductor *L_{tube}* or the first capacitor *C_{tube}*.

The resonant frequency of the system (*ω*) can be determined by overall system inductance *L* and the overall system capacitance *C* determined by the following expressions: $\begin{matrix}ω = \frac{1}{\sqrt{LC}} \\ \end{matrix}$ [5] $L = \frac{1}{\frac{1}{{L}_{tube}} + \frac{1}{{L}_{tank}}} and C = {C}_{tube} + {C}_{tank}$ [6]

As seen in the above equations where *Lₜₐₙₖ* and *Cₜₐₙₖ* are known and fixed by design, resonant frequency is influenced by both *L_{tube}* or *C_{tube}.* If *L_{tube}* is much larger than *C_{tube}* (for example, one or more orders of magnitude), then *L_{tube}* is therefore the dominant element in the tube model. A change in the measured resonant frequency can be associated with a change in the *L_{tube}* and *C_{tube}* can be ignored.

Because inductance has a relationship with permeability and water has a lower permeability than air, when the volume of condensate in the tube increases, *L_{tube}* will decrease. This will be reflected in the measured resonant frequency, which will correspondingly increase. The correct expression for *L_{tube}* (ignoring *C_{tube}*) is: ${L}_{tube} = \frac{{L}_{tank}}{\left({ω}^{2}{L}_{tank}{C}_{tank}\right) - 1}$ [7]

Since all parameters except *ω and L_{tube}* are fixed, if *L_{tube}* decreases, *ω* must increase.

Alternatively, if *C_{tube}* is much larger than *L_{tube}* (for example, one or more orders of magnitude), then *C_{tube}* is therefore the dominant element in the tube model. A change in the measured resonant frequency can be associated with a change in the *C_{tube}* and *L_{tube}* can be ignored.

Because capacitance has a relationship with permittivity and permittivity of water is much higher than air/bead material, *C_{tube}* will increase with moisture presence. This will be reflected in the measured resonant frequency, which will correspondingly decrease. Ignoring *L_{tube}, C_{tube}* can be approximated as: ${C}_{tube} = \frac{1}{{ω}^{2} {L}_{tank}} - {C}_{tank}$ [8]

In this case, only *ω and C_{tube}* will change, so the resonant frequency will decrease with increased tube capacitance.

In some implementations, the tube can be configured such that *C_{tube}* is much larger than the *L_{tube}.* In some implementations, the tube can be configured such that *L_{tube}* is much larger than the *C_{tube}.*

Connecting the resonant tank in parallel with any of the embedded tube wires to measure the tube inductance to contribute to the overall inductance of the LC tank takes advantage of the intrinsic inductances of an elongated wire coil and water, and specifically how their presence will affect the resonant frequency of the resonant tank when it is actively excited by an exciter module 615.

By measuring the resonant frequency of the resonant tank, where the inductance comprises a known nominal value of *Lₜₐₙₖ* and an unknown *L_{tube}* or *C_{tube}* (whichever was configured to be much larger) and a known nominal value of *Cₜₐₙₖ*, the controller of a device performing humidification can determine an approximate value of *L_{tube}* or *C_{tube}* which varies due to the presence of condensation in the tube. Thus, the value of *L_{tube}* or *C_{tube}* and/or how *L_{tube}* or *C_{tube}* changes over time can provide an indicator of condensation presence and/or the quantity of condensate in the tube.

Either or both the first inductor *L_{tube}* 603 and with a first capacitor *C_{tube}* 602 can be a combination of elements to provide the necessary inductance and/or capacitance for the first component 601. In some implementations either or both the second inductor *Lₜₐₙₖ* 611 and the second capacitor *Cₜₐₙₖ* 613 could be a combination of elements to provide the necessary inductance and/or capacitance for the second component 610.

In some implementations, the exciter module 615 (for example, a sensor) is not a part of the second component 610, but instead the exciter module 615 is a part of the first component 601. In other implementations, the exciter module 615 is optional and may not be a part of either the second component 610 or the first component 601.

In some implementations, the LC circuit 600 is instead an RLC circuit.

In some implementations, an alternative device or circuit for measuring the resonant frequency may be employed instead of either the exciter module 615, the controller 617, or the combination thereof. Example alternatives include any of the following or similar devices and circuits, or combination thereof: digital signal processors and analog operational amplifier circuits.

Some features of the moisture (for example, condensate) detection/sensing technologies disclosed herein may be implemented using alternative ways for detecting and monitoring moisture and/or humidity.

Although the moisture detection/sensing technologies have been described mainly with respect to the capacitance-based sensor signal, other sensor signals can be used, for example, changes in inductance, resistance, RF signal attenuation, and thermal conductivity, as described above. There may be some differences in the implementation of moisture detection depending on the sensor signal used. For example, an inductance-based approach still detects whether the temperature of the flow of gases is equal to or less than a dew point temperature by measuring changes in the resonant frequency of an LC or RLC circuit, which is dependent on the inductance. In this case, the system hardware and the detection criteria (implemented in software/firmware) may be similar to the capacitance-based approach, albeit with appropriate differences. Such differences may include different threshold values that are suited for inductance changes. As another example, if an RF signal attenuation approach is used, the moisture detection method may be more different from the capacitance-based approach, in part because such an approach requires active transmission of RF signals to function, and the relationship(s) between RF signal propagation qualities and moisture presence are different from those of electrical reactance (for example, capacitance and inductance).

### Tube Type Detection

In addition to moisture detection, the capacitance measurements disclosed herein can also be utilized to detect the type of tubes or conduits in the humidifier system. For example, the type of inspiratory tube used may be detected based on the capacitance measurements. Each tube may include two parallel wires, which may be heater wires or heater wire coils, or sensing wires. An example formula for the approximate capacitance between the two parallel wires or wire coils within the tube is shown below: $C = \left[\frac{{ε}_{r} {ε}_{0} π}{ln \left(\frac{2 s}{d}\right)}\right] D$

Where *C* is the capacitance between two parallel wires; *D* is the length of the individual wires within the tube; *s* is the distance between the centres of the two parallel wires; *d* is the diameter of the individual wires; *εᵣ* is the relative permittivity of the space surrounding the wire, and; *ε*₀ is the permittivity of free space. The above formula may approximate the capacitance between the two parallel wires when the diameter of both parallel wires is equal (d1=d2=d) and the distance between the two conductors is greater or far greater than their diameters (d<<s).

The capacitance values of the tube may be varied by varying any of the parameters in the formula shown above. In some examples, the distance between the two parallel heater wire coils may be varied. This would result in a change in the distance between the centres of the two parallel wires, *s*. In some examples, the length of two parallel heater wire coils may be varied. This may vary *D,* the length of the individual wires within the tube. In some examples, the dielectric material between two conductors may be adjusted. This may be done by using different materials for each tube type and/or by removing or adding dielectric materials in between the sensing wires in the tube. This may vary *εᵣ*, the relative permittivity of the space surrounding the wires. In some examples, the diameter of two parallel heater wires may be adjusted. This may vary *d*, the diameter of the individual wires.

FIG. 43 illustrates a schematic of a process to determine the tube type within the humidifier system. At block 4310, a controller of the system (for example, a controller of a humidifier) may measure the capacitance value of the tube. At block 4320, the controller may map the measured capacitance value to the tube type. The controller of the system may include a memory device that stores a look-up table or a database that allows the matching of the measured capacitance value to the tube type or the like. At block 4330, the controller may adjust control of the tube (for example, control of power to the heater wires or the set point of a sensor in the tube) and/or the heater base (or cartridge) (for example, adjust the power of the heater plate or the set points of sensors in the heater base or cartridge) based on the detected tube type. Optionally, the system may, before or after adjusting the control based on the tube type, query a user of the system as to the desired therapy or control mode (based on, e.g., patient type or therapy type or both) or system parameter settings (such as set points for temperature or flow sensors and the like) to confirm or approve the adjusted control. This may be accomplished via any suitable user interface, display, and/or input device as are known in the art and described herein.

Patient types may include, for example, infant or neonatal patients, pediatric patients, adult patients, or the like. Therapy types may include, for example, high flow gas therapy (generally greater than 15 liters/minute of gases or higher through an unsealed interface), non-invasive ventilation (generally through a sealed interface, such as a mask), invasive ventilation (where the patient's upper airway is bypassed), closed surgery (for laparoscopic or keyhole surgical procedures), open surgery (for surgeries with open surgical sites), anesthesia (generally for an apnoeic patient under general anesthesia via an unsealed interface at gas flows above 30 liters/minute), or the like.

### Resistance-Based Detection

Water and condensation may also be more conductive than the bead material. As such, resistance may be measured at certain segments or locations of the tube wire to determine if there is condensation present.

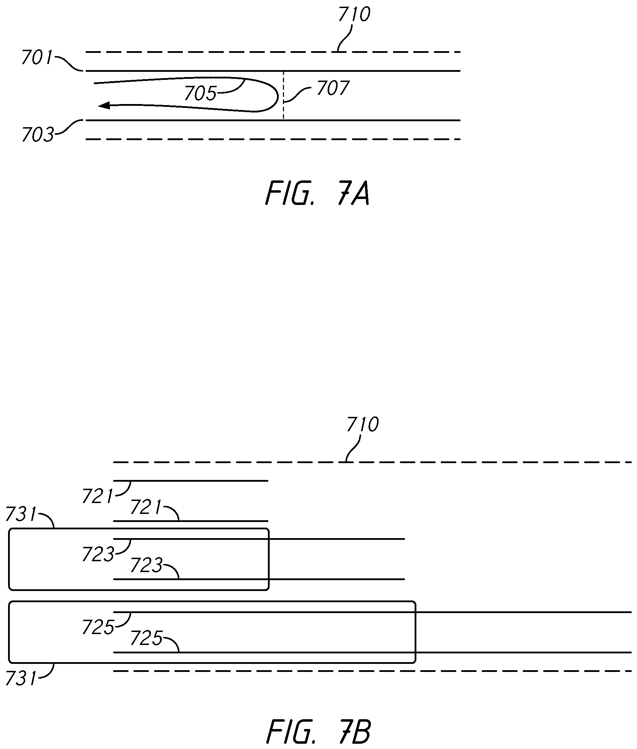

Fig. 7A is an example schematic of a resistance-based condensation detection system, which schematically illustrates a first detection wire 701 and a second detection wire 703 located in the bead 710. In some implementations, the first detection wire 701 and the second detection wire 703 may be same wire, or may be different wires. Moisture can be absorbed into the bead 710 which can create a low-resistance path 707 between the first detection wire 701 and the second detection wire 703, which would allow current 705 to flow between the first detection wire 701 and the second detection wire 703. Current 705 may be considered leakage current. The first detection wire 701 and the second detection wire 703 are connected to one or more sensors which can measure the resistance or current on the first detection wire 701 and/or the second detection wire 703.

In some implementations, the first detection wire 701 and the second detection wire 703 are running parallel to each other within the bead 710. In some implementations, the first detection wire 701 and the second detection wire 703 are running parallel to each other within the bead 710 such that the first detection wire 701 and the second detection wire 703 are equally distant from a center or centerline of the bead. In some implementations, the first detection wire 701 and the second detection wire 703 are running parallel to each other within the bead 710 such that the first detection wire 701 and the second detection wire 703 are not equally distant from a center or centerline of the bead.

In some implementations, the first detection wire 701 and the second detection wire 703 are not electrically connected to any other wire or component at one end of the bead 710, thus both detection wires would be open-circuit at one end of the bead so as not to allow current to flow along the first detection wire 701 and the second detection wire 703.

When more moisture is present, whether at one location or multiple locations, between the first detection wire 701 and the second detection wire 703 more current will be measured on the first detection wire 701 and/or the second detection wire 703 by the one or more sensors.

In some implementations, the first detection wire 701 and the second detection wire 703 can be a heater wire, thermistor wire, or any other wire in the bead 710.

Fig. 7B is an example schematic of a resistance-based condensation detection system, which schematically illustrates a first set of detection wires 721, a second set of detection wires 723, and a third set of detection wires 725 located in the bead 710. Moisture can be absorbed into the bead 710 which can create a low-resistance path between a set of detection wires, which would allow current to flow between the set of detection wires. For example, moisture may be absorbed into the bead 710 which creates a low-resistance path between the first set of detection wires 721 such that current flows between the first set of detection wires.

In some implementations, moisture can be absorbed into the bead 710 which can create a low-resistance path between one or more detection wires of different sets of detection wires, which would allow current to flow. For example, moisture may be absorbed into the bead 710 which creates a low-resistance path between one wire of the first set of detection wires 721 and another one wire of the second set of detection wires 723 such that current flows.

In some implementations, the first detection wire 701 and the second detection wire 703 are not electrically connected to any other wire or component at one end of the bead 710, thus both detection wires would be open-circuit at one end of the bead so as not to allow current to flow along the first detection wire 701 and the second detection wire 703.

In some implementations, the length of the first set of detection wires 721 is different from the length of the second set of detection wires 723. In some implementations, the length of the second set of detection wires 721 is different from the length of the third set of detection wires 723. For example, the first set of detection wires 721 may run one-third of the length of the bead 710, the second set of detection wires 723 may run two-thirds of the length of the bead 710, and the third set of detection wires 725 may run the full length of the bead 710. Any set of detection wires can be any length along the bead 710 or a portion of the bead 710.

In some implementations, the length of one wire of the first set of detection wires 721 is different from the length of another one wire of the first set of detection wires 721. For example, one wire of first set of detection wires 721 may run one-third of the length of the bead 710 while the another one wire of the first set of detection wires 721 may run two-thirds of the length of the bead 710. Any set of detection wires can be any length along the portion. Any wire of a set of detection wires can be any length along the bead 710 or a portion of the bead 710.

In some implementations, a longer set of detection wires may be insulated along their lengths for the length of a shorter set of detection wires which would prevent or mitigate current from flowing between wires of different sets along the length of the shorter set of detection wires. For example, the second set of detection wires 723 has insulation 731 along the length of the second set of detection wires 723 for the length of the first set of detection wires 721, and the third set of detection wires 725 has insulation 731 along the length of the third set of detection wires 725 for the length of the second of detection wires 723. This insulation can be any form of moisture insulation. Examples of moisture insulation can include a membrane or layer of material in the bead 710 impermeable by water, or a membrane or layer of material in the bead 710 that absorbs moisture less than other portions of the bead 710.

In some implementations, there are more than three sets of detection wires located in the bead 710. In some implementations, there are less than three sets of detection wires located in the bead 710.

In some implementations, any detection wire of any combination of the first set of detection wires 721, the second set of detection wires 723, and the third set of detection wires 725 can be a heater wire, thermistor wire, or any other wire in the bead 710.

It is to be understood that there can be more or less than three sets of detection wires. It is also to be understood that the above descriptions of the first set of detection wires 721, the second set of detection wires 723, and the third set of detection wires 725 are interchangeable with any other set of detection wires.

### Short-Circuit Based Detection

Additionally, the water or condensation can cause an electrical short-circuit between a wire or multiple wires at some threshold. This may happen because the water or condensation may be more conductive than the bead material. Fig. 8A is an example schematic of a short-circuit based condensation detection system, which schematically illustrates one or more detection wires 801 which are exposed to the lumen 811 of the conduit through the tube inner wall 810, wherein the one or more detection wires 801 are electrically connected to a power source in the heater base or sensor cartridge and a measurement component. The one or more detection wires 801 may have one or more exposed portions 813 to the lumen 811 of the conduit. In some implementations, the exposed portions 813 protrude from the one or more detection wires 801 into the lumen 811 of the conduit. The one or more detection wires 801 could be spaced throughout the tube, either for the entire length of the tube or only a portion of the length of the tube. In some implementations, the one or more detection wires are spaced periodically. The one or more detection wires 801 could be electrically connected to a power source. In some implementations, the power source electrically connected to the one or more detection wires 801 is one or more discrete power sources.

The one or more exposed portions 813 of the one or more detection wires 801 can be used to detect condensation. For example, there may be one or more exposed portions 813 of the one or more detection wires 801 which normally provides a measured resistance of 100 MΩ. When one or more of the exposed portions 813 are shorted together by moisture (for example, at potential short locations 815), however, the measured resistance may drop to 90 MΩ or lower. Alternatively, the measured current could change to show a short-circuit could be formed by condensation. Potential short locations 815 could be between one or more exposed portions 813 of different detection wires 801 or between multiple exposed portions of the same detection wire 801. Such short locations 815 could be used to detect condensation along the length of the tube. In some implementations, the one or more exposed portions 813 of the one or more detection wires 801 can be configured to detect moisture in a cross section of the tube, for example by having the one or more exposed portions at an equal length or near equal length along the tube.

The one or more detection wires 801 may be used to precisely find where the condensation is located. For example, there may be drop in resistance along two detection wires 801. This could mean that there is condensation around and/or between one or more exposed portions 813 of both detection wires causing a short-circuit.

Alternatively, the one or more detection wire 801 can be one or a combination of a heater wire, thermistor wire, or any other wire in the tube.

Fig. 8B is an example schematic of a short-circuit based condensation detection system, which schematically illustrates a detection wire 821 in the bead 830, wherein the detection wire 821 is electrically connected to a power source in the heater base or sensor cartridge and a measurement component. The detection wire 821 may have one or more exposed portions 833 to the bead channel 831. In some implementations, the exposed portions 833 protrude from the detection wire 821 into the bead channel 831. The detection wire 821 could be run through the bead 830, either for the entire length of the tube or only a portion of the length of the tube. For example, there may be one or more exposed portions 833 of the detection wire 821 which may provide a measured resistance of 100 MΩ. When one or more of the exposed portions 833 are shorted together by moisture (for example, at potential short locations 835), however, the measured resistance may drop to 90 MΩ or lower. Potential short locations 835 could short one or more of the exposed portions 833 that are in series with one another or in parallel with one another. Alternatively, the measured current could increase.

Water or condensation is more conductive than air. Similarly, water or condensation may be more conductive than the bead material. This would allow water or condensation to partially or completely short the circuit of the detection wire 821 at the exposed portions 833, thereby reducing a measured resistance or increasing a measured current at the measurement component to detect the water or condensation.

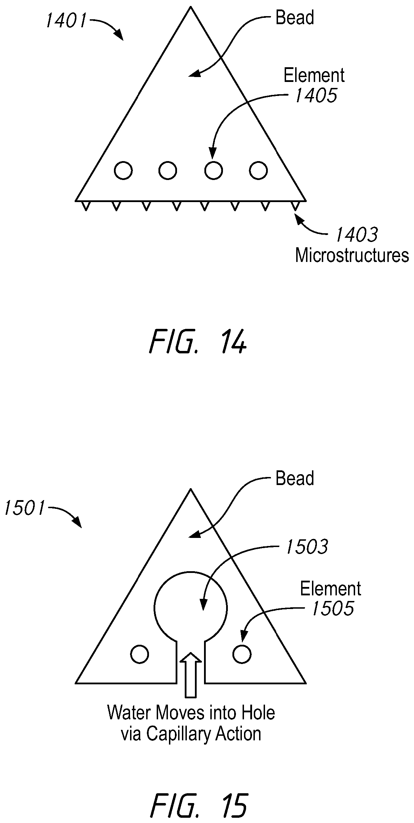

The bead 830 can have a condensation diversion channel (for example, the one or more openings 903 of Fig. 15 which will be described later. The segments of the detection wire 821 could be periodically exposed to the interior of the channel. In some implementations, the detection wire may not be a complete circuit (for example, open at the patient end). Thus, when moisture is diverted into the bead channel, the water may complete the circuit between exposed portions of the detection wire. This could be detected by measuring wire resistance, which could be very high in the absence of condensation in the bead channel, or current, which could be very low unless the circuit is complete.

In some implementations, the resistance-based condensation detection system may also be able to detect how much water or condensation is present by measuring the change in resistance or current at the measured point by the one or more detection wires 801, 821.

The resistance-based condensation detection system may be useful if the one or more detection points are located at known points in the tube where condensation tends to be quite high, for example at the tube mid-point which can be sagging leading to the pooling of condensate. The tube may be sagging due to arrangement of the humidifier and tube at the patients' bedside.

Alternatively, the detection wire 821 can be one or a combination of a heater wire, thermistor wire, or any other wire in the bead 830.

### Radio Frequency (RF) Attenuation Based Detection

Water and condensation are poor propagation mediums for high frequency signals. This attribute can be used to detect if condensation is present in the breathing tube.

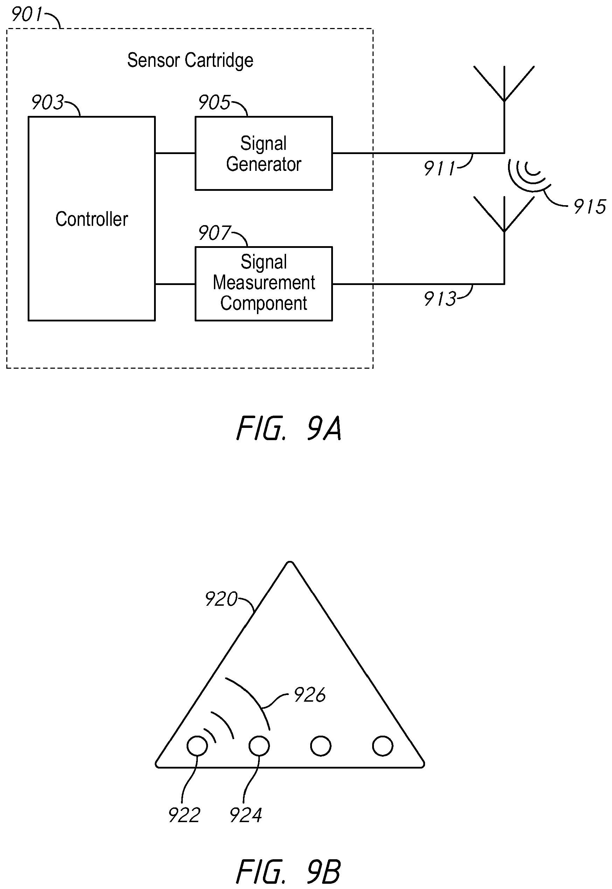

Fig. 9A is an example schematic of an RF attenuation-based condensation detection system, which schematically illustrates a sensor cartridge 901, a transmitter 911 (Tx), and a receiver 913 (Rx). The sensor cartridge 901 may have a controller 903, a signal generator 905 and a signal measurement component 907.

In the example schematic of Fig. 9A, the controller 903 is electrically connected to the signal generator 905 and the signal measurement component 907. The signal generator 905 is electrically connected to the transmitter 911. The signal measurement component 907 is electrically connected to the receiver 913.

The controller 903 can instruct the signal generator 905 to create a signal 915 to be transmitted by the transmitter 911. The signal 915 is then received by the receiver 913 and then measured at the signal measurement component 907. Once the signal 915 is measured, the signal measurement component 907 can communicate the measurement information of the signal 915 to the controller 903 to determine how to respond. When condensation is introduced to the transmission path between the transmitter 911 and the receiver 913, the condensation can lead to significantly increased signal attenuation of the signal 915 when received by the receiver 913. Attenuation is the reduction in signal magnitude or intensity when a signal is propagated through a medium. Thus, the received signal at the receiver 913 will be an attenuated version of the signal 915. The presence of water between the transmitter 911 and the receiver 913 can be detected by measuring the magnitude of the received signal. The magnitude of the received signal can also be used to determine how much water or condensation is present between the transmitter 911 and the receiver 913.

The frequency of the signal may encompass a broad spectrum of frequencies, namely any frequency where transmission through water or condensation would attenuate the signal. This could include, for example, a frequency band of 30 Hz - 300 GHz. In some implementations, the signal has a frequency in the 1-100 MHz band. In some implementations, the signal has a frequency of approximately 10 MHz. At approximately 10 MHz, the length of the antennas of the transmitter 911 and the receiver 913 may be one-quarter the signal wavelength. One-quarter wavelength antennas can be useful for resonance which may maximize the power of the signal 915 transmitted, and therefore the signal received. In some implementations, the resonant frequency of water (~2.45 GHz) is the frequency of the signal 915. Using the resonant frequency of water may yield the lowest signal-to-noise ratio at the receiver antenna, which may also increase sensitivity to condensation changes.

The signal generator 905 can be any suitable high frequency signal generator. Similarly, the signal measurement component 907 any suitable high frequency transducer such as an AM receiver, RF rectifier circuit or RF sampling ADC.

In some implementations, any combination of the controller 903, the signal generator 905, and the signal measurement component 907 could be located in the base (for example, the base 151 in Fig. 1B) or in a part of the breathing tube (for example, the breathing tube 159 in Fig. 1B).

Fig. 9B provides an example cross-section of a tube bead 920 with embedded wires including a heater wire 922 and a thermistor wire 924 in an RF attenuation-based condensation detection system. In this example, a radio-frequency signal is injected into the heater wire 922 by a signal generator (for example, the signal generator 905 of Fig. 9A), causing the heater wire 922 to act as a transmitter of a signal 926. The thermistor wire 924 acts as a receiver of the signal 926 and carries the signal to the signal measurement component (for example, the signal measurement component 907 of Fig. 9A).

In the example implementation of Fig. 9B, the heater wire 922 is adjacent to the thermistor wire 924. In some implementations, the heater wire 922 and the thermistor wire 924 are not adjacent wires.

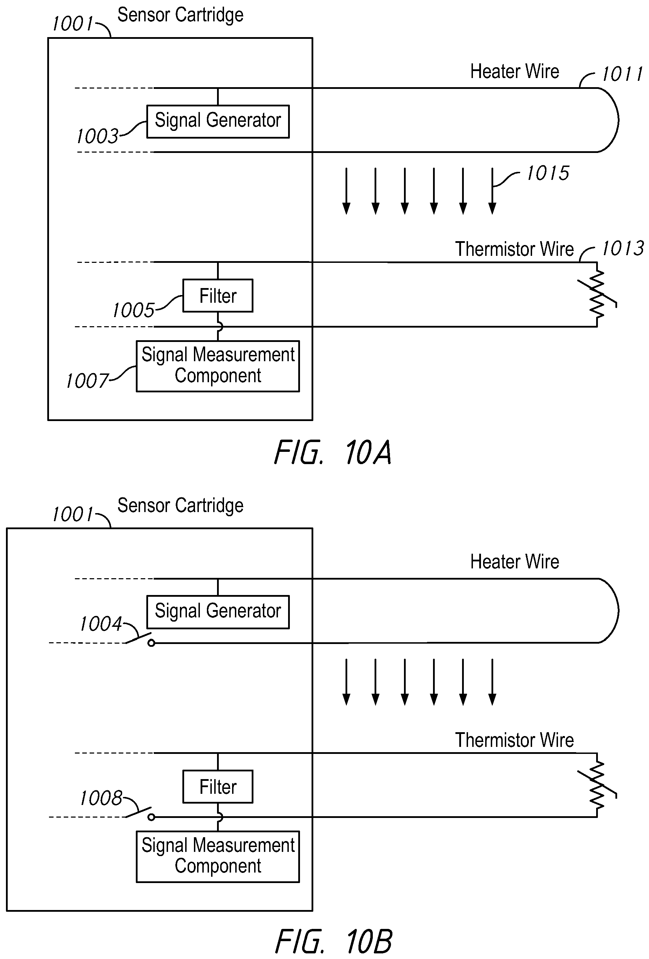

Fig. 10A is an example schematic of an RF attenuation-based condensation detection system which uses a heater wire 1011 as a transmitter (for example, the transmitter 911 of Fig. 9A) and a thermistor wire 1013 as a receiver (for example, the receiver 913 of Fig. 9A). Fig. 10A schematically illustrates a sensor cartridge 1001, a heater wire 1011, and a thermistor wire 1013. The sensor cartridge 1001 may have a signal generator 1003, a filter 1005 and a signal measurement component 1007.

In the example schematic of Fig. 10A, the signal generator 1003 is electrically connected to the heater wire 1011. The thermistor wire is electrically connected to the filter 1005 which is in turn electrically connected to the signal measurement component 1007. The signal generator 1003, the filter 1005, and the signal measurement component 1007 are electrically connected to other components in the sensor cartridge 1001 or other components of the humidifier.

The signal generator 1003 creates a signal 1015 to be transmitted by the heater wire 1011. The signal 1015 is then received by the thermistor wire 1013. The heater wire 1011 is used as a transmitter and the thermistor wire 1013 is used as a receiver as described with relation to Fig. 9A. The received signal may then be filtered by filter 1005 to eliminate extraneous frequencies other than the frequency or frequencies generated by the signal generator 1003 such that only an attenuated version of signal 1015 is measured at the signal measurement component 1007. Example filters can be high pass or bandpass filters. The filter can also be configured to filter out the mains frequency, wherein the mains frequency refers to the frequency of mains power. Mains power is power coming from a wall/plug socket or power grid.

As described with relation to Fig. 9A, the magnitude of the received signal can be used to detect the presence of water between the heater wire 1011 and the thermistor wire 1013 or determine the amount of water or condensation present therebetween.

The heater wire 1011 and the thermistor wire 1013 can be separate wires from other wires used to carry signals in the bead. In some implementations, the heater wire 1011 and the thermistor wire 1013 are not separate wires used to carry signals in the bead.

In some implementations, there is no filter 1005. As such, the signal measurement component 1007 measures whatever signal is on the thermistor wire 1013. In some implementations, there may be additional filters or alternative filter configurations to separate heater wire signals from other RF signals and/or separate thermistor wire signals from other RF signals. This allows for differentiation between condensation-detection signals on the heater wire and/or the thermistor wire from other RF signals, such as signals used for purposes other than the detection of condensation.

In Fig. 10A, the heater wire 1011 and thermistor wire 1013 are both electrically connected to the sensor cartridge 1001 and/or the humidifier. As such, both the heater wire 1011 and the thermistor wire 1013 form loop antennas. This implementation may be suitable for transmission and reception of a signal 1015 with a lower frequency, as loop antennas can be effective at frequencies below 30 MHz.

The signal generator 1003 can be a discrete signal generator from signal generators for other signals over the heater wire 1011 or thermistor wire 1013.

Fig. 10B is an example schematic of an RF attenuation-based condensation detection system similar to Fig. 10A. Again, the heater wire 1011 is used as the transmitter and the thermistor wire 1013 is used as the receiver. However, in the example implementation of Fig. 10B switches 1004, 1008 are employed to disconnect one end of each of the heater wire 1011 and the thermistor wire 1013 from the humidifier or other parts of the sensor cartridge 1001. By disconnecting one end of each of the heater wire 1011 and the thermistor wire 1013, monopole antennas are formed. In some implementations, the monopole antennas formed by the heater wire 1011 and the thermistor wire 1013 are quarter-wave monopole antennas. The switches 1004, 1008 are located in any one of the following: a heater base, a sensor cartridge, the conduit, an external component, or an intermediate connector. A neo-natal bubble tube with zone heating is an example intermediate connector.

With reference to Fig. 9B, the heater wire 1011 is adjacent to the thermistor wire 1013. In some implementations, the heater wire 1011 and the thermistor wire 1013 are not adjacent wires.

In some implementations, any combination of the switches 1004, 1008, the signal generator 1003, the filter 1005, and the signal measurement component 1007 are located in the base (for example, the heater base 151 in Fig. 1B) or another part of the humidifier (for example, the cartridge 155 in Fig. 1B). In some implementations, any combination of the switches 1004, 1008, the signal generator 1003, the filter 1005, and the signal measurement component 1007 are located in the breathing tube (for example, at an intermediate point of the inspiratory tube 159 in Fig. 1B). In some implementations, any combination of the switches 1004, 1008, the signal generator 1003, the filter 1005, and the signal measurement component 1007 are located in a cartridge (for example, the cartridge 155 in Fig. 1B) attachable to the base or tube. In some implementations, any combination of the switches 1004, 1008, the signal generator 1003, the filter 1005, and the signal measurement component 1007 are located between both the base and the tube.

### Thermally-Based Detection

Certain media or characteristics of media affect the efficiency and/or magnitude of heat transfer. Water has a thermal conductivity that may differ from the thermal conductivity of the bead material. Similarly, water has a specific heat capacity that may differ from the specific heat capacity of the bead material. The difference in thermal conductivity and/or the difference in specific heat capacity of water and the bead material can be used to detect if condensation is present in the breathing tube. When condensation is present around and/or in the bead, there may be improved thermal conduction between a pair of embedded tube wires.

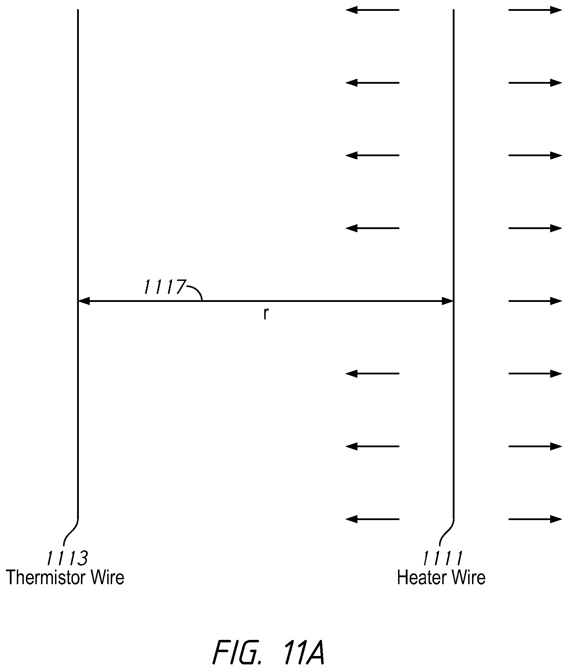

Fig. 11A is an example diagram of thermal conductivity between two wires. Fig. 11A illustrates a thermistor wire 1113, a heater wire 1111, and a radius r 1117 which represents the distance between the heater wire 1111 and the thermistor wire 1113.

The relationship between thermal conductivity and rate of change of temperature where a linear heat source is present is represented below $T \left(r, t\right) = \frac{\dot{q}}{4 πλ} ln \left[\frac{4 at}{{r}^{2}}-γ\right]$ [9]

Temperature *T* is a function of radius r and time *t* where *λ* is thermal conductivity, *q̇* is the specific heat output of a linear heat source, *γ* is Euler's constant and *a* is the thermal diffusivity.

Given a fixed radius r , temperature T may be measured at two discrete points in time and the temperature change can be readily computed without needing knowledge of thermal diffusivity or the actual radius: $Δ T = T \left(r, {t}_{2}\right) - T \left(r, {t}_{1}\right)$ [10]

Rearranging for thermal conductivity: $λ = \frac{\dot{q}}{4 π} \frac{ln \left(\frac{{t}_{2}}{{t}_{1}}\right)}{Δ T}$ [11]

This step requires an assumption of zero convective heat losses, which will be accurate provided measurements are taken within a linear region. There is a region where the change in temperature T has a linear relationship to the change in ln(t). This linear region can be time-dependent such that there is a time window after the initial temperature evolution, but prior to plateauing, where there is an approximately linear relationship between T and ln(t).

In some implementations, the condensation detection system uses thermal conductivity to detect the presence and/or amount of condensation present. For example, condensation can be detected by applying a step change in power to the heater wire 1111 and measuring the subsequent temperature rise in the thermistor wire 1113 or the heater wire 1111 itself.

Water can be detected in the bead, on the bead or somewhere else in the tube. Water in the bead will change the thermal conductivity the most, but thermal conductivity changes can be detected if water is anywhere in the tube.

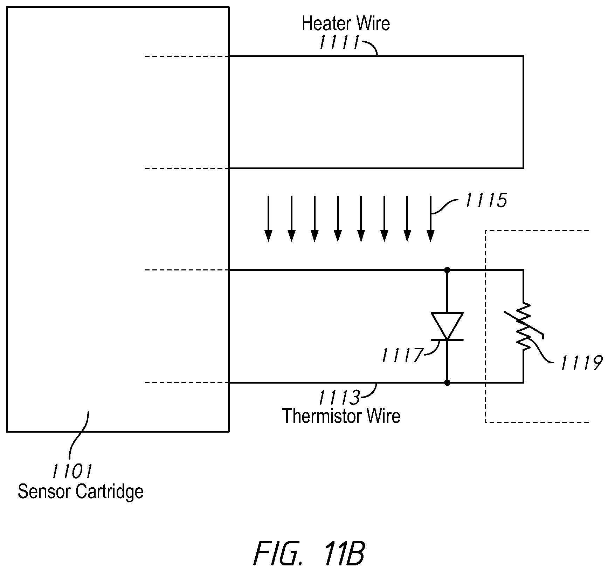

Fig. 11B is an example schematic of a thermal conductivity-based condensation detection system, which uses a heater wire 1111 as a heat source and a thermistor wire 1113. Fig. 11B schematically illustrates a sensor cartridge 1101, a heater wire 1111, and a thermistor wire 1113. The thermistor wire 1113 may include a bypass (shunt) diode 1117 and a thermistor 1119, which is bypassed by use of the bypass diode 1117.

In the example schematic of Fig. 11B, the sensor cartridge 1101 is electrically connected to both the heater wire 1111 and the thermistor wire 1113. In some implementations, one or both the heater wire 1111 and the thermistor wire 1113 are electrically connected to heater base or other components of the humidifier.

Condensation can be detected by applying a step change in power to the heater wire 1111 and measuring the subsequent temperature rise in the thermistor wire 1113 or the heater wire 1111 itself. In some implementations, the step change in power is for a fixed period of time. In some implementations, any change in the level of power may be applied whether negative or positive.

In some implementations, the temperature of the thermistor wire 1113 is measured. Here, the radius *r* between the linear heat source (heater wire 1111) and the body measured (thermistor wire 1113) is small but non-zero. Due to the linear proportional relationship between resistance and temperature of a conductor (both the heater wire 1111 and the thermistor wire 1113 can be conductors), temperature can be readily inferred by measuring wire resistance. However, it may be desirable to eliminate the contribution to resistance of the thermistor 1119 to the measured resistance of the thermistor wire 1113 via a bypass, for example using a parallel-connected diode 1117 at a known location along the tube, for example, adjacent to the thermistor 1119. By eliminating the resistance of the thermistor 1119, the resistance of the wire is the dominant contributor to the resistance measured. In some implementations, the bypass is proximate to the thermistor 1119 at the patient end of the tube.

There may be a delay in the response of the temperature of the thermistor wire 1113 to heat from the heater wire 1111. This delay in heating of the thermistor wire 1113 is due to the need for heat to be conducted away from the heater wire 1111 to the thermistor wire 1113 before observing heating at the thermistor wire 1113. This delay may overcome transient inconsistencies in temperature that can be observed briefly after the step change in power on the heater wire 1111.

In some implementations, the temperature of the heater wire 1111 itself is measured. Although the same principles described previously are applicable to this implementation, it is instead contemplated that the radius *r* (in the temperature expression [9]) is zero. When a step change in power is applied to the heater wire 1111, the thermal conductivity of the bead material will affect the degree of heat conduction or dissipation away from heater wire 1111. For example, the rate of heat conduction away from the heater wire 1111, or the dissipation of heat from the heater wire 1111, changes in relation to the amount of condensation present around the heater wire 1111. Therefore, the linear proportional relationship between resistance and temperature may again be used to infer temperature by measuring wire resistance. This implementation may be advantageous because it may not need additional hardware components to implement a condensation-detection system.

In some implementations, the thermistor wire 1113 may be energized and the temperature of the heater wire 1111 measured. In some implementations, multiple wires (for example, heater wires and/or thermistor wires) may be energized and the temperature of one wire is measured. In some implementations, one heater wire 1111 or thermistor wire 1113 may be energized and the temperatures of multiple wires (for example, heater wires and/or thermistor wires) is measured.

In some implementations, the heater wire 1111 and the thermistor wire 1113 are adjacent in the bead wiring arrangement. In some implementations, the heater wire 1111 and the thermistor wire 1113 are not adjacent in the bead wiring arrangement.

In some implementations, the step change is a step change in AC power to energize the heater wire 1111. In some implementations, the step change is in DC power. In some implementations, additional circuitry is required to applied to rectify the AC power to DC prior to energizing the heater wire 1111. In some implementations, multiple power signals are interleaved. For example, control power for powering circuits electrically connected to the wire and the step change in power are interleaved on the wire.

In some implementations, the temperature of the thermistor wire 1113 or the heater wire 1111 may be measured by the use of a device, for example thermocouples. In some implementations, the temperature of the thermistor wire 1113 or the heater wire 1111 may be measured by other known ways of measuring the temperature of a wire.

### Generally Applicable to All Methods of Condensation Detection

It is to be understood that although some wires may be described above as heating, sensing, heater, or thermistor wires, these wires are able to do any combination of such functions.

It is to be understood that these condensation detection systems are not mutually exclusive. Any of the condensation detection systems, including implementations of any of the condensation detection systems, may be used in combination with each other, for example to provide error-checking or redundancy. Any of the outputs of the condensation detection systems may be weighted relative to each other. In some implementations, certain condensation detection systems may be weighed more heavily due to increased accuracy or precision.

### Signal Generator

A signal generator can be used to generate a voltage signal. Any method of generating a signal may be used, including a pulse from an I/O pin of a controller. An exciter module can be a signal generator. Similarly, many of the above condensation detection methods may require multiple discrete signal generators or signal generators capable of producing multiple discrete signals.

In some implementations, a 5V square wave signal can be generated from the signal generator. One example signal generator is a pulse width modulated (PWM) heater wire current that generates a controllable periodic "pulse" to heat the breathing tube. In one such configuration, the time constant is shorter than the PWM period so that the capacitance fully discharges between consecutive "on" periods of the PWM signal. Alternatively, the duty cycle of the PWM heater wire current may be selectively or periodically set to 0% for two or more consecutive periods of the PWM heater wire current to fully discharge the capacitor and/or 100% for two or more consecutive periods to fully charge the capacitance and allow for measurement of the time constant as described in further detail below.

### Conduit Model

As discussed above, the conduit system can include an element resistance and inherent capacitance C but the element resistance can be ignored if resistor R is significantly larger than the element resistance.

### Detection Circuitry

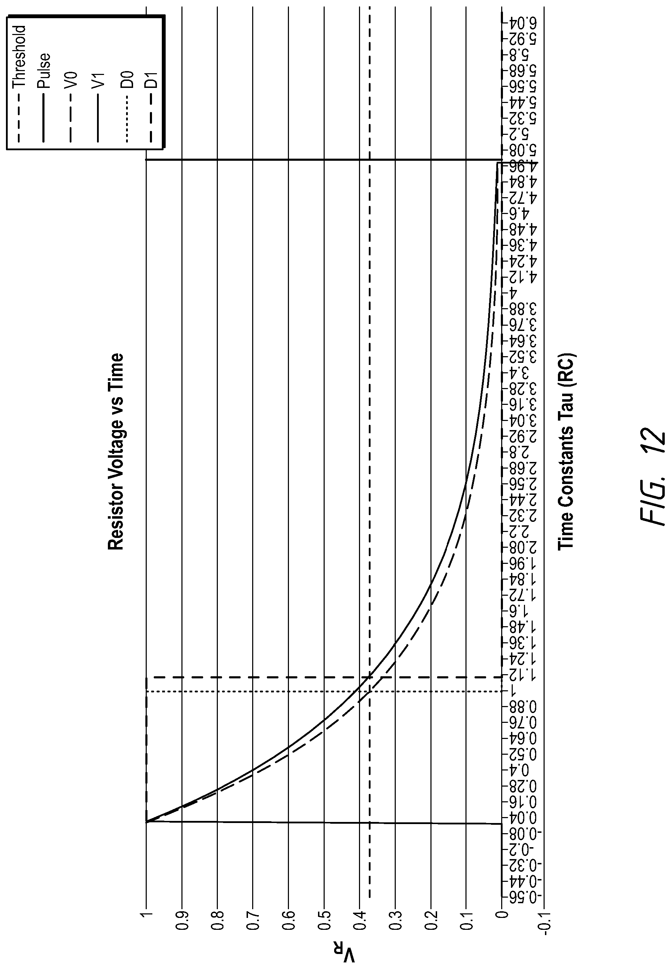

The detection circuitry can comprise a comparator. The comparator compares the voltage across the resistor R with a threshold voltage. The comparator may be configured to output a binary 1 (HIGH) if the voltage across the resistor is greater than the threshold voltage, and a binary 0 (LOW) if the voltage across the resistor is lower than the threshold voltage. The threshold voltage may correspond to *e⁻¹* or 36.8% of the voltage pulse applied to the element. However, any suitable alternative threshold voltage may be used. Fig. 12 compares the comparator output for a tube that is 'dry' and 'wet'. As illustrated in Fig. 12, the X axis and Y axis units have been normalized for ease of understanding, however, actual measurements may vary.

In Fig. 12, "Pulse" represents the signal generator voltage - for example, 5V square wave or pulse, which has been normalized in the diagram. "Threshold" represents a threshold voltage. The threshold voltage may be any value - for example, 36.8% of the signal generator voltage. "V0" and "V1" represent the voltage across the resistor R for a dry and wet tube, respectively, and "D0" and "D1" represent the detector output accordingly.

As illustrated in Fig. 12, D1 corresponds to a larger time constant than D0. The larger time constant is caused by differences in "dry" vs "wet" conditions within the tube, which causes differences in the absolute permittivity between the elements. The capacitor C is initially discharged. That is, the voltage across the capacitor C, *V_{C}*, is 0V. When the signal generator changes from 0V to +5V during the rise of the positive cycle, the capacitor acts as a short-circuit and so the voltage across the capacitor is 0V. That is, the first and second terminals of the capacitor are both at 5V. From *V_{R}* = *Vᵢₙ* - *V_{C}* we know that the instantaneous voltage across resistor R will therefore be the same as the signal generator output. The resultant current flowing through R dictates the rate at which capacitor C then charges. As capacitor C charges and the voltage across it increases, the voltage across the resistor R begins to decay. When the capacitor becomes fully charged then the voltage across resistor R becomes 0V. The reverse of this occurs when the signal generator voltage changes from +5V to 0V when the capacitor discharges. In this instance the resistor will have a negative voltage across it. The point at which the voltage crosses the threshold line again will cause the comparator output to be LOW. Thus, a pulse can be generated which represents the time constant of the tube. The duration of the time constant can be measured via a GPIO pin of a controller. Any other suitable method of measuring an interval or duration, for example an interval or duration of the generated pulse, may be used.

It will be appreciated that the threshold point (also referred to herein as a pre-determined threshold) may be selectable by any suitable means, including by the controller, based on any number of operational conditions including, for example and not to be limited to, the therapy type, patient type, ambient conditions, conduit type, length, and/or construction.

It should also be seen that multiple thresholds can be implemented such that the controller can respond differently depending on the degree of condensate in the tube, or to provide hysteresis between detection of 'wet' or 'dry' conditions. Additionally, the condensate level can be detected as a continuum such that the control algorithm could have a variable output dependent on the level of condensate and/or other inputs, rather than simply switching mode at a predefined threshold.

### Condensation Detection Process

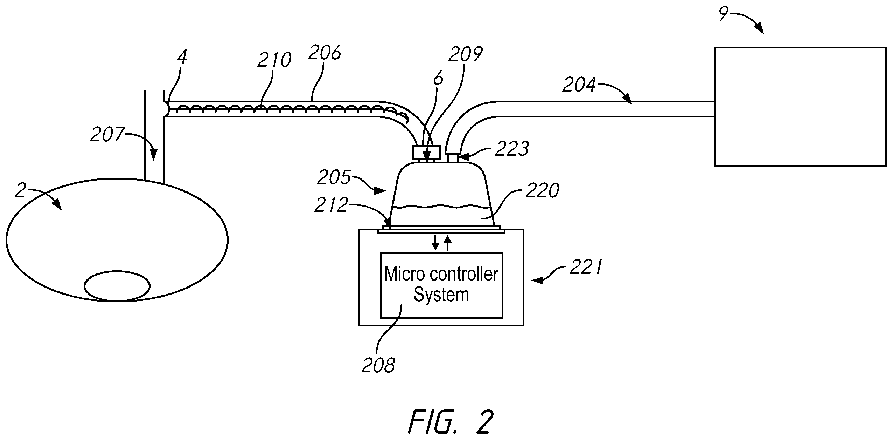

The respiratory assistance apparatus can switch between moisture detection and normal operational modes. When the respiratory assistance apparatus is in normal operational mode, it conducts normal operations as described in relation to Figure 1 - 2. When the respiratory assistance apparatus is in moisture detection mode, the respiratory assistance apparatus uses one or more of the above described methods of detecting moisture. In some implementations, there may be multiple moisture detection modes and/or multiple normal operational modes, for example a primary detection mode and a secondary detection mode. An example primary detection mode may involve detecting the presence of condensation while an example secondary detection may involve detecting the amount of condensation. These multiple moisture detection modes and/or multiple normal operational modes may carry out one or more moisture detection and/or normal operational operations.

In some implementations, the respiratory assistance apparatus switches periodically between moisture detection and normal operational modes. In some implementations, the respiratory assistance apparatus starts in moisture detection mode prior to entering normal operational mode. In some implementations, the respiratory assistance apparatus can be manually switched between moisture detection and normal operational modes. In some implementations, the existence of certain conditions may switch the respiratory assistance apparatus from certain moisture detection and/or normal operational modes to other moisture detection and/or normal operational modes.

Alternatively, the respiratory assistance apparatus can switch between moisture detection and normal operational operations. In some implementations, moisture detection is done in conjunction with or in parallel with normal operational mode. This could involve interleaving normal operating modes/operations and condensation detection modes/operations. For example, the respiratory assistance apparatus may use time-division multiplexing to interleave normal operating modes/operations and condensation detection modes/operations.

It should be known that the respiratory assistance apparatus can enter into other modes as well. The respiratory assistance apparatus can enter into other modes which may detect different conditions other than condensation or operate other functions of the respiratory assistance apparatus, for example detecting water-out, reverse flow, or other functions as described herein.

In some implementations, exiting moisture detection mode could be based on a pre-determined period of time that condensation is not detected, the meeting or non-meeting of certain thresholds, or sufficient successful tests for lack of condensation are passed.

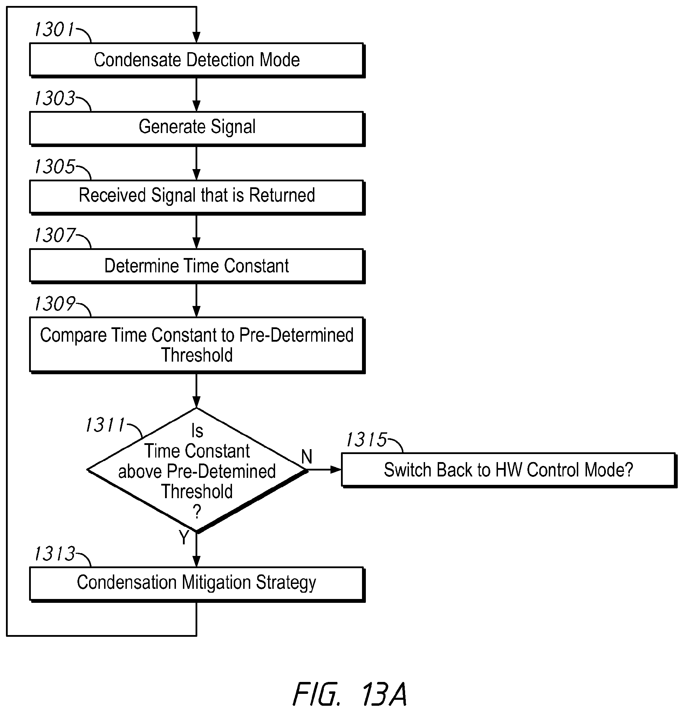

Fig. 13A illustrates a flow chart example algorithm to detect a presence of condensate using the inherent capacitance of at least two conductive elements in the conduit. The condensate detection may function independently of, or together with, normal operation. For example, normal operation may pause for a period of time while condensation detection is being performed. In another example, condensate detection may act in conjunction with normal operation, operating at a different frequency than normal operation.

As shown in Fig. 13A, the condensate detection mode operates by generating a signal 1303, receiving the signal that is returned 1305 in response to the generated signal, determining a time constant 1307, comparing the time constant to a pre-determined threshold 1309, and determining if the time constant is above the pre-determined threshold 1311. If the time constant is above the pre-determined threshold at 1311, then the system determines that an undesirable amount of condensate is present and condensation mitigation strategies are implemented 1313. If at 1311, the time constant is not above a pre-determined threshold, it is determined that an undesirable amount of condensate is not present in the tube and the system switches back to normal heater wire or sensor control mode at 1315. After implementing condensation mitigation strategies, the system switches back to normal heater wire or sensor control mode at 1315.

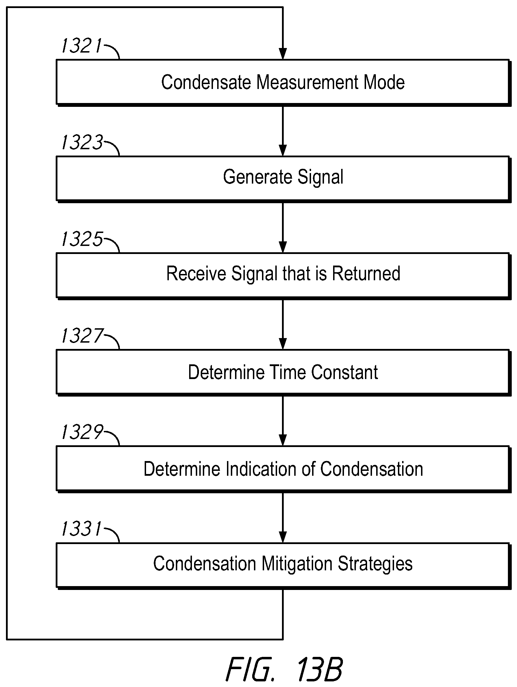

Alternatively, instead of a single pre-determined time constant threshold, multiple thresholds can be used with varying granularity (e.g., "dry," "low," "medium," and "high," or a scale of 1-10) to quantify a condensation condition or measure an amount of condensate. Fig. 13B illustrates a flow chart example algorithm to detect an indication of condensation. The indication can be a relative degree of condensation (for example, low, medium or high); a trend of condensation (for example, increasing or decreasing); an amount of condensation (for example 5ml of condensate); a percentage of the conduit or a portion of conduit that is experiencing condensation; or any combination of the above.

As shown in Fig. 13B, the condensate measurement mode operates by generating a signal 1323, receiving the signal that is returned 1325, determining a time constant 1427, determining an indication of condensation 1329, and implementing condensation mitigation strategies 1331.

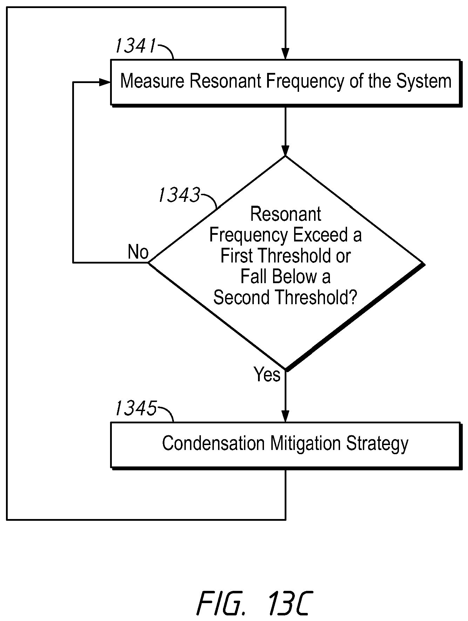

As shown in Fig. 13C, an example condensate detection mode for inductance-based detection which operates by measuring the resonant frequency 1341 of the system (for example, the inductance detection system 600 of Fig. 6), measuring the resonant frequency 1343 of the system, and determining whether the resonant frequency of the breathing tube exceeds a first threshold or falls below a second threshold 1343. If the resonant frequency exceeds the first threshold or falls below the second threshold at 1343, then the system determines that an undesirable amount of condensate is present and condensation mitigation strategies are implemented 1345 (for example setting a condensation alarm). If at 1345, the resonant frequency does not exceed the first threshold or fall below the second threshold, it is determined that an undesirable amount of condensate is not present in the tube and the system measures the resonant frequency 1341 again after a predetermined interval of time. After implementing condensation mitigation strategies, the system measures the resonant frequency 1341 again after a predetermined interval of time.

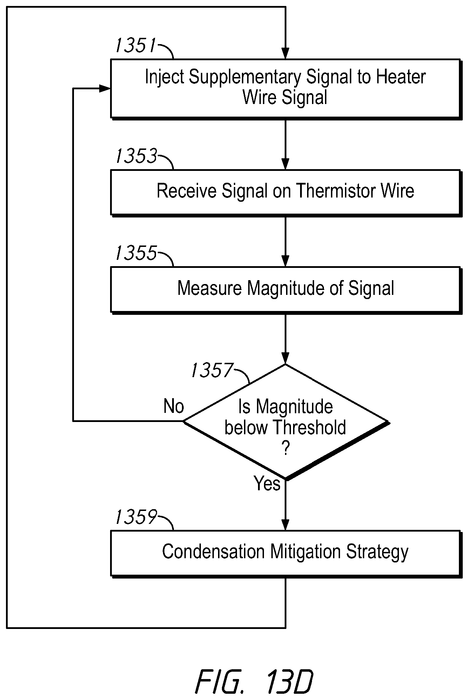

As shown in Fig. 13D, an example condensate detection mode for signal attenuation-based detection which operates by injecting a supplementary signal into a heater wire or heater wire signal 1351, receiving the signal on a thermistor wire 1353, measuring the magnitude of the signal received on the thermistor wire 1355, and determining if the magnitude is below a threshold 1357. If the magnitude of the received signal is below the pre-determined threshold at 1357, then the system determines that an undesirable amount of condensate is present and condensation mitigation strategies are implemented 1359 (for example setting a condensation alarm). If at 1357, the magnitude of the received signal is not below a pre-determined threshold, it is determined that an undesirable amount of condensate is not present in the tube and the system injects a supplementary signal into a heater wire or heater wire signal 1351 again after a predetermined interval of time. After implementing condensation mitigation strategies, the system injects a supplementary signal into a heater wire or heater wire signal 1351 again after a predetermined interval of time.

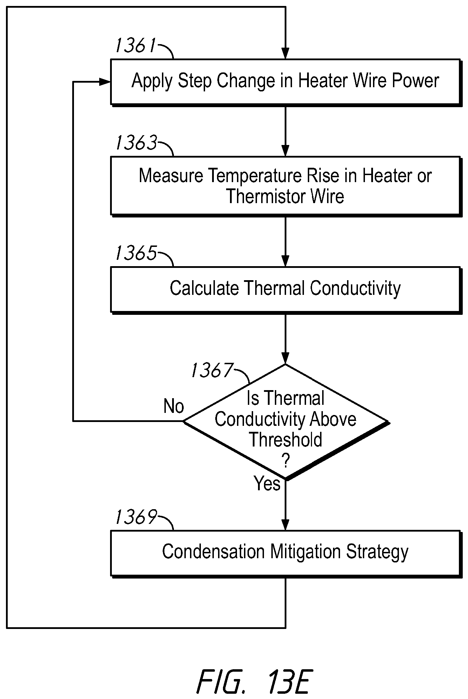

As shown in Fig. 13E, an example condensate detection mode for thermally-based detection which operates by applying a step change in a heater wire power 1361, measuring the temperature rise in the heater wire or a thermistor wire 1363, calculating the thermal conductivity of either the heater wire or the thermistor wire 1365, and determining whether the thermal conductivity of the heater wire or the thermistor wire is above a threshold 1367. If the thermal conductivity of the heater wire or the thermistor wire is above the pre-determined threshold at 1367, then the system determines that an undesirable amount of condensate is present and condensation mitigation strategies are implemented 1369 (for example setting a condensation alarm). If at 1367, the thermal conductivity of the heater wire or the thermistor wire is not above a pre-determined threshold, it is determined that an undesirable amount of condensate is not present in the tube and the system applies a step change in the heater wire power 1361 again after a predetermined interval of time. After implementing condensation mitigation strategies, the system applies a step change in the heater wire power 1361 again after a predetermined interval of time.

Note that for all methods disclosed, the condensate detection threshold may not be a strict magnitude threshold and may instead be a rate of change, sustained rate of change, sustained increase or decrease in magnitude, trend, or other statistical measure threshold. Multiple thresholds may be used - e.g. a soft threshold and a hard threshold. Thresholds may be upper or lower thresholds, and may be used to set an allowable range for the humidifier to operate within. Additionally, in response to the threshold being exceeded, any alternative response may be considered, not exclusively setting an alarm, such as the condensation mitigation strategies discussed below. For example, auditory, visual and/or audio-visual alerts, warnings or prompts may be triggered. Even further, the trigger may be used as feedback in a condensation mitigation strategy. Note that for all methods disclosed, if the method describes an upper threshold (for example, the method describes exceeding a threshold) the method may also use a lower threshold. Likewise, if the method describes a lower threshold (for example, the method describes falling below a threshold) the method may also use an upper threshold. Additionally, the predetermined intervals of time described in the methods above can be variable based on present conditions and/or user input.

Any of the methods disclosed may not have purely binary output indicating condensation presence. Using experimentally-determined values or known relationships stored in memory, any of the methods may be used to quantify the amount of condensation present (e.g. 10 mL, 15 mL, 20 mL, etc.). This memory can be located in the heater base, an external accessory, the sensor cartridge, the conduit, or an intermediate connector.

Alternatively, the detection of condensation is based on a difference on the previous and current duration of the time constant signal, not the absolute value of the duration. An increasing time constant is indicative of increasing condensate in the tube. A decreasing time constant is indicative of a drying tube. The time constant can also be correlated with any indication of condensation as discussed above. This approach has the advantage that it is unaffected by variations in the capacitance over time or from one tube to the next, without need for a calibration procedure as described below.

### Calibration

It should be understood that there will be inherent variability in the dry capacitance and time constant from one tube to another. Such variations may be due to different tube configurations or, for a given tube configuration, ambient conditions, component or manufacturing tolerances, or supplier/material changes, for example. As such, each tube can optionally include an indicator (such as a resistor value, capacitance, resonant frequency, or EPROM) which allows a gases supply system to identify the model of tube and/or the specific tube, and/or provides capacitance or time constant threshold information for that particular tube or tube model to the gases supply system. The gases supply system, for example the humidifier, may be configured to calibrate itself for use with a connected tube. The capacitance or time constant threshold information may be individually measured and programmed into an EPROM upon manufacture of the tube, or the humidifier or EPROM may be programmed with a nominal value (e.g. average or typical value) appropriate for that tube model. Alternatively, the gases supply system, such as the humidifier, may be programmed to perform a calibration routine (for example, initially measuring the time constant of a dry tube) while, or before, the humidifier warms up.

In some implementations, the characteristic dry capacitance may itself be used, at least in part, to identify a tube connected to the gases supply system. For example, tubes designed for adult respiratory therapy may have a dry capacitance, which is distinct from a dry capacitance of tubes designed for neonatal respiratory therapy. A humidifier may identify the tube by measuring the dry capacitance and select appropriate operating parameters accordingly. The appropriate operating parameters may be stored in a lookup table (LUT) of the humidifier controller, for example.

### Conduit Arrangements

Various arrangements can be used to improve condensation detection. The following arrangements can be used in conjunction with the composite conduit described in detail above, or with any other type of conduit including corrugated conduits, spiral conduits, extruded conduits or with any other conduit known to those of skill in the art. It is to be understood that each of the structures below can be integrated separately or combined together. Therefore, although the below structures are described separately, the structures or aspects of the structures can be mixed and combined together. Each of Figs. 14-25 illustrate, in cross-section, different implementations of the second elongate member 305 or bead 405 of the tubes of Figs. 3A and 4A, respectively, wherein the lumen would be positioned below the illustrated cross-section.

Fig. 14 illustrates a portion of an internal conduit wall 1401, such as a bead of a composite conduit, that comprises microstructures 1403 which may define one or more microchannels. The term "microstructure" as used herein refers to structures having dimensions of less than about 2.3 mm, and preferably in the range of 1 to 1000 microns (µm). It has been found that movement of liquid in a microchannel is primarily based on surface forces, rather than inertial forces or gravitational forces, and that surface forces generally dominate if the characteristic dimension of the microstructure is smaller than the capillary length of water which, at room temperature, is about 2.3 mm. It has been found that, to promote wicking, structures with high aspect ratios and/or high surface energy (equilibrium contact angles less than about π/2) are desirable. Surfactants can result in contact angles near 0°, so wicking can take place with ease. Microstructured or nanostructured bumps within the microchannels may act to pin the solid/liquid/vapor contact line, increase surface area, and/or act as nucleating sites for condensation.

The illustrated microstructures 1403 wick condensate along the length of the wall 1401 via the microchannels, distributing the condensate across a greater portion of elements 1405 in order to improve a capacitance measurement. Alternatively, or additionally, microstructures such as transverse microchannels may be provided on the internal surface of the first elongate member 303 of at least a portion of a composite tube, adjacent wall 1401, to transport liquid towards the elements 1405. A microchannel depth gradient may be used to control movement of a liquid in a particular direction, for example, towards the elements 1405. It has been found that liquids tend to move in the direction of the deeper channels. Gradients can also speed up or otherwise improve the wicking of liquid. Alternatively, or additionally, an internal surface of the first elongate member 303 and second elongate member 305 may respectively comprise hydrophilic and hydrophobic materials or coatings to direct condensate towards the elements 1405.

Fig. 15 illustrates a portion of an internal conduit wall 1501, such as a bead of a composite conduit, including one or more openings 1503 (such as, for example, dips, holes, key holes, channels or voids). These one or more openings 1503 can draw condensate into the one or more openings 1503, causing a change in capacitance between elements 1505. Condensate can be drawn in by, for example, capillary action or gravity, or openings 1503 may be filled with a permeable or absorptive material. A tube comprising such a bead may optionally further comprise microstructures on the internal surface of the first elongate member to transport condensate towards the one or more openings 1503. This may further improve the sensitivity and/or response time of the condensate detection algorithms.

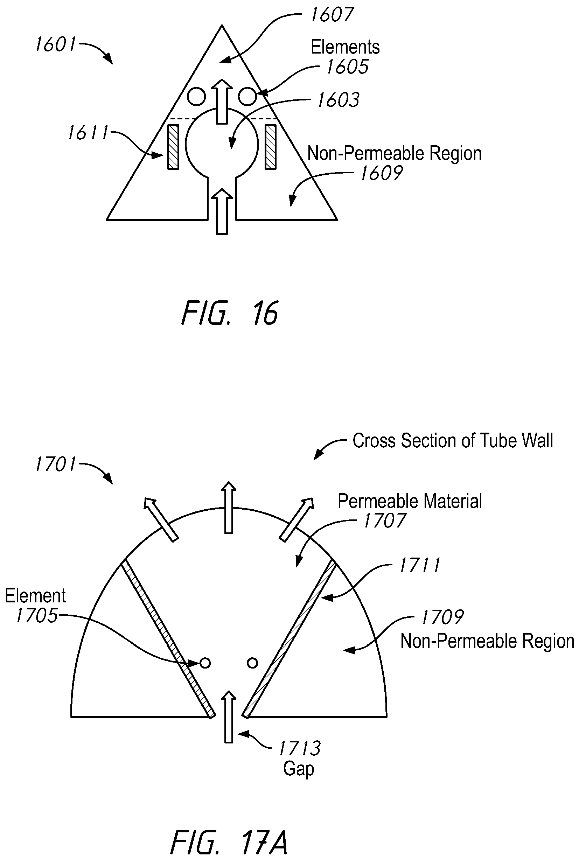

Fig. 16 illustrates a portion of an internal conduit wall 1601, such as a bead of a composite conduit, with one or more openings 1603, similar to those included in Fig. 15. In the example of Fig. 16, a portion of the conduit wall or bead comprises a permeable region 1607, such as a vapor and/or liquid permeable region. Condensate which accumulates in the openings 1603 may be dissipated by diffusion to the ambient environment. In this arrangement, two of the conductive elements 1611 may comprise substantially parallel plates or ribbon wires to increase their capacitive coupling. A heating loop or circuit may be formed by one of elements 1605 and one of elements 1611, and a sensing loop or circuit may be formed by the other of each of the elements 1605, 1611. Alternatively, both elements 1605 may comprise dedicated moisture detection elements. Capacitance may be detected between either the elements 1605 or elements 1611.

Fig. 17A illustrates a portion of an internal conduit wall 1701, such as a bead of a composite conduit such as those illustrated in Figs. 3B, 4A or 4B. The internal conduit wall 1701 comprises a non-permeable material 1709 and a gap 1713 in the non-permeable material leading to a widening permeable material 1707. The permeable material can be vapor permeable or liquid permeable or a combination of the two. For example, an inner portion of the permeable material 1707 can be liquid permeable while an outer portion can be only vapor permeable. The permeable material 1707 can also be separated from ambient air by a second non-permeable material. The permeable material 1707 can optionally be separated from the non-permeable material 1709 by electrical conductors 1711, with can take the form of conductive plates or ribbons. One conductor 1711 may form part of a heating circuit, with the other forming part of a sensing circuit or a moisture detection element or circuit. The increased surface area of the plates or ribbons increase the capacitive coupling therebetween. Alternatively, both conductors 1711 may comprise dedicated moisture detection elements. Elements 1705 can alternatively be comprised within the permeable material 1707. Water molecules can enter through the gap 1713 into the permeable material 1707. The relatively small gap 1713 at the lumen side of the conduit ensures that relatively little water vapor, for example humidity, is lost from the humidified breathing gases supplied to the patient. Liquid condensate, on the other hand, may be directed towards the gap 1713 by microstructures, openings or the like, to enhance sensitivity of the condensate detection algorithm to condensate. In a tube designed for use as an expiratory limb, in which drying of the expiratory gases may be desirable, the gap 1713 may be much larger or the nonpermeable material 1709 may be omitted entirely. The electrical conductors 1711 on either side of the permeable material can measure or infer the capacitance or change in capacitance which corresponds to the amount of condensation present between the electrical conductors 1711.

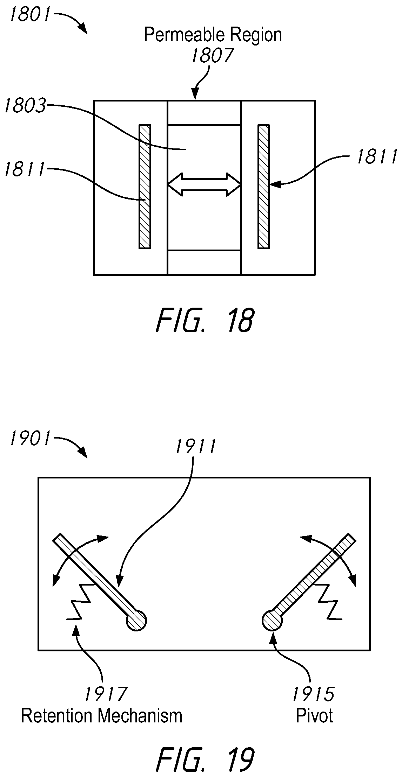

Configurations that cause a physical change in a distance, and therefore capacitance, between conductive elements (such as wires, filaments or plates among others discussed herein) can alternatively or additionally be used. In such arrangements, the capacitance may be negatively related to moisture. For example, Fig. 18 illustrates a portion of a conduit wall 1801, for example a bead of a composite conduit, which includes substantially parallel elements 1811 encapsulated in non-permeable regions, a permeable region 1807, and a hollow region 1803. Elements 1811 may each comprise dedicated moisture detection elements, which are not electrically coupled with each other, wherein each element 1811 effectively forms one of the parallel plates of the capacitor *C* 503 in the model of Fig. 5. The permeable region 1807 can be configured to change in length (e.g. swell) to physically change the distance of the elements 1811 in dependence upon a volume of condensate present in the tube. For example, the permeable region can have an accordion shape that lengthens/straightens when condensate permeates the permeable region 1807. The elements 1811 can be configured to move horizontally as illustrated or vertically or at an angle. Although this configuration is shown in a square or rectangular shape, it can be any shape that allows the elements to separate in some way with condensate. Further, the length of the permeable regions may be either positively or negatively related to moisture (for example, swelling or contracting, respectively, in the presence of condensate), although it is preferable that the effect upon capacitance between the elements 1811 complements, or far exceeds, that arising from the change in permittivity.

As another example, a permeable material comprised in the conduit wall can cause a change in an angle of elements. Fig. 19 illustrates such a configuration where elements 1911 can pivot as the permeable material swells. The elements can have a pivot point 1915 and/or a retention mechanism 1917. Capacitance between elements can be measured as the elements move closer or further apart. In a variant of the implementation of Fig. 18, the upper (outermost) permeable region 1807 may instead be a non-permeable material and/or a permeable material which does not change in size/shape in the presence of moisture. Elongation (or contraction) of the lower permeable region 1807 will therefore create an angle between the elements 1811, changing the capacitance therebetween. The non-permeable outer material will also minimize moisture loss and undesired drying of the respiratory gases.

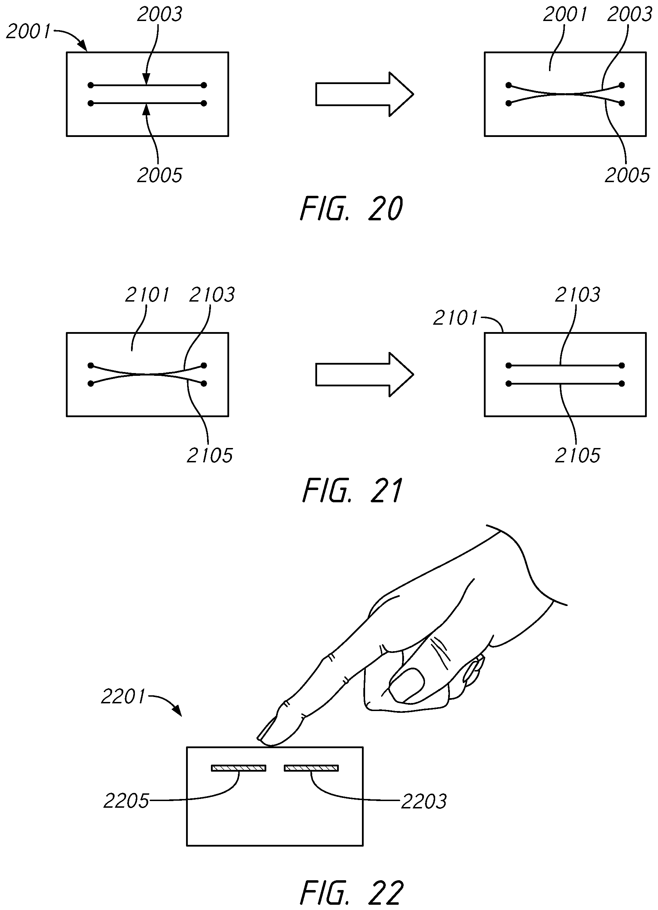

The swelling of the permeable material can also cause a closure or opening of a switch-like construction for binary condensate detection. For example, Figs 20 and 21 illustrate permeable wall portions of a conduit wall that include elements 2003, 2005, 2103, 2105. These elements either open or close in the presence of condensate as illustrated. When the elements 2003, 2005 or 2103, 2105 touch, closing the circuit, thus acting as a switch. The switch may be either normally open (NO), as shown in Fig. 20, or normally closed (NC), as shown in Fig. 21. The elements 2003, 2005, 2103, 2105 may be continuous along the length of the tube, or one or more discrete switches may be provided at particular locations along the length of the tube.

In some implementations, one or more Wheatstone bridge circuits with strain gauges positioned at one or more locations around the conduit. The one or more strain gauges can be located along the bead and configured such that if the bead changes in shape such as swelling or shrinking, the one or more strain gauges will create a signal through a transducer. This signal can be sent to the controller to indicate the presence and/or amount of condensation.

### Proximity Sensitive Exterior Conduit Wall

A proximity sensitive exterior of a conduit wall can also be included as an additional or alternative function to condensation detection. Detecting if there is bedding, patient limb, or other foreign object in contact or proximity with the exterior of the conduit can act as a safety mechanism. For example, it can prevent cases where the surface temperature of the conduit is too hot and could potentially burn the patient.

Permittivity is an electromagnetic property, which contributes to the capacitance of a capacitor and may be measured in farads per meter (F/m). Relative permittivity is a property of the dielectric material/medium in a capacitor. The presence of skin in close vicinity of a breathing conduit may result in changes to the relative permittivity of a dielectric medium, which can be detected or measured using capacitance-based sensing disclosed herein (for example, by measuring a time constant, a resonant frequency, a change in a time constant, or a change in a resonant frequency of the capacitor between the two electrically conductive elements disclosed herein). The permittivity thresholds can be used by a humidifier controller to determine whether there is presence of skin in close vicinity of the breathing conduit. The permittivity thresholds may also be used to distinguish different objects, such as skin versus a blanket. The detection of skin or other objects in close vicinity of the breathing conduit may be used to limit or control the heating elements of the breathing tube to reduce surface temperature and ensure exposed surface temperatures are safe, for example, there may be a 44°C limit for approximately 250mm of conduit near the patient or patient-end connector, whereas there may be a higher limit for the remainder of the tube may be for example 48°C. In the case of a dual-zone heated breathing conduit, only the heater wire(s) of the patient-proximate portion of the conduit may be limited as such.

Detecting if a person (such as a patient or operator) touches the conduit wall can be performed by having elements situated close to the outside surface of the conduit. The elements can be configured such that dielectric properties between the elements will change in response to skin touching the surface. Proximity of a foreign object may be distinguished from condensation based on a rate of change of capacitance, or by detecting capacitance between wires adjacent the lumen and wires adjacent the exterior surface of the tube.

Fig. 22 (not to scale) illustrates an example conduit configuration in which elements 2205 and 2203 are provided in substantially the same plane, parallel to the surface of the exterior conduit wall 2201. When a finger or other body part contacts the exterior surface, the dielectric properties will change causing a measurable change in capacitance.

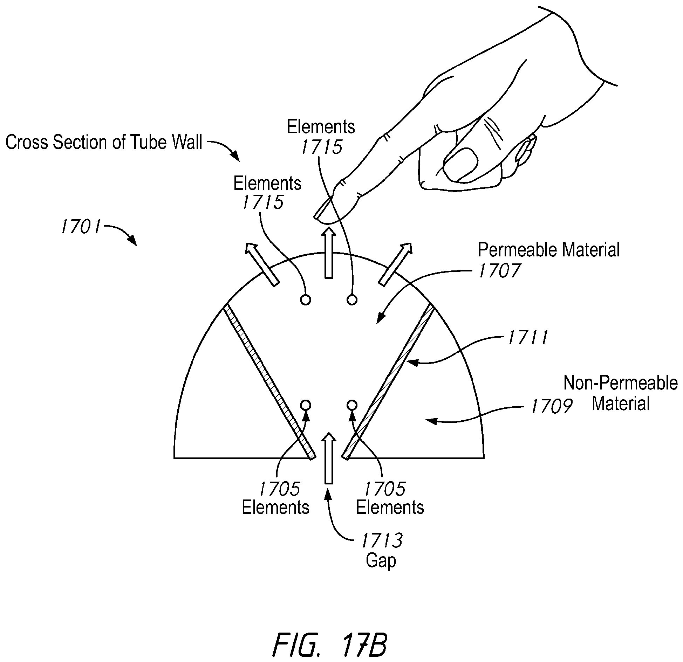

As another example, Fig. 17B illustrates the conduit wall portion of Fig. 17A. In Fig. 17B, additional elements 1715 are incorporated near an outer surface of the conduit walls. A touch from a body part, such as a finger, will cause a measurable change in capacitance.

In some embodiments, the detection of skin contact may also be used to generate an alarm, alert, warning, or the like after a predetermined period of time elapses while in contact. For example, an alarm may be raised if a contact is detected and lasts for longer than one minute, 10 minutes, or any other suitable duration. Contact may not need to be sustained, but intermittent over the duration to account for movement, signal noise, sensitivity, etc. Triggering of alarms may be based on both the duration of contact and the expected breathing conduit surface temperature. For example, if the conduit surface temperature is about 48°C, an alarm may be triggered after one minute of sustained or intermittent contact, whereas if the temperature is only about 43°C, a longer duration (for example, 10 minutes) may lapse before the alarm is triggered.

### Switched Circuit

In some arrangements, a tube may comprise a pair of wires which may be selectively electrically coupled with each other at a distal end of the tube. The wires may be closed to form a heating or sensing loop or circuit, and opened so that the pair of wires effectively form parallel plates of a capacitor.

The distal end of the tube may comprise a switch, such as a relay. The tube may further comprise a control wire or wires to enable a humidifier to control operation of the switch to selectively power the heating or sensing loop, or measure a capacitance between the wires.

The pair of wires may comprise an inductor selected to effectively provide a short circuit to relatively low frequencies, but an open circuit to relatively high frequencies. In particular, the inductor may be selected to have little, if any, effect upon a direct current (DC) or low frequency (e.g. 50/60 Hz ) alternating current (AC) heater wire current, but effectively blocks a high frequency (e.g., upwards of 1 kHz) signal which may be used to measure a capacitance between the pair of wires. The inductor may further be used to determine the capacitance between the wires by determining the resonant frequency of the circuit, as described above. A resonant frequency of a dry tube may further be used to identify a particular tube model and configure a humidifier or gases source accordingly.

### Mesh conductor

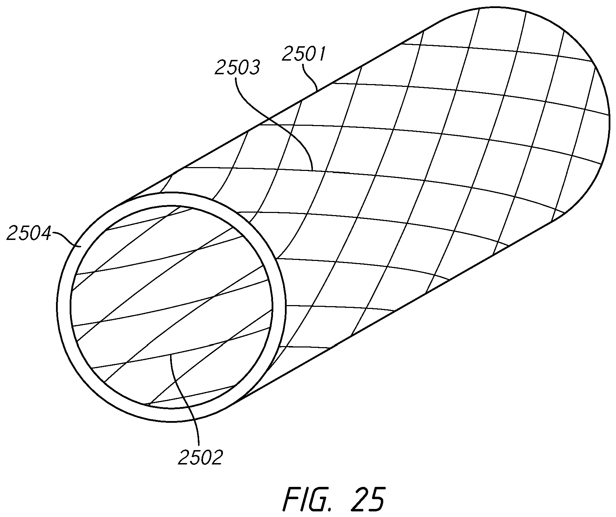

An electrically conductive mesh can alternatively or additionally be used to determine a presence of condensation. In one implementation, as illustrated in Fig. 25, a tube 2501 has an outer wall comprising a first (or inner) mesh 2502 and a second (or outer), coaxial, mesh 2503 separated by a permeable dielectric material 2504 or air gap. The tube may further comprise a non-permeable outer layer, particularly if used as an inspiratory conduit, to prevent excessive drying of the breathing gases. Condensate may be absorbed or diffused by the permeable dielectric material 2504, modifying the dielectric constant and thus the capacitance between the first and second meshes 2502, 2503. The meshes 2502, 2503 may increase the surface area of the conduit at which condensation can be detected. Either or both of the meshes 2502, 2503 may be replaced by a conductive foil, or a braided sheath. The inner foil may be perforated, or may comprise a helically-wound strip with a gap, to allow passage of condensate into the dielectric material 2504. Either or both of the meshes 2502, 2503 may optionally form part of a heating circuit. The increased surface area of the meshes 2502, 2503, compared to a heater wire, may provide more uniform heating and reduced condensation. Condensation may also be confirmed or detected from a power input to the heating circuit, as evaporative cooling of the condensate may increase a power requirement due to the larger surface area of the mesh.

### Detecting Location of Condensation

A location of condensation within the conduit can also be detected. For example, a conduit can include segmentations or zones to allow for determination of the location of condensation. For example, the segments can run a certain length of the conduit creating zones along the length of the conduit. As described above, a tube may comprise two or more consecutive and independently controllable zones. The capacitance in each zone may be checked independently. An increased capacitance in a zone towards a beginning or middle of the tube, where it may typically drape between a humidifier and a patient, may be indicative of mobile condensate pooling at a low point of the tube. An increased capacitance in a zone at the patient end of the tube, on the other hand, may be indicative of bodily fluids within the tube. The zones may be equal or unequal in length.

As disclosed above with respect to Fig. 20, the elements 2003, 2005 may form a NO switch or switches. In the presence of condensate, the switch closes to form a circuit. The length of the resulting circuit will be proportional to the distance of the detected condensate from the humidifier, and the location of condensate may be determined from a resistance of the resulting circuit.

Alternatively, a plurality of conductive elements, for example, wires, could extend different lengths down the tube to determine a general location of condensation within the tube.

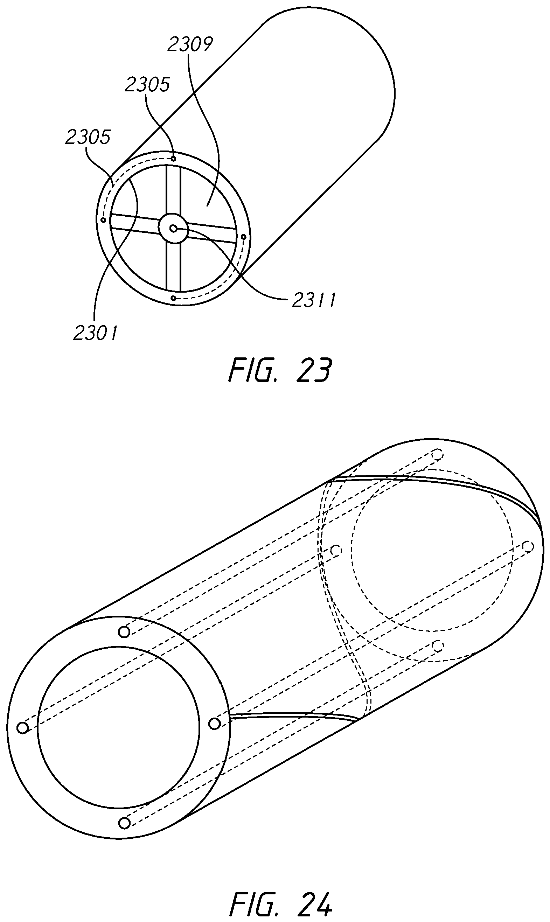

Alternatively or additionally, a circumferential location of condensate may be determined. A conduit can be separated into sectors, such as quadrants, running a length of the conduit (or a zone). For example, Fig. 23 shows a cross section of a conduit 2301 comprising quadrants 2309, wherein the elements 2305 are provided longitudinally, parallel to the lumen, and equidistantly spaced about the circumference of the tube. The elements 2305 can be used to detect a presence or quantity of condensation as discussed previously herein. The quadrants can be extruded with the conduit walls as part of the manufacturing process or added after the conduit is constructed. The quadrants can be used to determine a circumferential location where condensation is pooling (for example, the lower part of the tube) by measuring a capacitance between each of the adjacent wires. Alternatively, or additionally, the conduit may comprise a central wire 2311 suspended within the lumen, and a capacitance between the central wire and each of the circumferential wires may be determined. The quadrants can be combined with segments above to provide an even more accurate location of condensation. In a variation of the tube of Fig. 23, as shown in Fig. 24 (not to scale), an additional conductive element (for example, a mesh, ribbon or other structure) may be wound about the outside of the conduit wall with a pitch approximately equal to the length of the tube (or a zone of the tube). That is, the additional element preferably makes no more than one full turn around the tube from one end of the tube (or zone) to the other. A capacitance between the additional wound element and each of the embedded elements 2305 may be independently measured or inferred. An increase in capacitance between the additional wound element and any one or more of the elements 2305 is indicative of condensation at or near the point of the tube where the wound element crosses the respective element 2305. Although the tube of Fig. 24 comprises four embedded wires, further wires may be used for improved resolution. Similarly, more than one wound wire may be used.

Similarly, in a variation of the tube of Fig. 25, individual strands of either or both of the first and second meshes 2502, 2503 may be insulated and multiplexed. By selectively measuring or inferring a capacitance between each of the strands extending in a first direction with each of those extending in a different direction, a location or locations of condensate may be determined.

In yet other implementations, condensation in a particular location or locations may be determined by a tube configuration in which capacitance between conductors is dependent on moisture, or more dependent on moisture, only at a selected location(s) along the length of the tube. This may be achieved, for example, by varying the pitch, spacing, surface area, shape or alignment of the conductors along the length of tube; providing a permeable material between the conductors only at a selected location(s) along the length of the tube; providing openings only at a selected location(s) along the length of the tube; or providing a moisture-dependent switch at a selected location(s) along the length of the tube. For example, it may be desirable to detect condensate at a portion of the tube which, in use, generally drapes lowest and accumulates mobile condensate which may restrict or occlude the lumen, or to detect bodily fluids at the portion of the tube closest the patient. In other regions of the tube, the capacitance may be minimized or reduced to ameliorate errors in sensor readings.

### Other Methods of Moisture Detection

Alternatively or additionally to the above described moisture detection systems and methods, a conduit can also be configured to change color or transparency depending on a presence of moisture. For example, a conduit can be transparent when no condensation is present, but becomes opaque or brightly colored in the presence of condensation (or vice versa). Such changes provide a visual indication of the presence and/or location of moisture in the conduit to the patient, nurse, or other person.

The controller of the humidifier can respond to the detection of moisture (for example, condensate and/or water in the vapor permeable and/or liquid permeable material in a component of the humidifier) disclosed herein. The response can include various measures to reduce further condensation from occurring (or the likelihood thereof) and/or remove existing moisture from the breathing circuit. The application of moisture detection disclosed herein may improve the operation of a humidifier system, in terms of both humidification performance and safety, for example, by enabling more accurate management of condensation and/or control of humidity. Furthermore, the moisture detection system and methods disclosed herein can additionally or alternatively be applied in enthalpy estimation, water-out detection, zero/reverse flow detection, skin contact detection, etc.

### Moisture Management

In Figs. 13A-13E, implementing moisture (for example, condensation) mitigation strategies 1313, 1331, 1345, 1359, and 1369 can include reporting a presence or indication of condensation to an operator, reporting an amount of condensation to an operator, reporting whether the amount of condensation is increasing or decreasing, providing a visual guide to safely remove condensate from the conduit, providing visual or auditory alarms, or automatically implementing operational change to the gases supply system to, for example, reduce an absolute humidity or increase a temperature of the gases. When condensation mitigation strategies have been successful and reduced condensation to be below an undesirable amount, the system can report a lack of a presence or indication of condensation to an operator, report a decreased amount of condensation to an operator, report rate of decrease of condensation, report a decreasing trend, provide visual or auditory alarms, or automatically implementing operational change to the gases supply system to, for example, increase an absolute humidity or decrease a temperature of the gases. In some configurations, the system may, based on the detection of condensation as above, query the user (via e.g., the display or user interface) about whether they would like to change modes, take any action with respect to the circuit, or the like, and then follow the user's direction. Further, the system may make an appropriate suggestion of changed settings for the user to approve or confirm.

A controller of the humidifier can be configured (for example, by implementing software instructions stored in memory) to change certain operating parameters responsible for the delivery of humidity when an unacceptable volume or presence of moisture is detected using any of the previously described methods above. For example, the operating parameters may be changed when the duration of the time constant of the circuit contemplated by the capacitance-based detection method exceeds a pre-determined threshold. For example, a pre-determined threshold can be set to the duration of the time constant corresponding to a dry tube. When the duration of the time constant exceeds, or falls below depending on the detection method, the pre-determined threshold due to condensation build-up, the controller can change operating set points or parameters such as a chamber outlet setpoint to reduce humidity output from the chamber. For example, power (which as used in the present disclosure should be understood broadly to refer to an amount of energy per unit of time and may be supplied in accordance with a duty cycle, via pulse-width modulation, via a voltage regulator, or the like) supplied to the heater plate of the humidifier may be reduced to reduce the level of moisture added to the breathing gases supplied to the breathing tube and/or power supplied to the breathing tube heating wires may be increased to prevent the temperature of the humidified gases falling below their dew point. Once the duration of the time constant falls below, or exceeds depending on the detection method, the pre-determined threshold value (or after a period of time), the controller may resume normal/previous operation. The threshold may be varied, or a different threshold used, to provide hysteresis and avoid oscillation between operation modes.

When moisture is detected in a component of the humidifier system, the temperature of humidified gases in the breathing conduit is approximately equal to, marginally below, or below the dew point temperature of the gases. The component can include the conduit or a portion thereof, a connector, an adaptor, a portion of the humidification chamber that is exposed to the flow of gases, and/or the patient interface.

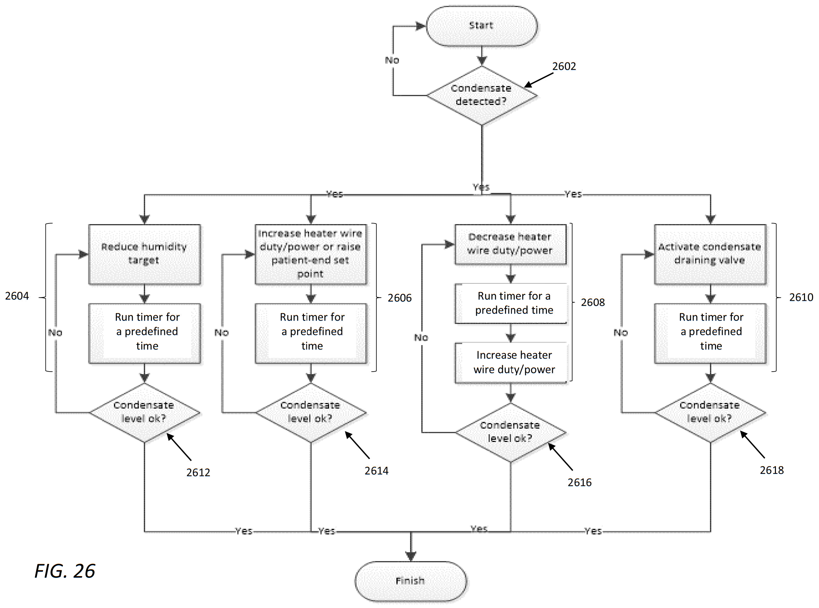

When moisture is detected, the controller of the humidifier can take various measures to manage the moisture, including reducing further condensation from occurring (or the likelihood thereof) and/or removing existing moisture from the breathing circuit. As shown in Figure 26, when any moisture (for example, condensate and/or water in the vapor permeable and/or liquid permeable material) is detected at decision block 2602, the controller of the humidifier can take one or more of a plurality of measures to update the operation of the humidifier to manage the detected moisture. The controller can detect that a predetermined level of moisture is present based on at least one sensor configured to output at least one sensor signal. The at least one sensor signal can include any of the moisture detection sensor signals disclosed herein, or any other suitable moisture detection sensors. Although, for some sensor types, the at least one sensor signal may include information based on moisture and humidity present in the component of the humidifier, any portion of the signal relating to the humidity of the gases in the component, which is orders of magnitude smaller than the portion of signal relating to the moisture, may be filtered from the at least one signal or ignored by the controller. The output of the at least one sensor signal may include at least one value indicative of moisture that may comprise a value indicative of condensate and/or a value indicative water in the vapor permeable and/or liquid permeable material. The sensor output may include a combination of the condensate and water in the vapor permeable and/or liquid permeable material. The controller may filter and/or otherwise process the sensor output to split the output into individual values for condensate and water in the vapor permeable and/or liquid permeable material. This may be based on, for example, ranges associated with the presence of each condensate and moisture or for example other signal processing techniques. The controller may determine that the vapor permeable and/or liquid permeable material is saturated when the sensor output reaches a predetermined threshold and/or when there is a predetermined amount of water present indicative of saturation of the vapor and/or liquid permeable material. In some cases, the controller may determine that condensate is present when the vapor permeable and/or liquid permeable material is saturated. In some cases, the sensor output may be able to determine a state of the moisture level in the tube. The state of the moisture level that can be determined by the controller may include the vapor permeable and/or liquid permeable material being saturated or unsaturated and/or the presence of condensate. The predetermined level of moisture may not need to be a physical amount (for example, 20 g or otherwise). The predetermined level of moisture can be experimentally determined based on the characteristics of the system and the particular sensor. The predetermined level of moisture can be indicative of a predetermined level of condensate in the component where the at least one sensor is located, a predetermined quantity of water in the vapor permeable and/or liquid permeable (that may for example be breathable) material of the component, or a sum of the condensate and the water. The controller can determine the presence of condensate in the component based on a comparison between the at least one value indicative of the amount of moisture present in the component and at least one condensate threshold. The controller can further determine the presence of a predetermined amount of water in a vapor permeable and/or liquid permeable material of the component of the system based on a comparison between the at least one value indicative of the amount of moisture present in the component and at least one water amount threshold. Optionally the predetermined amount of water can be indicative of saturation of the vapor and/or liquid permeable material.

The controller may continually and repeatedly evaluate for the moisture level if the predetermined level of moisture (also referred to as excess moisture, that is, moisture above a predetermined level, including condensate above a threshold, and/or water in the vapor and/or liquid permeable material above a threshold) is determined to be absent based on the level of moisture being above or below at least one threshold. An amount of moisture above at least one threshold may be condensate above a predetermined level or water in a vapor permeable and/or liquid permeable material above a predetermined level. The at least one threshold can be any of an absolute value threshold, a value change threshold, a percentage threshold (for example, about 80%, about 90%, about 100%, or any values in between) of a maximum sensor output, a percentage change threshold, a percentage change over time threshold, a gradient threshold, and/or a crossing threshold. The crossing threshold may relate to how the threshold may be triggered a predetermined number of times, which may be over the duration of a predetermined time period. The maximum sensor output may be, for example, an output indicating that the bead of a conduit is fully saturated with moisture. Alternatively, the maximum sensor output may be based on an acceptable or safe amount of condensate in the tube.

As long as a moisture detecting/sensing system or method is able to at least produce signals indicative of the presence of moisture in a breathing circuit, the moisture detecting/sensing system and method may be implemented for various features, for example, moisture management, humidity control, sensor substitution, water-out detection, and/or circuit condition detection. The scope of implementable features may be dependent on the information made available by the moisture detecting/sensing system and method.

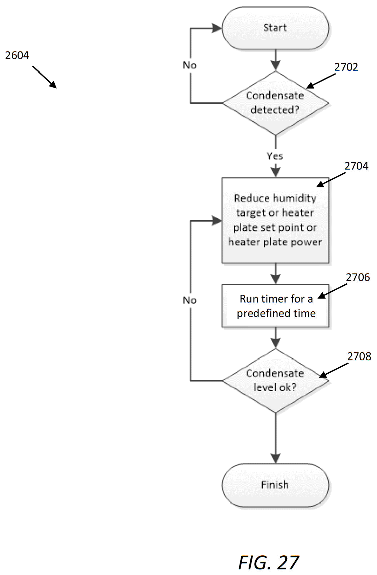

If the controller determines that excess moisture is present at decision block 2602, the controller can proceed to update the operation of the humidifier to manage the excess moisture. The updated operation can include any one or more of generating at least one notification and/or alarm (which may be displayed on the humidifier or another device, such as the ventilator or a central monitoring system), changing power to the heater plate and/or the heater wires, and/or changing a mode of the humidifier. The decision block 2602 can be implemented in all the example methods of managing the detected moisture, such as the decision block 2702, 2802, 2902, 3002 of Figs. 27-30. Although the figures of example methods of the present disclosure refer to "condensate," the methods can be implemented with any moisture (that is, including the condensate and the water in the vapor permeable and/or liquid permeable material of the humidifier system) detected using the system and methods disclosed herein.

Such moisture management methods can include, but are not limited to, reducing a humidity of the flow of gases 2604. The humidity may be a dew point of the flow of gases 2604, absolute humidity target of the flow of gases 2604, or relative humidity target of the flow of gases 2606 (each of which may likewise be approximated by a temperature value). The moisture management methods can actively drive moisture into the ambient air via a vapor permeable and/or liquid permeable material (for example, in the bead of a conduit or otherwise as disclosed herein) 2608, and actively drain condensate in the breathing circuit 2610. Active inducing of condensate may be part of a moisture management response or part of detecting a humidity of the flow of gases. The controller can also alternatively or additionally communicate with an external device to reduce moisture in the humidifier system. The humidifier controller may implement one or more of the moisture management methods disclosed herein in response to detecting excess moisture in the decision block 2602. These moisture management methods can be executed in any order and/or combination, independently and/or simultaneously. For example, upon detection of excess moisture, the controller may first activate a condensate draining valve as a preliminary response, with the aim to immediately reduce the volume of condensate. Following active draining, the controller may execute the remaining methods disclosed herein (for example, in parallel) to further eliminate the remaining moisture and/ prevent or reduce further formation of condensate. For example, the heater wire power may be controlled first, following the activation of a drain valve, to cause any residual moisture to be moved to the atmosphere, before turning to control measures that are more directed towards prevention of condensate formation. In other example configurations, certain steps may be included, omitted, or ordered such that a different moisture management response is achieved. Additional details of each of these methods will be described below, including descriptions with reference to Figs. 27-30.

After implementing the moisture management methods or responses disclosed herein, the controller can evaluate whether excess moisture is still present at decision blocks 2612, 2614, 2616, 2618, by using the same or similar threshold as applied at decision block 2602. The controller can determine that excess moisture is no longer present in the component in response to detecting an absence of moisture or a small amount of moisture present in the component. The small amount of moisture may include an acceptable mount of condensate for the particular system (that is, for example the humidifier, tube set and/or ventilator configuration), which may vary depending on the type of components in the system, therapy mode enabled, and the location of the moisture within the conduit. The small amount may be, for example, about 5 mL. The controller may have the ability to detect between non-mobile and mobile condensate, which may be based on sensor structure, sensor output filtering and/or signal processing, or at least one threshold or threshold range. The controller may have a further response when detecting mobile condensate and may further change at least one operating parameter of the conduit or the humidifier or add more than one response together. If excess moisture is no longer present, the controller can exit the process. By exiting the process, the controller can return to the normal operation (that is, operation without reduced/limited/disabled operating parameter limits introduced in response to moisture detection at decision block 2602) and/or revert to an original operating parameter of the humidifier (that is, before the controller proceeded to the decision block 2602). Alternatively, after exiting the process, the controller can repeat the same or another moisture detection process.

The moisture management responses disclosed herein may control the system to better control moisture in the system. Moisture management responses may ensure that moisture in the component does not further increase (for example to ensure that further condensate does not form). Moisture management may also allow for the condensate to re-evaporate. This may allow for the conduit to dry, for example via a drying strategy further described below.

### Absolute Humidity Reduction

Figs. 26-27 illustrate example methods 2604 of a humidifier controller updating operation of the humidifier to remove moisture and/or prevent further moisture from occurring in response to detection of moisture, by reducing the amount of humidity (that is, absolute humidity) imparted to the gases flowing through the humidification chamber. As shown in the method 2604 of Fig. 26, the controller can reduce the absolute humidity target of the flow of gases. The reduction of the absolute humidity can lower the chamber outlet dew point temperature, which may provide more buffer between the dew point temperature and the temperature of the gases. Gases having a lower absolute humidity but heated to the same temperature may be able to sustain more cooling before condensation begins to occur.

As shown in Fig. 27, the controller can reduce the absolute humidity target at step 2704 by decreasing the output of the heater plate. The decrease of the heater plate output can be achieved by the controller changing at least one of the following operating parameters of the heater plate: reducing set point(s) (for example, the chamber outlet or heater plate temperature set-point), and/or reducing power to the heater plate or limiting (or even disabling) the power to the heater plate. For example, by reducing the humidity target, for example a chamber outlet dew point set point can be reduced from about 37°C to about 36°C, about 35°C, about 34°C, etc.

The controller can maintain the changed operating parameter(s) of the heater plate from step 2704 for a predefined time using a timer at step 2706. After the predefined time, the controller can reevaluate at decision block 2708 whether excess moisture is present as described above with reference to decision block 2612. If excess moisture is still present, the controller can return to step 2704. If excess moisture is no longer present, the controller can exit the process 2604. By exiting the process 2604, the controller can return to the normal operation and/or revert to the original absolute humidity target of the flow of gases.

### Relative Humidity Reduction

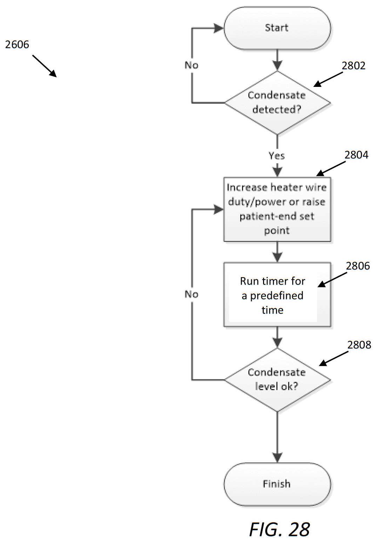

Figs. 26 and 28 illustrate an example method 2606 of the humidifier controller updating operation of the humidifier to remove moisture and/or prevent further condensation from occurring in response to detection of moisture, by reducing the relative humidity of the gases. The amount of water vapor or humidity with respect to the total water carrying capacity of the gases is relative humidity ("RH"). Decreasing the relative humidity of gases may provide additional buffer for cooling to occur before water vapor condenses out of the flow of gases. Furthermore, decreasing the relative humidity would result in the gases being less saturated. Therefore, for a given humidity, when the gases cool (for example, due to a cold draught), there may be a wider tolerance for the gases to cool before condensing.

The humidifier controller can reduce the relative humidity target by changing one or more operating parameters of the heater plate of the humidifier and/or the at least one heater wire of the conduit (for example, the inspiratory conduit.). As shown in the method 2606 of Fig. 26 and Fig. 28 at step 2804, the controller can increase the operating parameter(s) of the heater wire, for example, the heater wire power or duty cycle and/or the patient-end temperature set point, to heat up the gases, which may raise the vapor-carrying capacity of the flow of gases, assuming a constant absolute humidity, thereby reducing the relative humidity target. At step 2804, the humidifier controller can additionally or alternatively decrease or limit the operating parameter of the heater plate, for example, the heater plate temperature set point and/or power to the heater plate. Alternatively, in addition to the heater wire control changes, the heater plate may be disabled for a transitory period of time based on a timer, as further discussed below.

The controller can maintain the changed operating parameter(s) of the heater plate and/or heater wire at step 2804 for a predefined time using a timer at step 2806. After the predefined time, the controller can reevaluate at decision block 2808 whether excess moisture is present at decision block 2802 as described above with reference to decision block 2614. If excess moisture is still present, the controller can return to step 2804. If excess moisture is no longer present, the controller can exit the process 2606. By exiting the process 2606, the controller can return to the normal operation and/or revert to the original relative humidity target of the flow of gases.

The reduction of the relative humidity target can be implemented in a neonatal multi-zone breathing circuit used with an incubator to reduce formation of condensation in the breathing circuit. In such a humidifier system (which can include a zone-heating function as disclosed herein), a first segment of the inspiratory conduit is external to the incubator, which can be exposed to the ambient environment and a second segment of the inspiratory conduit is inside the incubator. For example, a conduit capable of multi-zone heating that is disclosed herein and in WO 2014/077706 A1 (the disclosure of which is incorporated herein by reference) can be used. Similarly, the first and second segments of the conduit may be capable of being heated independently or differently. The environment of the incubator may be warmer than the ambient environment. Therefore, the likelihood for the flow of gases to cool and condense within the second segment of the conduit may be lower. It may be difficult to determine the temperature of the flow of gases in the two environments, particularly when the conduit only has a patient-end sensor. Additionally, a chamber outlet sensor may only allow measurement of the gases at the beginning of the conduit and not at the location of the conduit just prior to the incubator. By reducing the relative humidity target, the controller may more accurately heat the conduit to reduce or prevent condensation. If moisture is detected in the first segment of the conduit using the moisture detection systems and methods disclosed herein, the controller can increase power to the heater wires in the first segment without changing power to the heater wires in the second segment. This may be beneficial because the power to the second segment may be unaffected and may not be heated excessively in response to detection of moisture in the first segment, which may result in a more accurate control of the temperature of the gases at the patient end.

Furthermore, this reduction of the relative humidity target may also be applicable to multi-zone adult breathing circuits, for example, where a blanket is unintentionally placed over a portion of the inspiratory conduit. If at least one temperature sensor at locations in the conduit (for example, at the patient-end) read higher than in a normal operation, heating of the gases by the heater wire(s) may be reduced, and condensation may occur. When this occurs, the portion of the breathing circuit where the temperature sensor reads a higher temperature may have the power reduced rather than reducing power to all the heater wires.

### Drying Strategies

As described above, the conduit of the breathing circuit can include a vapor permeable and/or liquid permeable material. This vapor and/or liquid permeable material can be part of the wall of a component, for example, a conduit. An example of this permeable material is shown in Fig. 4B. The bead 405 of the inspiratory conduit in Fig. 4B can include the vapor permeable and/or liquid permeable material. Alternatively or additionally, other components of the humidifier system (for example, connectors, adaptors, and/or the patient interface or a patient interfacing connecting tube), or other components of the tube (such as the hollow elongate member) may include a vapor permeable and/or liquid permeable material.

Using the bead 405 as an example, the humidifier controller can first remove the moisture in the conduit by allowing or driving the vapor permeable and/or liquid permeable material to be saturated with water, and then powering the heater wires of the conduit to cause movement of the water from the bead 405 into the atmosphere (for example by evaporation or by solution-diffusion).

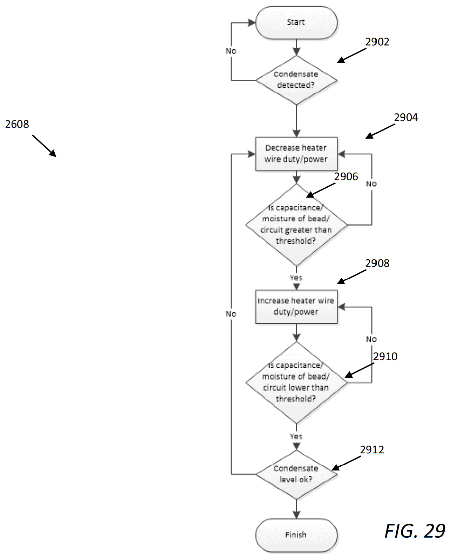

Figs. 26 and 29 illustrate example methods of conduit drying strategies as an example of a moisture management response. The controller may control one or more heaters to control drying of the conduit (that is, removing condensate and/or removing moisture in the component and/or the vapor and/or liquid permeable material). These strategies can increase a drying rate of the component and/or the vapor permeable and/or liquid permeable material via for example evaporation or solution-diffusion to the atmosphere to remove moisture from the component via the vapor permeable and/or liquid permeable material. The drying rate is the rate at which moisture (for example condensate and/or moisture in the vapor and/or liquid permeable material) can be transferred from the component across the vapor permeable and/or liquid permeable material to atmosphere, and/or from the vapor permeable and/or liquid permeable material to atmosphere.Upon detecting excess moisture (for example condensate present) at decision block 2902, the humidifier controller may decrease the heater wire duty cycle or power at step 2904. For example, the heater wire duty cycle can be reduced to any value between about 0-10W or heater wire power density can be reduced to about 3.0 W/m to about 7.0 W/m.

The controller can then monitor moisture (for example via a value indicative of the amount of moisture present in the component based at least in part on the at least one sensor signal - i.e. a capacitance) until it reaches a predetermined threshold. For example, the threshold may be a percentage threshold with value ranging between and including about 80% and about 100%, or above about 50% or above about 60%, or above about 70% of a predetermined maximum value which indicates the vapor permeable and/or liquid permeable material (for example the bead) is fully saturated with moisture. The predetermined threshold may be associated with a predetermined moisture saturation of the vapor and/or liquid permeable material. In some cases, the controller may control the heating operating parameter based on the capacitance to control the drying rate. The drying rate may relate to the transfer rate of water through the vapor permeable material and/or the evaporation rate of water through the liquid permeable material. For example, the controller may control the heater wire operating parameter to reach a sensor output (or for example a value indicative of moisture), which may correspond to a drying rate. When an amount of moisture is detected above a predetermined level, the controller may vary at least one operating parameter in an attempt to dry out the component. Such varying may include increasing and decreasing at least one operating parameter repeated or periodically, or varying the amplitude of the at least one operating parameter over time, and/or varying the frequency of increasing and decreasing the at least one operating parameter. As shown in Fig. 26, the controller can run a timer for a predefined time after step 2904 and assume the threshold has been reached. Additionally or alternatively, as shown in Fig. 29, the controller can determine whether capacitance and/or moisture of the bead and/or circuit is greater than a predetermined threshold at decision block 2906 after step 2904. The controller can implement decision block 2906 continuously or intermittently after step 2904. If the capacitance and/or moisture of the bead and/or circuit is not greater than the predetermined threshold, the controller may return to step 2904 to further reduce and/or maintain at least one operating parameter.

If the controller determines that capacitance and/or moisture of the bead and/or circuit is greater than a predetermined threshold (and/or if the predefined time has lapsed), at step 2908, the controller may increase the at least one operating parameter, for example, the heater wire duty or power. The heater wire operating parameter, for example heater wire power or duty cycle, may be increased such that the power provided is approximately 15-20W or higher or the power density to the heater wires may be increased to about 10 W/m to about 14 W/m, to drive moisture out of the vapor permeable and/or liquid permeable material and into the ambient environment around the conduit. The controller can run a timer for a predefined time after step 2908 and assume that the vapor permeable and/or liquid permeable material has dried to a dryness threshold. The dryness threshold may be when the capacitance has fallen below about 50%, or about 40% or about 30% about 20%, or about 15%, or lower, or otherwise. Additionally or alternatively, as shown in Fig. 29, the controller can determine whether capacitance and/or moisture of the vapor permeable and/or liquid permeable material and/or circuit is lower than a dryness threshold at decision block 2910. The controller can implement decision block 2910 continuously or intermittently after step 2908. If the capacitance and/or moisture of the vapor permeable and/or liquid permeable material and/or circuit is not lower than the dryness threshold, the controller may return to step 2908 to further increase the heater wire duty cycle or power and/or maintain the increased heater wire duty cycle or power for a longer period of time. The controller may repeat this process or any equivalent process variant multiple times, until the detected quantity of moisture (or an indicator thereof) is acceptable or falls below the dryness threshold. The dryness threshold may not be limited to the examples provided, but a level that may be determined to be acceptable for patient safety.

If the controller determines that the detected quantity of moisture in the vapor permeable and/or liquid permeable material and/or circuit is lower than the dryness threshold, the controller can reevaluate at decision block 2912 whether excess moisture is present in the humidifier system (that is, condensate within a component of the system and/or water within the within the vapor permeable and/or liquid permeable material) at decision block 2902 as described above with reference to decision block 2616, such as by comparing the level of moisture detected with a threshold. For example, the threshold may be a percentage threshold, for example, 0%, 15%, or 20% or any values in between. If excess moisture is still present, the controller can return to step 2904. If excess moisture is no longer present, the controller can exit the process 2608. By exiting the process 2608, the controller can return to the normal operation and/or revert to the original operating parameter(s) of the heater wire.

In some embodiments, the moisture in the vapor permeable and/or liquid permeable material may not reach saturation at decision block 2906 even after a period of time. This may indicate that there is no more excess moisture and normal operation may resume, or the condensation management measure may be exited. Furthermore, the capacitance thresholds may not be based on absolute values such as a percentage of known maximum capacity. The capacitance thresholds may be relative gradient thresholds. That is, the controller may identify a rate of change of capacitance to determine whether the vapor permeable and/or liquid permeable material is saturated or dry. For example, the controller may determine that a bead is saturated when a plateau or roll-off in capacitance or a value indicative of the amount of moisture is identified, as the bead will no longer be able to absorb more water and capacitance remains steady as a result.

Such methods of determining when to increase or decrease the heater wire power may be universal across different conduit models disclosed herein and/or eliminate error due to, for example manufacturing inconsistencies. Prior to running the humidification operation with a breathing tube, the controller may execute a self-calibration process where the controller checks one or more parameters (for example, at least the capacitance) to ascertain a set of "baseline" parameter values. The self-calibration process may optionally involve human interaction. For example, if anomalous parameter values are identified, a manual check may be requested by the controller.

### Automatic Condensate Draining

The humidifier system disclosed herein may further include a moisture draining assembly that can collect condensate and/or transport condensate back towards the humidification chamber. The moisture draining assembly can include a water trap that can collect the condensate formed in the humidifier system (for example, in the breathing circuit). The assembly can further include one or more controllable valves positioned at various locations in the breathing circuit. The assembly can be in communication with the humidifier controller, which can selectively activate the valve(s) to drain off condensation to one or more water traps or other suitable collection devices in response to detecting a threshold quantity of moisture. This threshold may or may not be the same as the threshold for the other moisture management measures disclosed herein.

For an inspiratory conduit, the water trap may be positioned at a location anywhere along the inspiratory conduit. Furthermore, in some embodiments, multiple water traps may be used. The location may be approximately one quarter of the distance between the humidifier outlet and the patient, where the moisture tends to pool (for example, due to sagging of the conduit), or the physically coolest point of the inspiratory portion of the breathing circuit in conventional clinical arrangements (and thus where moisture tend to accumulate). The location may also generally be cooler than downstream locations as the gases flow is heated by the breathing conduit as it travels.

For an expiratory conduit, the water trap may be positioned proximate to the ventilator (or other gases source), or approximately three-quarters of the distance between the patient and the ventilator.

Moisture, for example in the form of condensate, can be conveyed back to the humidification chamber. This may be directly from the conduit itself or via the water trap. The moisture draining assembly can include a moisture conveying assembly that can convey the collected moisture back to the chamber. The moisture conveying assembly can include a conveying conduit and/or a pump. For example, a peristaltic pump may be used to convey the moisture. Additionally, and/or alternatively, the peristaltic pump can be operated in reverse to pump moisture back into the conduit to increase the absolute humidity of the flow of gases. For example, the peristaltic pump can pump collected condensate to an ultrasonic vaporizer which may vaporize the condensate into airborne droplets. This may reduce the amount of condensate in the conduit and also further increase the absolute humidity of the gases.

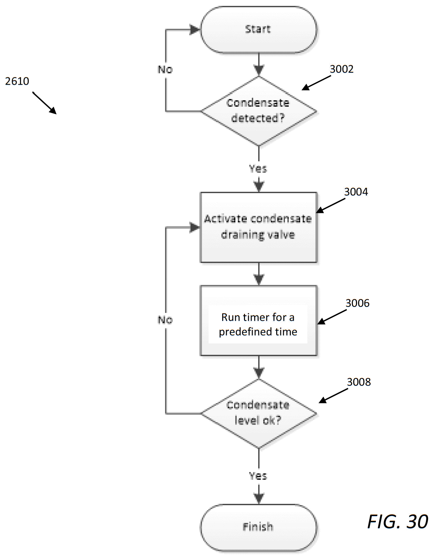

Figs. 26 and 30 illustrate an example method for automatic condensate draining. In response to detecting excess moisture in decision block 2602, 3002, the controller may activate one or more condensate draining valves at step 3004. The controller can maintain the valve(s) in the open position for a predefined time using a timer at step 3006. After the predefined time, the controller can reevaluate at decision block 2618, 3008 whether excess moisture is present as described above with reference to decision block 2618. If excess moisture is still present, the controller can return to step 3004. If excess moisture is no longer present, the controller can exit the process 2610. By exiting the process 2610, the controller can close the one or more valves.

### Chamber Overflow Detection

The controller can alternatively or additionally use the moisture detection disclosed herein to detect chamber overflow. Overflow of water from the chamber to the breathing circuit, for example, the inspiratory conduit, may occur due to excessive filing of the chamber, or tilting of the chamber during, for example, transport within a hospital. The water that overflows into the inspiratory conduit can be detected by any of the moisture detection systems and methods described herein. In some embodiments, additionally or alternatively, moisture sensing may be performed at or near the chamber end of a dry line or gases supply line conduit that is positioned between the gases source and the humidifier, or the chamber inlet itself, which may detect overflow into the dry line conduit. The component in which the at least one moisture detection sensor is located may be located near an outlet of the humidification chamber. The humidifier controller can determine a chamber overflow event based on a comparison between at least one threshold and the at least one value indicative of the amount of moisture present in the component. The at least one threshold associated with the chamber overflow event is higher than a threshold associated with the moisture management methods described above. For example, the controller can detect the change in a variable indicative of amount of moisture. In this application, the controller may detect sharp changes in the gradient of a measured capacitance, which can be indicative of a sudden increase in the amount of water in the inspiratory conduit. Alternatively, the controller can detect an overflow condition based on a consistent/steady high level of measured capacitance. Alternatively, if the controller is adapted to receive information relating to the location(s) of moisture accumulation, the controller may identify an overflow condition on the basis of a proportionally higher measured capacitance at a location proximate to the humidifier compared to a location further along the gases flow path, for example, along the conduit.

Additionally, and/or alternatively, the controller may generate a notification or alarm (for example, audio and/or visual) of the excess moisture and/or overflow condition. The notification or alarm can be raised by the humidifier, and/or output to a connected ventilator, a central monitoring system, remote device, or any other suitable device in communication with the humidifier.

### Humidity Control

Using moisture detection disclosed herein, humidity control may be improved by the controller determining (which can include estimating) a humidity of the flow of gases. The controller can filter the at least one moisture detection signal or signal process to extract a component relating to the humidity component of the signal. The humidity may be a relative humidity, absolute humidity, or dew point. The controller can control the heater of the humidifier and/or conduit based on the humidity to control a target humidity (for example via closed loop control).

When moisture is detected, a humidity sensing algorithm of the controller may assume the gases to be at 100% relative humidity at a known absolute humidity, or use a different control mechanism for controlling humidity. Alternatively or additionally, the controller can determine the dew point based on detection of moisture and the patient end temperature (or other gases temperature in the conduit) actively or passively.

The humidity of the flow of gases can be more finely controlled using moisture detection disclosed herein for humidity control. The controller can control humidity based on the humidity of the flow of gases, and/or simplify humidity control when the humidity of the flow of gases can be determined or estimated. For example, the amount of humidity that may need to be added by the humidification liquid can be more accurately determined and the heater plate power can be controlled more precisely to maximize humidification without exceeding 100% relative humidity using the determined or estimated dew point. In another example, power to the heater wires of the breathing conduit can be more precisely controlled to keep the gases temperature above the determined or estimated dew point, thereby preventing condensation from forming.

Condensation may form when the following equality is true: Gas Temperature = Dew Point Temperature (at 100% relative humidity). The controller may be able to assume that the gases temperature is above the dew point if no moisture is detected and control the operation of the humidifier based on this determination. The humidifier controller can test the above equality passively or actively. For example, the controller can wait for condensation to form (passive), or the controller can actively induce condensation to occur (active) to determine or estimate the dew point temperature. A passive approach may include periodically polling or otherwise checking a moisture detection module of the controller, while the active approach may include transiently lowering power to the heater wire to drop the gases temperature by a small amount that is sufficient to induce the gas-liquid state change for condensate to form. The patient-end temperature, or gases temperature in general, may be monitored concurrently.

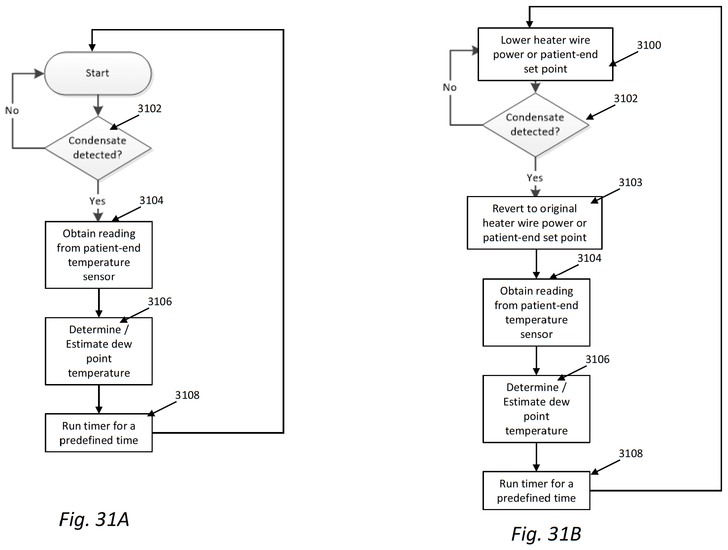

Fig. 31A illustrates an example passive method of determining or estimating the dew point temperature of the flow of gases. The controller can determine whether a predetermined level of moisture is present at decision block 3102 using any of the moisture detection systems or methods disclosed herein. After the humidifier determines that a predetermined level of moisture is present at decision block 3102, the controller can obtain readings from the patient-end temperature sensor 3104 at step 3104. The controller can then determine or estimate the dew point temperature of the flow of gases at step 3106. The determined or estimated dew point temperature may be the reading from the patient-end temperature sensor. The controller can wait for a period of time using a timer at step 3108 before restarting the passive method by returning to step 3102.

Fig. 31B illustrates an example active method of determining or estimating the dew point temperature of the flow of gases. The method in Fig. 31B includes all the steps of the passive method in Fig. 31A in addition to that the controller is configured to lower the heater wire duty cycle or power or the patient-end temperature set point at step 3100 before decision block 3102. This step can induce formation of condensate. For example, the patient-end temperature set point can be reduced from about 37°C to about 36°C, about 35°C, about 34°C, etc. The controller can revert to the original heater wire duty cycle or power or the patient-end temperature set point (that is, the operating parameter values prior to implementing the method in Fig. 31B) at step 3103, as soon as moisture is detected at decision block 3102. The temperature reading from the patient-end temperature sensor is read concurrently with moisture detection, which may allow for a specific temperature to be correlated to the point at which condensate begins to form.

Two further applications of humidity sensing, gases source or room-entrained air detection and humidity detection with the vapor permeable and/or liquid permeable materials will be described in greater detail below.

### Gases Source / Room-entrained Air Detection

Unlike dry air from a compressed gases source (for example, a bottle or wall source), ambient or "room" air has an intrinsic level of humidity. The humidifier may operate at a selected power level which would not be expected to result in condensation if the gases source is a bottle or wall source, but would result in condensation if the gases source is a room air entraining ventilator. The humidifier may not have a humidity sensor to measure the humidity of the room air before the humidifier begins warming the humidifying liquid contained by the humidification chamber. When a room-entraining ventilator (for example, a ventilator that entrains room air) is used with the humidifier, the humidifier does not know the incoming humidity and may add an excessive amount of absolute humidity/moisture to the flow of gases flowing through the chamber. The excess amount of moisture added to the flow of gases can increase condensate formation in the inspiratory tube.

The moisture detection of the present disclosure can be used to detect whether a bottle or wall source, or room air entraining gases source is used with the humidifier. For example, humidity of the ambient air can be estimated from a capacitance between conductors before the humidifier begins warming the humidifying liquid contained by the humidification chamber. Alternatively, the humidifier may initially operate for a predetermined period at a selected power level which would not be expected to result in condensation if the gases source is a bottle or wall source, but would result in condensation if the gases source is a room air entraining ventilator. The type of gases source may be determined by measuring the capacitance between conductors in the inspiratory tube after the predetermined period.

Furthermore, based on detection of the gases source, humidity control may change. In normal control, the controller may control to a target humidity, for example a particular dew point of 37°C dew point temperature. The controller may assume that incoming gases have no humidity. The controller may also set the patient-end temperature at a temperature above the dew point (for example, 40°C, which may be 3°C above the dew point). If condensation occurs under these circumstances, then the target humidity may be too high because incoming gases may have humidity present and the gases sources may be a room entraining ventilator. If the gases source is a room entraining ventilator, the controller may execute a control based on the chamber outlet set point as the controller may assume that the chamber outlet setpoint is equal to the dew point, assuming 100% relative humidity. The controller may then also apply a chamber outlet temperature limit for example 40°C, 42°C or 44°C. If the gases are dry, the controller can execute the normal humidity control (i.e. controlling to a specific dew point).

To provide a suitable level of humidification, the controller can vary the chamber outlet temperature to a desired dew point temperature depending on the chamber inlet temperature. For example, if the temperature of the gases flow at the chamber inlet is measured to be 37°C, the controller can control the chamber outlet temperature to be at least 37°C or higher to sufficiently humidify the gases. As noted above, the temperature of the gases equals the dew point when moisture is detected. Using this information, it may be assumed that it is safe to control the patient-end temperature to (for example) 37°C to maintain the same, sufficient level of humidity.

### Vapor Permeable and/or Liquid Permeable Conduit

Additionally or alternatively, detection or sensing of humidity, relative or absolute, can be used to control the breathability of the bead (or any other component including the vapor permeable and/or liquid permeable material) to remove moisture, for example, by changing the operating parameters of the heater wire as described above with reference to Figs. 26 and 29. For example, the capacitance-based moisture detection systems and methods (or any other moisture detection systems and methods disclosed herein) may be tuned to detect humidity (in addition or alternatively to detecting moisture).

As described above, the magnitude of capacitance or changes thereof (that is, the threshold) are multiple orders greater for moisture detection when compared to humidity sensing. The humidifier system may include multiple filters to select or reject frequency components (for example, RC, RL, LC, or RLC, or other circuits including a combination of passive and/or active energy-storing components) that could be selectively connected to capacitive sensing hardware, each tuned for the purpose of sensing moisture or gases humidity respectively. One or more controllable switches can switch these circuits on or off as needed. The filters, for example, tuning circuits, may be implemented digitally in software.

As described above, one factor for some vapor permeable materials in determining water transfer rate across the material is at least a function of the relative humidity ("RH") gradient between the environments inside and outside of the conduit. Water tends to move from areas with higher RH to lower RH. Therefore, the humidity sensing hardware and the controller can be used to ensure that the RH inside, and along, the conduit is always higher than the outside so that water moves in the direction from inside the conduit to outside the conduit. Using the dew point temperature determination or estimation disclosed herein, the controller can maintain or control this RH gradient along the length of the conduit to maintain or control breathability along the conduit. For example, the heater wires can be powered to maintain a baseline RH of the gases that can be above an assumed RH level of the atmosphere based on humidity sensing disclosed herein.

### Sensor Substitution/Additional Feedback

Additionally or alternatively, the controller can use moisture sensing and/or humidity detection disclosed herein to obtain other information which might not otherwise be available without particular sensors. The ability to obtain such information can reduce the number of sensors needed to effectively control gases delivery, which may provide cost-saving benefits and reduced system complexity.

### Enthalpy Estimation

Enthalpy, such as specific enthalpy, is a function of the dew point temperature. Latent heat, which is the more significant contributor of enthalpy than dry heat (that is, based on the temperature of the gases), can be calculated or inferred from the dew point temperature, for example, obtained from step 3106 in Figs. 31A-31B. The dew point temperature can be calculated or estimated by implementing the humidity sensing process described above, for example, with reference to Figs. 31A-31B. In one example, the enthalpy can be estimated using the following equation:$specific humidity of a gas = \left({M}_{wv}/{M}_{air}\right) \left({P}_{sat}\left({T}_{d}\right)/\left(P-{P}_{sat}\left({T}_{d}\right)\right)\right.$ where M_{wv} is the molecular weight of water vapor, Mₐᵢᵣ is the molecular weight of dry air, Pₛₐₜ(T_{d}) is the saturation vapor pressure at dew point, and P is the atmospheric pressure (assume 101.325 kPa). Pₛₐₜ(T_{d}) can be calculated using the equation Pₛₐₜ(T_{d}) = (611.21)x e^((18.678-T/234.5)(T/(T+257.154))), where T is the temperature of the gases in °C.

The controller can determine estimated enthalpy based on the determined or estimated dew point and control the dew point temperature target to reduce enthalpy delivered to the user if needed. This enthalpy estimation may be used in the heating element (the heater plate and/or heater wires) control system(s) of the humidifier controller to ensure enthalpy exceeding one or more safety limits is not delivered to the patient in normal use scenarios. Examples of the safety limits can include the standards-defined safety limits. Additionally, or alternatively, the enthalpy estimation can be coupled with additional or existing sensors (for example, temperature and/or flow rate sensors) to create a more robust functional safety system for single-fault scenarios.

### Feedback Control for Circuits without Patient-end Sensor(s)

For circuits without a patient-end sensor (for example, a temperature sensor), there may not be information relating to the gases delivered at the patient end available to the controller. However, the controller may determine the required heater wire duty cycle/power based on multiple factors. These factors may include flow rate, target patient-end temperature, chamber outlet temperature, and/or difference between the target patient-end temperature and chamber outlet temperature. By employing appropriate moisture detection technologies disclosed herein, an input signal may be made available to the controller.

Depending on the implementation, the input signal may include information relating to one or more of: presence of moisture (for example, above a threshold), moisture quantity/volume, and locations where moisture is present (as disclosed herein).

In one example, when moisture (for example, condensate) is detected, the heater wire power can be increased to reduce moisture. This power adjustment can be wound down when the quantity of moisture detected falls below a threshold. The threshold may also gradually wind down based on a computed or pre-determined rate such that the humidifier system ensures a sufficient quantity of moisture would evaporate or be according to one or more of the moisture management measures disclosed herein.

### Inspiratory Tube Heating Element Power Control Without Requiring Patient End Sensor Input

In some examples, a sensor at a patient end may not be required if the system can detect moisture (for example, condensate) has formed in the inspiratory tube because the detection of moisture is a measure of the condition of gas. This may be beneficial in situations where it may be undesirable to use a sensor at the patient end. These situations may include scenarios where the sensor may be affected by the environment it is in. For example, a temperature sensor at a patient end may be affected by its placement in an incubator, thus causing a reduction in heating to the inspiratory tube. This reduction in heating to the inspiratory tube can lead to condensation forming, which may be undesirable. Another example reason to not have a patient-end temperature sensor would be to reduce the cost of the conduit, which would be beneficial so long as patient safety and good performance of the system could still be controlled.

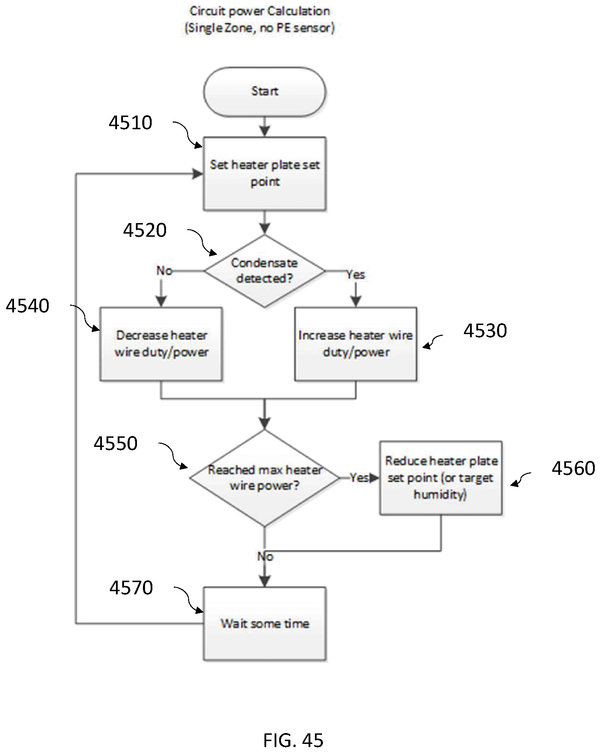

Fig. 45 illustrates a process or method to control power to a heating element of the inspiratory tube (e.g., a heater wire) in response to detection of moisture. The method can involve providing a maximum heater wire power. Once moisture (for example, condensate) is no longer detected, or the presence of moisture (for example, condensate) has fallen below a predetermined threshold, normal control may be resumed. As shown in Fig. 45, at block 4510, a controller of the humidifier system may set a heater plate set point (for example, a temperature set point). At block 4520, the controller may determine whether moisture (for example, condensate) is detected. This determination may include the controller measuring a condensate metric. This determination may further include the controller determining whether the measured condensate metric satisfies an expected condensate metric (for example, by being greater or below a predetermined threshold or threshold range). This determination can be made without input from a sensor at a patient end or at the outlet of the chamber of the humidification system. The measured condensate metric may not satisfy the expected condensate metric if the measured condensate metric is greater than a predetermined threshold or threshold range. If the controller detects moisture (for example, when the measured condensate metric is greater than the predetermined threshold), at block 4530, the controller may increase power to the inspiratory tube heating element. If the controller does not detect moisture (for example, when the measured condensate metric is less than the predetermined threshold), at block 4540, the controller may decrease or maintain power to the heating element. At block 4550, the controller may determine whether power to the inspiratory tube heating element is at a maximum. If power to the inspiratory tube heating element is at the maximum, at block 4560, the controller may reduce a setpoint of the heater plate of the humidifier of the humidifier system when the power to the heating element is at the maximum. This may include reducing the setpoint of the heater plate until the measured condensate satisfies the expected condensate metric. This may also include not further increasing the set point of the heater plate. If power to the inspiratory tube heating element is not at the maximum, at block 4570, the controller may wait for a predetermined period of time before returning to block 4510.

Certain inspiratory tubes may include heating elements arranged such that different sections of the inspiratory tube may be independently energized or controlled (e.g., increase power to that section) to remove moisture in a certain section. An example of such an inspiratory tube is a dual zone inspiratory tube, which may be used in neo-natal applications or other adult applications. The dual zone inspiratory tube may be used with an incubator. The section of the tube intended to be within the incubator may be referred to as the "outer section". The outer section is closer to a patient end of the tube. The section of the tube intended to be placed outside of the incubator may be referred to as an "inner section." The inner section is closer to a chamber end of the tube. As discussed above with respect to the one or more intermediate connectors in Fig. 3A, different section of the tube can be heated depending on the polarity of the section of the tube. the entire tube (including both the inner and the outer sections) is powered by an "outer loop" of a heating circuit that is designed to heat the tube. The inner section is powered by an "inner loop" of the heating circuit. It may be advantageous to measure moisture (for example, condensate) at those different sections of the inspiratory tube. If moisture is detected in the outer section, a controller of the humidifier system may increase power to the outer loop to reduce moisture built up in the outer section. If moisture is detected in the inner section, the controller may increase power to the inner loop instead of increasing power to the outer loop. Only increasing power to the inner loop can reduce the likelihood of overheating the outer section, which may already be at a higher temperature than the inner section due to the outer section being inside the incubator.

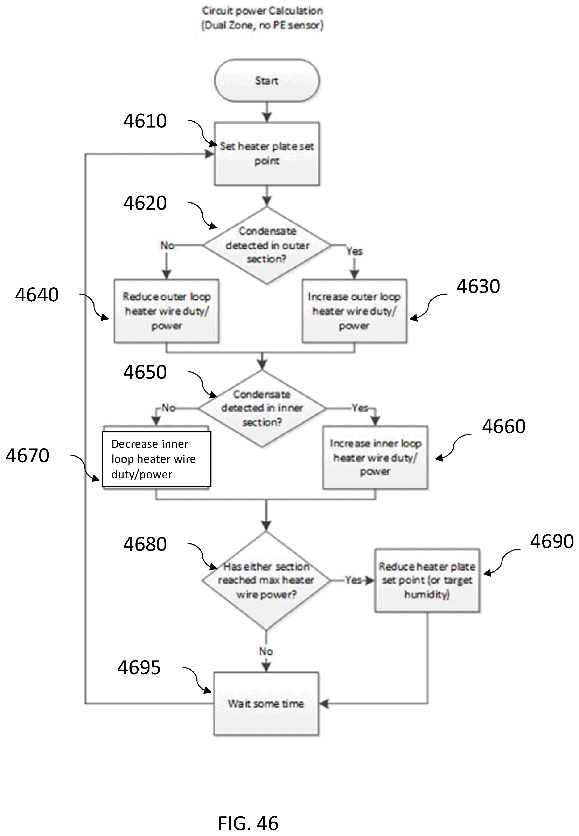

FIG. 46 illustrates a process or method to control power to a heating element of a dual zone inspiratory tube in response to detection of moisture. At block 4610, the controller of the humidifier system may set the heater plate to a set point (for example, a temperature set point). At block 4620, the controller may determine whether moisture is detected in an outer section of the breathing tube. This determination may further include the controller determining whether the measured condensate metric satisfies an expected condensate metric (for example, by being greater or below a predetermined threshold). This determination can be made without input from a sensor at a patient end or at the outlet of the chamber of the humidification system. The measured condensate metric may not satisfy the expected condensate metric if the measured condensate metric is greater than a predetermined threshold. If moisture is detected in the outer section of the breathing tube, at block 4630, the controller may increase the power directed to the heating element of the outer section of the breathing tube. This may increase the power to heat the entire breathing tube. If moisture is not detected in the outer section of the breathing tube, at block 4640, the controller may reduce the power to the outer loop. At block 4650, similar to the detection of moisture in the outer section at block 4620, the controller may determine whether moisture is detected in an inner section of the breathing tube. If moisture is detected in the inner section of the breathing tube, at block 4660, the controller may increase the power directed to the heating element of the inner section of the breathing tube (that is, the inner loop). If moisture is not detected in the inner section of the breathing tube, at block 4670, the controller may decrease the power to the inner loop. At block 4680, the controller may determine whether either the inner or the outer section has reached a maximum heating element power. If the heating element of either section has reached a maximum power limit, at block 4690, the controller may reduce the temperature setpoint of the heater plate of the humidifier. If the heating element of either section has not reached the maximum power limit, at block 4695, the controller may wait a predetermined period of time before returning to block 4610.

### Flow Sensor Replacement

The humidifier controller may no longer need flow sensor inputs for various humidity performance controls and water-out detection, as the ability to sense/infer the dew point temperature using humidity sensing disclosed herein allows the humidifier controller to control operation of the humidifier based on the saturation level of the flow of gases, regardless of the flow rate. As such, the chamber outlet flow sensor may be removed, which has benefits including cost savings and improving ease of manufacture.

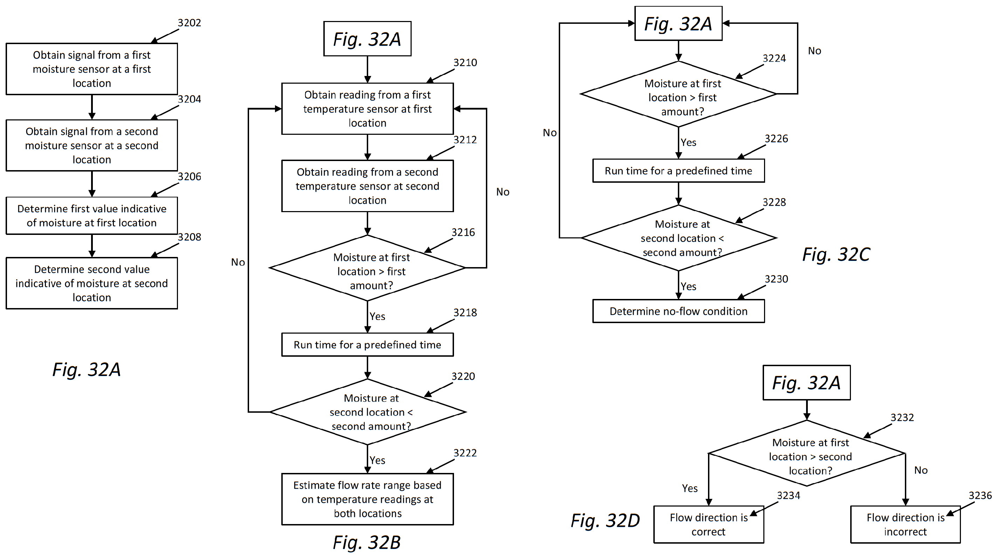

In addition, if the controller has information relating to at least two locations in the breathing circuit where water has condensed, and knowledge of a temperature gradient along the breathing conduit, the controller may determine the flow rate. As shown in Fig. 32A, the humidifier controller may receive at least one first sensor signal from a first sensor associated with a first location at step 3202 and at least one second sensor signal from a second sensor associated with a second location at step 3204. Each sensor signal can be indicative of the level or amount of moisture at the first or second location. The controller can determine a first value indicative of the level or amount of moisture at the first location based on the at least one first signal at step 3206 and a second value indicative of the level or amount of moisture at the second location based on the at least one second signal at step 3208. Each location can be associated with a different component of the humidifier system. The component can include one or more of a conduit or a portion thereof, a connector (which may connect two conduits or be a wye piece that connects an inspiratory conduit, an expiratory conduit, and a patient interface supply conduit), an adaptor, a portion of the humidification chamber that is exposed to the flow of gases, and/or the patient interface. The first location can be upstream of the second location during normal operation of the humidifier system. For example, the first location may be the chamber outlet. The second location may be at or near the patient end of an inspiratory conduit or a distal end of a patient interface supply conduit.

As shown in Fig. 32B, after having completed the steps in Fig. 32A, the controller can additionally receive readings from temperature sensors at the first and second locations respectively at steps 3210, 3212. If the controller first detects a first amount of moisture at the first location at decision block 3216, the controller can wait a period of time using a timer at step 3218 before proceeding to decision block 3220 to determine whether the moisture level or amount at the second location is below a second amount. If the level or amount of moisture at the second location is below the second amount at decision block 3220, the controller can determine that the flow rate of the gases is within a certain range at step 3222 based on the temperature readings at the first and second locations.

In an example, the controller may begin by detecting moisture formation in a breathing circuit zone and compare the patient-end temperature of that zone with an expected temperature. If the patient-end temperature of that zone is measured higher than a chamber-end temperature set point, assuming the gases leaving the chamber outlet are saturated, moisture (for example, condensate) may form increasingly further from the chamber outlet as the flow rate increases. Therefore, based on the locations where moisture is detected, the flow rate may be determined.

### No-Flow Detection

Additionally or alternatively, the controller may use moisture detection disclosed herein or sensed humidity levels to indicate that the humidifier is operating without flow (or with very low flow) being provided from a gases source, which can be a ventilator, a flow generator and/or the like.

As shown in Fig. 32A, the humidifier controller may receive at least one first sensor signal from a first sensor associated with a first location at step 3202 and at least one second sensor signal from a second sensor associated with a second location at step 3204. Each sensor signal can be indicative of the level or amount of moisture at the first or second location. The control can determine a first value indicative of the level or amount of moisture at the first location based on the at least one first signal at step 3206 and a second value indicative of the level or amount of moisture at the second location based on the at least one second signal at step 3208. Each location can be associated with a different component of the humidifier system. Additionally, the first and second location may be separate locations of the same component (e.g., a chamber end and a mid-point or patient end of a conduit). The component can include one or more of a conduit or a portion thereof, a connector (which may connect two conduits or be a wye piece that connects an inspiratory conduit, an expiratory conduit, and a patient interface supply conduit), an adaptor, a portion of the humidification chamber that is exposed to the flow of gases, and/or the patient interface. The first location can be upstream of the second location during normal operation of the humidifier system. After having completed the steps in Fig. 32A, as shown in Fig. 32C, the controller can proceed to decision block 3224. If the controller first detects a first amount of moisture at the first location at decision block 3224, the controller can wait a period of time using a timer at step 3226 before proceeding to decision block 3228 to determine whether the moisture level or amount at the second location is below a second amount. If the level or amount of moisture at the second location is below the second amount at decision block 3228, the controller can determine that no flow or a flow rate below a threshold is occurring in the system at step 3230. Due to natural movement of humid gases, moisture can be detected to some extent at the first location, even when there is no forced flow from a flow generator. The amount of moisture at the first location is expected to be higher than the second location.

In an example, upon successful detection of a first amount of moisture at the chamber-end of the breathing conduit, the chamber inlet, or the chamber outlet, the controller may allow a pre-determined time-period to elapse before checking whether a second amount of moisture is detected at the patient end. If the second amount (which may be a similar level as the first amount) of moisture is found at the patient end of the breathing conduit, the humidifier controller may assume that flow is present in the system. If the detected moisture is below the second amount or if there is an absence of moisture, it is likely that there is zero flow of gases through the breathing conduit, and the controller can generate a notification or trigger an alarm or alert accordingly. The notification or alarm may be displayed on the humidifier system or another device as disclosed herein.

If multiple moisture detection locations are provided (for example, along the breathing circuit), the above method may have improved accuracy, sensitivity, and/or detection times. The multiple moisture detection locations can be provided by including at least a third sensor providing a third signal based on the level of moisture present at a third location. The third location may allow for confirmation of no flow. The third location can be upstream of both the first and second location. For example, the first location may be at an inspiratory conduit; the second location may be at a chamber inlet; and the third location may be at an expiratory conduit.

This no-flow detection method may also be used as part of the humidity sensing method disclosed herein, for example, described with reference to Figs. 31A-31B. The estimated level of humidity at the patient end, for example, the estimated or determined dew point temperature, may be compared to the estimated level of humidity at the humidification chamber outlet. If the humidity at the patient end is significantly lower, this may suggest that there is no flow in the system. As a result, the controller may generate a notification and/or alarm as described above.

### Reverse Flow Detection

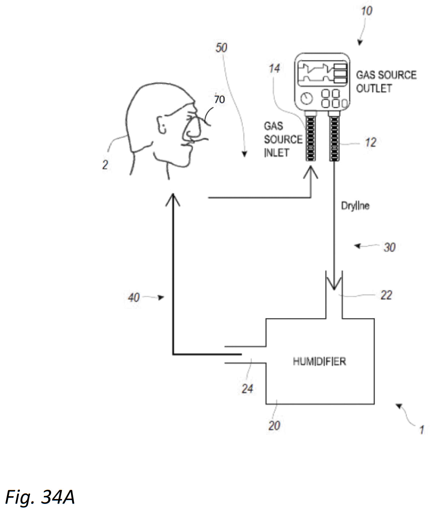

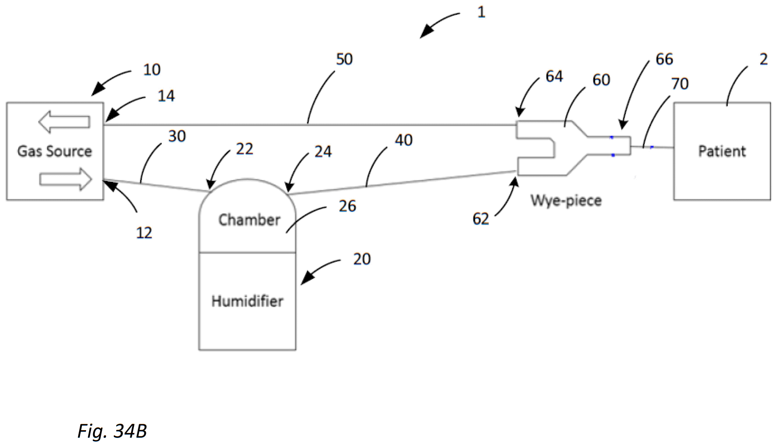

Additionally or alternatively, reverse flow detection may also be performed using at least one of the moisture detection methods. Reverse flow may be due to incorrect connection. Figs. 34A and 34B illustrate example humidifier systems with correct connections. The gases can flow in the direction of the arrows indicated in Figs. 34A and 34B. The humidification system 1 can include a gases source 10 in liquid communication with a humidifier 20 via a dryline conduit 30. In this disclosure the example of a gases source is a ventilator. Other gases sources may also be contemplated, for example, a wall gases source or a compressed gas tank. The dryline conduit 30 can be a tube that is shaped and configured to transfer gases from a gases source to the humidifier 20. The dryline conduit refers to a conduit (for example, a tube or a corrugated tube) that is used to transport unhumidified (for example, dry or ambient) gases from the gases source to the humidifier 20. When correctly connected (that is, in an operational configuration) the dry line conduit pneumatically connects between the gases source and the humidifier. The humidifier 20 can include various components, including, for example, a chamber 26 (see Fig. 34A), and a heat source. The heat source can include a humidifier heat source that is used to heat the contents of the chamber 26 in order to vaporize the contents of the chamber 26 such that the gases flowing through the chamber can be humidified by the vaporized contents. In one example the heat source is used to heat and vaporize water to humidify the gases. The heat source also heats the gases passing through the chamber 26 to a desired temperature (for example, a therapeutic temperature). Examples of the humidifier heat source can include chemical heaters, radiant heaters, induction heaters, and the like. By way of example, the heat source can be a heater plate using a resistive heater. The humidifier 20 can also optionally include one or more processors, such as hardware and/or software processors. The humidifier 20 can include a controller that can include one or more processors and memory. The controller can control operation of the humidifier 20, for example, the steady state operation of the humidifier 20. The gases source can be single direction gases sources, blower, ventilator unit, compressed air tanks, hospital wall gases sources, oxygen bottles, or pressurized gas bottles. Some gases sources may provide gases to the patient and withdraw gases from the patient, especially when the patient is sedated to simulate breathing and to encourage gases exchange within the patient. These systems generally require an inspiratory conduit and an expiratory conduit. The gases source can also include a high flow gases source configured to deliver high flow of air or gases, for example, in excess of 30L/min and/or up to 150L/min. The gases source can be configured to deliver gases flow rates in other ranges e.g. for neonatal applications the flow rates can be less than 8L/min. Gases supplied by the gases source can include either dry air, ambient air, oxygen, and/or a mixture of gases (for example, therapeutic gases or breathing gases). One or more controllers can control the gases source 10 to generate a gases flow at a desired flow rate, temperature, and/or pressure. The gases from a gases source outlet 12 can comprise dry gases.

The dry gases can be provided to a humidifier inlet 22 via the dryline conduit 30. The chamber 26 of the humidifier 20 can contain a liquid, such as water. The humidifier 20 can have a heat source such as a heater plate for vaporizing the water to humidify and heat the dry gases from the dryline conduit 30. Water can be supplied to the humidifier 20 from a water source. The humidified gases can leave a humidifier outlet 24 and enter an inspiratory conduit 40. The humidifier inlet 22 and outlet 24 can be the humidification chamber 26 inlet and outlet, respectively.

The inspiratory conduit 40 (that is, gases delivery conduit) can provide the humidified gases to a patient 2. The inspiratory conduit 40 can be a gas delivery conduit that carries gases from the humidifier to a patient interface 70. The inspiratory conduit 40 (that is, gas delivery conduit) can pneumatically couple the patient interface 70 and the humidifier 20. The inspiratory conduit 40 can be coupled to a patient interface 70. Although the patient 2 is illustrated as wearing a mask in Figure 1, the patient 2 can be wearing other types of patient interfaces 70 disclosed herein, such as a nasal cannula or an endotracheal tube. The patient interface 70 can also comprise an interface tube, which is a short section of tube, which may be heated or unheated, and the inspiratory conduit 40 can be coupled or connected to the interface tube. The short section of tube may be a breathable tube. The short section of tube can decouple the patient interface from the inspiratory conduit to prevent the patient interface from being dislodged. Alternatively, the inspiratory conduit 40 can be coupled to a wye-piece 60 (see Fig. 34B) at an inspiratory conduit connection port 62. The wye-piece 60 can be connected to the patient interface 70 at a patient interface connection portion 66. The inspiratory conduit 40 can have an inspiratory heat source to reduce or prevent condensate formation. Examples of the inspiratory conduit heat source can include a heater wire, heating tape, and/or water jacket heating. The humidification system 1 can include an expiratory conduit 50 (that is, a gases transport conduit or expired gases transport conduit). The expiratory conduit 50 can be a gas transport conduit that directs gases away from the patient. The expiratory conduit 50 can direct expired gases away from the patient and transport the expired gases to the gases source or to some other device (for example, a vent) that may release the gases to atmosphere. The expiratory conduit 50 can direct gases expired from the patient 2 back to a gases source inlet 14. The expiratory conduit 50 can include an expiratory conduit heat source, such as a heater wire, heating tape, and/or water jacket heating. Optionally, the expiratory conduit 50 can be formed of a vapor and/or liquid permeable material such that moisture within the expired gases are transferred from the expired gas to the atmosphere. In this regard, gases can be dried while travelling through the expiratory conduit 50. The expiratory conduit 50 can be coupled to the patient interface 70 via the wye-piece 60 at the expiratory conduit connection portion 64.

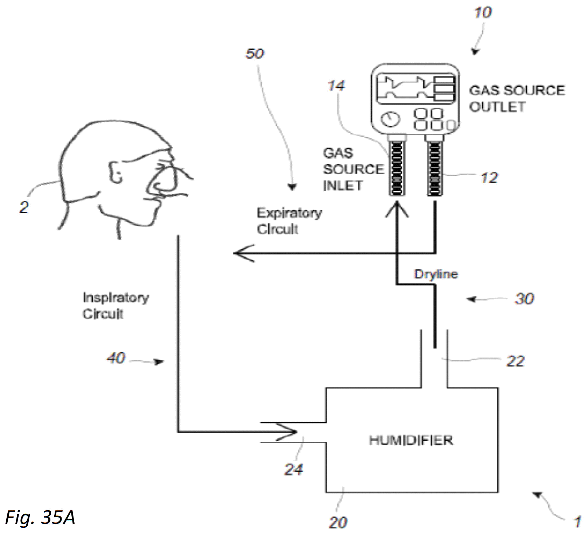

Figs. 35A-35D illustrate example humidifier systems with incorrect connections. These are examples only, and it should be appreciated that other conduit incorrect connections may be possible, such as connecting conduits backwards and/or in different locations in the circuit to where they should normally be connected.

As shown in Fig. 35A, connections of the dryline conduit 30 and the expiratory conduit 50 with the gases source 10 are reversed. Specifically, the dryline conduit 30 is incorrectly coupled to the gases source inlet 14 and the expiratory conduit 50 is incorrectly coupled to the gases source outlet 12. As a result, the dry gases can flow directly to the patient 2 in the expiratory conduit 30 without being humidified or heated because the dry gases do not pass through the humidifier 20. The dry gases can be heated by an expiratory heat source in the expiratory conduit 50, wherein the heating is not properly regulated by the controllers. Expired gases from the patient 2 can become humidified through the humidifier 30 before returning to the gases source 10, resulting in condensation forming in the gases source 10.

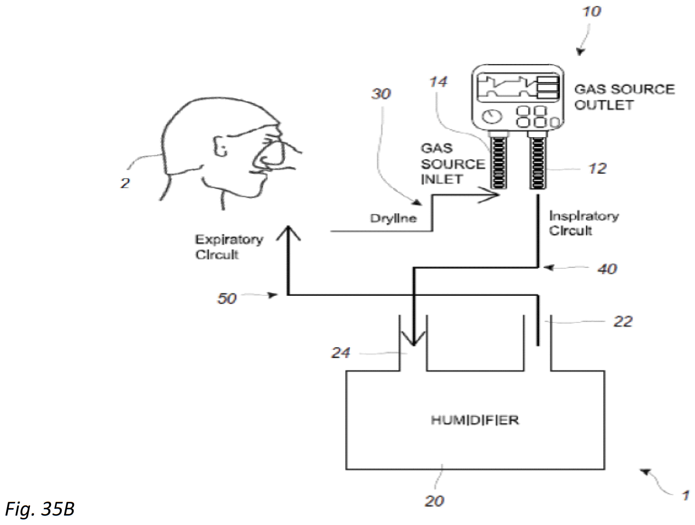

As shown in Fig. 35B, there is an incorrect flow condition in the humidifier 20. Specifically, the inspiratory conduit 40 is incorrectly coupled to the gases source outlet 12 and the humidifier outlet 24, rather than humidifier outlet 24 and patient 2. The expiratory conduit 50 is incorrectly coupled to the humidifier inlet port 22 and the patient 2, rather than patient 2 and gases source 10. The dryline conduit 30 is incorrectly coupled to the patient 2 and the gases source inlet 14, rather than gases source outlet 12 and humidifier inlet 22. The system receives outputs from the patient end sensor in the inspiratory conduit 40 and the sensors at the humidifier inlet and/or outlet 22, 24 that are not indicative of the actual patient end temperature, and/or inlet/outlet temperatures. The humidifier heat source and the inspiratory heat source may not function properly because of the incorrect outputs from the sensors, due to the incorrect feedback from the sensors. The gases leaving the humidifier inlet 22 for the patient 2 may not be heated because the expiratory conduit 50 may not have a heating wire, or may be heated by an expiratory conduit heat source in the expiratory conduit 50, wherein the heating is not properly regulated by the controllers. The expiratory conduit 50 transporting humidified gases from the humidifier 20 can cause the gases to lose moisture (that is, be dried out) before reaching the patient 2.

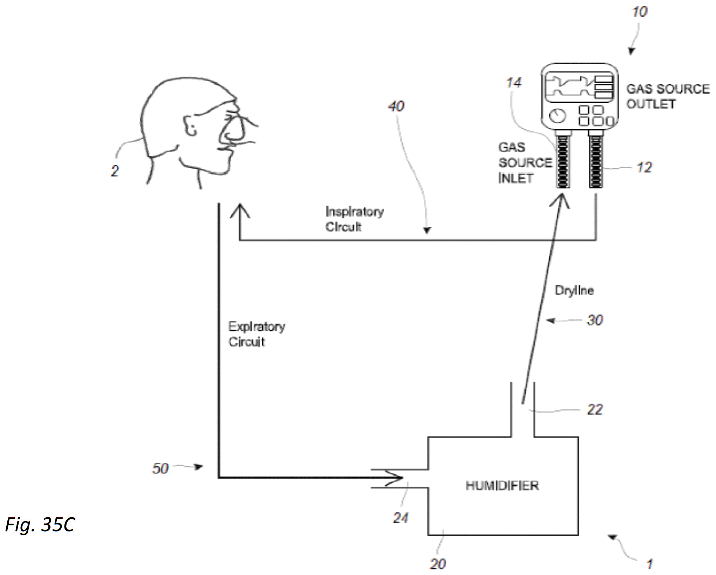

As shown in Fig. 35C, one of the errors includes connections of the gases source inlet 14 and outlet 12 being reversed. Specifically, the dryline conduit 30 is incorrectly coupled to the gases source inlet 14 and the humidifier inlet 22. Another error is that the expiratory conduit 50 is incorrectly coupled to the humidifier outlet 24 and the patient 2. Another error is that the inspiratory conduit 40 is incorrectly coupled to the patient 2 and the gases source inlet 12. As a result, the dry gases can flow directly to the patient 2 in the expiratory conduit 30 without being humidified or heated because the dry gases do not pass through the humidifier 20. The expired gases from the patient 2 can become humidified in the humidifier 30 before returning to the gases source 10. The configuration shown in Fig. 35C can result in humidified gases returning to the gases source without any drying of the gases. The excess moisture in the gases can potentially cause damage to the gases source due to condensation.

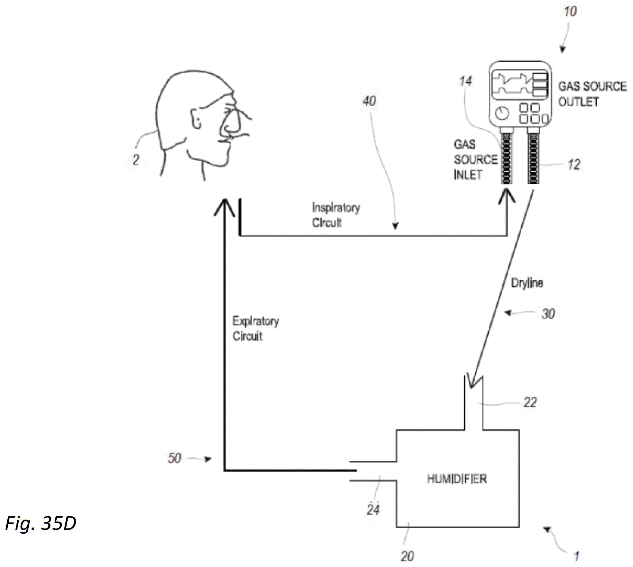

As shown in Fig. 35D, the gases flow in the normal direction, but there are errors in the connections. Specifically, the expiratory conduit 50 is incorrectly coupled to the humidifier outlet 24 and the patient 2. The inspiratory conduit 40 is incorrectly coupled to the gases source inlet 14 and the patient 2. That is, the inspiratory conduit 40 and expiratory conduit 50 have been switched from their intended positions in the breathing circuit. As a result, the patient end sensor in the inspiratory conduit 40 cannot properly measure the temperature of the gases delivered to the patient 2, but measures the temperature of the exhaled gases from the patient 2. The gases leaving the humidifier 20 cannot be heated to ensure that the patient end temperature reaches the patient end set-point. This can be due to the expiratory conduit 50 not having a heat source, or the expiratory conduit heat source not being properly energized by the controllers receiving the patient end temperature input from the sensor in the inspiratory conduit 40. The gases reaching the patient 2 can exceed or fall below the patient end set-point as the gases travel through the expiratory conduit 50. In the case of a breathable expiratory conduit, the gases delivered to the patient will also have sub-optimal humidity. The configuration shown in Fig. 35D can result in humidified gases delivered to the gases source 10. The inspiratory conduit 40 transporting expired gases may not be able to dry the gases like the expiratory conduit 50. Therefore, humidity can be retained in the inspiratory conduit 40 (incorrectly coupled to the gases source inlet 14 and the patient 2) and the moisture can be delivered to the gases source 10. As discussed above, this moisture can potentially cause damage to the gases source 10 due to condensation.

Reverse flow detection using at least one of the moisture detection methods will be described with reference to Fig. 32A and Fig. 32D. As shown in Fig. 32A, the humidifier controller may receive at least one first sensor signal from a first sensor associated with a first location at step 3202 and at least one second sensor signal from a second sensor associated with a second location at step 3204. Each sensor signal can be indicative of the level or amount of moisture at the first or second location. The control can determine a first value indicative of the level or amount of moisture at the first location based on the at least one first signal at step 3206 and a second value indicative of the level or amount of moisture at the second location based on the at least one second signal at step 3208. Each location can be associated with a different component of the humidifier system. The component can include one or more of a conduit or a portion thereof, a connector (which may connect two conduits or be a wye piece that connects an inspiratory conduit, an expiratory conduit, and a patient interface supply conduit), an adaptor, a portion of the humidification chamber that is exposed to the flow of gases, and/or the patient interface. The first location can be upstream of the second location during normal operation of the humidifier system for example as shown in Figure 34B. The first location may detect reverse flow if the upstream component humidity is above a predetermined threshold. As shown in Fig. 32D, after having completed the steps in Fig. 32A, the controller can proceed to decision block 3232. If the controller detects that the moisture level at the first (that is, upstream meant to be when connected correctly) location is less than the moisture level at the second (that is, meant to be downstream when connected correctly) location, the controller can determine that the flow direction is correct at step 3234. If the controller detects that the moisture level at the first (that is, meant to be upstream when connected correctly) location is greater than the moisture level at the second (that is, meant to be downstream, when connected correctly) location, , the controller can determine that the flow direction is incorrect at step 3236. As a result, the controller may generate a notification and/or alarm as described above. The first location may be upstream of the humidifier and the second location may be downstream of the humidifier. The upstream location may be the dryline and the downstream location may be the expiratory conduit.

Additionally or alternatively, the controller can receive a third sensor signal from a third sensor based on the moisture level at a third location. In an example, the controller may measure the levels of moisture at three locations: on the dry line; the inspiratory circuit, and the expiratory circuit. The dry line may be by the chamber inlet. In the case of reverse flow, the level of moisture in the expiratory circuit and inspiratory circuit may be lower than the level of moisture detected at the chamber inlet. The difference between detecting a water-out condition and a reverse flow condition is that in the reverse flow case, humidity at the patient end may still be detectable and/or moisture may still be present at the patient end. In contrast, in the water-out case, the patient end may be drier while the chamber outlet may have some humidity/moisture.

### External Device Communications

When moisture is detected in a component of the humidifier system, the humidifier may communicate with external devices, such as a gases source (for example, ventilators, gas mixers or other suitable flow/pressure generators), in addition or alternative to the moisture management methods disclosed herein. When a sufficient level of moisture has formed in a breathing circuit, the ventilator may detect/categorize the detected moisture as a blockage. Upon detecting moisture, the humidifier controller may control a function of the gases source. For example, the controller may communicate to the ventilator that the type of blockage is a result of moisture in the breathing circuit. This may allow the ventilator to respond accordingly. For example, the ventilator can increase the bias flow in an attempt to spread out the moisture along the conduit and/or to increase the evaporative effect to remove the moisture, or increase the flow rate to push the condensate to the water trap, which may prevent the mobile condensate from affecting the breathing pattern. Additionally or alternatively, the humidifier can directly send this moisture detection data to the ventilator, which can be displayed on the user interface of the ventilator, or such information could be communicated to other devices such as a central monitoring system.

The ventilator may execute additional functions to reduce or prevent condensation. These functions may include but are not limited to, for example, reducing tidal volume, increasing inspiratory rise time, reducing inspiratory to expiratory ratio, increasing the inlet gases temperature to minimize moisture in the circuit, and/or reducing incoming humidity by reducing the amount of entrained room air and/or switching to a "dry" air source (for example, a wall or bottle source).

### Water Out Detection

Because the humidifier controller may monitor moisture (for example, condensate) volume and even optionally the location, the controller may use moisture detection disclosed herein to controllably induce a safe or tolerable volume of moisture, for example condensate, to form for the purpose of detecting a water-out condition. The water out detection may be based on a determination of moisture and/or humidity, for example decreasing below a threshold. The threshold may be similar to the ones disclosed above with reference to moisture management responses and humidity sensing. The controller can detect moisture (for example, condensate) in normal use and identify a decrease in the amount of moisture (for example, condensate) indicative of water out condition. The controller can determine a decrease in the amount of moisture (for example, condensate) over time. In response to detecting the decrease, the controller can increase an operating parameter of the humidifier to increase the amount moisture (for example, condensate) in a component of the humidifier system. If no increase is detected, the controller can determine a water out condition. In some cases where the moisture detection sensor can determine a humidity level in the conduit (or other component, the water out condition may be determined based on a decrease in the humidity in the conduit (or other component). The water-out condition can be due to the chamber having no water or a very small amount (or low level) of water, for example, insufficient for humidifying the gases for the duration (or a remainder of the duration) of a respiratory therapy.

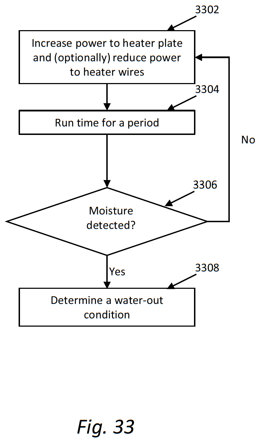

As shown in Fig. 33, to induce the safe or tolerable volume of condensate to form, the controller can change the operating parameter(s) of the humidifier, for example, by increasing power to the heater plate and optionally simultaneously lowering power to the heater wire at step 3302. The changed operating parameters can be maintained for a predetermined period using a timer at step 3304 before the controller proceeds to decision block 3306. If no moisture (for example, condensate) or insufficient level of moisture (for example, condensate) is detected at decision block 3306 after the changed operating parameters have been maintained for a period, the controller may determine that the water level in the humidification chamber is low or empty at step 3308.

The exact changes in heater plate and heater wire power may be pre-determined for a range of humidifier and ventilator operating parameters (for example, flow rate) such that an appropriate threshold for detection of moisture can be set. For example, it may be known that at a certain dew point setting, at steady-state, increasing heater plate power by a percentage of the heater plate power and decreasing heater wire power by a percentage of the heater wire power may induce a safe, small, but detectable quantity of moisture within an expected time frame. Limiting the changes to the operating parameters may be beneficial to minimize disruption to the gases flow delivery under normal operation and/or improve detection speed, accuracy, etc.

Alternatively, the controller may be calibrated such that the detection of (any) moisture (for example, condensate) formed as a result of increasing power to the heater plate and optionally reducing power to the heater wires can be detected quickly, before reaching an unsafe volume such that the changes to the operating parameters need not be as strictly limited. Optionally, this method may be interleaved with any of the moisture (for example, condensate) management (for example, reduction and/or removal) methods disclosed herein.

### Pre-Operation Circuit Condition Detection

The moisture (for example, condensate and/or water in the vapor permeable and/or liquid permeable material) detection methods can be used to detect presence of moisture in a conduit prior to use. When a conduit is first connected to the humidifier, any moisture (or moisture exceeding a small acceptable threshold quantity) is expected to absent in the conduit. The conduit would have been just removed from packaging or have undergone drying procedures if the conduit is reused. The presence of moisture (or moisture exceeding the small acceptable threshold quantity) may be a result of exposure to moisture during storage or transportation, extended exposure to the atmosphere (which contains an intrinsic level of humidity), inadequate drying procedures (in the case of re-used tubes), or the like. As described above, the small or acceptable threshold amount can be experimentally determined based on particular humidifier and/or conduit configurations.

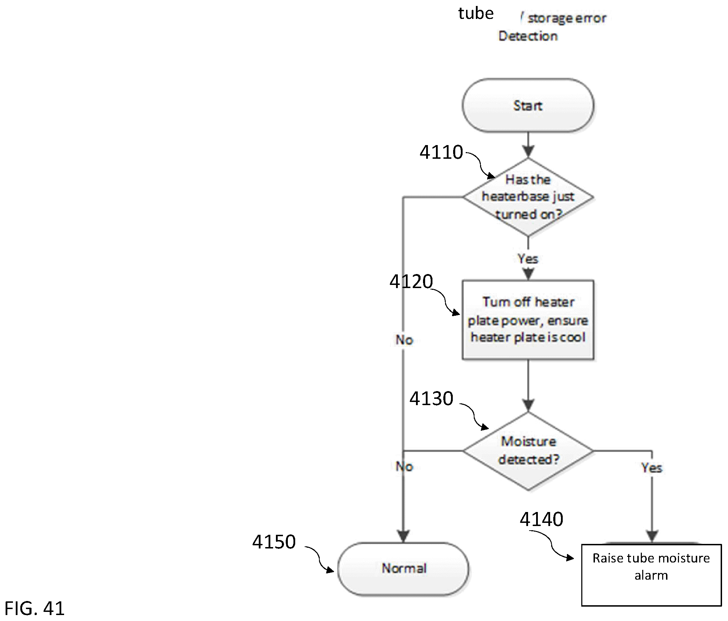

Moisture detection methods may be initiated by the humidifier controller as soon as the conduit is electrically connected with the humidifier. The controller may implement any of the moisture detection methods described previously to determine whether there is condensate or moisture present that may cause the humidifier to function improperly. Fig. 41 illustrates an example process that can be used to detected whether a tube of a humidifier system is contaminated. At block 4110, the controller of the humidifier system may determine a startup condition. In some examples, this may include the controller determining a duration of time for which the humidifier has been powered on. The controller may determine that the duration of time indicates the humidifier has just turned on. The controller may determine a duration of time for which the tube has been connected to the humidifier. In other words, the controller may determine whether the heater base has just powered on. If the startup condition is satisfied (for example, if the heater plate has just powered on and/or moisture is formed), at block 4120, the controller may reduce heating, such as turning off power to the heater plate of the humidifier, to ensure the heater plate is cool or at a sufficiently low temperature. This may include reducing power to the heater plate of the humidifier. At block 4130, the controller may determine whether moisture (for example, condensate) is detected. This may include the controller determining whether a measured condensate metric satisfies an expected condensate metric. In some examples, the measured condensate metric may not satisfy the expected condensate metric if the measured condensate metric is greater or lesser than the expected condensate metric. If moisture is detected, at block 4140, the controller may raise a tube moisture alarm. If moisture above the expected condensate metric is not detected, at block 4150, the controller may resume normal control of the humidifier system. Optionally, prior to block 4150 being performed, the controller may control heating to the tube to remove the condensate that has formed as a result of block 4120.

The example process at the start-up for moisture detection in the inspiratory tube can be implemented in conjunction with any of the other processes of moisture detection in the inspiratory tube disclosed herein. For example, in the no flow/water out detection processes described below, this contaminated tube detection can be implemented during the "wait for device warm-up" block.

The type of problem that may cause moisture to be present in the conduit can be distinguished by applying different thresholds to the detection algorithm. For example, a first threshold can be indicative of extended exposure to the atmosphere and a second threshold can be indicative of inadequate drying of the conduit. The controller may alert the user, for example in the form of a visual and/or audio alarm/message, if there is an undesirable level of moisture present in the conduit. The controller may prevent use of the conduit with the humidifier in response to determining the presence of moisture (or undesirable level of moisture) in the conduit. For example, the controller may prevent powering of the heater of the conduit and/or prevent use of the humidifier while connected to the conduit.

### Gas Type Detection and Room Entraining Vent Detection

Because different gases have different thermal properties, various levels of moisture (for example, condensate) may be present in a humidifier system based on the gas type (or blend) used. Example thermal properties of gases that may affect the presence of moisture for the type of gas can include, but are not limited to, thermal effusivity, thermal conductivity, heat capacity, etc. For example, there may be more moisture formed when a Heliox (a blend or combination of helium and oxygen) gas type is used as the gas in a Heliox mode rather than when air is used as the gas type (such as using a room entraining vent). The increase in presence of moisture when Heliox is used as the gas instead of air may be due to Helium having a higher thermal effusivity than air. Examples of gas types include various Heliox blends, ambient air, air blended with supplemental oxygen in various amounts, other blends of respiratory gases, or the like.

However, the controller of the humidifier system may not be able to determine whether certain detected behaviors or thermal properties are due to the gases of one type being humid or humidified gases, or due to the different gas types used. This is because humid/humidified gases of one type may have similar behaviors or thermal properties to various other gas types. Similarly, this may be because the controller may not be configured to determine multiple types of gases. This may limit the controller to controller the humidifier according to an expected gas type rather than an actual gas type, which can lead to increased condensation, for example. It may be important to distinguish a humid gas from another gas type to determine whether moisture is present and/or providing gases to the patient at desired or preferred conditions (e.g., preventing the formation of condensation). In some examples, the controller may check whether a room entraining vent is connected to the humidifier system. In some examples, the controller may additionally determine the gas type. The user may optionally be requested to enter or confirm the gas type determined. The controller can make those two determinations in any order.

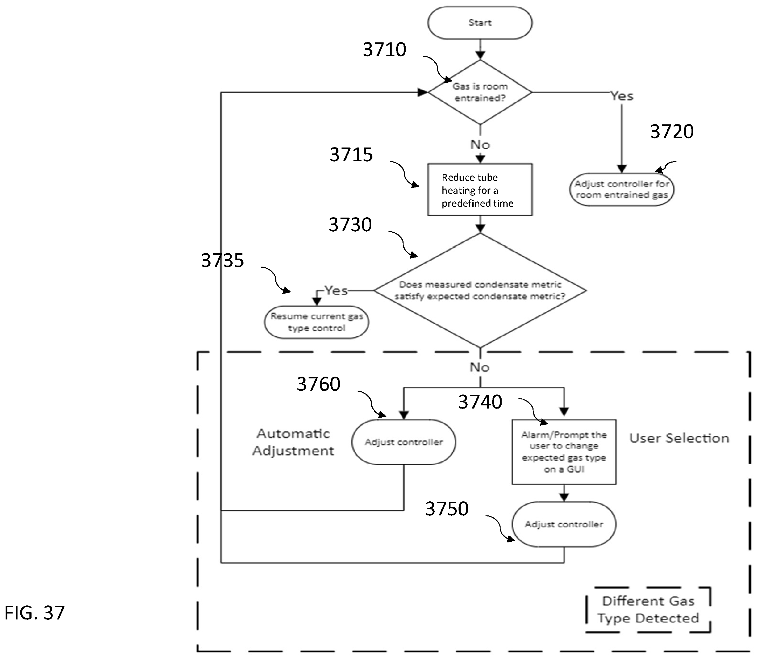

Example gas type detection and room entraining vent detection processes are shown in Fig. 37. As shown at block 3710, the controller of the humidifier system may determine whether the gas provided by the humidifier system is room entrained. If the gas is room entrained, at block 3720, the controller may adjust the operation of the humidifier for room entrained gas. If gas is not room entrained, at block 3715, the controller may reduce (which can include disable) heating of the inspiratory tube for a predetermined time. In some examples, this may include reducing power to a heater wire of the inspiratory tube coupled to the humidifier. This step allows for the gas to cool across the inspiratory tube and for moisture (for example, condensate) to form.

At block 3730, the controller may determine whether a measured condensate metric satisfies an expected condensate metric for the system. This determination can be made by measuring and/or performing any of the moisture (for example, condensate) detection methods as described herein. In some examples, the controller may compare a condensate level detected in the inspiratory tube to a predetermined threshold or threshold range. The predetermined threshold or range may be a function of various gas parameters, including but not limited to flow rate, gas temperature changes, ambient temperature, expected gas properties, etc. The measured condensate metric may not satisfy the expected condensate metric if the measured condensate metric, for example, the condensate level, is greater or lesser than the predetermined threshold or range.

At block 3735, if the measured condensate metric satisfies the expected condensate metric (that is, the amount of condensate formed is as expected), the controller may resume its current gas type control metrics. Optionally, prior to block 3735 being performed, the controller may control heating to the tube to remove the condensate that has formed as a result of block 3715. If the measured condensate metric does not satisfy the expected condensate metric (for instance, if the amount of condensate formed is more than expected), then the system may perform additional steps either automatically (block 3760) or via user selection (blocks 3740, 3750). For example, the controller may determine that Heliox is being used instead of air if an increase in humidity output is detected in the form of more condensation forming in the inspiratory tube. If there's more moisture (for example, condensate) formed, the controller can automatically be adjusted to operate in a Heliox mode at block 3760. Alternatively, at block 3740, upon the determination that the measured condensate metric does not satisfy the expected condensate metric (for instance, that there is more condensate than expected), the controller may alarm and/or prompt the user to change the expected gas type for the system, for example, via the graphic user interface of the humidifier system or the like. At block 3750, the controller can make the adjustment as disclosed at block 3760.

At block 3750 or 3760, the controller may output or further adjust the operation of the humidifier system based on the gas type detected. For example, after the controller has adjusted to operate in a Heliox mode, the controller can additionally disregard readings and/or measurements from a temperature-based flow sensor (for example, a hot wire anemometer) in the humidifier system. The controller can optionally disable the temperature based flow sensor at block 3750. When there is more moisture formed than expected, the temperature based flow sensor may be inaccurate. Instead, the controller can use a different form of calculating the flow rate, for example, using an algorithmic method that does not require further input from sensors.

Additionally or alternatively, the controller may also be adjusted such that the response of a heater plate temperature control is slowed. In some examples, the controller may adjust the PID coefficients, increase the control loop timing, and/or apply a maximum increase in the heater plate temperature setpoint to slow down the response to the heater plate temperature control.

### Detection of No Humidity (No Flow/Water Out/Reverse Flow) Using Heating Element Control

Power to certain heating elements can be reduced (including disabling those heating elements) and/or certain conditions can be determined based on whether moisture (for example, condensate has formed). However, when power to a heating element of a humidifier system is reduced (including disabled) and no moisture (for example, condensation) forms, this may be indicative of many scenarios including but not limited to, a no flow condition or a water out condition, or reverse flow conditions, etc.

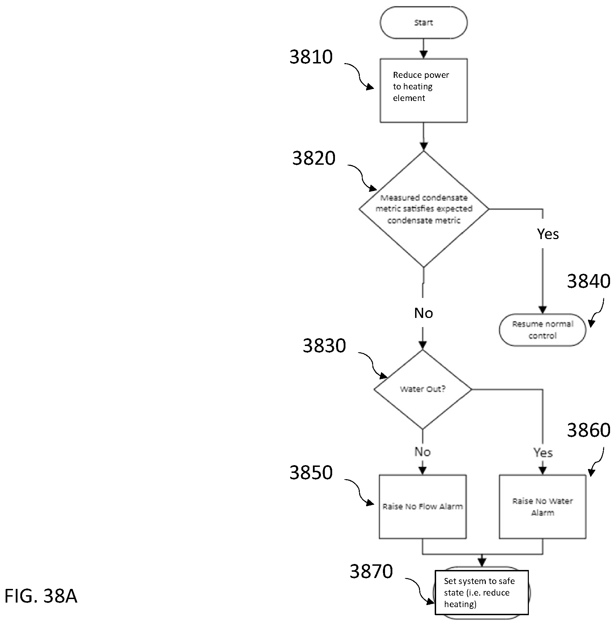

Fig. 38A illustrates an example process to determine whether there is a no flow or water out condition. At block 3810, the system can reduce (including disable) power to a heating element. The heating element may be a heater wire that is connected to the inspiratory tube. The power may be reduced for a predefined period of time. At block 3820, the controller may determine whether a measured condensate metric satisfies an expected condensate metric. With a reduction in power to the heating element of the inspiratory tube, more condensate is expected. That is, condensate may be expected to form as a result of block 3810. If no condensate has formed, or the level of condensate formed is not as expected, it may be inferred that no humidity is being delivered as a result of either no water being present in the chamber or no flow through the system. If the measured condensate metric satisfies the expected condensate metric, at block 3840, the controller resumes normal control (for example, by resuming the previous level of heating of the inspiratory tube). For example, the measured condensate metric may correspond to a measured condensate level and the expected condensate metric may be a predetermined threshold or threshold range. In this example, it may be expected that the measured condensate level may be greater than the predetermined threshold or range as a result of reducing power in block 3810 to the heater wire that is connected to the inspiratory tube.

If the measured condensate metric does not satisfy the expected condensate metric (for example, it is below a threshold or range such that the condensate level in the inspiratory tube is decreasing or at a minimum level) despite the reduced heating of the inspiratory tube, this may be indicative of either no flow through the system or water out in the chamber and the controller proceeds to block 3830. At block 3830, the controller may assess whether a water out condition is occurring. If there is not a water out condition, then the controller can infer a no flow condition. At block 3850, the controller may output a no flow alarm. If there is a water out condition, at block 3860, the controller may output a water out alarm.

Alternatively, the controller may determine whether there is a no flow condition first rather than determining whether there is a water out condition. The controller may determine a no flow condition with use of a flow sensor or an indirect means of determining flow. If there is no flow, the controller can output a water out alarm.

At block 3870 (after outputting either a water out or no flow alarm), the controller may set the humidifier system to a safe state. For example, the controller may discontinue power to heating elements of the humidifier system.

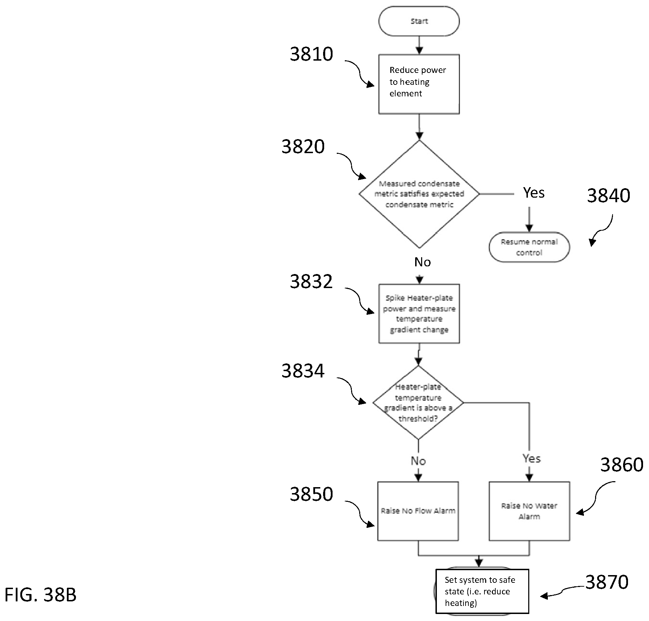

Fig. 38B illustrates an example process to determine whether there is a no flow or water out condition that has the same blocks as Fig. 38A except that block 3830 is replaced by blocks 3832 and 3834. At block 3832, if the measured condensate metric does not satisfy the expected condensate metric, the controller can increase the power to the heater plate of the humidifier and measure a change in the heater plate temperature gradient. The controller can measure the heater plate temperature using a temperature sensor located at or near the heater plate heating element. At block 3832, the controller may determine whether the heater plate temperature gradient is above a predetermined threshold. If the gradient is above the threshold, the controller proceeds to block 3860. If the gradient is not above the threshold, the controller proceeds to block 3850.

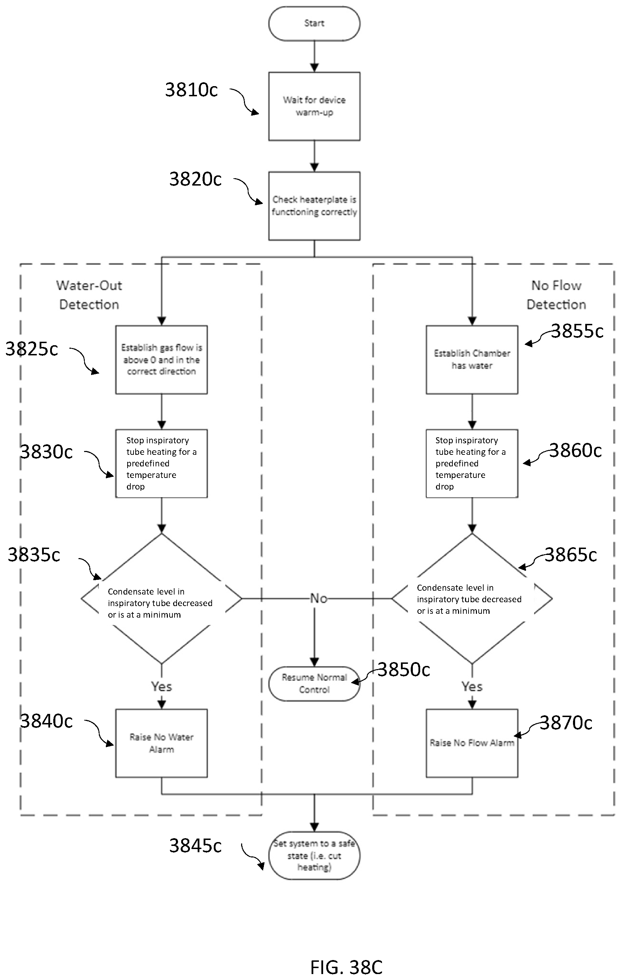

In some examples, the controller can further determine whether no humidity is a result of reverse flow, the humidifier being just powered on or warming up, or malfunctioning of the humidifier (for example, heater plate malfunction), as discussed below. Fig. 38C illustrates an example process for additional fault detection based on no flow/water out detection described above. In addition to detecting no flow or water out conditions, the process in Fig. 37C also includes determining whether the humidifier system is starting up and whether the humidifier system is functioning correctly. At block 3810c the controller of the humidifier system may wait for a period of time to allow the system to warm up. At block 3820c, the controller may check whether the heater plate is functioning correctly before proceeding to water out and no flow detection.

To detect whether a water out condition is present, at block 3825c, the controller may first establish a gas flow above zero and in the correct direction to rule out a no flow condition or a reverse flow condition. At block 3830c, the controller may stop the heating of the inspiratory tube until a predetermined temperature drop is achieved. At block 3835c, the controller may determine whether a condensate level in the inspiratory tube has decreased or is at a minimum level. If the condensate level in the inspiratory tube has not decreased or is not at a minimum level, at block 3850c, the controller may resume normal control (for example, by resuming heating of the inspiratory tube). Optionally, prior to block 3850c being performed, the controller may control heating to the tube to remove the condensate that has formed as a result of block 3830c. If the condensate level in the inspiratory tube has decreased or is at a minimum level, at block 3840c, the controller may output a water out alarm. After outputting the water out alarm, at block 3845c, the controller may set the operation of the humidifier system to a safe state. For example, the controller may discontinue power to heating elements of the humidifier system.

To detect whether a no flow condition is present, at block 3855c, the control may first establish that the humidification chamber has water to rule out a water out condition. At block 3860c, the controller may stop the heating of the inspiratory tube until a predetermined temperature drop is achieved. At block 3865c, the controller may determine whether a condensate level in the inspiratory tube has decreased or is at a minimum level. If the condensate level in the inspiratory tube has not decreased or is not at a minimum level, the controller proceeds to block 3850c. Optionally, prior to block 3850c being performed, the controller may control heating to the tube to remove the condensate that has formed as a result of block 3860c. If the condensate level in the inspiratory tube has decreased or is at a minimum level, at block 3870c, the controller may output a no flow alarm before proceeding to block 3845c.

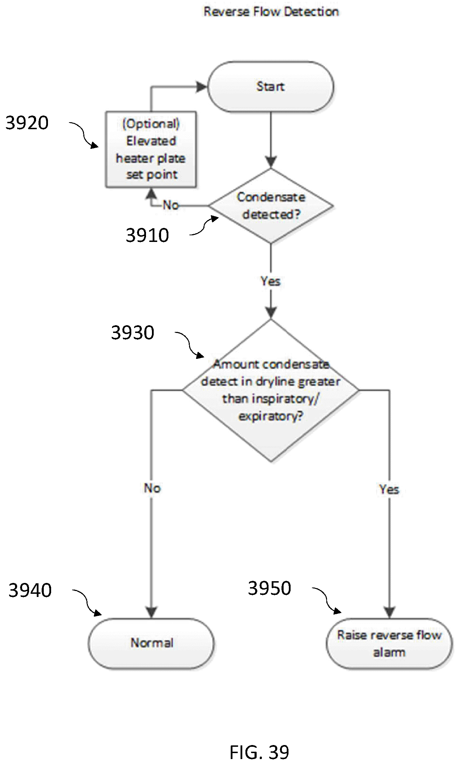

Fig. 39 illustrates a schematic to determine whether no humidity is a result of reverse flow. This process can be incorporated into the processes shown in Figs. 38A-38C for determining a no flow or water out condition. The reverse flow process includes determining whether there is condensate in the dryline or gases supply line which connects the gas source and the inlet of the humidification chamber, and/or whether there is more condensate in the dry line than in the inspiratory or expiratory tubes.

At block 3910, the controller may determine whether moisture (for example, condensate) is detected in the dryline. If condensate is not detected, the controller may return to the start of the process. Optionally, before restarting at block 3910, at block 3920, the controller may optionally elevate the heater plate temperature set point. This step may ensure there may be enough humidity present in the system so moisture (for example, condensate) will form at a location within the humidifier system. If moisture is detected, at block 3930, the controller may determine whether the condensate level detected in the dryline is greater than a condensate level in the inspiratory tube and/or expiratory tube. If the condensate level in the dryline is lower, at block 3940, the controller may resume as normal operation. If the condensate level in the dryline is greater, at block 3950, the controller may output a reverse flow alarm. The alarm may include an audio and/or visual message. *Covered Tube Detection*

During use of the humidifier system, a tube (for example, the inspiratory or expiratory tube) may be covered or partially covered, such as by clothing or bedding. A covered tube may produce and build up humidity within the tube because moisture cannot evaporate from the tube wall as fast as from an exposed tube, which uses a breathable material as disclosed herein. When any accumulated condensate does not evaporate within an expected time frame, this may indicate that the tube is covered. A covered tube can be undesirable. For example, the tube wall material may experience melt when heat cannot dissipate well when the tube is covered.

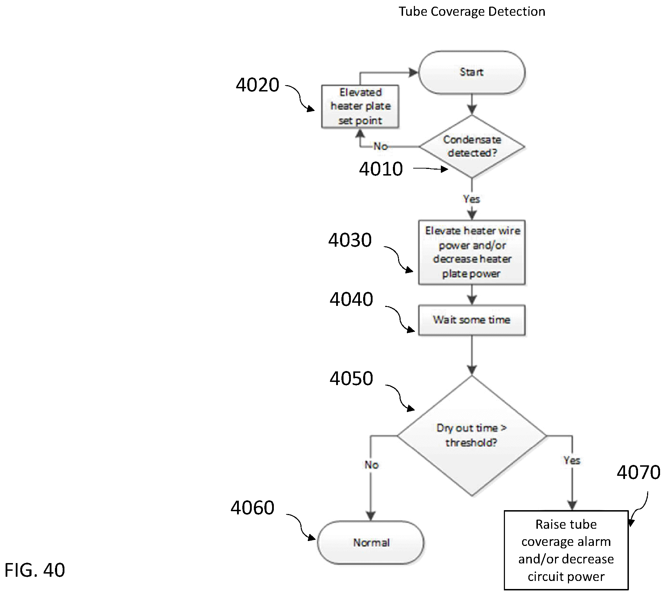

Fig. 40 illustrates an example process to detect whether a tube is covered. At block 4010, the controller may determine whether condensate is detected. The controller may determine whether condensate is detected by comparing a measured condensate metric within an inspiratory tube to an expected condensate metric of the inspiratory tube. In some examples, if the measured condensate metric does not satisfy an expected condensate metric, then condensate may be present. The measured condensate metric may not satisfy the expected condensate metric if the measured condensate metric is greater or lesser than a predetermined threshold.

If condensate is not detected, the controller may optionally proceed to block 4020. At block 4020, the controller may optionally elevate the heater plate temperature set point. This step may ensure there may be enough humidity present in the system so moisture (for example, condensate) will form at a location within the humidifier system. The amount of condensation may be based on at least ambient temperature. Therefore, this process can be more accurate for determining reverse flow when the ambient temperature is not excessively high (for example, not exceeding the bounds of a range between approximately 26°C to approximately 42°C). The controller may be limited in how much the heater plate temperature set point can be elevated to avoid heating the heater plate to a level that may be unsafe for the patient.

If moisture is detected, at block 4030, the controller may increase the power of the heater wire of the inspiratory tube and/or decrease the power of the heater plate. At block 4040, the control may wait a predetermined period of time. At block 4050, the controller may determine a duration of time to dry out condensation in the inspiratory tube, a dry out time. The controller may further determine whether the dry out time is greater than a predetermined threshold or threshold range. If the dry out time is not greater than the predetermined threshold or range (that is, the measured condensate metric satisfies the expected condensate metric), at block 4060, the controller may resume to normal (for example, returning to previous set points for the heater plate and the heater wire). If the dry out time is greater than the predetermined threshold or range (that is, the measured condensate metric does not satisfy the expected condensate metric), at block 4070, the controller may output a tube coverage alarm and/or decrease power to the heater wire of the tube.

### Incubator Detection

As disclosed herein, the tubes of the humidifier system may be partially located in an incubator during use. The moisture (for example, condensate) detection methods disclosed herein can be useful in determining whether an incubator is being used with the humidifier system. The incubator typically has a temperature greater than the ambient temperature. This may correlate with the condensate level present in a tube or a portion thereof within the incubator being lower than in the part of the tube that is outside the incubator. To detect the presence of an incubator, the controller may determine condensate levels in the two sections of the tube: a first section outside of the incubator and a second section inside the incubator. If the second section contains less condensate than the first section, an incubator may be present.

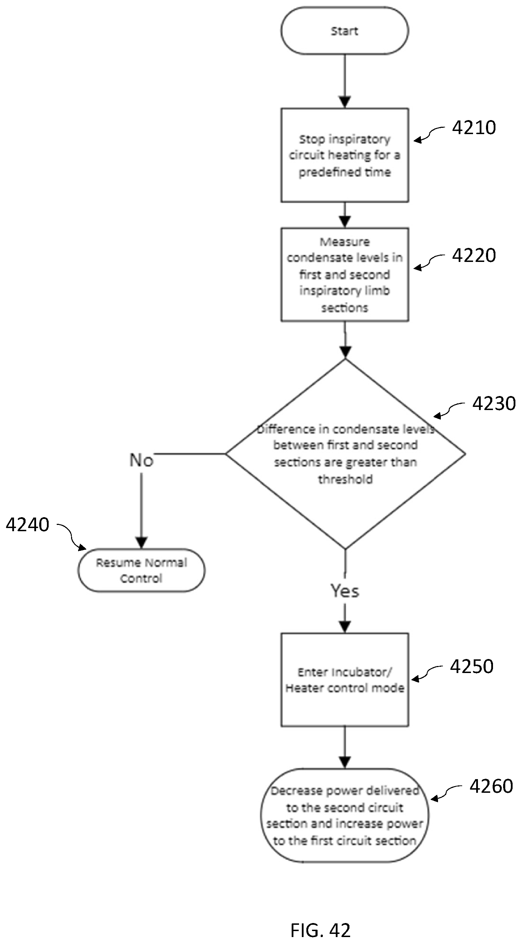

Fig. 42 illustrates an example process or method to determine whether an incubator is present within a humidifier system. At block 4210, the controller may reduce (including disabling) the heating of the inspiratory tube for a predetermined period of time. At block 4220, the controller may measure a first condensate metric in a first section of the inspiratory tube and a second condensate metric in a second section of the inspiratory tube. At block 4230, the controller may determine whether the measured condensate metrics satisfy an expected condensate metric. In some examples, this may include comparing the first condensate metric of the first section and the second condensate metric of the second section. The controller may determine whether the difference between the first condensate metric and the second condensate metric is greater a predetermined threshold or threshold range. If the difference is not greater than the predetermined threshold or range, at block 4240, the controller may resume normal operation of the system (for example, by resuming heating of the inspiratory tube). Optionally, prior to block 4240 being performed, the controller may control heating to the tube to remove the condensate that has formed as a result of block 4210. If the difference is greater than the predetermined threshold, at block 4250, the controller may enter an incubator and/or heater control mode before proceeding to block 4260. At block 4250, the controller may decrease power delivered to the heater wire at the second section and increase power delivered to the heater wire at the first section.

This process can be activated when the humidifier system is operating in a neonatal mode to confirm whether an incubator is being used. If an incubator is detected by the controller and further confirmed by the user (for example, via the GUI of the humidifier), the controller can adjust operation of the humidifier accordingly.

The tube may be further divided into more than two sections. Increasing the number of measurable sections may increase the resolution of the condensate level gradient across the tube. For example, the inspiratory tube may be split into three measurable sections and half of the inspiratory tube may be placed in a heated environment. In other words, two of the three measurable sections may be placed in the heated environment and one of the three sections may be placed outside of the incubator, or vice versa. The section that is completely and/or entirely in the heated environment may have the lowest condensate level. The section completely and/or entirely in the ambient environment may have the highest condensate level. The second partially in the heated environment and partially in the ambient environment may have an intermediate level of condensate.

### Tube Orientation Detection

When in use, the humidifier is generally placed lower in elevation than the patient. This is to ensure that if condensation is formed, the condensation may drain back to the humidifier instead of to the patient. The orientation of the tube may affect the drainage of the condensation so it may be desirable for the controller of the humidifier system to be able to determine the tube orientation. The tube orientation determination can be based on a comparison of the condensate measured from three locations: a patient end, a location within the inspiratory tube that is not near either ends of the tube (an intermediate location), and a near chamber outlet location. If the condensate level at the patient end is greater than the condensate level at the other two sections, this may indicate that the humidifier may be at an elevation above the location of the patient. The controller can alert the patient to reposition the humidifier to be below the patient.

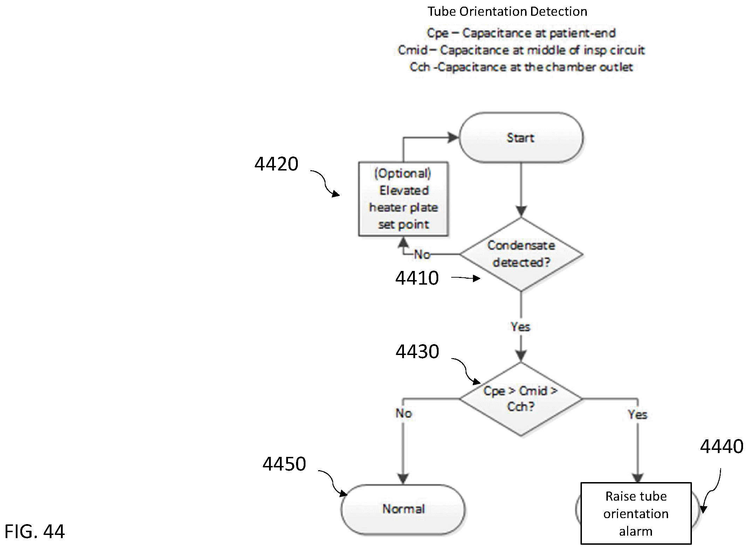

Fig. 44 illustrates an example process or method to determine the tube orientation within a humidifier system. At block 4410, the controller may determine whether condensate is detected in the inspiratory tube. This may include the controller measuring a condensate metric and determining whether the measuring condensate metric satisfies an expected condensate metric. The system may detect condensate if the measured condensate metric does not satisfy the expected condensate metric. The measured condensate metric may not satisfy the expected condensate metric if the measured condensate metric is greater or lesser than a predetermined threshold.

If condensate is not detected, the controller may return to the start of the process. Optionally, before restarting at block 4410, at block 4420, the controller may optionally elevate the heater plate temperature set point. This step may ensure there may be enough humidity present in the system so moisture (for example, condensate) will form at a location within the humidifier system. If moisture is detected, at block 4430, the controller may determine whether the capacitance value measured at the patient end Cpe is greater than the capacitance value measured at the intermediate location Cmid (for example, in the middle of the inspiratory tube) and whether the capacitance value measured at the intermediate location Cmid is greater than the capacitance value measured at the chamber outlet Cch. In other words, the measured condensate metric does not satisfy the expected condensate metric is determine by the measured condensate metric at a patient end of the inspiratory tube being greater than the measured condensate metric at an outlet of the humidification chamber and the measured condensate metric at the patient end may be greater than the measured condensate metric at the intermediate location. If the condition in block 4430 is satisfied, at block 4440, the controller may output a tube orientation alarm. If the condition in block 4430 is not satisfied, at block 4450, the controller may resume normal operation of the humidifier system. Optionally, prior to block 4450 being performed, the controller may control heating to the tube to remove the condensate that has formed as a result of block 4420.

### Alternative Implementations & Replaceable Features

The foregoing moisture detection systems and methods may be implemented by any humidifier or gases supply system which provides humidified gases in respiratory support or surgery, including, but not limited to, bi-level pressure respiratory support, CPAP respiratory support, or high flow nasal cannula respiratory support. The humidifier or gases supply system can include a gases sources in the form of a ventilator separate from the humidifier apparatus. Alternatively, the humidifier or gases supply system can include an integrated flow-generator and a humidifier apparatus in a single housing. The humidifier or gases supply system may include a single breathing conduit or tube, which is an inspiration conduit. Alternatively, the humidifier or gases supply system can include more than one breathing tube or conduit, such as the inspiratory conduit and an expiratory conduit.

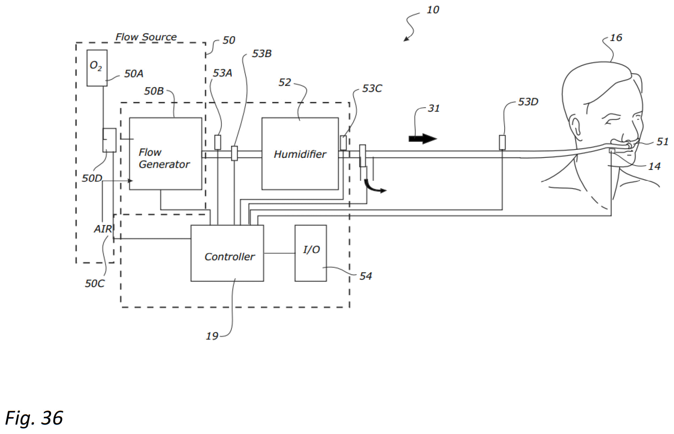

Fig. 36 illustrates an example humidifier system 10 that can provide high flow nasal cannula respiratory support. The system can provide a high flow gases flow 31 to a patient 16. The system can include a flow source 50, a humidifier 52 for humidifying the gases flow, an inspiratory tube, a conduit (e.g. 'dry' line or heated breathing tube), and a patient interface 51. The flow source 50 can include a wall supply of oxygen, tank of oxygen 50A, and/or a high flow apparatus with a flow generator 50B with optional air inlet 50C for entraining ambient air. In this example humidifier system 10, ambient air may contain a higher humidity content than in the example respiratory assistance apparatus of Fig, 1. Flow source 50 and humidifier 52 may be integrated into the same housing, or may be provided in separate housings. Oxygen (or other supplementary gas) source 50A can be connected to the flow generator via controllable valve/regulator/other gas flow control device 50D. The humidifier 52 can be connected between the flow source 50 and the patient 16. The system 10 can include one or more sensors 53A, 53B, 53B, 53D. The sensors can include flow, oxygen fraction, pressure, humidity, temperature, and/or other sensors. The sensors can also include patient physiological sensors, for example, for measuring patient heart rate, oxygen saturation, partial pressure of oxygen, respiratory rate, partial pressure of CO2, etc. The patient sensor may be an EEG sensor, torso band, etc. The system 10 can also include a sensor 14 at or near the patient interface 51 to measure the oxygen fraction inspired by the patient. The system can include a controller 19, which can be in communication with the flow source 50, humidifier 52, and/or sensor 14. The system can include an input/output (I/O) interface 54, which can be a display or input device, or both. The display can be a touch screen. The moisture detection systems and methods, and applications thereof as described above can be implemented in the system 10.

### Terminology

Although this disclosure has been described in the context of certain embodiments and examples, it will be understood by those skilled in the art that the disclosure extends beyond the specifically disclosed embodiments to other alternative embodiments and/or uses and obvious modifications and equivalents thereof. In addition, while several variations of the embodiments of the disclosure have been shown and described in detail, other modifications, which are within the scope of this disclosure, will be readily apparent to those of skill in the art. It is also contemplated that various combinations or sub-combinations of the specific features and aspects of the embodiments may be made and still fall within the scope of the disclosure. For example, features described above in connection with one embodiment can be used with a different embodiment described herein and the combination still fall within the scope of the disclosure. It should be understood that various features and aspects of the disclosed embodiments can be combined with, or substituted for, one another in order to form varying modes of the embodiments of the disclosure. Thus, it is intended that the scope of the disclosure herein should not be limited by the particular embodiments described above. Accordingly, unless otherwise stated, or unless clearly incompatible, each embodiment of this invention may comprise, additional to its essential features described herein, one or more features as described herein from each other embodiment of the invention disclosed herein.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without other input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

Any methods disclosed herein need not be performed in the order recited. The methods disclosed herein include certain actions taken by a practitioner; however, they can also include any third-party instruction of those actions, either expressly or by implication. For example, actions such as "controlling a motor speed" include "instructing controlling of a motor speed."

All of the methods and tasks described herein may be performed and fully automated by a computer system. The computer system may, in some cases, include multiple distinct computers or computing devices (for example, physical servers, workstations, storage arrays, cloud computing resources, etc.) that communicate and interoperate over a network to perform the described functions. Each such computing device typically includes a processor (or multiple processors) that executes program instructions or modules stored in a memory or other non-transitory computer-readable storage medium or device (for example, solid state storage devices, disk drives, etc.). The various functions disclosed herein may be embodied in such program instructions, and/or may be implemented in application-specific circuitry (for example, ASICs or FPGAs) of the computer system. Where the computer system includes multiple computing devices, these devices may, but need not, be co-located. The results of the disclosed methods and tasks may be persistently stored by transforming physical storage devices, such as solid state memory chips and/or magnetic disks, into a different state. In some embodiments, the computer system may be a cloud-based computing system whose processing resources are shared by multiple distinct business entities or other users.

The scope of the present disclosure is not intended to be limited by the specific disclosures of preferred embodiments in this section or elsewhere in this specification, and may be defined by claims as presented in this section or elsewhere in this specification or as presented in the future. The language of the claims is to be interpreted broadly based on the language employed in the claims and not limited to the examples described in the present specification or during the prosecution of the application, which examples are to be construed as non-exclusive.
This divisional application is divided from EP22892263.9 (the 'parent application') and the divisional specification as filed comprises the content of the parent application, including the original claims recited as 'representative features' below. The scope of this disclosure therefore includes the full content of the parent application. In any case, protection may be sought for any features disclosed in the parent application as filed.
1. A humidifier system configured to deliver a flow of gases to a user, the humidifier system comprising:
   a humidifier configured to heat and humidify the gases;
   a conduit configured to transport the gases from the humidifier to the user;
   at least one sensor configured to output at least one sensor signal based on an amount of moisture present in a component of the system; and
   a controller configured to control operation of the humidifier, wherein the controller is further configured to:
      determine at least one value indicative of the amount of moisture present in the component based at least in part on the at least one sensor signal, and
      execute one or more moisture management responses based on the at least one value indicative of the amount of moisture present in the component.
2. The humidifier system of Clause 1, wherein the conduit further comprises at least a first electrically conductive element and a second electrically conductive element, the at least one sensor signal comprising a signal generated using one or more of the at least first and second electrically conductive elements.
3. The humidifier system of Clauses 2, wherein the at least one signal is indicative of a capacitance between the first electrically conductive element and the second electrically conductive element.
4. The humidifier system of any of Clauses 2-3, wherein the at least one signal is indicative of a change in capacitance between the first electrically conductive element and the second electrically conductive element.
5. The humidifier system of any of Clauses 2-4, wherein the first electrically conductive element and the second electrically conductive element are separated by a distance configured to allow for a capacitive charge to be sensed between the first electrically conductive element and the second electrically conductive element.
6. The humidifier system of any of Clauses 2-5, further comprising a dielectric material located between the first electrically conductive element and the second electrically conductive element.
7. The humidifier system of any of Clauses 2-6, wherein the controller is configured to determine the at least one value indicative of moisture based on a comparison of a measurement of the first electrically conductive element and/or the second electrically conductive element.
8. The humidifier system of any of Clauses 2-7, wherein the at least one signal is indicative of a temperature of the first electrically conductive element or the second electrically conductive element.
9. The humidifier system of any of Clauses 2-8, wherein the at least one signal is indicative of a change in temperature of the first electrically conductive element or the second electrically conductive element.
10. The humidifier system of any of Clauses 2-8, wherein the at least one signal is indicative of a thermal conductivity of a medium between the first electrically conductive element and the second electrically conductive element, or the at least one signal is indicative of a thermal conductivity of a medium proximal to the first electrically conductive element or the second electrically conductive element.
11. The humidifier system of any of Clauses 2-10, wherein the at least one signal is indicative of a resistance of the first electrically conductive element or the second electrically conductive element.
12. The humidifier system of any of Clauses 2-11, wherein the first electrically conductive element or the second electrically conductive element comprise at least two portions that are electrically disconnected from one another.
13. A humidifier system configured to deliver a flow of gases to a user, the humidifier system comprising:
   a humidifier configured to heat and humidify the gases;
   at least one sensor configured to output at least one sensor signal based on an amount of moisture and/or humidity present in a component of the system; and
   a controller configured to control operation of the humidifier, wherein the controller is further configured to:
      determine a value indicative of the amount of moisture and/or humidity present in the component based on the at least one sensor signal, and
      determine a humidity of the flow of gases based on the value indicative of the amount of moisture and/or humidity present in the component.
14. The humidifier system of Clauses 13, wherein the conduit further comprises at least a first electrically conductive element and a second electrically conductive element, the at least one sensor signal comprising a signal generated using one or more of the at least first and second electrically conductive elements.
15. The humidifier system of Clauses 14, wherein the at least one signal is indicative of a capacitance between the first electrically conductive element and the second electrically conductive element.
16. The humidifier system of any of Clauses 14-15, wherein the at least one signal is indicative of a change in capacitance between the first electrically conductive element and the second electrically conductive element.
17. The humidifier system of any of Clauses 14-16, wherein the first electrically conductive element and the second electrically conductive element are separated by a distance configured to allow for a capacitive charge to be sensed between the first electrically conductive element and the second electrically conductive element.
18. The humidifier system of any of Clauses 14-17, further comprising a dielectric material located between the first electrically conductive element and the second electrically conductive element.
19. The humidifier system of Clause 18, wherein the dielectric material is vapor or liquid permeable.
20. The humidifier system of Clause 19, wherein the vapor permeable dielectric material allows evaporation of water to ambient air while inhibiting passage of liquid water and breathing gases to ambient air
21. The humidifier system of any of Clauses 14-20, wherein the controller is configured to determine the at least one value indicative of moisture based on a comparison of a measurement of the first electrically conductive element and/or the second electrically conductive element.
22. A humidifier system configured to deliver a flow of gases to a user, the humidifier system comprising:
   a humidifier configured to heat and humidify the gases;
   a first sensor associated with a first location, the first sensor configured to output at least one first sensor signal based on an amount of moisture present at the first location;
   a second sensor associated with a second location, the second sensor configured to output at least one second sensor signal based on an amount of moisture present at the second location; and
   a controller configured to control operation of the humidifier, wherein the controller is further configured to determine:
      a first value indicative of an amount of humidity and/or moisture present at the first location based on a first sensor signal, and
      a second value indicative of the amount of humidity and/or moisture present at the second location based on a second sensor.
23. The humidifier system of Clause 22, wherein the first location is associated with a first component of the system.
24. The humidifier system of Clause 22 or 23, wherein the second location is associated with a second component of the system.
25. The humidifier system of Clause 23 or 24, wherein the first and/or second components comprise at least one of:
   a conduit;
   a portion of the conduit;
   a connector;
   a portion of a chamber that is exposed to the flow of gases; and/or
   a patient interface.
26. The humidifier system of Clause 25, wherein the connector comprises one or more adapters.
27. The humidifier system of Clause 25 or 26, wherein the connector connects two conduits or is a wye piece that connects an inspiratory conduit, an expiratory conduit, and a patient interface supply conduit.
28. The humidifier system of any of Clauses 22-27, wherein the first location is upstream of the second location during normal operation of the system.
29. The humidifier system of any of Clauses 22-28, wherein the controller is configured to determine that no flow and/or a flow rate below a threshold is occurring in the system by:
   detecting a first predetermined amount moisture at the first location; and
   a predetermined time period after detecting the predetermined amount of moisture at the first location, determining that a second predetermined amount of moisture is not detected at a second location.
30. The humidifier system of any of Clauses 22-29, wherein the controller is configured to determine that a flow rate of the gases is in a range based on readings from a first temperature sensor at the first location and a second temperature sensor at the second location in response to detecting the first predetermined amount of moisture at the first location and not detecting the second predetermined amount of moisture at the second location a predetermined time period after detecting the predetermined amount of moisture at the first location.
31. The humidifier system of Clause 30, wherein the first temperature sensor is located at a chamber outlet of the humidifier.
32. The humidifier system of Clause 30 or 31, wherein the second temperature sensor is located at or near a patient end of an inspiratory conduit or a distal end of a patient supply conduit configured to transport the gases from the humidifier to the user.
33. The humidifier system of any of Clauses 22-32, wherein the controller is configured to determine that the flow direction is correct in response to the moisture at an upstream location being greater than the moisture at a downstream location.
34. The humidifier system of any of Clauses 22-33, wherein the controller is configured to determine that the flow direction is incorrect in response to the moisture at an upstream location being less than the moisture at a downstream location.
35. The humidifier system of Clause 33 or 34, wherein an inspiratory conduit comprises the upstream location.
36. The humidifier system of any of Clauses 33-35, wherein a conduit positioned between the gases source and the humidifier comprises the downstream location.
37. The humidifier system of any of Clauses 22-36, comprising a third sensor associated with a third location of the system, wherein the third sensor is configured to output at least one third sensor signal based on an amount of moisture present at the third location.
38. The third location of Clause 37, wherein the third location is upstream of the first location and the second location.
39. The humidifier system of any of Clauses 37-38, wherein the first location is at an inspiratory conduit.
40. The humidifier system of any of Clauses 37-39, wherein the third location is at an expiratory conduit.
41. The humidifier system of any of Clauses 37-40, wherein the second location is at a chamber inlet of a humidification chamber.
42. The humidifier system of any of Clauses 37-41, wherein the controller is configured to determine a flow direction based on a direction of the moisture detected from the humidifier, wherein the controller is configured to detect a reverse flow condition in response to moisture being detected in a component upstream of the humidifier when correctly connected.
43. A humidifier system useable in a gases supply system, the humidifier system comprising:
   a humidifier configured to heat and humidify the gases;
   a conduit configured to transport the gases from the humidifier to the user, the conduit further comprising:
      a first electrically conductive element; and
      a second electrically conductive element; and
   a controller configured to monitor capacitance and/or capacitance change of a capacitor formed between the first electrically conductive element and the second electrically conductive element to detect presence of skin in close vicinity or contact with the conduit.
44. The humidification system of Clause 43, wherein monitoring the capacitance and/or capacitance change comprises measuring a time constant, a resonant frequency, a change in a time constant, or a change in a resonant frequency of the capacitor formed between the first electrically conductive element and the second electrically conductive element.
45. The humidification system of Clause 43 or 44, wherein the controller is configured to detect a change in permittivity of the capacitor based on the monitored capacitance and/or capacitance change.
46. The humidification system of any of Clauses 43-45, wherein the controller is configured to compare the detected change in permittivity with one or more permittivity thresholds.
47. The humidification system of Clause 46, wherein the controller is configured to detect the presence of skin in close vicinity or contact with the conduit in response to the detected change in permittivity exceeding the one or more permittivity thresholds.
48. The humidification system of any of Clauses 43-47, wherein the controller is configured to output an alarm in response to detecting the presence of skin in close vicinity or contact with the conduit.
49. The humidification system of Clause 48, wherein the controller is configured to output the alarm in response to detecting the presence of skin in close vicinity or contact with the conduit continuously or intermittently for a predefined duration.
50. The humidification system of Clause 49, wherein the predefined duration is variable based on an expected conduit surface temperature.
51. The humidifier system of any of Clauses 43-50, wherein the conduit comprises a dielectric material between the first electrically conductive element and the second electrically conductive element.
52. The humidifier system of any of Clauses 43-51, wherein the first electrically conductive element and the second electrically conductive element are spirally wound about at least a length of the conduit.
53. The humidifier system of any of Clauses 43-52, wherein the first electrically conductive element and the second electrically conductive element are spirally wound within, through, or around the conduit.
54. The humidifier system of any of Clauses 43-53, wherein the first electrically conductive element and the second electrically conductive element form part of conduit walls.
55. The humidifier system of any of Clauses 43-54, wherein the first electrically conductive element is a sensing wire.
56. The humidifier system of any of Clauses 43-55, wherein the first electrically conductive element is a heater wire.
57. The humidifier system of any of Clauses 43-56, wherein the second electrically conductive element is a sensing wire.
58. The humidifier system of any of Clauses 43-57, wherein the second electrically conductive element is a heater wire.
59. The humidifier system of any of Clauses 43-58, wherein the first electrically conductive element and the second electrically conductive element are separated by a distance configured to allow for a capacitive charge to be sensed between the first electrically conductive element and the second electrically conductive element.
60. The humidifier system of any of Clauses 43-59, wherein the first electrically conductive element or the second electrically conductive element comprise at least two portions that are electrically disconnected from one another.
61. The humidifier system of Clause 60, wherein the at least two portions are in series with one another.
62. The humidifier system of Clause 60, wherein the at least two portions are in parallel with one another.
63. A method of determining a condition of a medical humidifier configured to provide gases to a patient, the method comprising:
   reducing power to a heater wire of a breathing tube coupled to the medical humidifier, wherein the medical humidifier comprises a heater plate and a temperature based flow sensor;
   measuring a condensate metric;
   determining whether the measured condensate metric satisfies an expected condensate metric; and
   outputting the condition of the medical humidifier to a controller of the medical humidifier in response to the measured condensate metric not satisfying the expected condensate metric, wherein outputting the condition of the medical humidifier comprises disregarding reading from the temperature based flow sensor for control of the medical humidifier.
64. The method of Clause 63, wherein determining whether the measured condensate metric satisfies the expected condensate metric comprises comparing a condensate level in the breathing tube to a predetermined threshold.
65. The method of Clause 64, wherein the measured condensate metric not satisfying the expected condensate metric comprises the condensate level in the breathing tube being greater than the predetermined threshold.
66. The method of any of Clauses 63-65, wherein outputting the condition of the medical humidifier further comprises disabling the temperature based flow sensor.
67. The method of any of Clauses 63-6600, wherein reducing power to the heater wire comprises disabling power to the heater wire.
68. The method of any of Clauses 63-67, wherein outputting the condition of the medical humidifier includes implementing an alternative method to determine a value indicative of a flow rate of the gases.
69. The method of any of Clauses 63-68, wherein the alternative method comprises an algorithmic method that does not require further input from sensors.
70. The method of any of Clauses 63-69, wherein outputting the condition of the medical humidifier includes adjusting a setting of the medical humidifier to be displayed on a user interface of the medical humidifier.
71. The method of Clause 70, wherein adjusting the setting of the medical humidifier includes at least one of:
   adjusting PID coefficients;
   increasing a control loop timing; and/or
   applying a maximum increase in a setpoint of the heater plate.
72. The method of any of Clauses 63-69, wherein the condition is a Heliox mode.
73. The method of any of Clauses 63-6900, wherein outputting the condition of the medical humidifier comprises determining a gases source type.
74. The method of any of Clauses 63-7300, wherein the power to the heater wire of the breathing tube is reduced or disabled for a predetermined period of time.
75. The method of any of Clauses 63-7400, further comprising removing condensate formed due to reducing power to the heater wire.
76. A method of determining a condition of a medical humidifier configured to provide gases to a patient, the method comprising:
   reducing power to a heater wire of a breathing tube coupled to the medical humidifier, wherein the medical humidifier comprises a heater plate;
   measuring a condensate metric;
   determining whether the measured condensate metric satisfies an expected condensate metric; and
   outputting the condition of the medical humidifier to a controller of the medical humidifier in response to the measured condensate metric not satisfying the expected condensate metric.
77. The method of Clause 76, wherein reducing the power to the heater wire of the breathing tube comprises disabling the power to the heater wire.
78. The method of Clause 76, wherein the power to the heater wire of the breathing tube is reduced for a predetermined period of time.
79. The method of any of Clause 76-780, wherein determining whether the measured condensate metric satisfies the expected condensate metric comprises comparing a condensate level in the breathing tube to a predetermined threshold.
80. The method of Clause 79, wherein the measured condensate metric not satisfying the expected condensate metric comprises the condensate level in the breathing tube being greater than the predetermined threshold.
81. The method of Clause 79, wherein the measured condensate metric not satisfying the expected condensate metric comprises the condensate level in the breathing tube being less than the predetermined threshold.
82. The method of Clause 81, wherein the condition comprises a reverse flow condition.
83. The method of Clause 82, wherein the measured condensate metric not satisfying the expected condensate metric comprises a condensate level of a dryline connected to the medical humidifier and a gases source is greater than a condensate level of the breathing tube.
84. The method of Clause 81, wherein outputting the condition further comprises outputting a reverse flow alarm.
85. The method of Clause 82, wherein the condition comprises the medical humidifier having just started up or is malfunctioning.
86. The method of any of Clauses 76-79, wherein the condition of the medical humidifier includes no presence of flow or water out.
87. The method of Clause 86, further comprising confirming the determination by performing a flow test or a water out test.
88. The method of any of Clauses 76-87, further comprising indicating the condition to the patient in an audio or visual message.
89. The method of any of Clauses 76-81, wherein the measured condensate metric not satisfying the expected condensate metric comprises a duration of time to dry condensation in the breathing tube being greater than a predetermined threshold duration of time.
90. The method of Clause 89, wherein in response to the duration of time to dry condensation in the breathing tube being greater than a predetermined threshold duration of time, outputting the condition comprising the breathing tube being covered.
91. The method of any of Clauses 76-81, wherein measuring a condensate metric comprises measuring a first condensate metric in a first section of an inspiratory limb and a second condensate metric in a second section of the inspiratory limb;
   wherein determining whether the measured condensate metric satisfies an expected condensate metric comprises comparing the first condensate metric of the first section of the inspiratory limb and the second condensate metric of the second section of the inspiratory limb,
   wherein the condition comprises an incubator being used with the medical humidifier.
92. The method of Clause 91, wherein the incubator is determined to be used in response to the first condensate metric of the first section being greater than the second condensate metric of the second section.
93. The method of any one of Clauses 91-92, wherein the first condensate metric and the second condensate metric each correspond to a presence of condensation.
94. The method of Clause 93, wherein the presence of condensation in the first section of the inspiratory limb and no presence of condensation in the second section of the inspiratory limb is indicative of the incubator being in use.
95. The method of any of Clauses 91-94, further comprising decreasing power delivered to the heater wire at the second section of the inspiratory limb when the incubator is used.
96. The method of any of Clauses 91-95, further comprising increasing power delivered to the heater wire at the first section of the inspiratory limb when the incubator is used.
97. The method of any of Clauses 76-81, wherein the condition is an orientation of the breathing tube.
98. The method of Clause 97, wherein the measured condensate metric not satisfying the expected condensate metric comprises the measured condensate metric at a patient end of the breathing tube being greater than the measured condensate metric at an outlet of a humidification chamber of the medical humidifier.
99. The method of Clause 97 or 98, wherein the measured condensate metric not satisfying the expected condensate metric comprises the measured condensate metric at the patient end being greater than the measured condensate metric at a region between the patient end and the outlet.
100. The method of any of Clauses 76-99, further comprising removing condensate formed by reducing power to the heater wire.
101. A method to determine a condition of a humidifier configured to provide gases to a patient, the method comprising:
   determining a startup condition;
   reducing power to a heater plate of a medical humidifier coupled to a breathing tube and comprising a heater plate;
   determining whether a measured condensate metric satisfies an expected condensate metric; and
   outputting the condition of the humidifier to a controller of the humidifier based on whether the measured condensate metric does not satisfy the expected condensate metric.
102. The method of Clause 101, wherein the startup condition comprises determining a duration of time for which the humidifier has been powered on and determining the duration of time indicating the humidifier has just turned on.
103. The method of Clause 101 or 102, further comprising resuming a state or normal control in response to the measured condensate metric satisfying the expected condensate metric.
104. The method of any of Clauses 101-103, wherein the condition includes an alarm that the breathing tube is contaminated.
105. The method of any one of Clauses 101-104, wherein the humidifier just been powered on.
106. The method of any of Clauses 101-105, further comprising powering off the heater plate and ensuring the heater plate is at a substantially cool temperature.
107. The method of any of Clauses 101-106, further comprising raising a circuit moisture alarm when condensate is detected.
108. A method of controlling power to a heating element of an inspiratory tube coupled to a humidifier of a humidifier system, the method comprising:
   measuring a condensate metric of the humidifier system;
   determining whether the measured condensate metric satisfies an expected condensate metric; and
   controlling power to the heating element based on at least whether the measured condensate metric satisfies the expected condensate metric.
109. The method of Clause 108, wherein determining whether the measured condensate metric satisfies the expected condensate metric comprises detecting whether the measured condensate metric is greater than a predetermined threshold.
110. The method of Clause 109, further comprising increasing power to the heating element in response to the measured condensate metric being greater than the predetermined threshold.
111. The method of Clause 109 or 110, further comprising decreasing power to the heating element in response to the measured condensate metric being less than the predetermined threshold.
112. The method of Clause 108, further comprising determining whether a power to the heating element is at a maximum.
113. The method of Clause 112, further comprising reducing a setpoint of a heater plate of the humidifier of the humidifier system when the power to the heating element is at the maximum.
114. The method of Clause 113, wherein reducing a setpoint of the heater plate is further based on determining that the measured condensate satisfies the expected condensate metric.
115. The method of any of clauses 108-114, wherein the inspiratory tube is sensorless at a patient end.
116. The method of any of Clauses 108-115, wherein whether the measured condensate metric satisfies the expected condensate metric is determined without an input from the sensor located at the patient end.
117. The method of any of Clauses 108-116, wherein the humidifier system is sensorless at an outlet of a humidification chamber of the system.
118. A humidifier system configured to deliver a flow of gases to a user, the humidifier system comprising:
   a humidifier configured to heat and humidify the gases;
   at least one sensor configured to output at least one sensor signal based on an amount of moisture present in a component of the system; and
   a controller configured to control operation of the humidifier, wherein the controller is further configured to:
      determine at least one value indicative of the amount of moisture present in the component based at least in part on the at least one sensor signal, and
      execute one or more moisture management responses based on the at least one value indicative of the amount of moisture present in the component.

## Claims

1. A humidifier system configured to deliver a flow of gases to a user, the humidifier system comprising:
a humidifier configured to heat and humidify the gases;
at least one sensor configured to output at least one sensor signal based on an amount of moisture present in a component of the system; and
a controller configured to control operation of the humidifier, wherein the controller is further configured to:
determine at least one value indicative of the amount of moisture present in the component based at least in part on the at least one sensor signal, and
execute one or more moisture management responses based on the at least one value indicative of the amount of moisture present in the component.

2. The humidifier system of claim 1, wherein the component comprises one or more of the following; the conduit; a portion of the conduit; a connector; a portion of a chamber that may be exposed to the flow of gases; a patient interface.

3. The humidifier system of Claim 1 or 2, comprising a conduit configured to transport the gases from the humidifier to the user, wherein the conduit further comprises at least a first electrically conductive element and a second electrically conductive element, the at least one sensor signal comprising a signal generated using one or more of the at least first and second electrically conductive elements.

4. The humidifier system of Claim 3, wherein the at least one signal is indicative of a capacitance and/or a change in capacitance between the first electrically conductive element and the second electrically conductive element.

5. The humidifier system of any of Claims 2-4, wherein the first electrically conductive element and the second electrically conductive element are separated by a distance configured to allow for a capacitive charge to be sensed between the first electrically conductive element and the second electrically conductive element.

6. The humidifier system of any of Claims 2-5, further comprising a dielectric material located between the first electrically conductive element and the second electrically conductive element.

7. The humidifier system of any of Claims 2-6, wherein the controller is configured to determine the at least one value indicative of moisture based on a comparison of a measurement of the first electrically conductive element and/or the second electrically conductive element.

8. The humidifier system of any of Claims 2-7, wherein the at least one signal is indicative of a temperature and/or a change of temperature of the first electrically conductive element or the second electrically conductive element.

9. The humidifier system of any of Claims 2-8, wherein the at least one signal is indicative of a thermal conductivity of a medium between the first electrically conductive element and the second electrically conductive element, or the at least one signal is indicative of a thermal conductivity of a medium proximal to the first electrically conductive element or the second electrically conductive element.

10. The humidifier system of any of Claims 2-9, wherein the first electrically conductive element or the second electrically conductive element comprise at least two portions that are electrically disconnected from one another.

11. A humidifier system of any one of Claims 1-10, wherein the controller is further configured to:
determine a value indicative of the humidity present in the component based on the at least one sensor signal, and
determine a humidity of the flow of gases based on the value indicative of the humidity present in the component.

12. The humidifier system of any of Claims 2-10, further comprising a dielectric material located between the first electrically conductive element and the second electrically conductive element.

13. The humidifier system of Claim 12, wherein the dielectric material is vapor or liquid permeable.

14. The humidifier system of Claim 13, wherein the vapor permeable dielectric material allows evaporation of water to ambient air while inhibiting passage of liquid water and breathing gases to ambient air

15. The humidifier system of Claim 11, wherein the controller is configured to determine the at least one value indicative of humidity based on a comparison of a measurement of the first electrically conductive element and/or the second electrically conductive element.
